(19) Europäisches Patentamt
European Patent Office
Office européen des brevets

(11) EP 0 272 009 B2

(12) NEW EUROPEAN PATENT SPECIFICATION

(45) Date of publication and mention of the opposition decision:
**03.10.2007 Bulletin 2007/40**

(45) Mention of the grant of the patent:
**04.03.1998 Bulletin 1998/10**

(21) Application number: **87310363.4**

(22) Date of filing: **24.11.1987**

(51) Int Cl.:
***C12Q 1/68*** (2006.01) ***G01N 33/53*** (2006.01)
***C12Q 1/04*** (2006.01) ***C12Q 1/00*** (2006.01)
***C07H 21/00*** (2006.01) *C12P 19/34* (2006.01)

# (54) Nucleic acid probes for detection and/or quantitation of non-viral organisms

Nukleinsäuresonden zum Nachweis und/oder zur Quantifizierung von nicht-viralen Organismen

Sondes d’acides nucléiques pour la détection et pour l’analyse quantitative d’organismes non viraux

(84) Designated Contracting States:
**AT BE CH DE ES FR GB GR IT LI LU NL SE**

(30) Priority: **24.11.1986 US 934244**
**07.08.1987 US 83542**

(43) Date of publication of application:
**22.06.1988 Bulletin 1988/25**

(73) Proprietor: **GEN-PROBE INCORPORATED**
**San Diego, CA 92121 (US)**

(72) Inventors:
- **Hogan, James J.**
  **Coronado, California 92118 (US)**
- **Smith, Richard D.**
  **Victoria, British Columbia V8X4N1 (CA)**
- **Kop, JoAnn**
  **Fremont, California 94536 (US)**
- **McDonough, Sherrol H.**
  **San Diego, California 92122 (US)**

(74) Representative: **Maschio, Antonio et al**
**D Young & Co**
**120 Holborn**
**London EC1N 2DY (GB)**

(56) References cited:
**EP-A- 0 133 671**
**EP-A- 0 232 085**
**EP-A- 0 245 129**
**EP-A- 0 250 662**
**EP-A- 0 277 237**
**WO-A-83/01073**
**WO-A-84/02721**

EP 0 272 009 B2

**Description**

BACKGROUND OF THE INVENTION

1. Field of the Invention

The inventions described and claimed herein relate to probes and assays based on the use of genetic material such as RNA. More particularly, the inventions relate to the design and construction of nucleic acid probes and hybridization of such probes to genetic material of target non-viral organisms in assays for detection and/or quantitation thereof in test samples of, e.g., sputum, urine, blood and tissue sections, food, soil and water.

2. Introduction

Two single strands of nucleic acid, comprised of nucleotides, may associate ("hybridize") to form a double helical structure in which the two polynucleotide chains running in opposite directions are held together by hydrogen bonds (a weak form of chemical bond) between pairs of matched, centrally located compounds known as "bases." Generally, in the double helical structure of nucleic acids, for example, the base adenine (A) is hydrogen bonded to the base thymine (T) or uracil (U) while the base guanine (G) is hydrogen bonded to the base cytosine (C). At any point along the chain, therefore, one may find the base pairs AT or AU, TA or UA, GC, or CG. One may also find AG and GU base pairs in addition to the traditional ("canonical") base pairs. Assuming that a first single strand of nucleic acid is sufficiently complementary to a second and that the two are brought together under conditions which will promote their hybridization, double stranded nucleic acid will result. Under appropriate conditions, DNA/DNA, RNA/DNA, or RNA/RNA hybrids may be formed.

Broadly, there are two basic nucleic acid hybridization procedures. In one, known as "in solution" hybridization, both a "probe" nucleic acid sequence and nucleic acid molecules from a test sample are free in solution. In the other method, the sample nucleic acid is usually immobilized on a solid support and the probe sequence is free in solution.

A probe may be a single strand nucleic acid sequence which is complementary in some particular degree to the nucleic acid sequences sought to be detected ("target sequences"). It may also be labelled. A background description of the use of nucleic acid hybridization as a procedure for the detection of particular nucleic acid sequences is described in EP-A-0131052.

WO84/02721 relates to a method for detecting, identifying and quantitating organisms and viruses. The document contains a description of the use of nucleic acid probe assays. It contains broad claims directed to a probe which "hybridizes to the rRNA of said group of non-viral organisms and does not detectably hybridize to rRNA from other non-viral organisms ....". The PCT application describes methods of obtaining such probes including Procedure A directed to obtaining clones containing potential probes and using screening by hybridization to select the probe, Procedure B which describes biologically synthesizing a group of probes and selecting a probe by hybridization procedures and Procedure C which involves selecting probes based upon sequence comparison.

EP-A-0133671 relates to a nucleic acid hybridization method for detecting bacteria in a test sample, employing a polynucleotide probe having a base sequence which is homologous with at least one base sequence in the nucleic acid of substantially all of the bacteria suspected to be present.

EP-A-0232085 discloses a DNA probe comprising a DNA sequence essentially complementary to a part of rRNA of a *Campylobacter* species which is capable of hybridizing to the rRNA of a *Campylobacter* species and not capable of hybridizing to the rRNA of a species other than *Campylobacter.*

EP-A-0245129 discloses a hybridization probe for detecting the existence of bacteria in a sample comprising particular pentadecadeoxyribonucleotides and portions thereof.

EP-A-0250662 relates to a method for determining the presence of a prokaryotic organism by contacting a sample containing the organism with a nucleic acid fragment from the organism and detecting the presence of hybridized oligonucleotides.

EP-A-0277237 is concerned with a method of assaying bacteria in a sample using a probe prepared by labelling a DNA or RNA, which DNA or RNA contains a base sequence complementary to a sequence of at least twelve bases of ribosomal RNA from *E. coli.*

Also described in those applications are methods for determining the presence of RNA-containing organisms in a sample which might contain such organisms, comprising the steps of bringing together nucleic acids from a sample and a probe comprising nucleic acid molecules which are shorter than the rRNA subunit sequence from which it was derived and which are sufficiently complementary to hybridize to the rRNA of one or more non-viral organisms or groups of non-viral organisms, incubating the mixture under specified hybridization conditions, and assaying the resulting mixture for hybridization of the probe and any test sample rRNA. The invention is described to include using a probe which detects only rRNA subunit subsequences which are the same or sufficiently similar in particular organisms or groups of organisms and is said to detect the presence or absence of any one or more of those particular organisms in a sample, even in the presence of many non-related organisms.

We have discovered and describe herein a method and means for designing and constructing DNA probes for use in detecting unique rRNA sequences in an assay for the detection and/or quantitation of any group of non-viral organisms. Some of the probes of the invention described herein may be used to detect and/or quantify a single species or strain of non-viral organism and others may be used to detect and/or quantify members of an entire genus or desired phylogenetic grouping.

According to the invention, there is provided a method for preparing a probe as defined in claim 1.

The invention further provides a hybridization assay probe as defined in claim 38 or claim 39.

## SUMMARY OF THE INVENTION

In a method of probe preparation and use, a single strand deoxyoligonucleotide of particular sequence and defined length is used in a hybridization assay to determine the presence or amount of rRNA from particular target non-viral organisms to distinguish them from their known closest phylogenetic neighbors. Probe sequences which are specific, respectively, for 16S rRNA variable subsequences of Mycobacterium avium, Mycobacterium intracellulare and the Mycobacterium tuberculosis-complex bacteria, and which do not cross react with nucleic acids from each other, or any other bacterial species or respiratory infectious agent, are described and claimed. A probe specific to a 23S rRNA variable region subsequence from the Mycobacterium tuberculosis-complex bacteria is also described and claimed, as are rRNA variable region probes useful in hybridization assays for the genus Mycobacterium (23S rRNA specific), Mycoplasma penumoniae (5S and 16S rRNA-specific), Chlamydia trachomatis (16S and 23S rRNA specific), Enterobacter cloacae (23S rRNA specific), Escherichia coli (16S rRNA specific), Legionella (16S and 23S rRNA specific), Salmonella (16S and 23S rRNA specific), Enterococci (16S rRNA specific), Neisseria gonorrhoeae (16S rRNA specific), Campylobacter (16S rRNA specific), Proteus mirabilis (23S rRNA specific), Pseudomonas (23S rRNA specific), fungi (18S and 28S rRNA specific), and bacteria (16S and 23S rRNA specific).

In one embodiment of the assay method, a test sample is first subjected to conditions which release rRNA from any non-viral organisms present in that sample. rRNA is single stranded and therefore available for hybridization with sufficiently complementary genetic material once so released. Contact between a probe, which can be labelled, and the rRNA target may be carried out in solution under conditions which promote hybridization between the two strands. The reaction mixture is then assayed for the presence of hybridized probe. Numerous advantages of the present method for the detection of non-viral organisms over prior art techniques, including accuracy, simplicity, economy and speed will appear more fully from the detailed description which follows.

## BRIEF DESCRIPTION OF THE DRAWING

Figure 1 is a chart of the primary structure of bacterial 16S rRNA for Escherichia coli, depicting standard reference numbers for base pairs.

Figure 2 is a chart of the primary structure of bacterial 23S rRNA for Escherichia coli, depicting standard reference numbers for base pairs.

Figure 3 is a chart of the primary structure of bacterial 5S rRNA for Escherichia coli, depicting standard reference numbers for base pairs.

Figure 4 is a chart of the primary structure for the 18S rRNA for Saccharomyces cerevisiae, depicting standard reference numbers for base pairs.

Figure 5 is a chart of the primary structure for the 28S rRNA for Saccharomyces cerevisiae, depicting standard reference numbers for base pairs.

Figure 6 is a diagram showing the locations in the 16S rRNA (using E. coli reference numbers) which differ bertween 12 different sets of related organisms. In Example 1, for example, 99.7 refers to the similarity in 16S rRNA between Clostridium botulinium and Clostridium subterminale.

Figure 7 is a diagram showing the locations in the first 1500 bases of 23S rRNA (using E.coli reference numbers) which differ between 12 different sets of related organisms.

Figure 8 is a diagram showing the locations in the terminal bases of 23S rRNA (using E.coli reference numbers) which differ between 12 different sets of related organisms.

Figure 9 is a schematic representation of the location of probes capable of hybridizing to the 165 rRNA.

Figure 10 is a schematic representation of the location of probes capable of hybridizing to the first 1500 bases of the 23S rRNA.

Figure 11 is a schematic representation of the location of probes capable of hybridizing to the terminal bases of 23S rRNA.

DETAILED DESCRIPTION OF THE INVENTION

Definitions

The following terms, as used in this disclosure and claims, are defined as:

nucleotide: a subunit of a nucleic acid consisting of a phosphate group, a 5' carbon sugar and a nitrogen containing base. In RNA the 5' carbon sugar is ribose. In DNA, it is a 2-deoxyribose. The term also includes analogs of such subunits.

nucleotide polymer: at least two nucleotides linked by phosphodiester bonds.

oligonucleotide: a nucleotide polymer generally about 10 to about 100 nucleotides in length, but which may be greater than 100 nucleotides in length.

nucleic acid probe: a single stranded nucleic acid sequence that will combine with a complementary single stranded target nucleic acid sequence to form a double-stranded molecule (hybrid). A nucleic acid probe may be an oligonucleotide or a nucleotide polymer.

hybrid: the complex formed between two single stranded nucleic acid sequences by Watson-Crick base pairings or non-canonical base pairings between the complementary bases.

hybridization: the process by which two complementary strands of nucleic acids combine to form double stranded molecules (hybrids).

complementarity: a property conferred by the base sequence of a single strand of DNA or RNA which may form a hybrid or double stranded DNA:DNA, RNA:RNA or DNA:RNA through hydrogen bonding between Watson-Crick base pairs on the respective strands. Adenine (A) usually complements thymine (T) or Uracil (U), while guanine (G) usually complements cytosine (C).

stringency: term used to describe the temperature and solvent composition existing during hybridization and the subsequent processing steps. Under high stringency conditions only highly homologous nucleic acid hybrids will form; hybrids without a sufficient degree of complementarity will not form. Accordingly, the stringency of the assay conditions determine the amount of complementarity needed between two nucleic acid strands forming a hybrid. Stringency is chosen to maximize the difference in stability between the target and the nontarget.

probe specificity: characteristic of a probe which describes its ability to distinguish between target and non-target sequences. Dependent on sequence and assay conditions. Probe specificity may be absolute (i.e., probe able to distinguish between target organisms and any nontarget organisms), or it may be functional (i.e., probe able to distinguish between the target organism and any other organism normally present in a particular sample). Many probe sequences can be used for either broad or narrow specificity depending on the conditions of use.

variable region: nucleotide polymer which differs by at least one base between the target organism and nontarget organisms contained in a sample.

conserved region: a region which is not variable.

sequence divergence: process by which nucleotide polymers become less similar during evolution.

sequence convergence: process by which nucleotide polymers become more similar during evolution.

bacteria: members of the phylogenetic group eubacteria, which is considered one of the three primary kingdoms.

Tm: temperature at which 50% of a probe is converted from the hybridized to the unhybridized form in the presence of a target.

thermal stability: a condition of hybridization incubation or separation incubation in which stable probe:target hybrids will form and in which no probe:non target hybrids can form by virtue of their instability. Factors which affect the thermal stability can affect probe specificity and therefore, must be controlled. Whether a probe sequence is useful to detect only a specific type of organism depends largely on the thermal stability difference between probe:target hybrids ("P:T") and probe:nontarget hybrids ("P:NT"). In designing probes, the Tm of P:T minus the Tm of P:NT should be as large as possible.

In addition to a novel method for selecting probe sequences, we have discovered that it is possible to create a DNA probe complementary to a particular rRNA sequence obtained from a single target microorganism and to successfully use that probe in a non-cross reacting assay for the detection of that single microorganism, even in the presence of its known, most closely related taxonomic or phylogenetic neighbors. With the exception of viruses, all prokaryotic organisms contain rRNA molecules including 5S rRNA, 16S rRNA, and a larger rRNA molecule known as 23S rRNA. Eukaryotes are known to have 5.0S, 5.8S, 18S and 28S rRNA molecules or analogous structures. (The term "16S-like" sometimes is used to refer to the rRNA found in the small ribosomal subunit, including 18S and 17S rRNA. Likewise the term "23S-like" rRNA sometimes is used to refer to the rRNA found in the large ribosomal subunit. "5S-like" rRNA sometimes is used to refer to rRNA found in the large ribosomal subunit. 5.8S rRNA is equivalent to the 5' end of the 23S-like rRNA.) These rRNA molecules contain nucleotide sequences which are highly conserved among all organisms thus far examined. There are known methods which allow a significant portion of these rRNA sequences to be determined. For example, complementary cligonucleotide primers of about 20-30 bases in length can be hybridized to universally conserved regions in purified rRNA that are specific to the 5S, 16S, or 23S subunits and extended with the enzyme reverse transcriptase. Chemical degradation or dideoxynucleotide-terminated sequencing reactions can be used to determine the nucleotide sequence of the extended product. Lane, D.J. et al., Proc. Nat'l Acad. Sci. USA 82, 6955-6959 (1985).

In our invention, comparison of one or more sequenced rRNA variable regions from a target organism to one or more rRNA variable region sequences from a closely related bacterial species is utilized to select a sequence unique to the rRNA of the target organism. rRNA is preferable to DNA as a probe target because of its relative abundance and stability in the cell and because of its patterns of phylogenetic conservation.

Notwithstanding the highly conserved nature of rRNA, we have discovered that a number of regions of the rRNA molecule which can vary in sequence, can vary even between closely related species and can, therefore, be utilized to distinguish between such organisms. Differences in the rRNA molecule are not distributed randomly across the entire molecule, but rather are clustered into specific regions. The degree of conservation also varies, creating a unique pattern of conservation across the ribosomal RNA subunits. The degree of variation and the distribution thereof, can be analyzed to locate target sites for diagnostic probes. This method of probe selection may be used to select more than one sequence which is unique to the rRNA of a target organism.

We have identified variable regions by comparative analysis of rRNA sequences both published in the literature and sequences which we have determined ourselves using procedures known in the art. We use a Sun Microsystems (TM) computer for comparative analysis. The compiler is capable of manipulating many sequences of data at the same time. Computers of this type and computer programs which may be used or adapted for the purposes herein disclosed are commercially available.

Generally, only a few regions are useful for distinguishing between closely related species of a phylogenetically conserved genus, for example, the region 400-500 bases from the 5' end of the 16S rRNA molecule. An analysis of closely related organisms (Figures <u>6, 7</u> and 8) reveals the specific positions (variable regions) which vary between closely related organisms. These variable regions of rRNA molecules are the likely candidates for probe design.

Figures 6, 7 and 8 display the variations in 16S and 23S rRNA's between two different bacteria with decreasing amounts of similarity between them. Closer analysis of these figures reveals some subtle patterns between these closely related organisms. In all cases studied, we have seen sufficient variation between the target organism and the closest phylogenetic relative found in the same sample to design the probe of interest. Moreover, in all cases studied to date, the per cent similarity between the target organism (or organisms) and the closest phylogenetically related organisms found in the same sample has been between 90% and 99%. Interestingly, there was enough variation even between the rRNA's of <u>Neisseria's gonorrhoeae</u> and <u>meningitidis</u> (See Example 21) to design probes - despite the fact that DNA: DNA homology studies suggested these two species might actually be one and the same.

These figures also show that the differences are distributed across the entire 16S and 23S rRNA's. Many of the differences, nonetheless, cluster into a few regions. These locations in the rRNA are good candidates for probe design, with our current assay conditions. We also note that the locations of these increased variation densities usually are situated in the same regions of the 16S and 23S rRNA for comparable per cent similarity values. In this manner, we have observed that certain regions of the 16S and 23S rRNA are the most likely sites in which significant variation exists between the target organism and the closest phylogenetic relatives found in a sample. We have disclosed and claimed species specific probes which hybridize in these regions of significant variation between the target organism and the closest phylogenetic relative found in a sample.

Figures 9, 10 and 11 are a schematic representation of the location of probes disclosed and claimed herein. Because 16S and 23S RNAs do not, as a rule, contain sequences of duplication longer than about six nucleotides in length, probes designed by these methods are specific to one or a few positions on the target nucleic acid.

The sequence evolution at each of the variable regions (for example, spanning a minimum of 10 nucleotides) is, for the most part divergent, not convergent. Thus, we can confidently design probes based on a few rRNA sequences which differ between the target organism and its phylogenetically closest relatives. Biological and structural constraints on the rRNA molecule which maintain homologous primary, secondary and tertiary structure throughout evolution, and the application of such constraints to probe diagnostics is the subject of ongoing study. The greater the evolutionary distance between organisms, the greater the number of variable regions which may be used to distinguish the organisms.

Once the variable regions are identified, the sequences are aligned to reveal areas of maximum homology or "match". At this point, the sequences are examined to identify potential probe regions. Two important objectives in designing a probe are to maximize homology to the target sequence(s) (greater than 90% homology is recommended) and to minimize homology to non-target sequence(s) (less than 90% non-homology to nontargets is recommended). We have identified the following useful guidelines for designing probes with desired characteristics.

First, probes should be positioned so as to minimize the stability of the probe:nontarget nucleic acid hybrid. This may be accomplished by minimizing the length of perfect complementarity to non-target organisms, avoiding G and C rich regions of homology to non-target sequences, and by positioning the probe to span as many destabilizing mismatches as possible (for example, dG:rU base pairs are less destabilizing than some others).

Second, the stability of the probe: target nucleic acid hybrid should be maximized. This may be accomplished by avoiding long A and T rich sequences, by terminating the hybrids with G:C base pairs and by designing the probe with an appropriate Tm. The beginning and end points of the probe should be chosen so that the length and %G and %C result in a TM about 2-10°C higher than the temperature at which the final assay will be performed. The importance and effect of various assay conditions will be explained further herein. Third, regions of the rRNA which are known to form strong structures inhibitory to hybridization are less preferred. Finally, probes with extensive self-complementarity should be avoided.

In some cases, there may be several sequences from a particular region which will yield probes with the desired hybridization characteristics. In other cases, one sequence may be significantly better than another which differs merely by a single base.

The following chart indicates how, for one embodiment of the invention useful in the detection of a nucleic acid in the presence of closely related nucleic acid sequences, unique sequences can be selected. In this example, rRNA sequences have been determined for organisms A-E and their sequences, represented numerically, are aligned as shown. It is seen that sequence 1 is common to all organisms A-E. Sequences 2-6 are found only in organisms A, B and C, while sequences 8, 9 and 10 are unique to organism A. Therefore, a probe complementary to sequences 8, 9 or 10 would specifically hybridize to organism A.

| Illustrative Pattern of Sequence Relationships Among Related Bacteria | | | | | | | | | | |
|---|---|---|---|---|---|---|---|---|---|---|
| Organism | rRNA Sequence | | | | | | | | | |
| A | 1 | 2 | 3 | 4 | 5 | 6 | 7 | 8 | 9 | 10 |
| B | 1 | 2 | 3 | 4 | 5 | 6 | 7 | 11 | 12 | 13 |
| C | 1 | 2 | 3 | 4 | 5 | 6 | 14 | 15 | 16 | 17 |
| D | 1 | 18 | 19 | 20 | 21 | 22 | 23 | 24 | 25 | 26 |
| E | 1 | 18 | 19 | 20 | 21 | 27 | 28 | 29 | 30 | 31 |

In cases where the patterns of variation of a macromolecule are known, for example, rRNA, one can focus on specific regions as likely candidates for probe design. However, it is not always necessary to determine the entire nucleic acid sequence in order to obtain a probe sequence. Extension from any single oligonucleotide primer can yield up to 300-400 bases of sequence. When a single primer is used to partially sequence the rRNA of the target organism and organisms closely related to the target, an alignment can be made as outlined above. Plainly, if a useful probe sequence is found, it is not necessary to continue rRNA sequencing using other primers. If, on the other hand, no useful probe sequence is obtained from sequencing with a first primer, or if higher sensitivity is desired, other primers can be used to obtain more sequences. In those cases where patterns of variation for a molecule are not well understood, more sequence data may be required prior to probe design.

Thus, in Examples 1-3 below, two 16S-derived primers were used. The first primer did not yield probe sequences which met the criteria listed herein. The second primer yielded probe sequences which were determined to be useful following characterization and testing for specificity as described. In Example 4, six 23S primers were used prior to locating the probe sequence set forth.

Once a presumptive unique sequence has been identified, a complementary DNA oligonucleotide is synthesized. This single stranded oligonucleotide will serve as the probe in the DNA/rRNA assay hybridization reaction. Defined oligonucleotides may be synthesized by any of several well known methods, including automated solid-phase chemical synthesis using cyanoethylphosphoramidite precursors. Barone, A.D. et al., Nucleic Acids Research 12, 4051-4060 (1984). In this method, deoxyoligonucleotides are synthesized on solid polymer supports. Release of the oligonucleotide from the support is accomplished by treatment with ammonium hydroxide at 60°C for 16 hours. The solution is dried and the crude product is dissolved in water and separated on polyacrylamide gels which generally may vary from 10-20% depending upon the length of the fragment. The major band, which is visualized by ultraviolet back lighting, is cut from the gel with a razor blade and extracted with 0.1M ammonium acetate, pH 7.0, at room temperature for 8-12 hours. Following centrifugation, the supernatant is filtered through a 0.4 micron filter and desalted on a P-10 column (Pharmacia). Other well known methods for construction of synthetic oligonucelotides may, of course, be employed.

Current DNA synthesizers can produce large amounts of synthetic DNA. After synthesis, the size of the newly made DNA is examined by gel filtration and molecules of varying size are generally detected. Some of these molecules represent abortive synthesis events which occur during the synthesis process. As part of post-synthesis purification, the synthetic DNA is usually size fractionated and only those molecules which are of the proper length are kept. Thus, it is possible to obtain a population of synthetic DNA molecules of uniform size.

It has been generally assumed, however, that synthetic DNA is inherently composed of a uniform population of molecules all of the same size and base sequence, and that the hybridization characteristics of every molecule in the preparation should be the same. In reality, preparations of synthetic DNA molecules are heterogeneous and are composed of significant numbers of molecules which, although the same size, are in some way different from each other and have different hybridization characteristics. Even different preparations of the same sequence can sometimes have different hybridization characteristics.

Accordingly, preparations of the same synthetic probe sequence can have different hybridization characteristics. Because cf this the specificity of probe molecules from different preparations can be different. The hybridization characteristics of each preparation should be examined in order to determine the hybridization conditions which must be used in order to obtain the desired probe specificity. For example, the synthetic probe described in Example 4 below has the specificity profile described in Table 14. These data were obtained by using the hybridization and assay conditions described. A separate preparation of this probe which has different hybridization characteristics may not have precisely the same specificity profile when assayed under the conditions presented in Example 4. Such probe preparations have been made. To obtain the desired specificity, these probes can be hybridized and assayed under different conditions, including salt concentration and/or temperature. The actual conditions under which the probe is to be used must be determined, or matched to extant requirements, for each batch of probe since the art of DNA synthesis is somewhat imperfect.

Following synthesis and purification of a particular oligonucleotide sequence, several procedures may be utilized to determine the acceptability of the final product. The first is polyacrylamide gel electrophrosis, which is used to determine size. The oligonucleotide is labelled using, for example, $^{32}$P-ATP and $T_4$ polynucleotide kinase. The labelled probe is precipitated in ethanol, centrifuged and the dried pellet resuspended in loading buffer (80% formamide, 20 mM NaOH, 1 mM EDTA, 0.1% Bromophenol blue and 0.1% xylene cyanol). The samples are heated for five minutes at 90°C and loaded onto a denaturing polyacrylamide gel. Electrophoresis is carried out in TBE buffer (0.1 M Tris HCl pH 8.3, 0.08 M boric acid, 0.002 M EDTA) for 1-2 hours at 1,000 volts. Following electrophoresis of the oligonucleotide the gel is exposed to X-ray film. The size of the oligonucleotide is then computed from the migration of oligonucleotide standards run concurrently.

The sequence of the synthetic oligonucleotide may also be checked by labelling it at the 5' end with $^{32}$P-ATP and $T_4$ polynucleotide kinase, subjecting it to standard chemical degradation techniques, Maxam, A.M. and Gilbert, W., Proc. Nat'l. Acad. Sci. USA 74, 560-564 (1980), and analyzing the products on polyacrylamide gels. Preferably, the nucleotide sequence of the probe is perfectly complementary to the previously identified unique rRNA sequence, although it need not be.

The melting profile, including the melting temperature (Tm) of the oligonucleotide/ rRNA hybrids should also be determined. One way to determine Tm is to hybridize a $^{32}$P-labelled oligonucleotide to its complementary target nucleic acid at 50°C in 0.1 M phosphate buffer, pH 6.8. The hybridization mixture is diluted and passed over a 2 cm hydroxyapatite column at 50°C. The column is washed with 0.1 M phosphate buffer, 0.02% SDS to elute all unhybridized, single-stranded probes. The column temperature is then dropped 15°C and increased in 5°C increments until all of the probe is eluted (single-stranded). At each temperature, unhybridized probe is eluted and the counts per minute (cpm) in each fraction determined. The number of cpm shown to be bound to the hydroxyapatite divided by the total cpm added to the column equals the percent hybridization of the probe to the target nucleic acid.

An alternate method for determining thermal stability of a hybrid is outlined below. An aliquot of hybrid nucleic acid is diluted into 1 ml of either 0.12M phosphate buffer, 0.2% SCS, 1mM EDTA, 1mM EGTA or an appropriate hybridization buffer. Heat this 1 ml of solution to 45 degrees C for 5 minutes and place it into a room temperature water bath to cool for 5 minutes. Assay this 1 ml of hybrid containing solution over a hydroxyapatite column, capturing the hybrid and washing away unbound probe. If a hybridization solution other than the 0.12M phosphate buffer is used, then a dilution of the hybridization solution into the 0.12M phosphate buffer cocktail will be necessary for binding. Keep taking aliquots of preformed hybrid and diluting into 1 ml of hybridization solution or into the standard 0.12M phosphate buffer solution described above while raising the heating temperature 5 degrees C at a time. Continue this until all of the hybrid is dissociated. The point where one half of the hybrid is converted to the dissociated form is considered the Tm. The Tm for a given hybrid will vary depending on the hybridization solution being used because the thermal stability depends upon the concentration of different salts, detergents, and other solutes which effect relative hybrid stability under thermal denaturation conditions.

Because the extent and specificity of hybridization reactions such as those described herein are affected by a number of factors, manipulation of one or more of those factors will determine the exact sensitivity and specificity of a particular probe, whether perfectly complementary to its target or not. For example, the base composition of the probe may be significant because G-C base pairs exhibit greater thermal stability as compared to A-T base pairs due to additional hydrogen bonding. Thus, hybridization involving complementary nucleic acids of higher G-C content will be stable at higher temperatures.

We have discovered that the length of the target nucleic acid sequence and, accordingly, the length of the probe sequence can also be important. While it is possible for nucleic acids that are not perfectly complementary to hybridize, the longest stretch of perfectly homologous base sequence will normally primarily determine hybrid stability. While oligonucleotide probes of different lengths and base composition may be used, oligonucleotide probes preferred in this invention are between about 15 and about 50 bases in length and are at least about 75-100% homologous to the target nucleic acid. For most applications 95-100% homology to the target nucleic acid is preferred.

Ionic strength and incubation temperature should also be taken into account in constructing a probe. It is known that the rate of hybridization will increase as ionic strength of the reaction mixture increases and that the thermal stability of hybrids will increase with increasing ionic strength. In general, optimal hybridization for synthetic oligonucleotide probes of about 15-50 bases in length occurs approximately 5°C below the melting temperature for a given duplex. Incubation at temperatures below the optimum may allow mismatched base sequences to hybridize and can therefore result in reduced specificity.

As to nucleic acid concentration, it is known that the rate of hybridization is proportional to the concentration of the two interacting nucleic acid species. Thus, the presence of compounds such as dextran and dextran sulphate are thought to increase the local concentration of nucleic acid species and thereby result in an increased rate of hybridization. Other agents which will result in increased rates of hybridization are specified in EP-A-0229442. On the other hand, chemical reagents which disrupt hydrogen bonds such as formamide, urea, DMSO, and alcohols will increase the stringency of hybridization.

Selected oligonucleotide probes may be labelled by any of several well known methods. Useful labels include radioisotopes as well as non-radioactive reporting groups. Isotopic labels include $^{3}H$, $^{35}S$, $^{32}P$, $^{125}I$ Cobalt and $^{14}C$. Most methods of isotopic labelling involve the use of enzymes and include the known methods of nick translation, end labelling, second strand synthesis, and reverse transcription. When using radio-labelled probes, hybridization can be detected by autoradiography, scintillation counting, or gamma counting. The detection method selected will depend upon the hybridization conditions and the particular radioisotope used for labelling.

Non-isotopic materials can also be used for labelling, and may be introduced by the incorporation of modified nucleotides through the use of enzymes or by chemical modification of the probe, for example, by the use of non-nucleotide linker groups. Non-isotopic labels include fluorescent molecules, chemiluminescent molecules, enzymes, cofactors, enzyme substrates, haptens or other ligands. We currently prefer to use acridinium esters.

In one embodiment of the DNA/rRNA hybridization assay invention, a labelled probe and bacterial target nucleic acids are contacted in solution. rRNA may be released from bacterial cells by a sonic disruption method. Other known methods for disrupting cells include the use of enzymes, osmotic shock, chemical treatment, and vortexing with glass beads. Following or concurrent with the release of rRNA, labelled probe may be added in the presence of accelerating agents and incubated at the optimal hybridization temperature for a period of time necessary to achieve significant hybridisation. Following this incubation period, hydroxyapatite may be added to the reaction mixture to separate the probe/rRNA hybrids from the non-hybridized probe molecules. The hydroxyapatite pellet is washed, recentrifuged and hybrids detected by means according to the label used.

Twenty-one embodiments illustrative of the claimed inventions are set forth below, in which a synthetic probe or probes complementary to a unique rRNA sequence from a target organism, or group of organisms is determined, constructed and used in a hybridization assay.

## DESCRIPTION OF PARTICULAR EMBODIMENTS

Mycobacterium are acid-fast, alcohol fast, aerobic, non-mobile bacilli. Their lipid content is high and their growth slow. Mycobacterium avium and Mycobacterium intracellulare are together referred to as M. avium-intracellulare because they are so difficult to differentiate. Recently, the M. avium complex, which includes M. intracellulare, was shown to be the second most commonly isolated, clinically significant Mycobacterium. Good, R.C. et al., J. Infect. Dis. 146, 829-833 (1982). More recent evidence indicates that these organisms are a common cause of opportunistic infection in patients with AIDS (acquired immune deficiency syndrome). Gill, V.J. et al., J. Clin. Microbio. 22, 543-546 (1985). Treatment of such infections in AIDS patients is difficult because these organisms are resistant to most antituberculosis drugs. Often a combination of five drugs are used in therapy. The severity of these infections also requires rapid diagnosis which, prior to the invention herein, was not available.

Members of the Mycobacterium tuberculosis complex (Mtb) include Mycobacterium tuberculosis, Mycobacterium bovis, Mycobacterium africanum and Mycobacterium microti. The first three are pathogenic for humans while the last is an animal pathogen. These organisms produce slowly developing granulomas on the skin or they may invade internal organs. Tuberculosis of the lungs can be disseminated to other parts of the body by the circulatory system, the lymph system, or the intestinal tract. Despite advances in public health and the advent of effective chemotherapy, Mycobacterial disease, tuberculosis in particular, continues to represent a major world-wide health problem.

The classical method for detecting bacteria in a test sample involves culturing of the sample in order to expand the number of bacterial cells present into observable colony growths which can be identified and enumerated. If desired, the cultures can also be subjected to additional testing in order to determine antimicrobial susceptibility. Currently, the most widely used procedures for the detection, isolation and identification of Mycobacterium species are the acid-fast bacilli (AFB) smear (using either the Ziehl-Neelsen or fluorochrome techniques), culture methods using Lowenstein-Jensen media and Middlebrook media, and biochemical tests. The AFB relies on the high lipid content of Mycobacterium to retain dye after exposure to acid-alcohol. While the AFB smear test is relatively rapid and simple to perform it does not always detect Mycobacteria and will not differentiate between Mycobacterium avium and non-tuberculosis species, between Mycobacterium intracellulare and non-tuberculosis species, or between Mycobacterium tuberculosis-complex bacilli and non-tuberculosis species. For accurate identification of the infecting Mycobacterial species the clinician must rely on culture results which can require anywhere from 3 to 8 weeks of growth followed by extensive biochemical testing. Other tests have been developed based on the detection of metabolic products from Mycobacterium using carbon-14 labelled substrates. In particular, the Bactec (TM) instrument can detect the presence of Mycobacterium within 6 to 10 days of the time of inoculation. Gill, V.J., supra. However, the test does not distinguish Mycobacterium species. It is often important to make this determination so that particular drugs to which the organism is susceptible may be prescribed. For traditional culture methods, this requires an additional 2 to 3 weeks and for the Bactec method, an additional 6 to 10 days.

In addition, specific embodiments for Mycoplasma pneumoniae, Mycobacterium, Legionella, Salmonella, Chlamydia trachomatis, Campylobacter, Proteus mirabilis, Enterococcus, Enterobacter cloacae, E. coli, Pseudomonas Group I, bacteria, fungi and Neisseria gonorrhoeae are set forth in the following examples.

As indicated by the below examples, the present invention has significant advantages over each of these prior art methods not only in the enhanced accuracy, specificity and simplicity of the test, but also in greatly reducing the time to achieve a diagnosis. The invention makes possible a definitive diagnosis and initiation of effective treatment on the same day as testing.

Example 1

Described below is the preparation of a single strand deoxyoligonucleotide of unique sequence and defined length which is labelled and used as a probe in a solution hybridization assay to detect the presence of rRNA from Mycobacterium avium. This unique sequence is specific for the rRNA of Mycobacterium avium and does not significantly cross-react under the hybridization conditions of this Example, with nucleic acids from any other bacterial species or respiratory infectious agent, including the closely-related Mycobacterium intracellulare. This probe is able to distinguish the two species, notwithstanding an approximate 98% rRNA approximate homology between the two species. In this Example, as well as in Examples 2 and 3, sequences for M. avium, M. tuberculosis complex, M. intracellulare and related organisms were obtained by using a specific primer to a highly conserved region in the 16S rRNA. The sequence of this primer, derived from E. coli rRNA, was 5'-GGC CGT TAC CCC ACC TAC TAG CTA AT-3'. 5 nanograms of primer was mixed with 1 microgram of each rRNA to be sequenced in the presence of 0.1M KCl and 20mM Tris-HCl pH 8.3 in a final volume of 10 microliters. The reactions were heated 10 min. at 45°C and then placed on ice. 2.5 microliters of $^{35}S$ dATP and 0.5 microliters of reverse transcriptase were added. The sample was aliquoted into 4 tubes, each tube containing either dideoxy A, G, T, or C. The concentrations of these nucleotides are set forth in Lane et al., supra. The samples were incubated at 40°C for 30 minutes, and were then precipitated in ethanol, centrifuged and the pellets lypholized dry. Pellets were resuspended in 10 microliters formamide dyes (100% formamide, 0.1% bromphenol blue and 0.1% xylene cyanol), and loaded onto 80 cm 8% polyacrylamide gels. The gels were run at 2000 volts for 2-4 hours.

Thus, nucleotide sequences for the 16S rRNA of Mycobacterium avium and what were considered to be its closest phylogenetic neighbors, Mycobacterium intracellulare and Mycobacterium tuberculosis, were determined by the method of Lane, D.J. et al., Proc. Nat. Acad. Sci. USA 82:6955 (1985). In addition to determining the rRNA sequences for the organisms noted above, a spectrum of clinically significant Mycobacterium were also sequenced. These included M. fortuitum, M. scrofulaceum and M. chelonae. Selected members of several genera closely related to Mycobacterium were also sequenced, including Rhodococcus bronchialis, Corynebacterium xerosis and Norcardia asteroides.

Partial rRNA sequences from the above organisms were aligned for maximum nucleotide homology, using commercially available software from Intelligenetics, Inc., 1975 El Canine Real West, Mountain View, California 94040-2216 (IFIND Program). From this alignment, regions of sequence unique to Mycobacterium avium were determined. The probe was selected so that it was perfectly complementary to a target nucleic acid sequence and so that it had a 10% or greater mismatch with the aligned rRNA from its known closest phylogenetic neighbor. A sequence 38 bases in length was chosen. The number of mismatched bases relative to the Mycobacterium avium sequence were as follows: Mycobacterium tuberculosis (8); Mycobacterium intracellulare (5); Mycobacterium scrofulaceum (6); Mycobacterium chelonae (12); and Mycobacterium fortuitum (10).

The following cDNA sequence was characterized by the criteria of length, Tm, and sequence analysis as described above before the Table headed "Illustrative patterns of Sequence Relation among related Bacteria and was determined to be specific for the rRNA of Mycobacterium avium:
ACCGCAAAAGCTTTCCACCAGAAGACATGCGTCTTGAG.
This sequence is complementary to a unique segment found in the 16S rRNA of Mycobacterium avium. The size of the probe is 38 bases. The probe has a Tm of 74°C and sequence analysis hy the method of Maxam & Gilbert (1980), supra, confirmed that the probe was correctly synthesized. The probe is capable of hybridizing to rRNA of M. avium in the region corresponding to bases 185-225 of E. coli 16S rRNA.

To demonstrate the reactivity of this sequence for Mycobacterium avium, it was tested as a probe in hybridization reactions under the following conditions. $^{32}P$-end-labeled oligonucleotide probes were mixed with 1 microgram ($7x10^{-13}$ moles) of purified rRNA from Mycobacterium avium and reacted in 0.12 M PB hybridization buffer (equimolar amounts of $Na_2HPO_4$ and $NaH_2PO_4$), 1 mM EDTA and 0.02% SDS (sodium dodecyl sulfate) at 65°C for 60 minutes in a final volume of 50 microliters. In separate tubes the probe was mixed with the hybridization buffer both with and without target present. Following separation on hydroxyapatite as outlined in the patents identified at page 2, supra, the hybrids were quantitated by scintillation counting. These results are presented in Table 1, showing that the probe has a high extent of reaction to homologous target and very little non-specific binding to the hydroxyapatite.

TABLE 1

| HYBRIDIZATION OF THE M. AVIUM PROBE TO HOMOLOGOUS TARGET rRNA* | | |
|---|---|---|
| | plus rRNA | minus rRNA |
| M. avium probe | 85-95% | 0.5% |

$$* \%Hybridization = \frac{\text{cpm bound to hydroxyapatite}}{\text{total cpm added to reaction}}$$

Specificity of the probe for M. avium was tested by mixing the $^{32}P$ labeled probe with rRNA released from cells of 29 other species of mycobacteria by sonic disruption techniques. 1 x $10^8$ cells were suspended in 0.1 ml 5% SDS and sonicated for 10 minutes at 50-60°C. 1.0 ml of hybridization buffer (45% sodium diisobutyl sulfosuccinate, 40 mM phosphate buffer pH 6.8 and 1 mM EDTA) was added and the mixture incubated for 60 minutes at 72°C. Following incubation, 4.0 ml of hydroxyapatite solution (0.14M sodium phosphate buffer, pH 6.8, 0.02% SDS and 1.0 gram hydroxyapatite per 50 mls solution) was added and incubated for 5 minutes at 72°C. The sample was centrifuged and the supernatant removed. 4.0 ml wash solution (0.14 M sodium phosphate pH 6.8) was added and sample was vortexed, centrifuged and the supernatant removed. The radioactivity bound to the hydroxyapatite was determined by scintillation counting. The results are shown in Table 2 and indicate that the probe is specific for Mycobacterium avium and does not react with any other mycobacterial species, including Mycobacterium intracellulare.

TABLE 2

| HYBRIDIZATION OF THE M. AVIUM PROBE TO MYCOBACTERIAL SPECIES | | |
|---|---|---|
| Organism | ATCC# | % Probe Bound |
| Mycobacterium africanum | 25420 | 1.0 |
| M. asiaticum | 25276 | 1.2 |
| M. avium | 25291 | 87.6 |
| M. bovis | 19210 | 1.2 |
| M. bovis (BCG) | 19015 | 1.0 |
| M. chelonae | 14472 | 0.9 |
| M. flavescens | 14474 | 0.9 |
| M. fortuitum | 6841 | 1.0 |
| M. gastri | 15754 | 1.2 |
| M. gordonae | 14470 | 1.2 |
| M. haemophilum | 29548 | 1.3 |
| M. intracallulare | 13950 | 1.5 |
| M. kansasii | 12478 | 1.2 |
| M. malmoense | 29571 | 1.2 |
| M. marinum | 827 | 1.2 |
| M. nonchromogenicum | 1930 | 1.1 |
| M. phlei | 11758 | 1.3 |

(continued)

| HYBRIDIZATION OF THE M. AVIUM PROBE TO MYCOBACTERIAL SPECIES | | |
|---|---|---|
| Organism | ATCC# | % Probe Bound |
| M. scrofulaceum | 19981 | 1.2 |
| M. shimoidei | 27962 | 2.3 |
| M. simiae | 25275 | 1.2 |
| M. smegmatis | el4468 | 1.0 |
| M. szulgai | 23069 | 1.0 |
| M. terrae | 15755 | 1.2 |
| M. thermoresistibile | 19527 | 1.3 |
| M. triviale | 23292 | 1.2 |
| M. tuberculosis (avirulent) | 25177 | 1.4 |
| M. tuberculosis (virulent) | 27294 | 1.1 |
| M. ulcerans | 19423 | 1.4 |
| M. vaccae | 15483 | 1.2 |
| M. xenopi | 19971 | 1.5 |

As shown in Table 3 the probe also did not react with the rRNA from any of the respiratory pathogens which were also tested by the method just described. Nor did the probe react with any other closely related or phylogenetically more diverse species of bacteria also tested by that method (Table 4).

TABLE 3

| HYBRIDIZATION OF M. AVIUM PROBE TO RESPIRATORY PATHOGENS | | |
|---|---|---|
| Organism | ATCC# | % Probe Bound |
| Corynebacterium xerosis | 373 | 0.7 |
| Fusobacterium nucleatum | 25586 | 1.3 |
| Haemophilum influenzae | 19418 | 1.3 |
| Klebsiella pneumoniae | 23357 | 1.8 |
| Legionella pneumophila | 33152 | 0.0 |
| Mycoplasma pneumoniae | 15531 | 3.0 |
| Neisseria meningitidis | 13090 | 0.0 |
| Pseudomonas aeruginosa | 25330 | 0.0 |
| Propionibacterium acnes | 6919 | 1.1 |
| Streptococcus pneumoniae | 6306 | 0.0 |
| Staphylococcus aureus | 25923 | 1.5 |

TABLE 4

| HYBRIDIZATION OF THE M. AVIUM PROBE TO A PHYLOGENETIC CROSS SECTION OF BACTERIAL SPECIES | | |
|---|---|---|
| Organism | ATCC# | % Probe Bound |
| Acinetobacter calcoaceticus | 33604 | 0.0 |
| Branhamella catarrhalis | 25238 | 0.6 |
| Bacillus subtilis | 6051 | 0.9 |
| Bacteroides fragilis | 23745 | 1.0 |
| Campylobacter jejuni | 33560 | 0.4 |
| Chromobacterium Violaceum | 29094 | 1.7 |
| Clostridium perfringens | 13124 | 2.1 |
| Deinococcus radiodurans | 35073 | 0.8 |
| Derxia gummosa | 15994 | 0.3 |
| Enterobacter aerogenes | 13048 | 0.6 |

(continued)

| HYBRIDIZATION OF THE M. AVIUM PROBE TO A PHYLOGENETIC CROSS SECTION OF BACTERIAL SPECIES | | |
|---|---|---|
| Organism | ATCC# | % Probe Bound |
| Escherichia coli | 11775 | 0.3 |
| Mycobacterium gordonae | 14470 | 1.9 |
| Mycoplasma hominis | 14027 | 3.3 |
| Proteus mirabilis | 29906 | 0.0 |
| Psudomonas cepacia | 11762 | 1.0 |
| Rahnella aquatilis | 33071 | 2.1 |
| Rhodospirillum rubrum | 11170 | 0.6 |
| Streptococcus mitis | 9811 | 0.9 |
| Vibrio parahaemolyticus | 17802 | 1.2 |
| Yersinia enterocolitica | 9610 | 0.4 |

Example 2

After the alignment described in Example 1, the following sequence was characterized by the aforementioned criteria of length, Tm and sequence analysis and was determined to be specific for Mycobacterium intracellulare:
ACCGCAAAAGCTTTCCACCTAAAGACATGCGCCTAAAG
The sequence is complementary to a unique segment found in the 16S rRNA of Mycobacterium intracellulare. The size of the probe was 38 bases. The probe has a Tm of 75°C and sequence analysis confirmed that the probe was correctly synthesized. The probe hybridizes to RNA of M. intracellulare in the region corresponding to bases 185-225 of E. coli 16S rRNA.

To demonstrate the reactivity of this sequence for the rRNA of Mycobacterium intracellulare, the probe was tested in hybridization reactions under the following conditions. $^{32}$P-end-labelled oligonucleotide probe was mixed with 1 microgram (7 x $10^{-13}$ moles) of purified rRNA from Mycobacterium intracellulare and reacted in 0.12 M PB (equimolar amounts of $Na_2HPO_4$ and $NaH_2PO_4$), 1 mM EDTA and 0.2% SDS (sodium dodecyl sulfate) at 65°C for 60 minutes in a final volume of 50 microliters. In separate tubes the probe was mixed with the hybridization buffer with and without target Mycobacterium intracellulare rRNA present. Following separation on hydroxyapatite as outlined previously the hybrids were quantitated by scintillation counting. These results are shown in Table 5.

TABLE 5

| HYBRIDIZATION OF THE M. INTRACELLULARE PROBE TO HOMOLOGOUS TARGET rRNA*/ | | |
|---|---|---|
| | plus rRNA | minus rRNA |
| M. intracellulare probe | 85-95% | 0.5% |
| $*\%\ Hybridization = \frac{\text{cpm bound to hydroxyapatite}}{\text{total cpm added to reaction}}$ | | |

These data shows that the probe has a high extent of reaction to its homologous target and very little non-specific binding to the hydroxyapatite.

Specificity of the Mycobacterium intracellulare probe was tested by mixing the $^{32}$P labelled probe with rRNA released from cells from 29 other species of mycobacteria by sonic disruption techniques. All hybridization assays were carried out as described in Example 1. Table 6 indicates that the probe is specific for Mycobacterium intracellulare and does not react with any other mycobacterial species, including Mvcobacterium avium. These results are impressive in view of the 98% rRNA homology to M. avium; 98% homology to M. kansasii; 98% homology to M. asiaticum; and 97% homology to M. tuberculosis.

TABLE 6

| HYBRIDIZATION OF THE M. INTRACELLULARE PROBE TO MYCOBACTERIAL SPECIES | | |
|---|---|---|
| Organism | ATCC# | % Probe Bound |
| Mycobacterium africanum | 25420 | 0.9 |
| M. asiaticum | 25276 | 1.1 |

(continued)

| HYBRIDIZATION OF THE M. INTRACELLULARE PROBE TO MYCOBACTERIAL SPECIES | | |
|---|---|---|
| Organism | ATCC# | % Probe Bound |
| M. avium | 25291 | 1.3 |
| M. bovis | 19210 | 1.1 |
| M. bovis (BCG) | 19015 | 1.2 |
| M. chelonae | 14472 | 1.0 |
| M. favescens | 14474 | 1.2 |
| M. fortuitum | 6841 | 1.3 |
| M. gastri | 15754 | 1.3 |
| M. gordonae | 14470 | 1.3 |
| M. haemophilum | 29548 | 0.9 |
| M. intracellulare | 13950 | 78.8 |
| M. kansasii | 12479 | 1.1 |
| M. Malmoense | 29571 | 1.0 |
| M. marinum | 827 | 0.9 |
| M. nonchromogenicum | 1930 | 1.0 |
| M. phlei | 11758 | 1.1 |
| M. scrofulaceum | 19981 | 1.0 |
| M. shimoidei | 27962 | 1.3 |
| M. simiae | 25275 | 1.1 |
| M. smegmatis | e14468 | 1.3 |
| M. szulgai | 23069 | 1.0 |
| M. terrae | 15755 | 1.4 |
| M. thermoresistibile | 19527 | 1.6 |
| M. triviale | 23292 | 1.3 |
| M. tuberculosis (avirulent) | 25177 | 1.2 |
| M. tuberculosis (virulent) | 27294 | 1.2 |
| M. ulcerans | 19423 | 1.1 |
| M. vaccae | 15483 | 1.0 |
| M. xenopi | 19971 | 1.2 |

As shown in Table 7 the probe did not react with the rRNA from any of the respiratory pathogens tested in the hybridization assay. Nor did the probe react with any other closely related or phylogenetically more diverse species of bacteria that were tested (Table 8).

TABLE 7

| HYBRIDIZATION OF THE M. INTRACELLULARE PROBE TO RESPIRATORY PATHOGENS | | |
|---|---|---|
| Organism | ATCC# | % Probe Bound |
| Corynebacterium xerosis | 373 | 2.2 |
| Fusobacterium nucleatum | 25586 | 1.5 |
| Haemophilum influenzae | 19418 | 1.3 |
| Klebsiella pneumoniae | 23357 | 1.2 |
| Legionella pneumophila | 33152 | 1.2 |
| Mycoplasma pneumoniae | 15531 | 3.2 |
| Neisseria meningitidis | 13090 | 1.1 |
| Pseudomonas aeruginosa | 25330 | 1.0 |
| Propionibacterium acnes | 6919 | 2.9 |
| Streptococcus pneumoniae | 6306 | 1.6 |
| Staphylococcus aureus | 25923 | 1.3 |

TABLE 8

| HYBRIDIZATION OF THE M. INTRACELLULARE PROBE TO A PHYLOGENETIC CROSS SECTION OF BACTERIAL SPECIES | | |
|---|---|---|
| Organism | ATTC# | % Probe |
| Acinetobacter calcoaceticus | 33604 | 1.5 |
| Branhamella catarrhalis | 25238 | 1.8 |
| Bacillus subtilis | 6051 | 1.7 |
| Bacteroides fragilis | 23745 | 1.9 |
| Campylobacter jejuni | 33560 | 1.9 |
| Chromobacterium violaceum | 29094 | 1.4 |
| Clostridium perfringens | 13124 | 2.1 |
| Deinococcus radiodurans | 35073 | 2.1 |
| Derxia gummosa | 15994 | 1.6 |
| Enterobacter aerogenes | 13048 | 1.3 |
| Escherichia coli | 11775 | 1.2 |
| Mycobacterium gordonae | 14470 | 2.3 |
| Mycoplasma hominis | 14027 | 2.6 |
| Proteus mirabilis | 29906 | 1.2 |
| Pseudomonas cepacia | 11762 | 1.7 |
| Rahnella aquatilis | 33071 | 1.5 |
| Rhodospirillum rubrum | 11170 | 1.4 |
| Strptococcus mitis | 9811 | 1.4 |
| Vibrio parahaemolyticus | 17802 | 2.5 |
| Yersinia enterocolitica | 9610 | 1.1 |

Example 3

After the alignment described in Example 1, the following sequence was characterized by the aforementioned three criteria of size, sequence and Tm, and was determined to be specific to the Mtb (Mycobacterium tuberculosis) complex of organisms, Mycobacterium tuberculosis, Mycobacterium africanum, Mycobacterium bovis, and Mycobacterium microti:

1. TAAAGCGCTTTCCACCACAAGACATGCATCCCGTG.

The sequence is complementary to a unique segment found in the 16S rRNA of the Mtb-complex bacteria. The size of the probe is 35 bases. The probe has a Tm of 72°C and sequence analysis confirmed that the probe was correctly synthesized. It is capable of hybridizing in the region corresponding to bases 185-225 of E. coli 16S rRNA.

To demonstrate the reactivity of this sequence for the Mtb complex the probe was tested in hybridization reactions under the following conditions. $^{32}$P-end-labelled oligonucleotide probe was mixed with 1 microgram (7 x $10^{-13}$ moles) of purified rRNA from Mycobacterium tuberculosis and reacted in 0.12 M PB hybridization buffer (equimolar amounts of $Na_2HPO_4$, and $NaH_2PO_4$), 1 mM EDTA and 0.2 SDS (sodium dodecyl sulfate) at 65°C for 60 minutes in a final volume of 50 microliters. In separate tubes the probe was mixed with the hybridization buffer with and without target rRNA from Mycobacterium tuberculosis present. Following separation on hydroxyapatite as outlined previously the hybrids were quantitated by scintillation counting. The results are shown in Table 9.

TABLE 9

| HYBRIDIZATION OF Mtb-COMPLEX 16S rRNA DNA PROBE TO HOMOLOGOUS TARGET rRNA*/ | | |
|---|---|---|
| | plus rRNA | minus rRNA |
| Mtb complex probe | 85-95% | 0.5% |
| *% Hybridization = $\frac{\text{cpm bound to hydroxyapatite}}{\text{total cpm added to reaction}}$ | | |

These data show that the probe has a high extent of reaction to homologous target and very little non-specific binding to the hydroxyapatite.

Specificity of the probe for the Mtb complex was tested by mixing the $^{32}P$ labelled probe with rRNA released from cells of the 4 Mtb complex bacilli and of 25 other mycobacterial species by sonic disruption techniques. All hybridization assays were carried out as described in Example 1. Table 10 indicates that the probe is specific for organisms within the Mtb complex and does not react with any other mycobacterial species.

TABLE 10

| HYBRIDIZATION OF Mtb-COMPLEX 16S rRNA DNA PROBE TO MYCOBACTERIAL SPECIES | | |
|---|---|---|
| Organism | ATCC# | % Probe Bound |
| Mycobacterium africanum | 25420 | 68.1 |
| M. asiaticum | 25276 | 3.4 |
| M. avium | 25291 | 0.9 |
| M. bovis | 19210 | 63.1 |
| M. chelonae | 14472 | 1.1 |
| M. flavescens | 14474 | 0.9 |
| M. fortuitum | 6841 | 1.1 |
| M. gastri | 15754 | 0.8 |
| M. gordonae | 14470 | 1.1 |
| M. haemophilum | 29548 | 0.8 |
| M. intracallulare | 13950 | 1.1 |
| M. kansasii | 12479 | 1.3 |
| M. malmoense | 29571 | 0.9 |
| M. marinum | 827 | 1.1 |
| M. nonchromogenicum | 1930 | 1.1 |
| M. phlei | 11758 | 1.3 |
| M. scrofulaceum | 19981 | 1.1 |
| M. shimoidei | 27962 | 1.0 |
| M. simiae | 25275 | 1.2 |
| M. smegmatis | el4468 | 0.9 |
| M. szulgai | 23069 | 1.1 |
| M. terrae | 15755 | 1.0 |
| M. thermoresistibile | 19527 | 1.0 |
| M. triviale | 23292 | 1.2 |
| M. tuberculosis (avirulent) | 25177 | 66.2 |
| M. tuberculosis (virulent) | 27294 | 62.4 |
| M. ulcerans | 19423 | 0.9 |
| M. vaccae | 15483 | 0.8 |
| M. xenopi | 19971 | 2.6 |

As shown in Table 11 the probe did not react with the rRNA from any of the respiratory pathogens tested in the hybridization assay. Nor did the probe react with any other closely related or phylogenetically more diverse species of bacteria that were tested (Table 12).

TABLE 11

| HYBRIDIZATION OF Mtb-COMPLEX 16S rRNA DNA PROBE TO RESPIRATORY PATHOGENS | | |
|---|---|---|
| Organism | ATCC# | % Probe Bound |
| Corynebacterium xerosis | 373 | 1.3 |
| Fusobacterium nucleatum | 25586 | 1.0 |
| Haemophilum influenzae | 19418 | 1.6 |
| Klebsiella pneumoniae | 23357 | 1.2 |
| Legionella pneumophila | 33152 | 1.4 |

(continued)

| HYBRIDIZATION OF Mtb-COMPLEX 16S rRNA DNA PROBE TO RESPIRATORY PATHOGENS | | |
|---|---|---|
| Organism | ATCC# | % Probe Bound |
| Mycoplasma pneumoniae | 15531 | 1.1 |
| Neisseria meningitidis | 13090 | 1.0 |
| Pseudomonas aeruginosa | 25330 | 1.7 |
| Propionibacterium acnes | 6919 | 1.2 |
| Streptococcus pneumoniae | 25923 | 0.9 |

TABLE 12

| HYBRIDIZATION OF THE Mtb-COMPLEX 16S rRNA DNA PROBE TO A PHYLOGENETIC CROSS SECTION OF BACTERIAL SPECIES | | |
|---|---|---|
| Organism | ATCC# | % Probe |
| Acinetobacter calcoaceticus | 33604 | 1.3 |
| Branhamella catarrhalis | 25238 | 1.5 |
| Bacillus subtilis | 6051 | 1.3 |
| Bacteroides fragilis | 23745 | 1.3 |
| Campylobacter jejuni | 33560 | 1.1 |
| Chromobacterium violaceum | 29094 | 1.0 |
| Clostridium perfringens | 13124 | 1.2 |
| Deinococcus radiodurans | 35073 | 1.0 |
| Derxia gummosa | 15994 | 1.0 |
| Enterobacter aerogenes | 13048 | 1.0 |
| Escherichia coli | 11775 | 1.0 |
| Mycobacterium gordonae | 14470 | 1.3 |
| Mycoplasma hominis | 14027 | 0.5 |
| Proteus mirabilis | 29906 | 1.0 |
| Pseudomonas cepacia | 11762 | 2.6 |
| Rahnella aquatilis | 33071 | 1.9 |
| Rhodospirillum rubrum | 11170 | 1.0 |
| Streptococcus mitis | 9811 | 1.1 |
| Vibrio parahaemolyticus | 17802 | 0.9 |
| Yersinia enterocolitica | 9610 | 1.1 |

Two derivatives of the probe of Example 3 (numbered 2-3 below) were made and tested:
2. CCGCTAAAGCGCTTTCCACCACAAGACATGCATCCCG
3. ACACCGCTAAAGCGCTTTCCACCACAAGACATGCATC.

All three probes have similar Tms (72°C; 73.5°C; and 72.3°C, respectively) and similar hybridization characteristics.

Hybridization to Mycobacterium tuberculosis complex organisms was 68-75% and non-specific hybridization to hydroxyapatite was less than 2%. Results of hybridization assay tests for these derivatives follow.

TABLE 13

| HYBRIDIZATION OF PROBE OF EXAMPLES 3 AND 2 DERIVATIVES THEREOF TO MYCOBACTERIAL SPECIES | | | | |
|---|---|---|---|---|
| Organism | ATCC# | Example % Probe 1 Bound | % Probe 2 Bound | % Probe 3 Bound |
| Mycobacterium africanum | 25420 | 68.1 | 69.4 | 70.6 |
| M. asiaticum | 25274 | 3.4 | 5.3 | 1.8 |

(continued)

| HYBRIDIZATION OF PROBE OF EXAMPLES 3 AND 2 DERIVATIVES THEREOF TO MYCOBACTERIAL SPECIES | | | | |
|---|---|---|---|---|
| Organism | ATCC# | Example % Probe 1 Bound | % Probe 2 Bound | % Probe 3 Bound |
| M. avium | 25291 | 0.9 | 1.6 | 1.4 |
| M. bovis | 19210 | 63.1 | 75.3 | 74 |
| M. chelonae | 14472 | 1.1 | 1.5 | 1.6 |
| M. flavescens | 14474 | 0.9 | 2.7 | 1.4 |
| M. fortuitum | 6841 | 1.1 | 3.6 | 1.5 |
| M. gastri | 15754 | 0.8 | 3.6 | 1.7 |
| M. gordonae | 14470 | 1.1 | 1.6 | 1.4 |
| M. haemophilum | 29548 | 0.8 | 3.2 | 1.7 |
| M. intracellulare | 13950 | 1.1 | 1.6 | 1.4 |
| M. kansasii | 12478 | 1.3 | 2.1 | 2.0 |
| M. malmoense | 29571 | 0.9 | 2.8 | 1.5 |
| M. marinum | 827 | 1.1 | 2.1 | 1.5 |
| M. nonchromogenicum | 1930 | 1.1 | 3.0 | 1.5 |
| M. phlei | 11758 | 1.3 | 1.3 | 1.1 |
| M. scrofulaceum | 19981 | 1.1 | 3.4 | 1.6 |
| M. shimoidei | 27962 | 1.0 | 2.7 | 1.6 |
| M. simiae | 25275 | 1.2 | 2.9 | 1.8 |
| M. smegmatis | e14468 | 0.9 | 1.5 | 1.2 |
| M. szulgai | 23069 | 1.1 | 3.6 | 1.1 |
| M. terrae | 15755 | 1.0 | 3.7 | 2.0 |
| M. thermoresistibile | 19527 | 1.0 | 1.6 | 1.3 |
| M. triviale | 23292 | 1.2 | 1.6 | 2.0 |
| M. tuberculosis (avirulent) | 25177 | 66.2 | 75 | 68 |
| M. tuberculosis (virulent) | 27294 | 62.4 | 74 | 75 |
| M. ulcerans | 19423 | 0.9 | 1.7 | 3.0 |
| M. vaccae | 15483 | 0.8 | 1.4 | 1.2 |
| M. xenopi | 19971 | 2.6 | 1.4 | 1.2 |

Example 4

The probe specific for the 23S rRNA of the M. tuberculosis complex was obtained by using a primer which was complementary to a highly conserved region of 23S rRNA. The sequence of this primer, derived from E. coli rRNA, was 5'-AGG AAC CCT TGG GCT TTC GG-3'. Five nanograms of this primer was mixed with 1 microgram of rRNA from M. tuberculosis and other closely related Mycobacterium and the procedure as described for Examples 1, 2 and 3 was followed. After alignment as described in Example 1, the following sequence was determined to be specific to the Mtb complex of organisms, Mycobacterium tuberculosis, Mycobacterium africanum, Mycobacterium bovis, and Mycobacterium microti:

TGCCCTACCCACACCCACCACAAGGTGATGT.

The sequence is complementary to a unique segment found in the 23S rRNA of the Mtb-complex bacteria. The oligonucleotide probe was characterized as previously described by the criteria of length, Tm and sequence analysis. The size of the probe is 31 bases. The probe has a Tm of 72.5°C and sequence analysis confirmed that the probe was correctly synthesized. It is capable of hybridizing in the region corresponding to bases 1155-1190 of E. coli 23S rRNA.

To demonstrate the reactivity of this sequence for the Mtb complex the probe was tested in hybridization reactions under the following conditions. $^{32}P$-end-labelled oligonucleotide probes were mixed with 1 microgram ($7\times10^{-13}$ moles) of purified rRNA from Mycobacterium tuberculosis and reacted in 0.12 M PB hybridization buffer (equimolar amounts of $Na_2HPO_4$, and $NaH_2PO_4$), 1 mM EDTA and 0.2 SDS (sodium dodecyl sulfate) at 65°C for 60 minutes in a final volume of 50 microliters. In separate tubes the probe was mixed with the hybridization buffer with and without target rRNA from Mycobacterium tuberculosis present. Following separation on hydroxyapatite as outlined previously the hybrids were quantitated by scintillation counting. The results are shown in Table 14.

TABLE 14

| HYBRIDIZATION OF THE Mtb-COMPLEX 23S rRNA DNA PROBE TO HOMOLOGOUS TARGET rRNA | | |
|---|---|---|
| | plus rRNA | minus rRNA |
| Mtb complex 23S probe | 94% | 1.2% |

These data show that the probe has a high extent of reaction to homologous target and very little non-specific binding to the hydroxyapatite.

Specificity of the probe for the Mtb complex was tested by mixing the $^{32}P$ labelled probe with rRNA released from cells of the four Mtb complex bacilli and of 25 other mycobacterial species by sonic disruption techniques. All hybridization assays were carried out as described in Example 1. Table 14 indicates that the probe is specific for organisms within the Mtb complex and does not react with any other mycobacterial species.

TABLE 15

| HYBRIDIZATION OF Mtb-COMPLEX 23S rRNA DNA PROBE TO MYCOBACTERIAL SPECIES | | |
|---|---|---|
| Organism | ATCC# | % Probe Bound |
| Mycobacterium africanum | 25420 | 33.6 |
| M. asiaticum | 25276 | 1.2 |
| M. avium | 25291 | 1.0 |
| M. bovis | 19210 | 32.0 |
| M. chelonae | 14472 | 1.2 |
| M. flavescens | 14474 | 1.2 |
| M. fortuitum | 6841 | 1.3 |
| M. gastri | 15754 | 1.1 |
| M. gordonae | 14470 | 1.2 |
| M. haemophilum | 29548 | 1.2 |
| M. intracellulare | 13950 | 1.1 |
| M. kansasii | 12479 | 1.3 |
| M. malmoense | 29571 | 1.3 |
| M. marinum | 827 | 1.2 |
| M. nonchromogenicum | 1930 | 1.0 |
| M. phlei | 11758 | 1.0 |
| M. scrofulaceum | 19981 | 1.1 |
| M. shimoidei | 27962 | 1.2 |
| M. simiae | 25275 | 1.3 |
| M. smegmatis | el4468 | 1.1 |
| M. szulgai | 23069 | 1.1 |
| M. terrae | 15755 | 1.0 |
| M. thermoresistibile | 19527 | 1.2 |
| M. triviale | 23292 | 1.0 |
| M. tuberculosis (avirulent) | 25177 | 33.7 |
| M. tuberculosis (virulent) | 27294 | 38.1 |
| M. ulcerans | 19423 | 1.3 |
| M. vaccae | 15483 | 1.0 |
| M. xenopi | 19971 | 1.3 |

Example 5

Three additional Mycobacterium tuberculosis complex probes, Examples 5-7 herein, were identified using two unique primers complementary to 23S rRNA. The first sequence is:

CCATCACCACCCTCCTCCGGAGAGGAAAAGG

The sequence of this Example 5 was obtained using a 23S primer with the sequence 5'-GGC CAT TAG ATC ACT CC-3'. It was characterized and shown to be specific for the Mycobacterium tuberculosis complex of organisms including Mycobacterium tuberculosis, Mycobacterium africanum and Mycobacterium bovis. This sequence, from 23S rRNA, is 31 bases in length and has a Tm of 72°C. This probe is capable of hybridizing to RNA of the aforementioned organisms in the region corresponding to bases 540-575 of E. coli 23S rRNA.

To demonstrate the reactivity and specificity of this probe for Mycobacterium tuberculosis complex, it was tested as a probe in hybridization reactions under the following conditions. $^{32}P$-end-labeled oligonucleotide probe was mixed with rRNA released from cells of 30 species of mycobacteria by sonic disruption techniques. 3 x $10^7$ cells were suspended in 0.1 ml 5% SDS and sonicated for 15 minutes at 50-60°C. One ml of hybridization buffer (45% diisobutyl sulfosuccinate, 40 mM phosphate buffer pH 6.8, 1 mM EDTA, 1 mM EGTA) was added and the mixture incubated at 72°C for 2 hours. Following incubation, 4 ml of 2% (w/v) hydroxyapatite, 0.12M sodium phosphate buffer pH6.8, 0.02% SDS, 0.02% sodium azide was added and incubated at 72°C for 5 minutes. The sample was centrifuged and the supernatant removed. Four ml wash solution (0.12M sodium phosphate buffer pH6.8, 0.02% SDS, 0.02% sodium azide) was added and the sample was vortexed, centrifuged and the supernatant removed. The radioactivity bound to the hydroxyapatite was determined by scintillation counting. The results are shown in Table 16 and indicate that the probe is specific for the Mycobacterium tuberculosis complex of organisms.

TABLE 16

HYBRIDIZATION OF THE M. TUBERCULOSIS COMPLEX PROBE OF EXAMPLE 5 TO MYCOBACTERIAL SPECIES

| Organism | ATCC # | % Probe Bound |
|---|---|---|
| Mycobacterium africanum | 25420 | 18.0 |
| M. asiaticum | 25274 | 2.6 |
| M. avium | 25291 | 3.4 |
| M. bovis | 19210 | 21.7 |
| M. bovis (BCG) | 35734 | 35.3 |
| M. chelonae | 14472 | 3.8 |
| M. flavescens | 14474 | 2.3 |
| M. fortuitum | 6841 | 1.8 |
| M. gastri | 15754 | 2.2 |
| M. gordonae | 14470 | 2.8 |
| M. haemophilum | 29548 | 2.8 |
| M. intracellulare | 13950 | 2.1 |
| M. kansasii | 12478 | 1.6 |
| M. malmoense | 29571 | 2.3 |
| M. marinum | 827 | 2.1 |
| M. nonchromogenicum | 1930 | 2.3 |
| M. phlei | 11758 | 2.1 |
| M. scrofulaceum | 19981 | 2.2 |
| M. shimoidei | 27962 | 1.9 |
| M. simiae | 25275 | 2.2 |
| M. smegmatis | e14468 | 2.0 |
| M. szulgai | 23069 | 2.2 |
| M. terrae | 15755 | 2.2 |
| M. thermoresistible | 19527 | 2.2 |
| M. triviale | 23292 | 2.0 |
| M. tuberculosis (avirulent) | 25177 | 26.4 |
| M. tuberculosis (virulent) | 27294 | 36.6 |
| M. ulcerans | 19423 | 2.5 |
| M. vaccae | 15483 | 2.4 |
| M. xenopi | 19971 | 2.8 |

Table 16 shows that the probe also did not cross react with RNA from any of the closely related organisms tested by the method just described.

TABLE 17

| HYBRIDIZATION OF THE M. TUBERCULOSIS COMPLEX PROBE OF EXAMPLE 5 TO PHYLOGENETICALLY CLOSELY RELATED ORGANISMS | | |
|---|---|---|
| Organism | ATCC# | % Probe Bound |
| Actinomadura madurae | 19425 | 2.1 |
| Actinoplanes italicus | 10049 | 3.1 |
| Arthrobacter oxidans | 14358 | 2.1 |
| Brevibacterium linens | e9172 | 1.9 |
| Corynebacterium xerosis | 373 | 2.2 |
| Dermatophilus congolensis | 14367 | 2.2 |
| Microbacterium lacticum | 8180 | 2.1 |
| Nocardia asteroides | 19247 | 2.0 |
| Nocardia brasiliensis | 19296 | 2.2 |
| Nocardia otitidis-caviarum | 14629 | 2.0 |
| Nocardioposis dassonvillei | 23218 | 4.0 |
| Oerskovia turbata | 33225 | 2.2 |
| Oerskovia xanthineolytica | 27402 | 2.0 |
| Rhodococcus aichiensis | 33611 | 1.9 |
| Rhodococcus aurantiacus | 25938 | 2.0 |
| Rhodococcus bronchialis | 25592 | 2.1 |
| Rhodococcus chubuensis | 33609 | 2.3 |
| Rhodococcus equi | 6939 | 2.4 |
| Rhodococcus obuensis | 33610 | 2.2 |
| Rhodococcus sputi | 29627 | 2.3 |

Example 6

The second Mycobacterium tuberculosis complex probe was obtained using a 23S primer with the sequence 5' CCT GAT TGC CGT CCA GGT TGA GGG AAC CTT TGG G-3'. Its sequence is:
CTGTCCCTAAACCCGATTCAGGGTTCGAGGTTAGATGC
This sequence, from 23S rRNA, is 38 bases in length and has a Tm of 75°C. It hybridizes in the region corresponding to bases 2195-2235 of E. coli 23S rRNA.

Like the complex probe in Example 5, this sequence was characterized and shown to be specific for the Mycobacterium tuberculosis complex of organisms including Mycobacterium tuberculosis, Mycobacterium africanum and Mycobacterium bovis.

To demonstrate the reactivity and specificity of the probe of this Example 6 to Mycobacterium tuberculosis complex, it was tested as a probe in hybridization reactions under the following conditions described for the probe in Example 5. The results are shown in Table 18 and indicate that the probe is specific for the Mycobacterium tuberculosis complex of organisms with the exception of Mycobacterium thermoresistibile, a rare isolate which is not a human pathogen.

TABLE 18

| HYBRIDIZATION OF THE M. TUBERCULOSIS COMPLEX PROBE OF EXAMPLE 6 TO MYCOBACTERIAL SPECIES | | |
|---|---|---|
| Organism | ATCC # | % Probe Bound |
| Mycobacterium africanum | 25420 | 56.0 |
| M. asiaticum | 25274 | 3.1 |
| M. avium | 25291 | 2.6 |
| M. bovis | 19210 | 48.0 |

(continued)

| HYBRIDIZATION OF THE M. TUBERCULOSIS COMPLEX PROBE OF EXAMPLE 6 TO MYCOBACTERIAL SPECIES | | |
|---|---|---|
| Organism | ATCC # | % Probe Bound |
| M. bovis (BCG) | 35734 | 63.0 |
| M. chelonae | 14472 | 2.8 |
| M. flavescens | 14474 | 2.8 |
| M. fortuitum | 6841 | 3.0 |
| M. gastri | 15754 | 3.2 |
| M. gordonae | 14470 | 3.0 |
| M. haemophilum | 29548 | 3.0 |
| M. intracellulare | 13950 | 3.6 |
| M. kansasii | 12478 | 3.9 |
| M. malmoense | 29571 | 2.9 |
| M. marinum | 827 | 2.9 |
| M. nonchromogenicum | 1930 | 4.8 |
| M. phlei | 11758 | 2.9 |
| M. scrofulaceum | 19981 | 2.6 |
| M. shimoidei | 27962 | 3.6 |
| M. simiae | 25275 | 3.3 |
| M. smegmatis | el4468 | 3.0 |
| M. szulgai | 23069 | 2.8 |
| M. terrae | 15755 | 2.8 |
| M. thermoresistibile | 19527 | 11.7 |
| M. triviale | 23292 | 3.2 |
| M. tuberculosis (avirulent) | 25177 | 65.0 |
| M. tuberculosis (virulent) | 27294 | 53.0 |
| M. ulcerans | 19423 | 2.5 |
| M. vaccae | 15483 | 2.8 |
| M. xenopi | 19971 | 3.3 |

Table 19 shows that the probe also did not cross react with RNA from any of the phylogenetically closely related organisms tested by the method just described.

TABLE 19

| HYBRIDIZATION OF THE M. TUBERCULOSIS COMPLEX PROBE OF EXAMPLE 6 TO PHYLOGENETICALLY CLOSELY RELATED ORGANISMS | | |
|---|---|---|
| Organism | ATCC # | % Probe Bound |
| Actinomadura madurae | 19425 | 1.3 |
| Actinoplanes italicus | 10049 | 0.6 |
| Arthrobacter oxidans | 14358 | 1.1 |
| Brevibacterium linens | e9172 | 0.8 |
| Corynebacterium xerosis | 373 | 1.0 |
| Dermatophilus congolensis | 14367 | 0.6 |
| Microbacterium lacticum | 8180 | 1.9 |
| Nocardia asteroides | 19247 | 0.9 |
| Nocardia brasiliensis | 19296 | 0.8 |
| Nocardia otitidis-caviarum | 14629 | 1.5 |
| Nocardioposis dassonvillei | 23218 | 0.5 |
| Oerskovia turbata | 33225 | 0.3 |
| Oerskovia xanthineolytica | 27402 | 0.8 |

(continued)

| HYBRIDIZATION OF THE M. TUBERCULOSIS COMPLEX PROBE OF EXAMPLE 6 TO PHYLOGENETICALLY CLOSELY RELATED ORGANISMS | | |
|---|---|---|
| Organism | ATCC # | % Probe Bound |
| Rhodococcus aichiensis | 33611 | 1.6 |
| Rhodococcus aurantiacus | 25938 | 0.7 |
| Rhodococcus bronchialis | 25592 | 1.5 |
| Rhodococcus chubuensis | 33609 | 0.8 |
| Rhodococcus equi | 6939 | 0.3 |
| Rhodococcus obuensis | 33610 | 0.8 |
| Rhodococcus sputi | 29627 | 1.4 |

Example 7

The following additional Mycobacterium tuberculosis complex probe also has been identified using a 23S primer with the same sequence as that of Example 6, namely, 5'-CCT GAT TGC CGT CCA GGT TGA GGG AAC CTT TGG G-3': AGGCACTGTCCCTAAACCCGATTCAGGGTTC.

This sequence, from 23S rRNA is 31 bases in length and has a Tm of 71°C. It hybridizes in the region corresponding to bases 2195-2235 of E. coli 23S rRNA. As is the case with the Mycobacterium tuberculosis complex probes of Examples 5 and 6 herein, this sequence also was characterized and shown to be specific for the Mycobacterium tuberculosis complex of organisms, including Mycobacterium tuberculosis, Mycobacterium africanum and Mycobacterium bovis.

To demonstrate the reactivity and specificity of this probe for Mycobacterium tuberculosis complex, it was tested as a probe in hybridization reactions under the conditions described for the probe of Example 5. Table 20 shows that the probe is specific for the Mycobacterium tuberculosis complex of organisms.

TABLE 20

| HYBRIDIZATION OF THE MYCOBACTERIUM TUBERCULOSIS COMPLEX PROBE OF EXAMPLE 7 TO MYCOBACTERIAL SPECIES | | |
|---|---|---|
| Organism | ATCC # | % Probe Bound |
| Mycobacterium africanum | 25420 | 43.0 |
| M. asiaticum | 25274 | 0.6 |
| M. avium | 25291 | 0.7 |
| M. bovis | 19210 | 43.0 |
| M. bovis (BCG) | 35734 | 46.0 |
| M. chelonae | 14472 | 0.6 |
| M. flavescens | 14474 | 0.6 |
| M. fortuitum | 6841 | 0.5 |
| M. gastri | 15754 | 0.9 |
| M. gordonae | 14470 | 0.7 |
| M. haemophilum | 29548 | 0.6 |
| M. intracellulare | 13950 | 0.6 |
| M. kansasii | 12478 | 0.9 |
| M. malmoense | 29571 | 0.8 |
| M. marinum | 827 | 0.7 |
| M. nonchromogenicum | 1930 | 0.8 |
| M. phlei | 11758 | 0.6 |
| M. scrofulaceum | 19981 | 0.7 |
| M. shimoidei | 27962 | 0.8 |
| M. simiae | 25275 | 0.7 |
| M. smegmatis | e14468 | 0.6 |
| M. szulgai | 23069 | 0.6 |

(continued)

| HYBRIDIZATION OF THE MYCOBACTERIUM TUBERCULOSIS COMPLEX PROBE OF EXAMPLE 7 TO MYCOBACTERIAL SPECIES | | |
|---|---|---|
| Organism | ATCC # | % Probe Bound |
| M. terrae | 15755 | 0.7 |
| M. thermoresistibile | 19527 | 0.9 |
| M. triviale | 23292 | 0.7 |
| M. tuberculosis (avirulent) | 25177 | 40.0 |
| M. tuberculosis (virulent) | 27294 | 50.0 |
| M. ulcerans | 19423 | 0.7 |
| M. vaccae | 15483 | 0.4 |
| M. xenopi | 19971 | 0.6 |

Table 21 shows that the probe also did not cross react with RNA from any of the closely related organisms tested by the method just described.

TABLE 21

| HYBRIDIZATION OF THE M. TUBERCULOSIS COMPLEX PROBE OF EXAMPLE 7 TO PHYLOGENETICALLY CLOSELY RELATED ORGANISMS | | |
|---|---|---|
| Organism | ATCC # | % Probe Bound |
| Actinomadura madurae | 19425 | 1.0 |
| Actinoplanes italicus | 10049 | 0.6 |
| Arthrobacter oxidans | 14358 | 0.4 |
| Brevibacterium linens | e9172 | 0.8 |
| Corynebacterium xerosis | 373 | 0.6 |
| Dermatophilus congolensis | 14367 | 0.8 |
| Microbacterium lacticum | 8180 | 0.5 |
| Nocardia asteroides | 19247 | 0.7 |
| Nocardia brasiliensis | 19296 | 0.5 |
| Nocardia otitidis-caviarum | 14629 | 0.6 |
| Nocardioposis dassonvillei | 23218 | 0.6 |
| Oerskovia turbata | 33225 | 0.8 |
| Oerskovia xanthineolytica | 27402 | 0.6 |
| Rhodococcus aichiensis | 33611 | 0.7 |
| Rhodococcus aurantiacus | 25938 | 0.7 |
| Rhodococcus bronchialis | 25592 | 0.6 |
| Rhodococcus chubuensis | 33609 | 0.6 |
| Rhodococcus equi | 6939 | 0.6 |
| Rhodococus obuensis | 33610 | 0.6 |
| Rhodococcus sputi | 29627 | 0.9 |

Notably, overlapping probes may have identical specificity. Compare, for example, the probes of Examples 6 and 7:

Ex. 6 CTGTCCCTAAACCCGATTCAGGGTTCGAGGTTAGATGC

Ex. 7 AGGCACTGTCCCTAAACCCGATTCAGGGTTC

There may be several sequences from a particular region which will yield probes with the desired hybridization characteristics. In other cases, one probe sequence may be significantly better than another probe differing by a single base. In general, the greater the sequence difference (% mismatch) between a target and nontarget organism, the more likely one will be able to alter the probe without affecting its usefulness for a specific application. This phenomenon also was demonstrated by the derivative probes in Example 3.

In Example 7, five bases were added to the 5' end of the probe in Example 6, and 12 bases were removed from the 3' end. The two probes have essentially identical hybridization characteristics.

Example 8

The Mycobacterium genus is particularly difficult to distinguish from Nocardia, Corynebacterium and Rhodococcus. These genera have common antigens, precipitins and G & C counts. Despite the fact that these organisms also exhibit 92-94% rRNA homology to the above listed organisms, we have designed probes which detect all members of the genus Mycobacterium without cross reacting to the related genera.

In addition to the Mycobacterium species probes already disclosed, four probes specific for members of the Mycobacterium genus were identified using one primer complementary to 16S rRNA and one primer complementary to 23S rRNA. Sequence 1 was obtained using a 16S primer with the sequence 5'-TTA CTA GCG ATT CCG ACT TCA-3'. Sequences 2, 3 and 4 were obtained using a 235 primer with the sequence 5'-GTG TCG GTT TTG GGT ACG-3'. Sequence 1 is capable of hybridizing to RNA of the genus Mycobacterium in the region corresponding to bases 1025-1060 of E. coli 16S rRNA. Sequences 2-4 hybridize in regions corresponding to the following bases of E. coli 23S rRNA in our numbering system (See Figure 2); 1440-1475; 1515-1555; 1570-1610 in our numbering system.

The following sequences were characterized and shown to be specific for the genus Mycobacterium:

1. CCA TGC ACC ACC TGC ACA CAG GCC ACA AGG
2. GGC TTG CCC CAG TAT TAC CAC TGA CTG GTA CGG
3. CAC CGA ATT CGC CTC AAC CGG CTA TGC GTC ACC TC
4. GGG GTA CGG CCC GTG TGT GTG CTC GCT AGA GGC

Sequence 1, from 16S rRNA, is 30 bases in length and has a Tm of 73°C. Sequence 2, from 23S rRNA, is 33 bases in length and has a Tm of 75°C. Sequence 3, from 23S rRNA, is 35 bases in length and has a Tm of 76°C. Sequence 4, from 23S rRNA, is 33 bases in length and has a Tm of 73°C.

To demonstrate the reactivity and specificity of probe 1 for members of the genus Mycobacterium, it was tested as a probe in hybridization reactions under the following conditions. $^{125}$I-labeled oligonucleotide probes were mixed with rRNA released from cells of 30 species of mycobacteria by sonic disruption techniques. 3 x $10^7$ cells were suspended in 0.1 ml 5% SDS and sonicated for 15 minutes at 50-60°C. One ml of hybridization buffer (45% diisobutyl sulfosuccinate, 40 mM sodium phosphate pH6.8, 1 mM EDTA, 1 mM EGTA) was added and the mixture incubated at 72°C for 2 hours. Following incubation, 2 g/l of separation solution (containing 2.5 g/l cationic magnetic microspheres, 0.17M sodium phosphate buffer pH6.8, 7.5% Triton X-100 (TM), 0.02% sodium azide) was added and incubated at 72°C for 5 minutes. The RNA:probe hybrids, bound to the magnetic particles, were collected and the supernatant removed. One ml wash solution (0.12M sodium phosphate buffer pH6.8, 14% diisobutyl sulfosuccinate, 5% Triton X-100, 0.02% sodium azide) was added, the particles collected and the supernatant removed. This step was repeated two times. The radioactivity bound to the magnetic particles was determined in a gamma counter. The results are shown in Table 22 and indicate that the probes hybridize to organisms in the genus Mycobacterium and that a combination of probes will detect all members of the genus. Table 23 shows that the probes do not react with other closely related bacteria.

TABLE 22

| HYBRIDIZATION OF THE MYCOBACTERIUM PROBES 1-4 TO MYCOBACTERIAL SPECIES | | | | | |
|---|---|---|---|---|---|
| Organism | ATCC# | % Probe 1 Bound | % Probe 2 Bound | % Probe 3 Bound | % Probe 4 Bound |
| Mycobacterium africanum | 25420 | 41.5 | 14.7 | 17.9 | 26.7 |
| M. asiaticum | 25274 | 31.8 | 20.2 | 7.9 | 0.1 |
| M. avium | 25291 | 11.7 | 34.7 | 10.1 | 1.6 |
| M. bovis | 19210 | 19.4 | 28.4 | 44.6 | 20.9 |
| M. bovis (BCG) | 35734 | 30.0 | 35.5 | 17.8 | 5.6 |
| M. chelonae | 14472 | 8.6 | 0.7 | 6.3 | 0.2 |
| M. flavescens | 14474 | 29.8 | 17.7 | 2.3 | 0.9 |
| M. fortuitum | 6841 | 34.7 | 2.2 | 4.8 | 0.2 |
| M. gastri | 15754 | 27.6 | 65.1 | 9.6 | 22.3 |
| M. gordonae | 14470 | 50.7 | 55.2 | 3.1 | 0.4 |
| M. haemophilum | 29548 | 40.7 | 60.7 | 0.4 | 12.4 |
| M. intracellulare | 13950 | 38.8 | 48.3 | 0.9 | 5.4 |
| M. kansasii | 12478 | 53.4 | 27.3 | 24.5 | 27.8 |
| M. malmoense | 29571 | 3.1 | 38.4 | 0.8 | 1.5 |
| M. marinum | 827 | 41.7 | 4.1 | 4.8 | 0.1 |

(continued)

| HYBRIDIZATION OF THE MYCOBACTERIUM PROBES 1-4 TO MYCOBACTERIAL SPECIES | | | | | |
|---|---|---|---|---|---|
| Organism | ATCC# | % Probe 1 Bound | % Probe 2 Bound | % Probe 3 Bound | % Probe 4 Bound |
| M. non-chromogenicum | 1930 | 35.0 | 42.9 | 0.5 | 16.4 |
| M. phlei | 11758 | 23.7 | 0.6 | 1.8 | 0.6 |
| M. scrofulaceum | 19981 | 35.1 | 66.9 | 0.9 | 26.4 |
| M. shimoidei | 27962 | 34.6 | 1.4 | 1.3 | 4.8 |
| M. simiae | 25275 | 45.9 | 44.0 | 5.3 | 0.1 |
| M. smegmatis | e14468 | 31.3 | 4.0 | 5.6 | 0.1 |
| M. szulgai | 23069 | 19.4 | 22.3 | 1.5 | 3.0 |
| M. terrae | 15755 | 25.6 | 21.7 | 0.4 | 12.3 |
| M. thermo-resistibile | 19527 | 20.3 | 34.5 | 3.1 | 17.6 |
| M. triviale | 23292 | 37.3 | 4.6 | 4.3 | 0.1 |
| M. tuberculosis (avirulent) | 25177 | 38.5 | 26.3 | 11.3 | 23.0 |
| M. tuberculosis (virulent) | 27294 | 13.8 | 12.4 | 38.4 | 22.3 |
| M. ulcerans | 19423 | 33.9 | 28.7 | 0.4 | 8.9 |
| M. vaccae | 15483 | 8.8 | 36.2 | 4.8 | 3.2 |
| M. xenopi | 19971 | 38.4 | 2.1 | 3.8 | 0.2 |

TABLE 23

| HYBRIDIZATION OF THE MYCOBACTERIUM PROBES 1-4 TO PHYLOGENETICALLY CLOSELY RELATED ORGANISMS | | | | | |
|---|---|---|---|---|---|
| Organism | ATCC# | % Probe 1 Bound | % Probe 2 Bound | % Probe 3 Bound | % Probe 4 Bound |
| Actinomadura madurae | 19425 | 0.2 | 0.3 | 0.2 | 0.1 |
| Actinoplanes italicus | 10049 | 0.4 | 0.5 | 0.3 | 0.2 |
| Arthrobacter oxidans | 14358 | 0.2 | 0.4 | 0.3 | 0.1 |
| Brevibacterium linens | e9172 | 0.3 | 0.3 | 0.3 | 0.1 |
| Corynebacterium xerosis | 373 | 0.4 | 0.3 | 0.3 | 0.1 |
| Dermatophilus congolensis | 14367 | 0.4 | 0.6 | 0.3 | 0.2 |
| Microbacterium lacticum | 8180 | 0.2 | 0.3 | 0.2 | 0.1 |
| Nocardia asteroides | 19247 | 0.3 | 0.3 | 0.4 | 0.1 |
| Nocardia brasiliensis | 19296 | 0.4 | 0.3 | 0.6 | 0.1 |
| Nocardia otitidiscaviarum | 14629 | 0.4 | 0.4 | 1.0 | 0.3 |
| Nocardioposis dassonvillei | 23218 | 0.3 | 0.2 | 0.3 | 0.1 |
| Oerskovia turbata | 33225 | 0.2 | 0.2 | 0.3 | 0.1 |

(continued)

| HYBRIDIZATION OF THE MYCOBACTERIUM PROBES 1-4 TO PHYLOGENETICALLY CLOSELY RELATED ORGANISMS | | | | | |
|---|---|---|---|---|---|
| Organism | ATCC# | % Probe 1 Bound | % Probe 2 Bound | % Probe 3 Bound | % Probe 4 Bound |
| Oerskovia xanthineolytica | 27402 | 0.2 | 0.3 | 0.3 | 0.1 |
| Rhodococcus aichiensis | 33611 | 0.4 | 0.2 | 0.3 | 0.2 |
| Rhodococcus aurantiacus | 25938 | 0.3 | 0.4 | 0.3 | 0.2 |
| Rhodococcus bronchialis | 25592 | 0.4 | 0.3 | 0.3 | 0.1 |
| Rhodococcus chubuensis | 33609 | 0.6 | 0.4 | 0.3 | 0.3 |
| Rhodococcus equi | 6939 | 0.4 | 0.4 | 0.4 | 0.5 |
| Rhodococcus obuensis | 33610 | 0.5 | 0.5 | 0.3 | 0.1 |
| Rhodococcus sputi | 29627 | 0.4 | 0.5 | 0.4 | 0.3 |

Example 9

Mycoplasmas are small, aerobic bacteria lacking cell walls. Mycoplasma pneumoniae is estimated to cause 8-15 million infections per year. The infections may be asymptomatic or range in severity from mild to severe bronchitis and pneumonia. The organism is believed to cause about 10% of pneumonias in the general population and 10-50% of the pneumonias of members of groups in prolonged, close contact such as college students and military personnel.

Diagnosis until now has required isolation of the organism in culture or demonstration of an increase in antibody titer. Culturing of the organism involves inoculation of respiratory tract specimens onto agar or biphasic media containing bacterial growth inhibitors. Examination for growth at 3-4 and 7-10 days is used to establish the presence or absence of any mycoplasma. Mycoplasma pneumoniae must then be identified by hemadsorption (the ability of M. pneumoniae to adhere to sheep or guinea pig erythrocytes), hemolysis (the ability of M. pneumoniae to produce beta hemolysis of sheep or guinea pig erythrocytes in blood agar), growth inhibition by specific antibodies, or immunofluorescence with specific antibodies. The present invention has significant advantages over each of these prior art methods both because of the simplicity of the test and because of the greatly reduced time necessary to achieve a diagnosis.

A probe specific for the 5S rRNA of M. pneumoniae was obtained by a comparison of known rRNA sequences. The particular sequences aligned were from M. pneumoniae, M. gallisepticum and Ureaplasma urealyticum (Rogers, M.J. et al. 1985, Proc. Natl. Acad. Sci. USA, 82 (1160-1164), M. capricolum (Hori, H. et al. 1981, Nucl. Acids Res. 9, 5407-5410) and Spiroplasma sp. (Walker, R.T. et al. 1982 Nucl. Acids Res. 10, 6363-6367). The alignments were performed as described above and outlined at page 6. 5S rRNA can be isolated and sequenced as outlined in Rogers et al., or a primer can be made which is complementary to a conserved region in the 5S rRNA and sequencing performed as outlined in Examples 1-4. The conserved region of 5S rRNA is documented in Fox, G.E. and Woese, C.R., 1975, Nature 256: 505-507. The following sequence was determined to be specific for Mycoplasma pneumoniae: GCTTGGTGCTTTCCTATTCTGCTGAAACAGCTACATTCGGC.

The sequence is complementary to a unique segment found in the 5S rRNA of Mycoplasma pneumoniae in the region corresponding to bases 65-108 of E. coli 5S rRNA, and was selected by comparison to 5S rRNA sequences from Mycoplasma gallisepticum, Spiroplasma mirum and Ureaplasma urealyticum. The oligonucleotide probe was characterized as described above. The size of the probe was 42 bases. The probe has a Tm of 71.5°C.

To demonstrate the reactivity of this sequence for Mycoplasma pneumoniae, the probe was tested in hybridization reactions under the following conditions. $^{32}$P-end-labelled oligonucleotide probe was mixed with 1 microgram ($7\times10^{-13}$ moles) of purified rRNA from Mycoplasma pneumoniae and reacted in 0.12 M PB (equimolar amounts of $Na_2HPO_4$ and $NaH_2PO_4$), 1 mM EDTA and 0.2% SDS (sodium dodecyl sulfate) at 65°C for 60 minutes in a final volume of 50 microliters. In separate tubes the probe was mixed with the hybridization buffer with and without target Mycoplasma pneumoniae rRNA present. Following separation on hydroxyapatite as outlined previously the hybrids were quantitated by scintillation counting. These results are shown in Table 24.

TABLE 24

| HYBRIDIZATION OF THE M. PNEUMONIAE 5S rRNA DNA PROBE TO HOMOLOGOUS TARGET rRNA*/ | | |
|---|---|---|
| | plus rRNA | minus rRNA |
| M. pneumoniae 5S probe | 85-95% | 0.5% |
| *% Hybridization = $\frac{\text{cpm bound to hydroxyapatite}}{\text{total cpm added to reaction}}$ | | |

This data shows that the probe has a high extent of reaction to its homologous target and very little non-specific binding to the hydroxyapatite.

Specificity of the M. pneumoniae 5S probe was tested by mixing the $^{32}P$ labelled probe with rRNA released from cells from other Mycoplasma species. All hybridization assays were carried out as described in Example 1. Table 25 indicates that the probe is specific for Mycoplasma pneumoniae and does not react with any other Mycoplasma species.

TABLE 25

| HYBRIDIZATION OF M. PNEUMONIAE PROBE TO OTHER MYCOPLASMA SPECIES | | |
|---|---|---|
| Acholeplasma laidlawii | 14089 | 3.3 |
| M. buccale | 23636 | 1.7 |
| M. capricolum | 23205 | 2.4 |
| M. columbinsale | 33549 | 1.4 |
| M. faucium | 25293 | 1.4 |
| M. fermentans | 15474 | 1.0 |
| M. gallisepticum | 19610 | 1.8 |
| M. gallopavonis | 33551 | 1.6 |
| M. genitalium | 3353c | 1.7 |
| M. hominis | 14027 | 1.3 |
| M. orale | 23714 | 1.8 |
| M. pneumontae | 15531 | 78.0 |
| M. primatum | 15497 | 1.6 |
| M. salivarium | 23064 | 0.6 |
| Spiroplasma mirum | | 2.3 |

As shown in Table 26, the probe did not react with any other closely related or phylogenetically diverse species of bacteria.

TABLE 26

| HYBRIDIZATION OF M. PNEUMONIAE PROBE TO A PHYLOGENETIC CROSS SECTION OF BACTERIA | | |
|---|---|---|
| Organism | ATCC# | % Probe Bound |
| Corynebacterium xerosis | 373 | 1.4 |
| Haemophilus influenzae | 19418 | 1.4 |
| Klebsiella pneumoniae | 23357 | 1.3 |
| Legionella pneumophila | 33152 | 1.8 |
| Mycobacterium tuberculosis (avir) | 25177 | 1.6 |
| Mycoplasma pneumoniae | 15531 | 52.0 |
| Neisseria meningitidis | 13077 | 0.6 |
| Propionibacterium acnes | 6919 | 2.0 |
| Pseudomonas aeruginosa | 25330 | 1.6 |
| Staphylococcus aureus | 12598 | 2.0 |
| Streptococcus pneumoniae | c6306 | 1.9 |

Four additional probe sequences (numbered 2-5 below) specific for Mycoplasma pneumoniae were obtained by utilizing four unique primers complementary to conserved regions on 16S rRNA. The regions correspond, respectively, to bases 190-230; 450-490; 820-860; and 1255-1290 of E. coli 16S rRNA. Probe sequence #1 was obtained using a primer with the sequence 5'-GGCCGTTACCCCACCTACTAGCTAAT-3'. Probe sequence #2 was obtained using a primer with the sequence 5'-GTATTACCGCGGCTGCTGGC-3'. Probe sequence #3 was obtained using a primer with the sequence 5'-CCGCTTGTGCGGGCCCCGTCAATTC-3'. Probe sequence #4 was obtained using a primer with the sequence 5'-CGATTACTAGCGATTCC-3'. Sequencing reactions were performed as outlined in previous examples. The M. pneumoniae sequences were compared with sequences from Mycoplasma genitalium, Mycoplasma capricolum, Mycoplasma gallisepticum and Spiroplasma mirum.

The following probe sequences were characterized by criteria described in Example one of the patent application and were shown to be specific for Mycoplasma pneumoniae:

2. AATAACGAACCCTTGCAGCTCCTTTCAACTTTGAT
3. CAGTCAAACTCTAGCCATTACCTGCTAAAGTCATT
4. TACCGAGGGGATCGCCCCGACAGCTAGTAT
5. CTTTACAGATTTGCTCACTTTTACAAGCTGGCGAC.

Probe #2 is 35 bases in length and has a Tm of 67°C. Probe #3 is 35 bases in length and has a Tm of 66°C. Probe #4 is 30 bases in length and has a Tm of 69°C. Probe #5 is 35 bases long with a Tm of 66°C.

When the four probes were mixed and used in hybridization assays at 60°C in the same manner as previous examples, they were found to be specific for M. pneumoniae. The probes do not cross react with other respiratory pathogens or with any organism representing the bacterial phylogenetic tree (Table 28).

TABLE 27

| HYBRIDIZATION OF MYCOPLASMA PNEUMONIAE PROBES 2-5 TO MYCOPLASMA SPECIES | | |
|---|---|---|
| Organism | ATCC# | % Probe Bound |
| Acholeplasma axanthum | 27378 | 0.34 |
| Acholeplasma laidlawii | 14089 | 0.30 |
| Mycoplasma arginini | 23838 | 0.20 |
| Mycoplasma arthritidis | 19611 | 0.49 |
| Mycoplasma bovigenitalium | 19852 | 0.18 |
| Mycoplasma bovis | 25523 | 0.43 |
| Mycoplasma buccale | 23636 | 0.37 |
| Mycoplasma californicum | 33451 | 0.79 |
| Mycoplasma capricolum | 23205 | 0.38 |
| Mycoplasma columbinasale | 33549 | 0.54 |
| Mycoplasma columborale | 29258 | 0.50 |
| Mycoplasma faucium | 25293 | 0.45 |
| Mycoplasma fermentans | 15474 | 0.27 |
| Mycoplasma gallisepticum | 19610 | 0.25 |
| Mycoplasma gallopavonis | 33551 | 0.47 |
| Mycoplasma genitalium | 33530 | 2.5 |
| Mycoplasma hominis | 14027 | 0.52 |
| Mycoplasma hyorhinis | 17981 | 0.46 |
| Mycoplasma orale | 23714 | 0.56 |
| Mycoplasma pneumoniae | 15531 | 34.0 |
| Mycoplasma primatum | 15497 | 0.71 |
| Mycoplasma pulmonis | 19612 | 0.68 |
| Mycoplasma salivarium | 23064 | 0.46 |
| Spiroplasma citri | 29416 | 0.60 |
| Spiroplasma mirum | 29335 | 0.52 |

TABLE 28

| HYBRIDIZATION OF MYCOPLASMA PNEUMONIAE PROBES 2-5 WITH OTHER BACTERIA | | |
|---|---|---|
| Organism | ATCC# | % Probe Bound |
| Actinomyces israelii | 10049 | 1.0 |
| Bacteroides fragilis | 23745 | 1.4 |
| Bifidobacterium breve | 15700 | 1.0 |
| Bordetella bronchiseptica | 10580 | 0.9 |
| Clostridium inocuum | 14501 | 1.0 |
| Clostridium pasteurianum | 6013 | 0.9 |
| Clostridium perfringens | 13124 | 1.1 |
| Clostridium ramosum | 25582 | 1.0 |
| Corynebacterium xerosis | 373 | 0.8 |
| Erysipelothrix rhusiopathiae | 19414 | 1.1 |
| Escherichia coli | 11775 | 1.0 |
| Haemophilus influenzae | 19418 | 0.9 |
| Klebsiella pneumoniae | 15531 | 1.0 |
| Lactobacillus acidophilus | 4356 | 1.4 |
| Legionella pneumophila | 33154 | 0.8 |
| Listeria monocytogenes | 15313 | 1.2 |
| Moraxella osloensis | 19976 | 1.1 |
| Mycobacterium tuberculosis | 25177 | 1.0 |
| Neisseria meningitidis | 13077 | 1.0 |
| Pasteurella multocida | 6529 | 1.6 |
| Peptococcus magnus | 14955 | 0.9 |
| Propionibacterium acnes | 6919 | 1.1 |
| Pseudomonas aeruginosa | 25330 | 1.0 |
| Staphylococcus aureus | 12600 | 1.0 |
| Streptococcus faecalis | 19433 | 1.5 |
| Streptococcus mitis | 9811 | 1.0 |
| Streptococcus pneumoniae | 6306 | 1.0 |
| Streptococcus pyogenes | 19615 | 1.1 |

Example 10

The genus Legionella contains 22 species which are all potentially pathogenic for humans. These organisms cause Legionnaires' disease, an acute pneumonia, or Pontiac fever, an acute, non-pneumonic, febrile illness that is not fatal.

Legionella species have also been shown to be responsible for nosocomial pneumonia occuring predominantly among immunocompromised patients.

Legionellosis, which includes Legionnaires' disease and Pontiac fever, is diagnosed on the basis of clinical symptoms, either direct or indirect fluorescence antibody tests, and by culture using a buffered charcoal yeast extract (BCYE) agar containing selective antimicrobial agents. There is no single definitive genus test known in the prior art. (See Bergey's Manual of Systematic Bacteriology at page 283, (ed. 1984)). The fluorescent antibody tests are not able to identify all species of Legionella, but only those few for which antibodies exist. The culture method is not definitively diagnostic for Legionella species.

The oligonucleotide sequences described below, when used as probes in a nucleic acid hybridization assay, accurately identify all species of Legionella. This assay is more sensitive than culture or antibody tests and shortens significantly the time of identification and, thus, diagnosis. The assay, therefore, represents a significant improvement over prior diagnostic methods.

Three probe sequences specific for the genus Legionella were obtained by utilizing three unique primers complementary to conserved regions on both 16S and 23S rRNA. Sequence 1 was obtained by using a 16S primer with the sequence 5'-TCT ACG CAT TTC ACC GCT ACA C-3'. Probe sequence 2 was obtained with a 23S primer of sequence 5'-CAG TCA GGA GTA TTT AGC CTT-3'. Probe sequence 3 was obtained with a 16S primer of sequence 5'GCT CGT TGC GGG ACT TAA CCC ACC AT-3'. Sequencing with these primers was performed as described for previous examples.

The following three sequences were characterized by the criteria described in Example 1 and were shown to be specific for the genus Legionella. The phylogenetically nearest neighbors Escherichia coli, Pseudomonas aeruginosa, Vibrio parahaemolyticus and Acinetobacter calcoaceticus were used as comparisons with sequences from Legionella species.

1. TACCCTCTCCCATACTCGAGTCAACCAGTATTATCTGACC
2. GGATTTCACGTCTCCCGGCCTACTTGTTCGCGTCCCTAGTTC
3. CATCTCTGCAAAATTCACTGTATGTCAAGGGTAGGTAAGG.

Sequence 1, from 16S rRNA, is 40 bases in length and has a Tm of 72°C. Sequence 2, from 235 rRNA, is 42 bases in length and has a Tm of 73°C. Sequence 3, from 16S rRNA, is 40 bases in length and has a Tm of 68°C. These sequences are capable of hybridizing to RNA of the genus Legionella in the regions corresponding respectively to, 630-675 of E. coli 16s rRNA; 350-395 of E. coli 23s rRNA; and 975-1020 of E. coli 16s rRNA. When mixed together the probes had a combined average Tm of 73°C. Analysis on polyacrylamide gels showed that each probe was the correct length and sequence analysis demonstrated that each was the correct sequence of bases.

When the three probes were mixed and used in a hybridization assay, they were found to be specific for the genus Legionella (Tables 29 and 30) and did not cross react with other respiratory pathogens or with any selected organism from the phylogenetic tree (Tables 31 and 32). Use of more than one probe, i.e., a mixture of probes, can result in increased assay sensitivity and/or in an increase in the number of non-viral organisms to be detected.

TABLE 29

| HYBRIDIZATION OF LEGIONELLA PROBES TO HOMOLOGOUS TARGET rRNA | | |
|---|---|---|
| | plus rRNA | minus rRNA |
| Legionella probe | 80% | 1.0% |

TABLE 30

| HYBRIDIZATION OF LEGIONELLA PROBES TO LEGIONELLA SPECIES | | |
|---|---|---|
| Organism | ATCC# | % Probes Bound |
| L. anisa | 35292 | 42.0 |
| L. bozemanii | 33217 | 58.0 |
| L. cherrii | 35252 | 69.0 |
| L. dumoffii | 33279 | 57.0 |
| L. erythra | CDC#9PIW044C | 26.0 |
| L. feeleii | 35303 | 59.0 |
| L. hackeliae | 35250 | 47.0 |
| L. jamestowniensis | 35298 | 20.0 |
| L. jordanis | 33623 | 50.6 |
| L. longbeachae | 33484 | 48.0 |
| L. maceachernii | 35300 | 25.0 |
| L. micdadei | 33704 | 38.0 |
| L. oakridgensis | 33761 | 44.0 |
| L. parisiensis | 9060 | 69.0 |
| L. pneumophila 1* | 6736 | 75.0 |
| " 2 | | 64.0 |
| " 3 | | 73.0 |
| " 4 | | 73.0 |
| " 5 | | 78.0 |
| " 6 | | 75.0 |
| " 7 | | 73.0 |
| " 8 | | 63.0 |
| " 11 | | 75.0 |
| L. rubrilucens | 35304 | 12.0 |

(continued)

| HYBRIDIZATION OF LEGIONELLA PROBES TO LEGIONELLA SPECIES | | |
|---|---|---|
| Organism | ATCC# | % Probes Bound |
| L. sainthelensi | 35248 | 61.0 |
| L. sainticrucis | 35301 | 24.0 |
| L. spiritensis | CDC#MSH9 | 55.0 |
| L. steigerwaltii | 7430 | 56.0 |
| L. wadsworthii | 33877 | 37.0 |
| *The numbers 1-8 and 11 are serotypes of L. pneumophila. | | |

TABLE 31

| HYBRIDIZATION OF LEGIONELLA PROBES TO RESPIRATORY PATHOGENS | | |
|---|---|---|
| Organisms | ATCC# | % Probe Bound |
| Corynebacterium xerosis | 373 | 2.1 |
| Haemophilus influenzae | 19418 | 2.3 |
| Klebsiella pneumoniae | 23357 | 2.0 |
| Mycoplasma pneumoniae | 15531 | 2.3 |
| Neisseria meningitidis | 13090 | 2.2 |
| Pseudomonas aeruginosa | 25330 | 1.2 |
| Propionibacterium acnes | 6919 | 1.6 |
| Streptococcus pneumoniae | 6306 | 0.8 |
| Staphylococcus aureus | 25923 | 1.6 |

TABLE 32

| HYBRIDIZATION OF LEGIONELLA PROBES TO A PHYLOGENETIC CROSS SECTION OF BACTERIAL SPECIES | | |
|---|---|---|
| Organisms | ATCC# | % Probe Bound |
| Acinetobacter calcoaceticus | 33604 | 1.4 |
| Branhamella catarrhalis | 25238 | 2.0 |
| Bacillus subtilis | 6051 | 1.9 |
| Bacteroides fragilis | 23745 | 2.2 |
| Campylobacter jejuni | 33560 | 1.2 |
| Chromobacterium violaceum | 29094 | 1.3 |
| Clostridium perfringens | 13124 | 1.9 |
| Deinoccoccus radiodurans | 35073 | 1.8 |
| Derxia gummosa | 15994 | 2.0 |
| Enterobacter aerogenes | 13048 | 1.4 |
| Escherichia coli | 11775 | 1.2 |
| Mycoplasma hominis | 14027 | 1.1 |
| Proteus mirabilis | 29906 | 1.4 |
| Pseudomonas cepacia | 11762 | 1.1 |
| Rahnella aquatilis | 33071 | 1.7 |
| Rhodospirillum rubrum | 11170 | 2.0 |
| Streptococcus mitis | 9811 | 2.0 |
| Vibrio parahaemolyticus | 17802 | 2.0 |
| Yersinia enterocolitica | 9610 | 1.2 |

Three additional probe sequences (numbered 4-6) specific for the genus Legionella were obtained by utilizing two primers complementary to conserved regions on 23S rRNA. Sequence 4 was made from a 23S primer with the sequence 5'-CCT TCT CCC GAA GTT ACG G-3'. Probe sequences 5 and 6 were made from a 23S primer of sequence 5'-AAG CCG GTT ATC CCC GGG GTA ACT TTT-3'. Sequencing with these primers was performed as described for previous examples.

The following three sequences were characterized by the criteria previously described and were shown to be specific for the genus Legionella. The phylogenetically nearest neighbors Escherichia coli, Pseudomonas aeruginosa, Vibrio parahaemolyticus and Actinetobacter calcoaceticus were used for comparisons with sequences from Legionella species.

4. GCG GTA CGG TTC TCT ATA AGT TAT GGC TAG C
5. GTA CCG AGG CTA CCT TTG TGC T
6. CAC TCT TGG TAC GAT GTC CGA C

Probe 4, complementary to 23S rRNA in the region corresponding to bases 1585-1620 of E. coli 23S rRNA, is 31 bases long and has a Tm of 67°C. Probe 5, complementary to 235 rRNA in the region corresponding to bases 2280-2330 of E. coli 23S rRNA, is 22 bases long and has a Tm of 66°C. Probe 6, complementary to 23S rRNA in the same region as Probe 5, is 22 bases long and has a Tm of 63°C.

When the three probes were mixed with probe 3 above and used in a hybridization assay as described for probes 1-3, they were found to be specific for the genus Legionella (Table 33) and did not cross react with other respiratory pathogens or with any selected organism from the phylogenetic tree (Tables 34 and 35). Using more than one probe, i.e., a mixture of probes, can improve assay sensitivity and/or increase the number of non-viral organisms detected.

TABLE 33

| HYBRIDIZATION OF LEGIONELLA PROBES TO LEGIONELLA SPECIES | | |
|---|---|---|
| Organism | ATCC# | % Probes Bound |
| L. anisa | 35292 | 29.6 |
| L. bozemanii | 33217 | 35.5 |
| L. cherrii | 35252 | 29.2 |
| L. dumoffii | 33279 | 26.0 |
| L. erythra | 35303 | 32.0 |
| L. feelii | CDC#9P1WO44C | 32.0 |
| L. hackeliae | 35250 | 39.0 |
| L. jamestowniensis | 35298 | 31.2 |
| L. jordanis | 33623 | 25.7 |
| L. longbeachae | 33484 | 27.6 |
| L. maceahernii | 35300 | 39.3 |
| L. micdadei | 33204 | 31.0 |
| L. oakridgensis | 33761 | 24.4 |
| L. parisiensi | 35299 | 31.2 |
| L. pneumophilia 1* | 33153 | 40.0 |
| " 2 | 33154 | 38.5 |
| " 3 | 33155 | 44.6 |
| " 4 | 33156 | 48.6 |
| " 5 | 33216 | 32.0 |
| " 6 | 33215 | 43.0 |
| " 7 | 33823 | 29.5 |
| " 8 | 35096 | 37.6 |
| " 11 | 43130 | 44.5 |
| L. rubrilucens | 35304 | 30.1 |
| L. sainthelensis | 35248 | 27.0 |
| L. sainticrucis | 35301 | 22.0 |
| L. spiritensis | CDC#MSH9 | 40.5 |
| L. steigerwaltii | 35302 | 31.7 |

(continued)

| HYBRIDIZATION OF LEGIONELLA PROBES TO LEGIONELLA SPECIES | | |
|---|---|---|
| Organism | ATCC# | % Probes Bound |
| L. wadsworthii | 33877 | 30.0 |
| *The numbers 1-8 and 11 are serotypes of L. pneumophilia. | | |

TABLE 34

| HYBRIDIZATION OF LEGIONELLA PROBES TO RESPIRATORY PATHOGENS | | |
|---|---|---|
| Organisms | ATCC# | % Probe Bound |
| Corynebacterium xerosis | 373 | 0.13 |
| Haemophilum influenzae | 19418 | 0.12 |
| Klebsiella pneumoniae | 23357 | 0.13 |
| Neisseria meningitidis | 13090 | 0.14 |
| Pseudomonas aeruginosa | 25330 | 0.13 |
| Propionibacterium acnes | 6919 | 0.11 |
| Streptococcus pneumoniae | 6306 | 0.08 |
| Staphylococcus aureus | 25923 | 0.15 |

TABLE 35

| HYBRIDIZATION OF LEGIONELLA PROBES TO A PHYLOGENETIC CROSS SECTION OF BACTERIAL SPECIES | | |
|---|---|---|
| Organisms | ATCC# | % Probe Bound |
| Acinetobacter calcoaceticus | 33604 | 0.12 |
| Branhamella catarrhalis | 25238 | 0.13 |
| Bacillus subtilis | 6051 | 0.09 |
| Bacteroides fragilis | 23745 | 0.12 |
| Campylobacter jejuni | 33560 | 0.06 |
| Chromobacterium violaceum | 29094 | 0.33 |
| Clostridium perfringens | 13124 | 0.07 |
| Deinoccoccus radiodurans | 35073 | 0.11 |
| Derxia gummosa | 15994 | 0.15 |
| Enterobacter aerogenes | 13048 | 0.26 |
| Escherichia coli | 11775 | 0.09 |
| Mycoplasma hominis | 14027 | 0.09 |
| Proteus mirabilis | 29906 | 0.09 |
| Pseudomonas cepacia | 17762 | 0.20 |
| Rahnella aquatilis | 33071 | 0.15 |
| Rhodospirillum rubrum | 11170 | 0.13 |
| Streptococcus mitis | 9811 | 0.07 |
| Vibrio parahaemolyticus | 17802 | 0.11 |
| Yersinia enterocolitica | 9610 | 0.19 |

Example 11

Chlamydia are gram-negative, non-motile, obligate intracellular bacteria. The species C. trachomatis is associated with endemic trachoma (the most common preventable form of blindness), inclusion conjunctivitis and lymphogranuloma venereum (LGV). It is a major cause of nongonococcal urethritis in men and may cause cervicitis and acute salpingitis in women. Eye disease or chlamydial pneumonia may develop in newborns passing through the infected birth canal.

There are several methods known in the art for identification of C. trachomatis in the urogenital tract, for example, by direct immunofluorescent staining or enzyme immunoassay of clinical specimens. The method of choice, however, remains culture of the organism in cycloheximide treated McCoy cells. Cell culture is followed by morphological or fluorescent antibody staining for confirmation of the organism's identity.

The oligonucleotide sequences described below, when used as probes in nucleic acid hybridization assay, accurately identify Chlamydia trachomatis isolates. This assay test is equal in sensitivity to culture or antibody tests and, in the case of culture, significantly shortens the time to identification, and thus, diagnosis.

Kingsbury, D.T., and E. Weiss, 1968 J. Bacteriol. 96: 1421-23 (1968); Moulder, J.W., ASM News, Vol.50, No. 8, (1984) report a mere 10% DNA homology between C. trachomatis and C. psittaci. Moreover, these reports show that different C. trachomatis strains differ in DNA homology. Weisberg, W.G. et. al, J. Bacteriol. 167:570-574 (1986) published the 16S rRNA sequences of C. psittaci and noted that C. trachomatis and C. psittaci share a greater than 95% rRNA homology. From these reports, it may be inferred that it would be difficult to invent (1) probes capable of hybridizing to all strains of C. trachomatis; and (2) probes capable of distinguishing between C. trachomatis and C. psittaci. The following probes accomplish both objectives.

Ten probe sequences specific for Chlamydia trachomatis were made using seven unique primers complementary to conserved regions of both 16S and 23S rRNA. Probe sequence 1 was obtained from a 16S primer of sequence 5'-TCT ACG CAT TTC ACC GCT ACA C-3'. Probe sequence 2 was obtained with a 16S primer of sequence 5'-CCG CTT GTG CGG GCC CCC GTC AAT TC-3'. Sequences 3 and 4 were obtained using a 16S primer with the sequence 5'-GGC CGT TAC CCC ACC TAC TAG CTA AT-3'. Probe sequences 5 and 6 were obtained with a 23S primer of sequence 5'-CTT TCC CTC ACG GTA-3'. Probe sequences 7 and 8 were obtained with a 235 primer of sequence 5'-CCT TCT CCC GAA GTT ACG G-3'. Probe sequence 9 was obtained with a 235 primer of sequence 5'-TCG GAA CTT ACC CGA CAA GGA ATT TC-3'. Probe sequence 10 was obtained with a primer of sequence 5'-CTA CTT TCC TGC GTC A-3'.

The following ten sequences were characterized using the criteria described in Example 1 and were shown to be specific for the rRNA of Chlamydia trachomatis. The phylogenetically nearest neighbor Chlamydia psittaci was used for comparison with Chlamydia trachomatis sequence.

1. CCG ACT CGG GGT TGA GCC CAT CTT TGA CAA
2. TTA CGT CCG ACA CGG ATG GGG TTG AGA CCA TC
3. CCG CCA CTA AAC AAT CGT CGA AAC AAT TGC TCC GTT CGA
4. CGT TAG TCG GAT GCC CAA ATA TCG CCA CAT TCG
5. CAT CCA TCT TTC CAG ATG TGT TCA ACT AGG AGT C C T GAT CC
6. GAG GTC GGT CTT TCT CTC CTT TCG TCT ACG
7. CCG TTC TCA TCG CTC TAC GGA CTC TTC CAA TCG
8. CGA AGA TTC CCC TTG ATC GCG ACC TGA TCT
9. CCG GGG CTC CTA TCG TTC CAT AGT CAC CCT AAA AG
10. TAC CGC GTG TCT TAT CGA CAC ACC CGC G

Sequence 1, from 16S rRNA, is 30 bases in length and has a Tm of 66°C. Sequence 2, from 16S rRNA, is 32 bases in length and has a Tm of 67°C. Sequence 3, from 16S rRNA, is 39 bases in length and has a Tm of 70°C. Sequence 4, from 16S rRNA, is 33 bases in length and has a Tm of 69°C. Sequence 5, from 23S rRNA, is 41 bases in length and has a Tm of 71°C. Sequence 6, from 23S rRNA, is 30 bases in length and has a Tm of 72°C. Sequence 7, from 23S rRNA, is 33 bases in length and has a Tm of 72 C. Sequence 8, from 23S rRNA, is 30 bases in length and has a Tm of 71°C. Sequence 9, from 23S rRNA is 35 bases in length and has a Tm of 74°C. Sequence 10 is 28 bases in length and has a Tm of 72°C.

The reactivity and specificity of the probes was tested hybridization assays. $^{32}$P-end-labeled oligonu-cleotide probes 1 and 2 were mixed with purified RNA or RNA released from at least $10^7$ organisms in 0.55 ml of 41% diisobutyl sulfosuccinate, 3% sodium dodecyl sulfate, 0.03 M sodium phosphate pH 6.8, 1mM EDTA, 1mM EGTA at 60°C (probe 1) or 64°C (probe 2) for 1 hour. Hybrids were bound to hydroxyapatite as described in previous examples and the amount of radioactivity bound was determined by scintillation counting. Table 36 shows that probes 1 and 2 hybridize well to all serotypes of C. trachomatis tested. Probe 1 does not react with any strain of C. psittaci tested and probe 2 does not react with two of the strains. Probe 2 does react with the ovine polyarthritis strain of C. psittaci, an organism which is not known to infect humans. Table 37 demonstrates the reactivity and specificity of probes 3-9 when $^{125}$I-labeled and used as a mix. In this case, the hybrids were bound to cationic magnetic particles. These probes hybridize well to all strains of C. trachomatis tested and not to any strains of C. psittaci. Probes 3-9 were further tested against a panel of organisms commonly found in the urogenital tract (Table 38) and a phylogenetic cross section of organisms (Table 39). In all cases, the probes were shown to be specific. Probe 10 is 25% non-homologous to C. psittaci and also should be specific for C. trachomatis.

TABLE 36

| HYBRIDIZATION OF CHLAMYDIA TRACHOMATIS PROBES 1 AND 2 TO CHLAMYDIA RNA | | | |
|---|---|---|---|
| | | % Probe Bound | |
| Organism | ATCC# | Probe 1 | Probe 2 |
| Chlamydia trachomatis serotype C | VR578 | 22 | 39 |
| Chlamydia trachomatis serotype E | VR348B | 27 | 48 |
| Chlamydia trachomatis serotype G | VR878 | 20 | 44 |
| Chlamydia trachomatis serotype I | VR880 | 20 | 42 |
| Chlamydia trachomatis serotype K | VR887 | 28 | 45 |
| Chlamydia psittaci guinea pig conjunctivitis strain | VR813 | 1.2 | 1.4 |
| Chlamydia psittaci ovine abortion strain | VR656 | 1.0 | 3.0 |
| Chlamydia psittaci ovine polyarthritis strain | VR619 | 1.1 | 35.3 |

TABLE 37

| HYBRIDIZATION OF CHLAMYDIA TRACHOMATIS PROBES 3-9 WITH CHLAMYDIA rRNA | | | |
|---|---|---|---|
| Organism | Serovar | ATCC# | Ratio Counts Bound* |
| C. trachomatis | A | | 689 |
| C. trachomatis | B | | 560 |
| C. trachomatis | Ba | | 1066 |
| C. trachomatis | C | VR548 | 962 |
| C. trachomatis | D | | 1192 |
| C. trachomatis | E | VR348 | 1022 |
| C. trachomatis | F | | 391 |
| C. trachomatis | G | VR878 | 874 |
| C. trachomatis | H | | 954 |
| C. trachomatis | I | VR880 | 943 |
| C. trachomatis | J | | 482 |
| C. trachomatis | K | VR887 | 999 |
| C. trachomatis | L1 | | 638 |
| C. trachomatis | L2 | | 501 |
| C. trachomatis | L3 | VR903 | 821 |
| C. psittaci | | VR125 | 1.6 |
| C. psittaci | | VR629 | 0.9 |
| C. psittaci | | VR656 | 1.3 |
| C. psittaci | | VR813 | 1.2 |
| *Ratio = $\frac{\text{counts bound when RNA present}}{\text{counts bound when no RNA present}}$ | | | |

TABLE 38

| HYBRIDIZATION OF CHLAMYDIA TRACHOMATIS PROBES 3-9 TO ORGANISMS FOUND IN THE UROGENITAL TRACT. | | |
|---|---|---|
| Organism | ATCC# | Ratio Counts Bound* |
| Achromobacter xylosoxidans | 27061 | 1.9 |
| Acinetobacter lwoffii | 15309 | 1.2 |
| Branhamella catarrhalis | 25238 | 1.2 |
| Candida albicans | 18804 | 2.4 |
| Flavobacterium meningosepticum | 13253 | 1.1 |
| Gardnerella vaginalis | 14018 | 1.3 |
| Lactobacillus acidophilus | 4356 | 0.8 |
| Listeria monocytogenes | 15313 | 0.7 |
| Mycobacterium smegmatis | 14468 | 1.1 |
| Moraxella osloensis | 19976 | 1.3 |
| Neisseria gonorrhoeae | 19424 | 2.3 |
| Pasteurella multocida | 6529 | 1.0 |
| Peptostreptococcus anaerobius | 27337 | 1.2 |
| Streptococcus agalactiae | 13813 | 4.0 |
| Streptococcus faecalis | 19433 | 2.6 |

$$*Ratio = \frac{\text{counts bound when RNA present}}{\text{counts bound when no RNA present}}$$

TABLE 39

| HYBRIDIZATION OF CHLAMYDIA TRACHOMATIS PROBES 3-9 TO PHYLOGENETICALLY DIVERSE ORGANISMS. | | |
|---|---|---|
| Organism | ATCC# | Ratio Counts Bound* |
| Bacillus subtilis | 6051 | 2.2 |
| Bacteroides fragilis | 23745 | 1.6 |
| Campylobacter jejuni | 33560 | 1.4 |
| Chromabacterium violaceum | 29094 | 1.4 |
| Deinococcus radiodurans | 35073 | 1.8 |
| Derxia gummosa | 15994 | 1.3 |
| Enterobacter aerogenes | 13048 | 1.9 |
| Escherichia coli | 11775 | 1.9 |
| Mycoplasma hominis | 14027 | 1.3 |
| Pseudomonas cepacia | 17762 | 2.2 |
| Proteus mirabilis | 29906 | 2.2 |
| Rahnella aquatilis | 33071 | 1.9 |
| Rhodospirillum rubrum | 11170 | 1.9 |
| Vibrio parahaemolyticus | 17802 | 2.0 |
| Yersinia enterocolitica | 9610 | 2.5 |

$$*Ratio = \frac{\text{counts bound when RNA present}}{\text{counts bound when no RNA present}}$$

Example 12

Campylobacters are motile, microaerophilic, gram negative curved rods. The genus is quite diverse and distinct from other genera. Although the genus is well defined, some revision is occurring at the species level (Romaniuk, P.J. et al., J. Bacteriol. 169:2137-2141 (1987). Three Campylobacter species, Campylobacter jejuni, C. coli and C. laridis, cause enteritis in humans. The disease includes diarrhea, fever, nausea, abdominal pain and in some cases, vomiting. These organisms cause an estimated 2 million infections per year in the United States (estimate based on the number of Salmonella and Shigella induced cases of diarrheal disease). Other members of the genus cause septicemias in humans and abortion and infertility in sheep and cattle.

Diagnosis of Campylobacter enteritis is currently dependent upon growth and isolation of the organism in culture, followed by a number of biochemical tests. Optimum growth of campylobacters requires special conditions such as low oxygen tension and high temperature (42°C). No single set of conditions is recommended for isolation of all Campylobacter species.

The oligonucleotide sequences listed below, when used in a hybridization assay, hybridize to the 16S rRNA of the Campylobacter species of interest. The present invention has significant advantages over the prior art methods of detection of Campylobacter because one probe can detect all Campylobacters of interest; the other two probes detect the enteric Campylobacters and one can detect human isolates of Campylobacter. In addition, the probes have advantages over the prior art in terms of ease of the assay and greatly reduced time to identification and therefore, diagnosis.

The four probes which hybridize to the 16S rRNA of Campylobacter species of interest were constructed using three unique primers complementary to 16S rRNA. Sequences 1 and 2 were made using a 16S primer with the sequences 5'-GTA TTA CCG CGG CTG CTG GCA C-3'. Sequence 3 was made using a 16S primer with the sequence 5'-CCG CTT GTG CGG GCC CCC GTC AAT TC-3'. Sequence 4 was made with a 16S primer with the sequence 5'-GCT CGT TGC GGG ACT TAA CCC AAC AT-3'.

The following sequences were characterized and shown to hybridize to Campylobacter jejuni, C. coli and C. laridis. The phylogenetically nearest neighbors Vibrio parahaemolyticus and Wollinella succinogenes were used for comparison with the campylobacter sequences.

1. CGC TCC GAA AAG TGT CAT CCT CC
2. CCT TAG GTA CCG TCA GAA TTC TTC CC
3. GCC TTC GCA ATG CGT ATT CTT GGT G
4. GGT TCT TAG GAT ATC AAG CCC AGG

Sequence 1, from 16S rRNA, is 23 bases in length and has a Tm of 65°C. Sequence 2, from 16S rRNA, is 26 bases in length and has a Tm of 64°C. Sequence 3, from 16S rRNA, is 25 bases in length and has a Tm of 66°C. Sequence 4, from 16S rRNA, is 24 bases in length and has a Tm of 61°C. Sequence 1 is capable of hybridizing in the region corresponding to bases 405-428 of E. coli 16S rRNA; Sequence 2 is capable of hybridizing in the region corresponding to bases 440-475 of E. coli 16S rRNA; Sequence 3 is capable of hybridizing in the region corresponding to bases 705-735 of E. coli 16S rRNA; Sequence 4 is capable of hybridizing in the region corresponding to bases 980-1010 of E. coli 16S rRNA.

The reactivity and specificity of the probes for campylobacter was tested in hybridization assays. $^{32}$P-end-labeled oligonucleotide probes were mixed with purified RNA or RNA released from cells in 0.1% sodium dodecyl sulfate. 0.5 ml of hybridization solution (41% diisobutyl sulfosuccinate, 30mM sodium phosphate, pH 6.8, 0.7% sodium dodecyl sulfate, 1mM EDTA, 1mM EGTA) was added and the mixture incubated at 60°C for 1 to 1.5 hour. Following incubation, 2 to 2.5 ml of separation solution (2% hydroxyapatite, 0.12 M sodium phosphate, pH6.8, 0.02% sodium dodecyl sulfate) was added and the mixture incubated at 60°C for five minutes. The sample was centrifuged and the supernatant removed. 2.5 m of wash solution (0.12 M sodium phosphate, pH6.8, 0.02% sodium dodecyl sulfate) was added and the sample mixed, centrifuged and the supernatant removed. The radioactivity bound to the hydroxyapatite was determined by scintillation counting.

Table 40 indicates that the probes hybridize well to the Campylobacter species of interest, C. jejuni, C. coli and C. laridis. Probe 1 detects all of the Campylobacter species tested, probes 2 and 4 detect only the enteric campylobacters, and probe 3 detects all of the Campylobacter species except C. sputorum, an organism isolated from cattle. Thus all of the probes are useful for identifying campylobacter in stool samples. The choice of which probe to use for other applications would depend upon the level of specificity required (i.e., enteric campylobacters, or all Campylobacter species).

TABLE 40

| HYBRIDIZATION OF CAMPYLOBACTER PROBES 1-4 TO CAMPYLOBACTER SPECIES. | | | | | |
|---|---|---|---|---|---|
| | | % Probe Bound (*) | | | |
| Organism | ATCC# | 1 | 2 | 3 | 4 |
| Campylobacter coli | 33559 | 64 | 70 | 52 | 49 |
| C. fetus subsp. fetus | 27374 | 68 | 0.1 | 66 | 0.5 |
| C. fetus subsp. venerealis | 19438 | 66 | 0.7 | 54 | 1.2 |

(continued)

| HYBRIDIZATION OF CAMPYLOBACTER PROBES 1-4 TO CAMPYLOBACTER SPECIES. | | | | | |
|---|---|---|---|---|---|
| | | % Probe Bound (*) | | | |
| Organism | ATCC# | 1 | 2 | 3 | 4 |
| C. jejuni | 33560 | 63 | 76 | 51 | 56 |
| C. laridis | 35221 | 74 | 73 | 64 | 52 |
| C. sputorum subsp. bubulus | 33562 | 71 | 3.0 | 2.5 | 0 |
| (*) % Probe Bound = cpm bound to hybroxyapatite-cpm bound when no RNA present/total cpm used in the assay | | | | | |

Table 41 shows that the probes do not hybridize to closely related organisms or organisms found in the gastrointestinal tract.

TABLE 41

| HYBRIDIZATION OF CAMPYLOBACTER PROBES 1-4 TO CLOSELY RELATED ORGANISMS AND ORGANISMS FOUND IN THE GASTRO-INTESTINAL TRACT. | | | | | |
|---|---|---|---|---|---|
| | | % Probe Bound (*) | | | |
| Organism | ATCC# | 1 | 2 | 3 | 4 |
| Bacteroides fragilis | 25285 | 0 | 0.2 | 0.7 | 0 |
| Escherichia coli | 11775 | 1.3 | 0.5 | 0.5 | 0 |
| Salmonella typhimurium | 14028 | 0 | 0 | 0.3 | 0 |
| Shigella boydii | 29929 | 0 | 0.2 | 0.5 | 0 |
| Shigella dysenteriae | 13313 | 0 | 0.7 | 0.2 | 0 |
| Shigella flexneri | 29903 | 0 | 0 | 0.5 | 0 |
| Shigella sonnei | 29930 | 0 | 0 | 0.1 | 0 |
| Vibrio parahaemolyticus | 17802 | 0 | 1.9 | 0.1 | 0 |
| Wollinella succinogenes | 29543 | 0.4 | 2.1 | 2.2 | 0 |
| Yersinia pseudotuberculosis | 29833 | 0.6 | 0.2 | 1.7 | 0.3 |
| (*) % probe bound = cpm bound to hydroxyapatite-cpm bound when no RNA present/total cpm used in the assay | | | | | |

The probes specific for the enteric Campylobacters, probes 2 and 4, were further tested and shown not to react with rRNAs of other organisms found in the gastrointestinal tract.

TABLE 42

| HYBRIDIZATION OF CAMPYLOBACTER PROBES 2 AND 4 TO ORGANISMS FOUND IN THE GASTROINTESTINAL TRACT. | | | |
|---|---|---|---|
| | | % Probe Bound (*) | |
| Organism | ATCC# | Probe 2 | Probe 4 |
| Citrobacter diversus | 27156 | 0 | 0 |
| Clostridium perfringens | 13124 | 0 | 0 |
| Enterobacter cloacae | 13047 | 0 | 0 |
| Klebsiella pneumoniae | 23357 | 0 | 0.5 |
| Proteus mirabilis | 25933 | 0 | 0 |
| Serratia marcescens | 13880 | 0 | 0 |

(continued)

| HYBRIDIZATION OF CAMPYLOBACTER PROBES 2 AND 4 TO ORGANISMS FOUND IN THE GASTROINTESTINAL TRACT. | | | |
|---|---|---|---|
| | | % Probe Bound (*) | |
| Organism | ATCC# | Probe 2 | Probe 4 |
| Staphylococcus aureus | e12600 | | |
| Staphylococcus epidermidis | 14990 | 0 | 0.3 |
| Streptococcus bovis | 33317 | 0 | 0 |
| (*) % probe bound = cpm bound to hydroxyapatite-cpm bound when no RNA present/total cpm used in the assay | | | |

Example 13

Streptococci are gram positive, oxidase negative coccoid bacteria. The genus has been divided into 18 groups, A-R, on the basis of group-specific carbohydrates. Group D streptococci are further subdivided into the enteroccocci (S. faecium, S. faecalis, S.avium and S. gallinarum and the non-enterococci S. bovis, S. equinus. S. faecium, S. faecalis and S. avium are considered the medically important enteroccocci. Some species of streptococcus are human pathogens; others are normal flora in the mouth and intestine but are capable of causing disease when introduced to other sites. Two examples are S. faecium and S. faecalis which are normally found in the intestine but may spread to cause bacteremia, wound infections, and as many as 10% of the urinary tract infections in the United States.

Current methods of detection of enterococci require culture of the specimen for 18-72 hours followed by a battery of biochemical tests. The oligonucleotide sequence shown below, when used in a hybridization assay, accurately detects Streptococcus faecalis, S. avium, and S. faecium. The probe of the invention does not cross react with other Streptococci or Staphylococci which are very closely related in DNA homology. (Kiepper-Baez, 1981, 1982, Schliefer 1984.) The current invention also reduces the number of tests which must be run on a sample and greatly reduces the time to identification and thus, diagnosis. This represents a significant improvement over prior art methods.

The probe sequence was identified using a primer complementary to 16S rRNA with the sequence 5'-CCG CTT GTG CGG GCC CCC GTC AAT TC-3'. The following sequence was characterized and shown to be specific for three enterococci, S. faecium, S. faecalis and S. avium. The phylogenetically nearest neighbors S. agalactiae, S. bovis, S. pneumoniae and S. pyogenes were used for comparison with the sequences of interest.

1. TGC AGC ACT GAA GGG CGG AAA CCC TCC AAC ACT TA

The sequence is 35 bases in length and has a Tm of 72°C. It is capable of hybridizing in the region corresponding to bases 825-860 of E. coli 16S rRNA. To demonstrate the reactivity and specificity of the probe, it was used in a hybridization assay with purified RNA or RNA released from cells. A suspension containing at least $10^7$ cells in 2% sodium dodecyl sulfate was vortexed in the presence of glass beads. 0.1 ml of suspension was mixed with 0.1 ml of hybridization buffer (0.96 M sodium phosphate, pH 6.8, 0.002 M EDTA, 0.002 M EGTA) and incubated at 65°C for 2 hours. After incubation, 5 ml of 2% hydoxyapatite, 0.12 M sodium phosphate pH 6.8, 0.02% sodium dodecyl sulfate was added and the mixture was incubated at 65°C for 10 minutes. The sample was centrifuged and the supernatant removed. Five ml of wash solution (0.12 M phosphate buffer, pH 6.8, 0.02% sodium dodecyl sulfate) was added and the samples were vortexed, centrifuged, and the supernatant removed. The amount of radioactivity bound to the hydroxyapatite was determined by scintillation counting. Table 43 shows that the probe reacts well with S. faecium, S. faecalis, and S. avium, and does not react with other closely related organisms.

TABLE 43

| HYBRIDIZATION OF THE ENTEROCOCCUS PROBE TO CLOSELY RELATED ORGANISMS. | | |
|---|---|---|
| Organism | ATCC# | % Probe Bound |
| Staphylococcus aureus | 12600 | 1.4 |
| Streptococcus agalactiae | 13813 | 1.5 |
| Streptococcus avium | 14025 | 22.7 |
| Streptococcus bovis | 33317 | 1.4 |
| Streptococcus faecalis | 19433 | 45.3 |
| Streptococcus faecium | 19434 | 43.0 |
| Streptococcus mitis | 9811 | 1.5 |

(continued)

| HYBRIDIZATION OF THE ENTEROCOCCUS PROBE TO CLOSELY RELATED ORGANISMS. | | |
|---|---|---|
| Organism | ATCC# | % Probe Bound |
| Streptococcus pneumoniae | 6306 | 1.5 |
| Streptococcus pyogenes | 19615 | 1.3 |

Example 14

Pseudomonads are gram-negative, nonsporeforming, nonfermentative bacilli. Pseudomonads are common inhabitants of soil and water and rarely infect healthy individuals. When the organisms encounter already compromised patients, they can cause a variety of clinical syndromes including wound infections, post-surgical infections, septicemia, infant diarrhea and respiratory and urinary tract infections. Members of the genus Pseudomonas are particularly important to identify in a clinical sample because of the resistance of the organisms to antibiotics. Nucleic acid homology studies have divided the genus into five homology classes known as RNA groups I-V. Eighty-three percent of all clinical isolates of Pseudomonas are from RNA group I and Pseudomonas aeruginosa is by far the most common species isolated.

Current methods of detection of pseudomonas require culture of a patient sample for 24-72 hours, followed by a battery of biochemical tests. The oligonucleotide sequence below, when used in a hybridization assay, detects the clinically important group I pseudomonas. The present invention reduces the number of tests which must be run on a single sample, and reduces the time to detection. This represents a significant improvement over prior art methods.

The sequence was obtained with a primer complementary to a conserved region on 23S rRNA with the sequence 5'-CTT TCC CTC ACG GTA-3'. The following sequence was shown to detect group I pseudomonads:

1. CAG ACA AAG TTT CTC GTG CTC CGT CCT ACT CGA TT

The probe is 35 bases in length and has a Tm of 70°C. It is capable of hybridizing to the RNA of group I Pseudomonas in the region corresponding to bases 365-405 of E. coli 23S rRNA. To demonstrate the reactivity and specificity of the probe, it was used in a hybridization assay. $^{32}$P-end-labeled oligonucleotide was mixed with RNA released from at least $10^7$ organisms by standard methods in 0.48 M sodium phosphate pH 6.8, 1% sodium dodecyl sulfate, 1 mM EDTA, 1 mM EGTA and incubated at 65°C for two hours. After incubation, the RNA:DNA hybrids were bound to hydroxyapatite as described for previous examples and the radioactivity bound was determined by scintillation counting. Table 44 demonstrates that the probe reacted well with all 8 species of group I pseudomonads that were tested. The probe did not react with RNA from group II or group V organisms. A low reaction was seen with Pseudomonas acidovorans, a group III organism which represents < 1% of all isolates of nonfermentative bacilli from clinical samples. Table 45 demonstrates that the probe does not react with other closely related organisms which were tested.

TABLE 44

| HYBRIDIZATION OF PSEUDOMONAS GROUP I PROBE TO PSEUDOMONAS RNAs | | | |
|---|---|---|---|
| Organism | Group | ATCC# | % Probe* Bound |
| Pseudomonas alcaligenes | I | 14909 | 24 |
| Pseudomonas aeruginosa | I | 10145 | 83 |
| Pseudomonas denitrificans | I | 13867 | 83 |
| Pseudomonas fluorescens | I | 13525 | 82 |
| Pseudomonas mendocina | I | 25411 | 79 |
| Pseudomonas pseudoalcaligenes | I | 17440 | 78 |
| Pseudomonas putida | I | 12633 | 80 |
| Pseudomonas stutzeri | I | 17588 | 84 |
| Pseudomonas cepacia | II | 25416 | 0 |
| Pseudomonas pickettii | II | 27511 | 1.0 |
| Pseudomonas acidovorans | III | 15668 | 11 |
| Pseudomonas maltophilia | V | 13637 | 0.2 |
| *% Probe Bound = counts bound when RNA present - counts bound when no RNA present/ total counts used in the assay | | | |

TABLE 45

| HYBRIDIZATION OF PSEUDOMONAS GROUP I PROBE TO RNAs OF CLOSELY RELATED ORGANISMS | | |
|---|---|---|
| Organism | ATTCC# | % Probe* Bound |
| Acinetobacter calcoaceticus | 23055 | 1.6 |
| Legionella pneumophila | 33155 | 0.6 |
| Moraxella phenylpyruvica | 23333 | 0.3 |
| Morganella morganii | 25830 | 0 |
| Vibrio parahaemolyticus | 17802 | 0.6 |
| *% Probe Bound = counts bound when RNA present - counts bound when no RNA present/total counts used in the assay | | |

Example 15

Examples 15-18 disclose probes for the Enterobacteriaceae, all of which are highly related at the DNA level. Even fewer differences exist at the rRNA level. For example, Proteus vulgaris 16S rRNA is 93% homologous to E. coli. These factors illustrate the difficulties associated with making rRNA probes specific for this group of organisms. Nevertheless, we have invented probes for Enterobacter cloacae, Proteus mirabilis, Salmonella and E. coli.

Members of the genus Enterobacter are motile, gram negative, non-spore-forming bacilli which belong in the family Enterobacteriaceae. The genus is a large and heterogeneous group. Eight species have been defined but only 5 are clinically significant. Enterobacter cloacae and E. aerogenes are the most common isolates and are associated with genitourinary, pulmonary, blood, central nervous system and soft tissue infections in humans.

The current method for identifying Enterobacter cloacae from patient samples involves culture of the specimen on agar plates for 18-24 hours, followed by a battery of biochemical tests. The oligonucleotide sequence described below, when used as a probe in a nucleic acid hybridization assay, accurately identifies Enterobacter cloacae. The present invention reduces the number of tests which must be run on a single sample, the time to identification and therefore, diagnosis, and thus represents a significant improvement over prior art methods.

The probe specific for Enterobacter cloacae was obtained with a primer complementary to a conserved region of 23S rRNA with the sequence 5'-CAG TCA GGA GTA TTT AGC CTT-'3.

The following sequence was characterized and shown to be specific for E. cloacae. The phylogenetically nearest neighbors Escherichia coli, Klebsiella pneumoniae, Proteus vulgaris, Salmonella enteritidis, and Citrobacter freundii were used as comparisons with the sequence of E. cloacae.

1. GTG TGT TTT CGT GTA CGG GAC TTT CAC CC

The probe is 29 bases in length and has a Tm of 68°C. It is capable of hybridizing to RNA of E. cloacae in the region corresponding to bases 305-340 of E. coli 23S rRNA. To demonstrate the reactivity and specificity of the probe for E. cloacae, it was used in a hybridization assay. $^{32}$P-end-labeled oligonucleotide probe was mixed with RNA released from at least $10^7$ organisms in 1% sodium dodecyl sulfate, 0.48 M sodium phosphate, pH 6.8 (0.2 ml final volume) and incubated at 60°C for 2 hours. Following incubation, 5 ml of 2% hydoxyapatite, 0.12 M sodium phosphate pH 6.8, 0.02% sodium dodecyl sulfate was added and the mixture incubated at 60°C for 10 minutes. The sample was centrifuged and the supernatant removed. Five ml of wash solution (0.12 M sodium phosphate, pH 6.8, 0.02% sodium dodecyl sulfate) was added, the sample vortexed, centrifuged and the supernatant removed. The amount of radioactivity bound to the hydroxyapatite was determined by scintillation counting. The results are shown in Table 46 and demonstrates that the probe reacts well with E. cloacae and does not react with the RNA of closely related organisms.

TABLE 46

| HYBRIDIZATION OF ENTEROBACTER CLOACAE PROBE TO CLOSELY RELATED ORGANISMS | | |
|---|---|---|
| Organisms Name | ATCC# | % Probe Bound |
| Citrobacter freundii | 8090 | 1.8 |
| Enterobacter aerogenes | 13048 | 1.4 |
| Enterobacter cloacae | 13047 | 27. |
| Escherichia coli | 11775 | 1.0 |
| Klebsiella pneumoniae | 13883 | 1.7 |
| Proteus mirabilis | 29906 | 0.9 |

(continued)

| HYBRIDIZATION OF ENTEROBACTER CLOACAE PROBE TO CLOSELY RELATED ORGANISMS | | |
|---|---|---|
| Organisms Name | ATCC# | % Probe Bound |
| Proteus vulgaris | 13315 | 0.6 |
| Providencia stuartii | 29914 | 1.1 |

Table 47 shows that the probe does not react with the RNA of organisms found in urine.

TABLE 47

| HYBRIDIZATION OF ENTEROBACTER CLOACAE PROBE TO ORGANISMS FOUND IN URINE. | | |
|---|---|---|
| Organisms Name | ATCC# | % Probe Bound |
| Candida albicans | 18804 | 0.8 |
| Candida krusei | 34135 | 0.8 |
| Candida parapsilosis | 22019 | 0.9 |
| Candida tropicalis | 750 | 1.1 |
| Pseudomonas aeruginosa | 10145 | 1.0 |
| Serratia marcescens | 13880 | 1.6 |
| Staphylococcus aureus | 12600 | 1.7 |
| Staphylococcus epidermidis | 14990 | 1.4 |
| Streptococcus agalactiae | 13813 | 2.5 |
| Streptococcus faecium | 19434 | 1.5 |
| Torulopsis glabrata | 2001 | 0.9 |

Example 16

Members of the genus Proteus are motile, gram negative, non-sporeforming bacilli which belong in the family Enterobacteriaceae. Four species of Proteus have been described and three of them, Proteus mirabilis, P. vulgaris, and P. penneri, cause human disease.

The most common type of proteus infection involves the urinary tract, but septicemia, pneumonia and wound infections also occur. Proteus mirabilis is the species most often isolated and may account for up to 10% of all acute, uncomplicated urinary tract infections. Species, rather than genus level identification of the causative organism is desirable because of differential antibiotic susceptibility among the species.

The current method for identifying Proteus mirabilis from patient samples involves culture of the specimen on agar plates for 18-24 hours, followed by a battery of biochemical tests. The oligonucleotide sequence described below, when used as a probe in a nucleic acid hybridization assay, accurately identifies Proteus mirabilis. The present invention reduces the number of tests which must be run on a sample, the time to identification and therefore, diagnosis and treatment. This represents a significant improvement over prior art methods.

The probe specific for Proteus mirabilis was obtained with a primer complementary to a conserved region of 23S rRNA with the sequence 5'-CAG TCA GGA GTA TTT AGC CTT-3'.

The following sequence was characterized and shown to be specific for P. mirabilis. The phylogenetically nearest neighbors Escherichia coli, Klebsiella pneumoniae. Proteus vulgaris and Salmonella enteritidis were used as comparisons with the sequence of Proteus mirabilis.

1. CCG TTC TCC TGA CAC TGC TAT TGA TTA AGA CTC

1. CCG TTC TCC TGA CAC TGC TAT TGA TTA AGA CTC This probe is capable of hybridizing to the RNA of P. mirabilis in the region corresponding to base 270-305 of E. coli 23s rRNA. The probe is 33 bases in length and has a Tm of 66°C. To demonstrate the reactivity and specificity of the probe for P. mirabilis, it was used in a hybridization assay. $^{32}$P-end-labeled oligonucleotide probe was mixed with RNA released from at least $10^7$ organisms in 1% sodium dodecyl sulfate, 0.48 M sodium phosphate, pH 6.8, 1 mM EDTA, 1 mM EGTA (0.2 ml final volume) and incubated at 64°C for 2 hours. Following incubation, 5 ml of 2% hydroxyapatite, 0.12 M sodium phosphate pH 6.8, 0.02% sodium dodecyl sulfate was added and the mixture incubated at 64°C for 10 minutes. The sample was centrifuged and the supernatant removed. Five ml of wash solution (0.12 M sodium phosphate, pH 6.8, 0.02% sodium dodecyl sulfate) was added, the sample vortexed, centrifuged and the supernatant was removed. The amount of radioactivity bound to the hydroxyapatite was determined by scintillation counting. The results are shown in Table 48 and demonstrate that the probe reacts well with P. mirabilis and does not react with 27 other closely related bacteria. Table 49 shows that the probe does not react with 24 other phylogenetically diverse bacteria and two yeasts tested in the same manner as the organisms in Table 48.

TABLE 48

| HYBRIDIZATION OF PROTEUS MIRABILIS PROBE TO CLOSELY RELATED ORGANISMS | | |
|---|---|---|
| Organism Name | ATCC# | % Probe Bound |
| Citrobacter diversus | 27156 | 1.1 |
| Citrobacter freundii | 8090 | 1.1 |
| Citrobacter freundii | 6750 | 1.0 |
| Enterobacter aerogenes | 13048 | 1.0 |
| Enterobacter agglomerans | 27155 | 1.0 |
| Enterobacter cloacae | e13047 | 1.1 |
| Enterobacter gergoviae | 33028 | 1.0 |
| Enterobacter sakazakii | 29544 | 1.1 |
| Escherichia coli | 10798 | 1.2 |
| Escherichia coli | 11775 | 1.2 |
| Escherichia coli | 29417 | 1.2 |
| Klebsiella oxytoca | 13182 | 1.0 |
| Klebsiella ozaenae | 11296 | 1.1 |
| Klebsiella planticola | 33531 | 0.9 |
| Klebsiella pneumoniae | 13883 | 1.3 |
| Klebsiella pneumoniae | 23357 | 1.1 |
| Klebsiella rhinoscleromatis | 13884 | 1.2 |
| Klebsiella terrigena | 33257 | 1.1 |
| Klebsiella trevisanii | 33558 | 1.0 |
| Kluyvera ascorbata | 33433 | 0.9 |
| Proteus mirabilis | 25933 | 69.0 |
| Proteus penneri | 33519 | 2.5 |
| Proteus vulgaris | 13315 | 1.7 |
| Providencia alcalifaciens | 9886 | 1.1 |
| Providencia rettgeri | 29944 | 1.3 |
| Providencia stuartii | 29914 | 1.1 |
| Salmonella arizonae | 29933 | 1.1 |
| Salmonella enteritidis | 13076 | 0.8 |

TABLE 49

| HYBRIDIZATION OF PROTEUS MIRABILIS PROBE TO PHYLOGENETICALLY DIVERSE ORGANISMS | | |
|---|---|---|
| Organism Name | ATCC# | % Probe Bound |
| Acinetobacter calcoaceticus | 33604 | 0.8 |
| Bacillus subtilis | 6051 | 1.2 |
| Bacteroides fragilis | 23745 | 0.9 |
| Branhamella catarrhalis | 25238 | 0.7 |
| Campylobacter jejuni | 33560 | 1.0 |
| Candida krusei | 34135 | 0.8 |
| Chromobacterium violaceum | 29094 | 1.1 |
| Clostridium perfringens | 13124 | 0.9 |
| Deinococcus radiodurans | 35073 | 0.8 |
| Derxia gummosa | 15994 | 0.8 |
| Hafnia alvei | 13337 | 0.9 |

(continued)

| HYBRIDIZATION OF PROTEUS MIRABILIS PROBE TO PHYLOGENETICALLY DIVERSE ORGANISMS | | |
|---|---|---|
| Organism Name | ATCC# | % Probe Bound |
| Morganella morganii | 25830 | 0.9 |
| Pseudomonas aeruginosa | 10145 | 1.0 |
| Pseudomonas cepacia | 17762 | 0.9 |
| Rahnella aquatilis | 33071 | 0.9 |
| Rhodospirillum rubrum | 11170 | 0.8 |
| Serratia marcescens | 13880 | 0.9 |
| Serratia odorifera | 33077 | 0.9 |
| Staphylococcus aureus | e12600 | 0.8 |
| Staphylococcus epidermidis | 14990 | 0.8 |
| Streptococcus mitis | 9811 | 0.8 |
| Streptococcus pneumoniae | e6306 | 0.9 |
| Torulopsis glabrata | 2001 | 0.9 |
| Vibrio parahaemolyticus | 17802 | 0.8 |
| Xanthomonas maltophilia | 13637 | 1.1 |
| Yersinia enterocolitica | 9610 | 0.8 |

Example 17

Members of the genus Salmonella are motile, gram negative, non-sporeforming bacilli which belong in the family Enterobacteriaceae. All salmonellae are highly related and some microbiologists consider them to be one species. Five subgroups have been identified using nucleic acid homology studies and over 1400 different serotypes have been described. All serotypes have been implicated in human enteric disease ranging from self-limited gastroenteritis with mild symptoms, to severe gastroenteritis with bacteremia, to typhoid fever, a potentially life-threatening illness. S. cholerasuis, S. paratyphi A and S. typhi are the serotypes most often associated with severe disease and bacteremia. Diagnosis of Salmonella-induced enteritis is dependent upon detection of the organism in stool samples. Because infection occurs primarily by ingestion of contaminated milk, food and water, methods for identifying Salmonella in these products before release to consumers is critical.

Current methods for detection of members of the genus Salmonella involve culture of the specimen for 1-3 days on selective media followed by a battery of biochemical tests. Often an enrichment step is needed to isolate Salmonella from clinical samples or food products. The oligonucleotide sequences shown below, when used in a hybridization assay, accurately identify members of the genus Salmonella. The present inventive probes are specific for all members of the genus and do not react with the other closely related Enterobacteriaceae genera. These probes reduce the number of tests which must be run on a sample and greatly reduce the time to identification. This represents a significant improvement over prior art methods.

The probes specific for the genus Salmonella were obtained with two primers complementary to 16S and 23S rRNA. Sequence 1 was obtained using a 16S primer with the sequence 5' TTA CTA GCG ATT CCG ACT TCA 3'. Sequence 2 was obtained using a 23S primer with the sequence 5' CAG TCA GGA GTA TTT AGC CTT 3'. The following sequences were characterized and shown to be specific for the genus Salmonella:

1. CTC CTT TGA GTT CCC GAC CTA ATC GCT GGC
2. CTC ATC GAG CTC ACA GCA CAT GCG CTT TTG TGT A

Sequence 1, from 16S rRNA, is 30 bases in length and has a Tm of 73°C. Sequence 2, from 23S rRNA, is 34 bases long and has a Tm of 71°C. These probes are capable of hybridizing in the regions corresponding to bases 1125-1155 of E. coli 16S rRNA and 335-375 of E. coli 23S rRNA, respectively. To demonstrate the reactivity and specificity of probe 1 for members of the genus Salmonella, $^{32}$P-end-labeled oligonucleotide was tested as a probe in a hybridization reaction. Purified RNA, or RNA released from at least $10^7$ organisms by standard methods, was mixed with 1 ml hybridization buffer (final concentration 43% diisobutyl sulfosuccinate, 60ml sodium phosphate pH 6.8, 1mM EDTA, 1mM EGTA) and incubated at 72°C for 2-12 hours. Following incubation, 5 ml of separation solution (2% hydroxyapatite, 0.12 M sodium phosphate, pH 6.8, 0.02% sodium dodecyl sulfate) was added and the sample were mixed, incubated at 72°C for 5 minutes, centrifuged and the supernatants removed. Four ml of wash solution (0.12 M sodium phosphate pH 6.8, 0.02% sodium dodecyl sulfate) was added and the samples were vortexed, centrifuged, and the supernatants removed. The amount of radioactivity bound to the hydroxyapatite was determined by scintillation counting. The results shown in Table 50 indicate that a combination of the two probes hybridized to the 5 subgroups of Salmonella and to all 31 of the serotypes which were tested.

TABLE 50

| HYBRIDIZATION OF SALMONELLA PROBES 1 AND 2 TO MEMBERS OF THE GENUS SALMONELLA | | | | |
|---|---|---|---|---|
| | | | % Probe Bound | |
| Subgroup | Organism | ATCC# | Probe 1 | Probe 2 |
| I | Salmonella choleraesuis | 10708 | 24 | 40 |
| I | Salmonella enteritidis | 13076 | 15 | 67 |
| I | Salmonella paratyphi A | 9150 | 1.4 | 70 |
| I | Salmonella sp. serotype anatum | 9270 | 40 | 26 |
| I | Salmonella sp. serotype cubana | 12007 | 54 | 35 |
| I | Salmonella sp. serotype give | 9268 | 12 | 40 |
| I | Salmonella sp. serotype heidelberg | 8326 | 53 | 33 |
| I | Salmonella sp. serotype illinois | 11646 | 36 | 46 |
| I | Salmonella sp. serotype montevideo | 8387 | 35 | 32 |
| I | Salmonella sp. serotype newington | 29628 | 52 | 34 |
| I | Salmonella sp. serotype newport | 6962 | 3.4 | 36 |
| I | Salmonella sp. serotype putten | 15787 | 34 | 39 |
| I | Salmonella sp. serotype saintpaul | 9712 | 28 | 30 |
| I | Salmonella sp. serotype senftenberg | 8400 | 38 | 43 |
| I | Salmonella sp. serotype simsbury | 12004 | 29 | 29 |
| I | Salmonella sp. serotype sloterdijk | 15791 | 34 | 30 |
| I | Salmonella sp. serotype thompson | 8391 | 32 | 41 |
| I | Salmonella sp. serotype vellore | 15611 | 35 | 2.6 |
| I | Salmonella typhi . | 19430 | 7.0 | 21 |
| I | Salmonella typhimurium | 14028 | 69 | 69 |
| II | Salmonella salamae | 6959 | 3.0 | 46 |
| II | Salmonella sp. serotype maarssen | 15793 | 6.6 | 30 |
| III | Salmonella arizonae | 33952 | 2.9 | 38 |
| III | Salmonella arizonae | 12324 | 5.5 | 42 |
| III | Salmonella arizonae | 29933 | 2.3 | 62 |
| III | Salmonella arizonae | 29934 | 63 | 12 |
| III | Salmonella arizonae | 12323 | 4.0 | 39 |
| III | Salmonella arizonae | 12325 | 51 | 1.9 |
| IV | Salmonella sp. serotype harmelen | 15783 | 5.8 | 8.0 |
| IV | Salmonella sp. serotype ochsenzoll | 29932 | 7.5 | 40 |
| V | Salmonella sp. serotype bongor | cdc1319 | 60 | 1.8 |

The specificity of the probes for members of the genus Salmonella was demonstrated with hybridization reactions containing RNA from organisms closely related to Salmonella. The results are shown in Table 51.

TABLE 51

| HYBRIDIZATION OF SALMONELLA PROBES 1 AND 2 TO RNA OF CLOSELY RELATED ORGANISMS | | | |
|---|---|---|---|
| | | % Probe Bound | |
| Organism | ATCC# | Probe 1 | Probe 2 |
| Citrobacter freundii | 6750 | 2.2 | 0 |
| Edwardsiella tarda | 15947 | 0 | 0 |
| Enterobacter agglomerans | 27155 | 0.6 | 0 |

(continued)

| HYBRIDIZATION OF SALMONELLA PROBES 1 AND 2 TO RNA OF CLOSELY RELATED ORGANISMS | | | |
|---|---|---|---|
| | | % Probe Bound | |
| Organism | ATCC# | Probe 1 | Probe 2 |
| Enterobacter cloacae | 13047 | 0 | 0 |
| Enterobacter sakazakii | 29544 | 0 | 0 |
| Escherichia coli | 10798 | 0 | 0 |
| Escherichia coli | 29417 | 0 | 0 |
| Klebsiella pneumoniae | 23357 | 0.7 | 0 |
| Kluyvera ascorbata | 33433 | 0 | 0.5 |
| Proteus mirabilis | 25933 | 0.2 | 0 |
| Shigella flexneri | 29903 | 0 | 0 |
| *% Probe Bound = counts bound to hydroxyapatite - counts bound when no RNA present/total counts used in assay | | | |

Table 52 shows that Salmonella probes 1 and 2 do not hybridize to phylogenetically diverse organisms.

TABLE 52

| HYBRIDIZATION OF SALMONELLA PROBES 1 AND 2 TO RNA OF A PHYLOGENETIC CROSS SECTION OF ORGANISMS | | | |
|---|---|---|---|
| | | % Probe Bound* | |
| Organism | ATCC# | Probe 1 and Probe 2 | |
| Acinetobacter calcoaceticus | 33604 | 1.1 | 0.1 |
| Bacillus subtilis | 6051 | 0 | 0.5 |
| Bacteroides fragilis | 23745 | 0.1 | 0 |
| Branhamella catarrhalis | 25238 | 0.9 | 0 |
| Campylobacter jejuni | 33560 | 0 | 0.2 |
| Candida krusei | 34135 | 0.4 | 0.3 |
| Chromobacterium violaceum | 29094 | 1.7 | 0 |
| Clostridium perfringens | 13124 | 0.3 | 0 |
| Deinococcus radiodurans | 35073 | 1.6 | 0.1 |
| Derxia gummosa | 15994 | 1.2 | 0 |
| Hafnia alvei | 13337 | 1.8 | 0 |
| Morganelli morganii | 25830 | 0 | 1.1 |
| Pseudomonas aeruginosa | 10145 | 0.5 | 0.7 |
| Pseudomonas cepacia | 17762 | 0 | 0 |
| Pseudomonas maltophilia | 13637 | 1.9 | 0 |
| Rahnella aquatilis | 33071 | 1.2 | 0.3 |
| Rhodospirillum rubrum | 11170 | 0.9 | 0 |
| Serratia marcescens | 13880 | 0 | 0 |
| Serratia odorifera | 33077 | 2.6 | 0.2 |
| Staphylococcus aureus | e12600 | 0.2 | 0 |
| Staphylococcus epidermidis | 14990 | 0 | 0 |
| Streptococcus mitis | 9811 | 1.2 | 0.7 |
| Streptococcus pneumoniae | e6306 | 0 | 0 |
| Torulopsis glabrata | 2001 | 0 | 0 |
| Vibrio parahaemolyticus | 17802 | 0 | 0.2 |

(continued)

| HYBRIDIZATION OF SALMONELLA PROBES 1 AND 2 TO RNA OF A PHYLOGENETIC CROSS SECTION OF ORGANISMS | | | |
|---|---|---|---|
| | | % Probe Bound* | |
| Organism | ATCC# | Probe 1 and Probe 2 | |
| Yersinia enterocolitica | 9610 | 0 | 0 |
| *% Probe Bound = Counts bound to hydroxyapatite - counts bound when no RNA present/total counts used in assay | | | |

Example 18

Escherichia coli is a gram negative, nonsporeforming bacillus which belongs in the family Enterobacteriaceae. Five species of Escherichia have been described: E. coli, which accounts for >99% of the clinical isolates, E. hermanii, E. blattae, E. vulneris and E. fergusonii. E. coli is a leading cause of urinary tract infections, bactermia and neonatal meningitidis, and can cause a type of gastroenteritis known as traveller's diarrhea.

The current method for identifying E. coli from patient samples involves culture of the specimen on agar plates for 18-72 hours, followed by a battery of biochemical tests on isolated colonies. The oligonucleotide sequence described below, when used as a probe in a nucleic acid hybridization assay, accurately detects E. coli even in the presence of other organisms. The present invention reduces the number of tests which must be run on a sample and reduces the time to identification and therefore diagnosis and treatment. This represents a significant improvement over prior art methods.

The probe specific for E. coli was derived from the published E coli sequence (Brosius, et al. Proc. Natl. Acad. Sci. U.S.A. 75:4801-4805 (1978)), using Proteus vulgaris (Carbon, et al., Nuc. Acids Res. 9:2325-2333 (1981)), Klebsiella pneumoniae, Salmonella enteritidis, Enterobacter gergoviae and Citrobacter freundii for comparison. The probe sequence is shown below.

1. GCA CAT TCT CAT CTC TGA AAA CTT CCG TGG

It hybridizes to RNA of E. coli in the region of 995-1030 of 16S rRNA. The probe is 30 bases in length and has a $T_m$ of 66°C. To demonstrate the reactivity and specificity of the probe for E. coli, it was used in a hybridization assay. $^{32}$P-end-labeled oligonucleotide probe was mixed with two unlabeled oligonucleotides of sequence 5'-TGG ATG TCA AGA CCA GGT AAG GTT CTT CGC GTT GCA TCG-3' and 5'-CTG ACG ACA GCC ATG CAG CAC CTG TCT CAC GGT TCC CGA AGG CA-3' and with purified RNA, or RNA released from cells with detergent and heat, in 1% sodium dodecyl sulfate (SDS), 0.48 M sodium phosphate pH 6.8, 1mM EDTA, 1mM EGTA (0.2 ml final volume) and incubated at 60°C for 2 hours. Following incubation, 5 ml of 2% hydroxyapatite, 0.12 M sodium phosphate pH 6.8, 0.02% sodium dodecyl sulfate was added and the mixture incubated at 60°C for 10 minutes. The sample was centrifuged and the supernatant removed. Five ml of wash solution (0.12 M sodium phosphate, pH 6.8, 0.02% sodium dodecyl sulfate) was added, the sample vortexed, centrifuged and the supernatant was removed. The amount of radioactivity bound to the hydroxyapatite was determined by scintillation counting.

An example of a use for this probe would be to detect E. coli in urine samples. Table 53 shows that the probe detects 7 out of 8 strains of E. coli tested. This probe also reacts with E. fergusonii, an organism which would only rarely be found in urine.

Table 54 shows that the probe does not react with any other genus tested except Shigella, another organism rarely isolated from urine. These results show that the probe will be useful in detecting E. coli from urine samples.

TABLE 53

| HYBRIDIZATION OF E. coli TO ESCHERICHIA SPECIES | | |
|---|---|---|
| Organism | ATCC# | % Probe Bound |
| Escherichia coli | 10798 | 70 |
| E. coli | 11775 | 67 |
| E. coli | 23722 | 58 |
| E. coli | 25404 | 68 |
| E. coli | 25922 | 55 |
| E. coli | 29417 | 72 |
| E. coli | 33780 | 0.8 |
| E. coli | 35150 | 45 |

(continued)

| HYBRIDIZATION OF E. coli TO ESCHERICHIA SPECIES | | |
|---|---|---|
| Organism | ATCC# | % Probe Bound |
| E. fergusonii | 35469 | 55 |
| E. hermanii | 33650 | 0.7 |
| E. vulneris | 33821 | 0.8 |

TABLE 54

| HYBRIDIZATION OF THE E. coli PROBE TO CLOSELY RELATED ORGANISMS | | |
|---|---|---|
| Organism | ATCC# | % Probe Bound |
| Citrobacter freundii | 6750 | 0.8 |
| Citrobacter freundii | 8090 | 0.9 |
| Citrobacter freundii | 29221 | 0.6 |
| Citrobacter freundii | 33128 | 0.6 |
| Enterobacter aerogenes | 13048 | 1.2 |
| Enterobacter agglomerans | 27155 | 0.9 |
| Enterobacter cloacae | 13047 | 0.9 |
| Enterobacter gergoviae | 33023 | 0.7 |
| Enterobacter sakazakii | 29544 | 0.6 |
| Klebsiella oxytoca | 13182 | 0.7 |
| Klebsiella pneumoniae | 13883 | 0.7 |
| Proteus mirabilis | 29906 | 0.7 |
| Proteus vulgaris | 13315 | 0.8 |
| Shibella boydii | 8700 | 76 |
| Shigella dysenteriae | 13313 | 0.8 |
| Shigella flexneri | 29903 | 71 |
| Shigella sonnei | 29930 | 75 |

Example 19

The bacteria encompass a morphologically and physiologically diverse group of unicellular organisms which occupy most natural environments. Although many bacteria are harmless or beneficial to their environment or host, some are harmful and cause disease. The presence of any bacteria in some locations is undesirable or indicative of disease (e.g., culture media, pharmaceutical products, body fluids such as blood, urine or cerebrospinal fluid, and tissue biopsies). Low levels of bacteria are considered acceptable in other products such as drinking water and food products. Accordingly, there is a need for a means for detecting and quantitating bacteria in a sample.

The current method of detection and quantitation of total bacteria in a sample requires culture on multiple types of media under different conditions of temperature and atmosphere. To date, no single test exists to detect or quantitate all bacteria. The oligonucleotide sequences shown below, when used in a hybridization assay, detect a broad phylogenetic cross section of bacteria. The present invention reduces the number of tests which need to be performed and also reduces the time required for the assay. Comparison of the hybridization results from an unknown sample to a set of standards will allow some quantitation of the number of bacteria present. This represents a significant improvement over prior art methods.

The bacterial probes were designed following examination of published sequences of rRNA and sequences determined at Gen-Probe. The sequences used for the comparison include Agrobacterium tumefaciens (Yang et al., Proc. Natl. Acad. Sci. U.S.A., 82:4443, (1985)), Anacystis nidulans (Tomioka and Sugiura. Mol. Gen. Genet. 191:46, (1983), Douglas and Doolittle Nuc. Acids Res. 12:3373, (1984)), Bacillus subtilis (Green et al., Gene 37:261. (1985)), Bacillus stearothermophilus (Kop et al., DNA 3:347, (1984)), Bacteroides fragilis (Weisburg et al., J. Bacteriol. 164:230, (1985)), Chlamydia psittaci (Weisburg et al., J. Bacteriol. 167:570. (1986)), Desulfovibrio desulfuricans (Oyaizu and Woese, System. Appl. Microbiol. 6:257, (1985)); Escherichia coli, (Brosius et al., Proc. Natl. Acad. Sci. U.S.A. 77:201, (1980)); Flavobacterium heparinum (Weisburg et al., J. Bacteriol. 164:230, (1985)); Heliobacterium chlorum (Woese et al., Science 229:762, (1985)); Mycoplasma PG50 (Frydenberg and Christiansen, DNA 4:127, (1985)); Proteus vulgaris (Carbon et al., Nuc. Acids Res. 9:2325, (1981)); Pseudomonas testosteroni (Yang et al., Proc. Natl. Acad. Sci. U.S.A. 82:4443, (1985)); Rochalimaea quintana (Weisburg et al., Science 230:556, (1985)); Saccharomyces cerevisiae (Rubstov et al., Nuc. Acids Res. 8:5779, (1980); Georgiev et al., Nuc. Acids Res. 9:6953, (1981)); and human (Torczynski et al., DNA 4:283, (1985); Gonzalez et al., Proc. Natl. Acad. Sci. U.S.A. 82:7666, (1985)).

The following sequences were shown to hybridize to a broad phylogenetic cross section of bacteria and not to yeast or human rRNA:

1. CCA CTG CTG CCT CCC GTA GGA GTC TGG GCC
2. CCA GAT CTC TAC GCA TTT CAC CGC TAC ACG TGG
3. GCT CGT TGC GGG ACT TAA CCC AAC AT
4. GGG GTT CTT TTC GCC TTT CCC TCA CGG
5. GGC TGC TTC TAA GCC AAC ATC CTG
6. GGA CCG TTA TAG TTA CGG CCG CC
7. GGT CGG AAC TTA CCC GAC AAG GAA TTT CGC TAC C

Probe 1 is 30 bases long and has a Tm of 70°C. Probe 2 is 33 bases long and has a Tm of 69°C. Probe 3 is 26 bases long and has a Tm of 67°C. Probe 4 is 27 bases long and has a Tm of 69°C. Probe 5 is 24 bases long and has a Tm of 66°C. Probe 6 is 23 bases long and has a Tm of 62°C. Probe 7 is 34 bases long and has a Tm of 66°C. Probes 1-3 hybridize to 16S rRNA in the following regions, respectively, (corresponding to E. coli bases) 330-365; 675-715; and 1080-1110. Probes 4-7 hybridize to 23S rRNA in the following regions, respectively, (corresponding to E. coli bases) 460-490; 1050-1080: and 1900-1960 (probes 6 and 7). The oligonucleotides interact with regions on the rRNA which are highly conserved among eubacteria. This means that they can be used as par-bacterial probes in a hybridization assay. A second use is as a tool to obtain rRNA sequence. For example, an oligonucleotide can be hybridized to the rRNA of interest and extended with reverse transcriptase. The sequence of the resulting DNA can be determined and used to deduce the complementary rRNA sequence as described in the Detailed Description of the Invention.

One application of the invention is to detect bacteria in urine (bacteriuria). To demonstrate the reactivity and specificity of the probes for bacteria found in urine, they were used in hybridization assays. $^{32}$P-end-labeled or $^{125}$I-labeled oligonucleotide probes were mixed with RNA released from cells by standard methods (e.g, sonic disruption techniques, detergent with glass beads, or enzymatic lysis). Probe was mixed with RNA in 0.48 M sodium phosphate, pH 6.8, 1 mM EDTA, 1 mM EGTA, 1% sodium dodecyl sulfate (0.2 ml final volume) and hybridized at 60°C for 2 hours. Five ml of 2% hydroxyapatite, 0.12 M sodium phosphate pH 6.8, 0.02% sodium dodecyl sulfate was added and the mixture incubated at 60°C for 10 minutes. The mixture was centrifuged and the supernatant removed. Five ml of wash solution (0.12 M sodium phosphate, pH 6.8, 0.02% sodium dodecyl sulfate) was added and the sample was mixed, centrifuged and the supernatant removed. The amount of radioactivity bound to the hydroxyapatite was determined by scintillation counting. Tables 55-68 demonstrate the specificity of these probes and show that a combination of probes could be used to detect all bacteria wich have been tested.

Table 55 shows that probe 1 hybridizes to the RNA of bacteria commonly isolated frome urine and does not detect yeast RNA. Table 56 shows that probe 1 detects phylogenetically diverse bacteria and does not hybridize to human RNA.

TABLE 55

| HYBRIDIZATION OF BACTERIAL PROBE 1 TO RNA OF ORGANISMS FOUND IN URINE | | |
|---|---|---|
| Organism | ATCC# | % Probe* Bound |
| Candida albicans | 18804 | 2.6 |
| Candida krusei | 34135 | 2.2 |
| Candida parapsilosis | 22019 | 2.9 |
| Candida tropicalis | 750 | 2.5 |
| Citrobacter freundii | 8090 | 69 |
| Enterobacter aerogenes | 13048 | 70 |
| Enterobacter cloacae | 13047 | 71 |
| Escherichia coli | 11775 | 67 |
| Klebsiella oxytoca | 13182 | 70 |
| Klebsiella pneumoniae | 13883 | 72 |
| Morganella morganii | 25830 | 66 |
| Proteus mirabilis | 29906 | 71 |

(continued)

| HYBRIDIZATION OF BACTERIAL PROBE 1 TO RNA OF ORGANISMS FOUND IN URINE | | |
|---|---|---|
| Organism | ATCC# | % Probe* Bound |
| Proteus vulgaris | 13315 | 67 |
| Providencia stuartii | 29914 | 69 |
| Pseudomonas aeruginosa | 10145 | 76 |
| Pseudomonas fluorescens | 13525 | 73 |
| Serratia marcescens | 13880 | 66 |
| Staphylococcus aureus | 12600 | 57 |
| Staphylococcus epidermidis | 14990 | 68 |
| Streptococcus agalactiae | 13813 | 68 |
| Streptococcus faecalis | 19433 | 51 |
| Streptococcus faecium | 19434 | 53 |
| Torulopsis glabrata | 2001 | 2.3 |
| Ureaplasma urealyticum | 27618 | 54 |

TABLE 56

| HYBRIDIZATION OF BACTERIAL PROBE 1 TO RNAs OF A CROSS SECTION OF PHYLOGENETICALLY DIVERSE ORGANISMS. | | |
|---|---|---|
| Organism | ATCC# | % Probe* Bound |
| Acinetobacter calcoaceticus | 23055 | 65 |
| Bacillus subtilis | 6051 | 73 |
| Bacteroides fragilis | 23745 | 61 |
| Branhamella catarrhalis | 25238 | 72 |
| Campylobacter jejuni | 33560 | 64 |
| Chlamydia trachomatis | VR878 | 14 |
| Chromabacterium violaceum | 29094 | 71 |
| Clostridium perfringens | 13124 | 74 |
| Corynebacterium xerosis | 373 | 38 |
| Deinococcus radiodurans | 35073 | 47 |
| Derxia gummosa | 15994 | 65 |
| Gardnerella vaginalis | 14018 | 67 |
| Hafnia alvei | 13337 | 60 |
| Lactobacillus acidophilus | 4356 | 56 |
| Moraxella osloensis | 19976 | 61 |
| Mycobacterium smegmatis | 14468 | 47 |
| Mycoplasma hominis | 14027 | 58 |
| Neisseria gonorrhoeae | 19424 | 58 |
| Rahnella aquatilis | 33071 | 74 |
| Rhodospirillum rubrum | 11170 | 73 |
| Vibrio parahaemolyticus | 17802 | 75 |
| Human | | 2.5 |

Table 57 shows that Probe 2 hybridizes to the RNA of bacteria commonly found in urine except Ureaplasma urealyicum and does not hybridize to yeast rRNA.

TABLE 57

| HYBRIDIZATION OF BACTERIAL PROBE 2 TO RNA OF ORGANISMS FOUND IN URINE | | |
|---|---|---|
| Organism | ATCC# | %Probe* Bound |
| Candida albicans | 18804 | 2.5 |
| Candida krusei | 34135 | 1.8 |
| Candida parapsilosis | 22019 | 1.6 |
| Candida tropical is | 750 | 1.4 |
| Citrobacter freundii | 8090 | 61 |
| Enterobacter aerogenes | 13048 | 57 |
| Enterobacter cloacae | 13047 | 61 |
| Escherichia coli | 11775 | 67 |
| Klebsiella oxytoca | 13182 | 67 |
| Klebsiella pneumoniae | 13883 | 51 |
| Morganella morganii | 25830 | 69 |
| Proteus mirabilis | 29906 | 69 |
| Proteus vulgaris | 13315 | 69 |
| Providencia stuartii | 29914 | 66 |
| Pseudomonas aeruginosa | 10145 | 59 |
| Pseudomonas fluorescens | 13525 | 58 |
| Serratia marcescens | 13880 | 64 |
| Staphylococcus aureus | 12600 | 60 |
| Staphylococcus epidermidis | 14990 | 60 |
| Streptococcus agalactiae | 13813 | 54 |
| Streptococcus faecalis | 19433 | 37 |
| Streptococcus faecium | 19434 | 58 |
| Torulopsis glabrata | 2001 | 1.5 |
| Ureaplasma urealyticum | 27618 | 3.2 |

Table 58 shows that probe 2 detects phylogenetically diverse bacteria and does not hybridize to human rRNA.

TABLE 58

| HYBRIDIZATION OF BACTERIAL PROBE 2 TO RNAs OF A CROSS SECTION OF PHYLOGENETICALLY DIVERSE ORGANISMS. | | |
|---|---|---|
| Organism | ATCC# | % Probe* Bound |
| Acinetobacter calcoaceticus | 23055 | 76 |
| Bacillus subtilis | 6051 | 75 |
| Bacteroides fragilis | 23745 | 2.0 |
| Branhamella catarrhalis | 25238 | 70 |
| Campylobacter jejuni | 33560 | 2.5 |
| Chlamydia trachomatis | VR878 | 16 |
| Chromobacterium violaceum | 29094 | 61 |
| Clostridium perfringens | 13124 | 66 |
| Corynebacterium xerosis | 373 | 3.8 |
| Deinococcus radiodurans | 35073 | 6.0 |
| Derxia gummosa | 15994 | 61 |
| Gardnerella vaginalis | 14018 | 2.0 |
| Hafnia alvei | 13337 | 72 |
| Lactobacillus acidophilus | 4356 | 50 |
| Moraxella osloensis | 19976 | 64 |
| Mycobacterium smegmatis | 14468 | 19 |

(continued)

| HYBRIDIZATION OF BACTERIAL PROBE 2 TO RNAs OF A CROSS SECTION OF PHYLOGENETICALLY DIVERSE ORGANISMS. | | |
|---|---|---|
| Organism | ATCC# | % Probe* Bound |
| Mycoplasma hominis | 14027 | 34 |
| Neisseria gonorrhoeae | 19424 | 71 |
| Rahnella aquatilis | 33071 | 77 |
| Rhodospirillum rubrum | 11170 | 1.5 |
| Vibrio parahaemolyticus | 17802 | 73 |
| Yersinia enterocolitica | 9610 | 76 |
| Human | | 2.0 |

Table 59 shows that probe 3 hybridizes to the RNA of bacteria commonly found in urine and does not detect yeast rRNA.

TABLE 59

| HYBRIDIZATION OF BACTERIAL PROBE 3 TO RNA OF ORGANISMS FOUND IN URINE. | | |
|---|---|---|
| Organism | ATCC# | % Probe* Bound |
| Candida albicans | 18804 | 1.4 |
| Candida krusei | 34135 | 1.5 |
| Candida parapsilosis | 22019 | 2.2 |
| Candida tropical is | 750 | 2.6 |
| Citrobacter freundii | 8090 | 79 |
| Enterobacter aerogenes | 13048 | 40 |
| Enterobacter cloacae | 13047 | 44 |
| Escherichia coli | 11775 | 67 |
| Klebsiella oxytoca | 13182 | 38 |
| Klebsiella pneumoniae | 13883 | 45 |
| Morganella morganii | 25830 | 57 |
| Proteus mirabilis | 29906 | 40 |
| Proteus vulgaris | 13315 | 51 |
| Providencia stuartii | 29914 | 54 |
| Pseudomonas aeruginosa | 10145 | 61 |
| Pseudomonas fluorescens | 13525 | 56 |
| Serratia marcescens | 13880 | 54 |
| Staphylococcus aureus | 12600 | 37 |
| Staphylococcus epidermidis | 14990 | 20 |
| Streptococcus agalactiae | 13813 | 34 |
| Streptococcus faecalis | 19433 | 20 |
| Streptococcus faecium | 19434 | 47 |
| Torulopsis glabrata | 2001 | 1.9 |
| Ureaplasma urealyticum | 27618 | 26 |

Table 60 shows that probe 3 detects phylogenetically diverse bacteria and does not hybridize to human rRNA.

TABLE 60

| HYBRIDIZATION OF BACTERIAL PROBE 3 TO RNAs OF A CROSS SECTION OF PHYLOGENETICALLY DIVERSE ORGANISMS. | | |
|---|---|---|
| Organism Name | ATCC# | % Probe Bound |
| Acinetobacter calcoaceticus | 23055 | 69 |
| Bacillus subtilis | 6051 | 35 |

(continued)

| HYBRIDIZATION OF BACTERIAL PROBE 3 TO RNAs OF A CROSS SECTION OF PHYLOGENETICALLY DIVERSE ORGANISMS. | | |
|---|---|---|
| Organism Name | ATCC# | % Probe Bound |
| Bacteroides fragilis | 23745 | 1.2 |
| Branhamella catarrhalis | 25238 | 43 |
| Campylobacter jejuni | 33560 | 55 |
| Chlamydia trachomatis | VR878 | 42 |
| Chromobacterium violaceum | 29094 | 69 |
| Clostridium perfringens | 13124 | 62 |
| Corynebacterium xerosis | 373 | 23 |
| Deinococcus radiodurans | 35073 | 30 |
| Derxia gummosa | 15994 | 67 |
| Gardnerella vaginalis | 14018 | 40 |
| Hafnia alvei | 13337 | 56 |
| Lactobacillus acidophilus | 4356 | 36 |
| Moraxella osloensis | 19976 | 64 |
| Mycobacterium smegmatis | 14468 | 77 |
| Mycoplasma hominis | 14027 | 1.5 |
| Neisseria gonorrhoeae | 19424 | 26 |
| Rahnella aquatilis | 33071 | 66 |
| Rhodospirillum rubrum | 11170 | 51 |
| Vibrio parahaemolyticus | 17802 | 68 |
| Yersinia enterocolitica | 9610 | 68 |
| Human | | 0.9 |

Table 61 shows that probe 4 hybridizes to the RNA of bacteria commonly found in urine and does not detect yeast rRNA.

TABLE 61

| HYBRIDIZATION OF BACTERIAL PROBE 4 TO RNA OF ORGANISMS FOUND IN URINE. | | |
|---|---|---|
| Organism Organism | ATCC# | % Probe Bound |
| Candida albicans | 18804 | 4.5 |
| Candida krusei | 34135 | 2.5 |
| Candida parapsilosis | 22019 | 2.7 |
| Candida tropicalis | 750 | 2.5 |
| Citrobacter freundii | 8090 | 55 |
| Enterobacter aerogenes | 13048 | 52 |
| Enterobacter cloacae | 13047 | 57 |
| Escherichia coli | 11775 | 70 |
| Klebsiella oxytoca | 13182 | 70 |
| Klebsiella pneumoniae | 13883 | 43 |
| Morganella morganii | 25830 | 74 |
| Proteus mirabilis | 29906 | 74 |
| Proteus vulgaris | 13315 | 73 |
| Providencia stuartii | 29914 | 73 |
| Pseudomonas aeruginosa | 10145 | 76 |
| Pseudomonas fluorescens | 13525 | 79 |
| Serratia marcescens | 13880 | 74 |
| Staphylococcus aureus | 12600 | 73 |
| Staphylococcus epidermidis | 14990 | 73 |
| Streptococcus agalactiae | 13813 | 70 |

(continued)

| HYBRIDIZATION OF BACTERIAL PROBE 4 TO RNA OF ORGANISMS FOUND IN URINE. | | |
|---|---|---|
| Organism Organism | ATCC# | % Probe Bound |
| Streptococcus faecalis | 19433 | 37 |
| Streptococcus faecium | 19434 | 63 |
| Torulopsis glabrata | 2001 | 2.2 |
| Ureaplasma urealyticum | 27618 | 43 |

Table 62 shows that probe 4 detects phylogenetically diverse bacteria and does not hybridize to human rRNA.

TABLE 63

| HYBRIDIZATION OF BACTERIAL PROBE 4 TO RNAs OF A CROSS SECTION OF PHYLOGENETICALLY DIVERSE ORGANISMS | | |
|---|---|---|
| Organism Name | ATCC# | % Probe Bound |
| Acinetobacter calcoaceticus | 23055 | 69 |
| Bacillus subtilis | 6051 | 55 |
| Bacteroides fragilis | 23745 | 3.0 |
| Branhamella catarrhalis | 25238 | 59 |
| Campylobacter jejuni | 33560 | 65 |
| Chlamydia trachomatis | VR878 | 50 |
| Chromobacterium violaceum | 29094 | 61 |
| Clostridium perfringens | 13124 | 57 |
| Corynebacterium xerosis | 373 | 9.5 |
| Deinococcus radiodurans | 35073 | 63 |
| Derxia gummosa | 15994 | 65 |
| Gardnerella vaginalis | 14018 | 57 |
| Hafnia alvei | 13337 | 67 |
| Lactobacillus acidophilus | 4356 | 68 |
| Moraxella osloensis | 19976 | 68 |
| Mycobacterium smegmatis | 14468 | 28 |
| Mycoplasma hominis | 14027 | 74 |
| Neisseria gonorrhoeae | 19424 | 76 |
| Rahnella aquatilis | 33071 | 68 |
| Rhodospirillum rubrum | 11170 | 59 |
| Vibrio parahaemolyticus | 17802 | 75 |
| Yersinia enterocolitica | 9610 | 74 |
| Human | | 2.8 |

Table 63 shows that probe 5 hybridizes to the RNA of bacteria commonly found in urine and does not detect yeast rRNA.

TABLE 63

| HYBRIDIZATION OF BACTERIAL PROBE 5 TO RNA OF ORGANISMS FOUND IN URINE. | | |
|---|---|---|
| Organism | ATCC# | % Probe Bound |
| Candida albicans | 18804 | 1.8 |
| Candida krusei | 34135 | 1.7 |
| Candida parapsilosis | 22019 | 2.2 |
| Candida tropicalis | 750 | 1.8 |
| Citrobacter freundii | 8090 | 39 |
| Enterobacter aerogenes | 13048 | 38 |

(continued)

| HYBRIDIZATION OF BACTERIAL PROBE 5 TO RNA OF ORGANISMS FOUND IN URINE. | | |
|---|---|---|
| Organism | ATCC# | % Probe Bound |
| Enterobacter cloacae | 13047 | 43 |
| Escherichia coli | 11775 | 31 |
| Klebsiella oxytoca | 13182 | 38 |
| Klebsiella pneumoniae | 13883 | 66 |
| Morganella morganii | 25830 | 50 |
| Proteus mirabilis | 29906 | 44 |
| Proteus vulgaris | 13315 | 52 |
| Providencia stuartii | 29914 | 44 |
| Pseudomonas aeruginosa | 10145 | 47 |
| Pseudomonas fluorescens | 13525 | 25 |
| Serratia marcescens | 13880 | 35 |
| Staphylococcus aureus | 12600 | 26 |
| Staphylococcus epidermidis | 14990 | 37 |
| Streptococcus agalactiae | 13813 | 29 |
| Streptococcus faecalis | 19433 | 14 |
| Streptococcus faecium | 19434 | 33 |
| Torulopsis glabrata | 2001 | 2.2 |
| Ureaplasma urealyticum | 27618 | 73 |

Table 64 shows that probe 5 detects phylogenetically diverse bacteria and does not hybridize to human RNA.

TABLE 64

| HYBRIDIZATION OF BACTERIAL PROBE 5 TO RNAs OF A CROSS SECTION OF PHYLOGENETICALLY DIVERSE ORGANISMS | | |
|---|---|---|
| Organism | ATCC# | % Probe Bound |
| Acinetobacter calcoaceticus | 23055 | 20 |
| Bacillus subtilis | 6051 | 53 |
| Bacteroides fragilis | 23745 | 44 |
| Branhamella catarrhalis | 25238 | 22 |
| Campylobacter jejuni | 33560 | 35 |
| Chromabacterium violaceum | 29094 | 59 |
| Clostridium perfringens | 13124 | 63 |
| Corynebacterium xerosis | 373 | 1.7 |
| Deinococcus radiodurans | 35073 | 5.7 |
| Derxia gummosa | 15994 | 14 |
| Gardnerella vaginalis | 14018 | 1.6 |
| Hafnia alvei | 13337 | 44 |
| Lactobacillus acidophilus | 4356 | 1.5 |
| Moraxella osloensis | 19976 | 7.2 |
| Mycobacterium smegmatis | 14468 | 39 |
| Mycoplasma hominis | 14027 | 21 |
| Neisseria gonorrhoeae | 19424 | 40 |
| Rahnella aquatilis | 33071 | 55 |
| Rhodospirillum rubrum | 11170 | 17 |
| Vibrio parahaemolyticus | 17802 | 66 |
| Yersinia enterocolitica | 9610 | 64 |
| Human | | 1.6 |

Table 65 shows that probe 6 hybridizes to the RNA of bacteria commonly found in urine and does not detect yeast rRNA.

TABLE 65

| HYBRIDIZATION OF BACTERIAL PROBE 6 TO RNA OF ORGANISMS FOUND IN URINE | | |
|---|---|---|
| Organism | ATCC# | % Probe Bound |
| Candida albicans | 18804 | 3.0 |
| Candida krusei | 34135 | 2.0 |
| Candida parapsilosis | 22019 | 2.2 |
| Citrobacter freundii | 8090 | 54 |
| Enterobacter aerogenes | 13048 | 50 |
| Enterobacter cloacae | 13047 | 58 |
| Escherichia coli | 11775 | 63 |
| Klebsiella oxytoca | 13182 | 54 |
| Klebsiella pneumoniae | 13883 | 55 |
| Morganella morganii | 25830 | 60 |
| Proteus mirabilis | 29906 | 64 |
| Proteus vulgaris | 13315 | 67 |
| Providencia stuartii | 29914 | 64 |
| Pseudomonas aeruginosa | 10145 | 65 |
| Pseudomonas fluorescens | 13525 | 31 |
| Serratia marcescens | 13880 | 67 |
| Staphylococcus aureus | 12600 | 53 |
| Staphylococcus epidermidis | 14990 | 34 |
| Streptococcus agalactiae | 13813 | 31 |
| Streptococcus faecium | 19434 | 18 |
| Torulopsis glabrata | 2001 | 2.5 |

Table 66 shows that probe 6 detects some phylogenetically diverse bacteria and does not hybridize to human rRNA.

TABLE 66

| HYBRIDIZATION OF BACTERIAL PROBE 5 TO RNAs OF A CROSS SECTION OF PHYLOGENETICALLY DIVERSE ORGANISMS. | | |
|---|---|---|
| Organism | ATCC# | % Probe Bound |
| Acinetobacter calcoaceticus | 23055 | 73 |
| Bacteroides fragilis | 23745 | 7.0 |
| Branhamella catarrhalis | 25238 | 4.0 |
| Deinococcus radiodurans | 35073 | 5.5 |
| Derxia gummosa | 15994 | 3.0 |
| Gardnerella vaginalis | 14018 | 2.0 |
| Hafnia alvei | 13337 | 3.5 |
| Lactobacillus acidophilus | 4356 | 17 |
| Moraxella osloensis | 19976 | 62 |
| Mycoplasma hominis | 14027 | 44 |
| Rahnella aquatilis | 33071 | 56 |
| Yersinia enterocolitica | 9610 | 50 |
| Human | | 4.0 |

Table 67 shows that probe 7 hybridizes to the RNA of bacteria commonly found in urine and does not detect yeast rRNA.

TABLE 67

| HYBRIDIZATION OF BACTERIAL PROBE 7 TO RNA OF ORGANISMS FOUND IN URINE | | |
|---|---|---|
| Organism | ATCC# | % Probe Bound |
| Candida albicans | 18804 | 2.1 |
| Candida krusei | 34135 | 2.0 |
| Candida tropicalis | 750 | 2.2 |
| Citrobacter freundii | 8090 | 67 |
| Enterobacter aerogenes | 13048 | 69 |
| Enterobacter cloacae | 13047 | 78 |
| Escherichia coli | 11775 | 75 |
| Klebsiella oxytoca | 13882 | 79 |
| Klebsiella pneumoniae | 13883 | 77 |
| Morganella morganii | 25830 | 76 |
| Proteus mirabilis | 29906 | 77 |
| Proteus vulgaris | 13315 | 79 |
| Providencia stuartii | 29914 | 64 |
| Pseudomonas aeruginosa | 10145 | 76 |
| Pseudomonas fluorescens | 13525 | 78 |
| Serratia marcescens | 13880 | 66 |
| Staphylococcus aureus | 12600 | 71 |
| Staphylococcus epidermidis | 14990 | 75 |
| Streptococcus agalactiae | 13813 | 70 |
| Streptococcus faecalis | 19433 | 58 |
| Streptococcus faecium | 19434 | 68 |
| Torulopsis glabrata | 2001 | 2.4 |
| Ureaplasma urealyticum | 27618 | 21 |

Table 68 shows that probe 7 detects phylogenetically diverse bacteria and does not hybridize to human rRNA.

TABLE 68

| HYBRIDIZATION OF BACTERIAL PROBE 7 TO RNAs OF A CROSS SECTION OF PHYLOGENETICALLY DIVERSE ORGANISMS | | |
|---|---|---|
| Organism | ATCC# | % Probe Bound |
| Acinetobacter calcoaceticus | 23055 | 86 |
| Bacillus subtilis | 6051 | 83 |
| Bacteroides fragilis | 23745 | 69 |
| Branhamella catarrhalis | 25238 | 74 |
| Campylobacter jejuni | 33560 | 5.3 |
| Chlamydia trachomatis | VR878 | 41 |
| Chromobacterium violaceum | 29094 | 69 |
| Clostridium perfringens | 13124 | 68 |
| Corynebacterium xerosis | 373 | 23 |
| Deinococcus radiodurans | 35073 | 70 |
| Derxia gummosa | 15994 | 69 |
| Gardnerella vaginalis | 14018 | 68 |
| Hafnia alvei | 13337 | 77 |
| Moraxella osloensis | 19976 | 68 |
| Mycobacterium smegmatis | 14468 | 64 |
| Mycoplasma hominis | 14027 | 4.0 |
| Neisseria gonorrhoeae | 19424 | 53 |

(continued)

| HYBRIDIZATION OF BACTERIAL PROBE 7 TO RNAs OF A CROSS SECTION OF PHYLOGENETICALLY DIVERSE ORGANISMS | | |
|---|---|---|
| Organism | ATCC# | % Probe Bound |
| Rahnella aquatilis | 33071 | 72 |
| Rhodospirillum rubrum | 11170 | 73 |
| Vibrio parahaemolyticus | 17802 | 67 |
| Yersinia enterocolitica | 9610 | 66 |
| Human | | 2.2 |

Example 20

Fungi encompass a morphologically and physiologically diverse group of simple eucaryotic organisms. We estimate, using published sequences of three fungi, Neurospora crassa, Podospora, and Saccharomyces, that the rRNA of fungi are 58-60% homologous to E. coli and 84-90% homologous to one another. Some fungi grow as single cells (yeasts), others as multinuclear filaments (molds) and still others can grow as either single cells or multicellular filaments (dimorphic fungi). Although many fungi are harmless inhabitants of their environments, others are harmful and cause disease. The presence of any fungi in some locations is undesirable or indicative of disease (e.g., culture media, pharmaceutical products, body fluids such as blood, urine or cerebrospinal fluid, and tissue biopsies). Low levels of fungi are considered acceptable in other products such as drinking water and food products. This has created the need for a means of detecting and quantitating fungi in a sample.

The current methods for detecting and quantifying fungi involve microscopic examination of samples and culture on different media. Although most yeasts can be grown from clinical samples in a matter of days, some filamentous fungi take up to four weeks culture time, after which special staining procedures, biochemical analysis and antigen tests are performed. The oligonucleotide sequences below, when used in a hybridization assay, detect the five yeasts most commonly isolated in the clinical setting, Candida albicans, Torulopsis glabrata, Candida tropicalis, Candida parapsilosis and Candida krusei. Five other fungi representing the Trichosporon, Blastomyces, Cryptococcus and Saccharomyces genera are also detected. The present invention allows one step detection of these organisms rnd, in relation to culture, reduces the time to identification or elimination of these fungi as the cause of an infection. This represents a significant improvement over prior art methods.

The four probes vhich hybridize to the organisms of interest were identified using 3 primers complementary to conserved regions on 18S or 28S rRNA. Sequence 1 was obtained using an 18S primer with the sequence 5'-AGA ATT TCA CCT CTG-3'. Sequence 2 was obtained using a 28S primer with the sequence 5'-CCT TCT CCC GAA GTT ACG G-3'. Sequences 3 and 4 were obtained with a 28S primer with the sequence 5'-TTC CGA CTT CCA TGG CCA CCG TCC-3'. The following sequences were characterized and shown to hybridize to fungal rRNA. The sequences of Saccharomyces cerevisiae, Saccharomyces carlsbergensis, Escherichia coli and human rRNA were used for comparison with the sequences of interest.

1. CCC GAC CGT CCC TAT TAA TCA TTA CGA TGG
2. CGA CTT GGC ATG AAA ACT ATT CCT TCC TGT GG
3. GCT CTT CAT TCA ATT GTC CAC GTT CAA TTA AGC AAC AAG G
4. GCT CTG CAT TCA AAC GTC CGC GTT CAA TAA AGA AAC AGG G

Sequence 1, from 18S rRNA, is 30 bases in length and has a Tm of 68°C. Sequence 2, from 235 rRNA, is 32 bases in length and has a Tm of 67°C. Sequence 3, from 23S rRNA, is 40 bases in length and has a Tm of 66°C. Sequence 4, from 23S rRNA, is 40 bases in length and has a Tm of 68°C. Sequence 1 hybridizes in the region corresponding to position 845-880 of Saccharomyces cerevisiae 18S rRNA. Sequence 2 hybridizes in the region corresponding to position 1960-2000 of Saccharomyces cerevisiae 28S rRNA and sequences 3 and 4 hybridize in the region of 1225-1270 of the 28S rRNA.

To demonstrate the reactivity and specificity of these probes for fungal RNA, they were used in hybridization assays. $^{32}P$- or $^{125}I$-labeled oligonucleotide probes were mixed with purified RNA or RNA released from cells by standard lysis techniques in 0.2 ml of 0.48M sodium phosphate pH 6.8, 1% sodium dodecyl sulfate, 1mM EDTA, 1mM EGTA and incubated at 60°C for 2 hours. Following incubation, 5 ml of 2% hydroxyapatite, 0.12 M sodium phosphate pH 6.8, 0.02% sodium dodecyl sulfate was added and the samples incubated 10 minutes at 60°C. The samples were centrifuged and the supernatants removed. Five ml of 0.12M sodium phosphate pH 6.8, 0.02% sodium dodecyl sulfate was added, the samples were mixed, centrifuged and the supernatants removed. The results are shown in Table 69. Probe 1 detects all ten fungi which were tested, probe 2 detects all six of the yeasts which were tested, probe 3 detects five of the six yeasts, and probe 4 detects C. krusei only. Thus probe 4 could be used to detect and identify C. krusei in samples, probe 1, 2 or combination of 3 and 4 could be used to detect the yeasts, and probe 1 could be used to detect any of the ten organisms listed in Table 69.

One potential use for these probes is to identify yeasts in urine samples or other normally sterile body fluids. The probes were hybridized to a panel of bacteria lost commonly isolated from urine and shown not to react (Table 70). Table 71 shows that the probes do not hybridize to phylogenetically diverse bacteria or to human RNA.

TABLE 69

| HYBRIDIZATION OF YEAST PROBES TO YEAST RNA | | | | | |
|---|---|---|---|---|---|
| | | % Probe Bound | | | |
| Organism | ATCC# | #1 | #2 | #3 | #4 |
| Blastomyces dermatitidis | C.I. | 25 | 1.4 | 1.5 | 1.5 |
| Candida albicans | 18804 | 40 | 63 | 56 | 2.0 |
| C. krusei | 34135 | 73 | 62 | 2.2 | 70 |
| C. parapsilosis | 22019 | 71 | 63 | 65 | 2.0 |
| C. tropicalis | 750 | 62 | 71 | 71 | 2.0 |
| Cryptococcus laurentii | C.I. | 43 | 1.4 | 1.5 | 1.5 |
| Cryptococcus neoformans | C.I. | 60 | 1.3 | 1.5 | 1.6 |
| Torulopsis glabrata | 2001 | 61 | 44 | 62 | 2.0 |
| Trichosporon beigelii | C.I. | 57 | 1.3 | 2.1 | 1.5 |
| Saccharomyces cerevisiae | C.I. | 41 | 67 | 53 | 1.9 |
| C.I. = Clinical isolate | | | | | |

TABLE 70

| HYBRIDIZATION OF FUNGAL PROBES 1-4 TO RNA OF ORGANISMS FOUND IN URINE | | | | | |
|---|---|---|---|---|---|
| | | % Probe Bound | | | |
| Organism | ATCC# | #1 | #2 | #3 | #4 |
| Citrobacter freundii | 8090 | 1.5 | 1.7 | 1.5 | 2.1 |
| Enterobacter aerogenes | 13048 | 2.5 | 1.9 | 2.0 | 2.0 |
| Enterobacter cloacae | 13047 | 2.5 | 1.6 | 2.6 | 2.0 |
| Escherichia coli | 11775 | 3.0 | 2.0 | 1.6 | 1.5 |
| Klebsiella oxytoca | 13182 | 2.5 | 2.2 | 2.5 | 2.0 |
| Klebsiella pneumoniae | 13883 | 2.5 | 2.2 | 2.1 | 2.0 |
| Morganella morganii | 25830 | 2.0 | 2.8 | 1.7 | 1.9 |
| Proteus mirabilis | 29906 | 2.5 | 1.9 | 2.3 | 2.0 |
| Proteus vulgaris | 13315 | 2.0 | 2.2 | 2.0 | 1.5 |
| Providencia stuartii | 29914 | 3.0 | 1.7 | 2.8 | 2.0 |
| Pseudomonas aeruginosa | 10145 | 2.0 | 1.9 | 1.3 | 2.0 |
| Pseudomonas fluorescens | 13525 | 2.5 | 2.7 | 2.1 | 2.0 |
| Serratia marcescens | 13880 | 2.5 | 1.7 | 1.8 | 2.0 |
| Staphylococcus aureus | 12600 | 2.0 | 1.7 | 1.8 | 2.0 |
| Staphylococcus epidermidis | 14990 | 3.0 | 1.5 | 1.3 | 2.0 |

(continued)

| HYBRIDIZATION OF FUNGAL PROBES 1-4 TO RNA OF ORGANISMS FOUND IN URINE | | | | | |
|---|---|---|---|---|---|
| | | % Probe Bound | | | |
| Organism | ATCC# | #1 | #2 | #3 | #4 |
| Streptococcus agalactiae | 13813 | 2.5 | 1.9 | 1.3 | 2.5 |
| Streptococcus faecalis | 19433 | 1.7 | 3.3 | 3.5 | 1.9 |
| Streptococcus faecium | 19434 | 2.0 | 2.9 | 2.1 | 1.5 |
| Ureaplasma urealyticum | 27618 | 2.1 | 3.1 | 2.4 | 1.8 |

TABLE 71

| HYBRIDIZATION OF FUNGAL PROBES 1-4 TO RNAs OF A CROSS SECTION OF PHYLOGENETICALLY DIVERSE ORGANISMS | | | | | |
|---|---|---|---|---|---|
| | | % Probe Bound | | | |
| Organism | ATCC# | #1 | #2 | #3 | #4 |
| Acinetobacter calcoaceticus | 23055 | 2.5 | 2.5 | 2.0 | 1.9 |
| Bacillus subtilis | 6051 | 2.0 | 2.8 | 2.4 | 2.4 |
| Bacteroides fragilis | 23745 | 2.0 | 2.2 | 2.5 | 2.3 |
| Branhamella catarrhalis | 25238 | 2.5 | 3.2 | 1.8 | 1.7 |
| Campylobacter jejuni | 33560 | 2.5 | 2.1 | 2.0 | 1.9 |
| Chlamydia trachomatis | VR878 | 3.1 | 3.1 | 1.8 | 2.7 |
| Chromobacterium violaceum | 29094 | 2.5 | 1.7 | 2.0 | 2.2 |
| Clostridium perfringens | 13124 | 1.9 | 2.3 | 1.8 | 1.8 |
| Corynebacterium xerosis | 373 | 1.6 | 4.8 | 1.8 | 1.1 |
| Deinococcus radiodurans | 35073 | 2.0 | 1.6 | 2.1 | 0.8 |
| Derxia gummosa | 15994 | 3.0 | 1.5 | 1.7 | 1.8 |
| Gardnerella vaginalis | 14018 | 2.0 | 2.2 | 1.3 | 1.2 |
| Hafnia alvei | 13337 | 1.0 | 2.5 | 1.7 | 1.6 |
| Lactobacillus acidophilus | 4356 | 2.0 | 2.7 | 2.0 | 1.9 |
| Moraxella osloensis | 19976 | 2.0 | 2.1 | 1.9 | 1.8 |
| Mycobacterium smegmatis | 14468 | 1.6 | 1.8 | 1.8 | 1.7 |
| Mycoplasma hominis | 14027 | 1.5 | 1.8 | 1.6 | 1.5 |
| Neisseria gonorrhoeae | 19424 | 2.0 | 2.7 | 1.6 | 1.6 |
| Rahnella aquatilis | 33071 | 2.0 | 2.7 | 2.3 | 2.1 |
| Rhodospirillum rubrum | 11170 | 2.0 | 1.8 | 1.6 | 1.5 |

(continued)

| HYBRIDIZATION OF FUNGAL PROBES 1-4 TO RNAs OF A CROSS SECTION OF PHYLOGENETICALLY DIVERSE ORGANISMS | | | | | |
|---|---|---|---|---|---|
| | | % Probe Bound | | | |
| Organism | ATCC# | #1 | #2 | #3 | #4 |
| Vibrio parahaemolyticus | 17802 | 2.5 | 3.1 | 1.7 | 1.6 |
| Yersinia enterocolitica | 9610 | 2.0 | 1.8 | 2.3 | 2.2 |
| Human | | 2.0 | 1.8 | 2.1 | 3.0 |

Two derivatives of this probe also were made:
CCCGACCCTCCCTATTAATCATTACGATCCTCCTAGAAAC
CCCGACCGTCCCTATTAATCATTACGATGG
The first derivative works well at 65°C, the second at 60°C.

Example 21

Gonorrhea is one of the most commonly reported bacterial infections in the United States, with over two million cases reported annually. This sexually transmitted disease usually results in anterior urethritis in males and involves the cervix in females. While severe complications and even sterility can occur in untreated individuals, asymptomatic infections are common, resulting in carriers who unknowingly spread the disease.

The causative agent, Neisseria gonorrhoeae, is a gram negative, oxidase positive diplococcus with stringent growth requirements. The method used for diagnosis depends on the site of infection and the patient symptoms. Gonococcal urethritis in males is diagnosed with good sensitivity and specificity using gram stain. Culture, requiring 24-72 hours, usually must be performed to confirm diagnosis of gonorrhea from all females and asymptomatic males. Following the detection of the organism from growth in culture, Neisseria gonorrhoeae must be identified by further tests such as carbohydrate degradation, coagglutination, fluorescent antibody screens or chromogenic enzyme substrate assays.

Neisseria gonorrhoeae is particularly difficult to detect and distinguish using a nucleic acid probe because it is very closely related to N. meningitidis. Data published in Kingsbury, D.T., J. Bacteriol. 94:870-874 (1967) shows a DNA: DNA homology for the two species of approximately 80-94%. Under guidelines established by the Ad Hoc Committee on Reconciliation of Approaches to Bacterial Systematics, Int'l J. System. Bacteriol. 37:463-464 (1987), the phylogenetic definition of a species generally means 70% or greater DNA:DNA homology. Despite the fact that these organisms may be considered to be the same species under established principles, we were able to make probes capable of distinguising them.

As expected, the rRNA homology between N. gonorrhoeae and N. meningitidis is even greater because of known conserved regions. We noted a 1.0% difference between the 16S and a 1.1% difference between the 235 rRNA sequences of N. gonorrhoeae and N. meningitidis using our sequencing data.

Making a probe for N. gonorrhoeae was complicated by the fact that in some sites where N. meningitidis and N. gonorrhoeae differed, other Neisseria species were similar to N. gonorrhoeae. The few mismatches which exist between these two species are in the most variable regions, i.e., regions which vary not only between species, but also from strain to strain. Despite the fact that some believed the species could not be distinguished at all, and others believed that rRNA was too conserved to be useful in probe diagnostics, we were able to make probes capable of differentiating N. gonorrhoeae and and N. meningitidis.

The present invention has significant advantages over each of the prior art methods; the probes are more specific and much faster than culture methods. It also is believed that the probes are more sensitive, (i.e., able to detect a smaller number of organisms in a clinical sample) than prior art methods.

The primers used to identify these probe sequences had the following sequences:
1. GGCCGTTACCCCACCTACTAGCTAAT
2. GTATTACCGCGGCTGCTGGCAC
3. CCTCGTTCCGGGACTTAACCCACCAT

Each of the rRNA sites chosen to target had at least two mismatches to E. coli, N. meningitidis, N. cinerea, N. lactamica, N. mucosa, and Kingella kingae.

Oligonucleotides complementary to sequences adjacent to the probe regions were synthesized and used in the hydridization mix.

The following sequences were characterized and shcwn to be specific for Neisseria gonorrhoeae. The phylogenetically nearest neighbors Neisseria meningitidis, N. lactamica, N. cinerea, N. mucosa, and Kingella kingae were used for comparison with the N. gonorrhoeae sequence.

1. CCG CCG CTA CCC GGT AC
2. TCA TCG GCC GCC GAT ATT GGC
3. GAG CAT TCC GCA CAT GTC AAA ACC AGG TA

Sequence 1, complementary to 16S rRNA in the region 125-150, is 17 bases in length and has a Tm of 56°C. Sequence 2, complementary to 16S rRNA in the region 455-485, is 21 bases in length and has a Tm of 63°C. Sequence 3, complementary to 16S rRNA in the region 980-1015, is 29 bases in length and has a Tm of 57°C.

The reactivity and specificity of the probes for Neisseria gonorrhoeae was demonstrated with a hybridization assay. The three oligonucleotide probes were iodinated and mixed with unlabeled oligonucleotides of sequence 5'-CCC CTG CTT TCC CTC TCT AGA CGT ATG CGG TAT TAG CTG ATC TTT CG-3', 5'-GCC TTT TCT TCC CTG ACA AAA GTC CTT TAC AAC CCG-3', 5'-GGC ACG TAG TTA GCC GGT GCT TAT TCT TCA GGT AC-3', and 5'-GGT TCT TCG CGT TGC ATC GAA TTA ATC CAC ATC ATC CAC CGC-3', and with purified RNA in 0.48 M sodium phosphate, ph6.8, 0.5% sodium dodecyl sulfate (SDS) and incubated at 60°C for one hour. Following incubation, 4 ml of 2% hydroxyapatite, 0.12 M sodium phosphate pH6.8, 0.02% SDS was added and the mixture was incubated at 60°C for 5 minutes. The samples were centrifuged and the supernatants were removed. Five ml of wash solution (0.12 M sodium phosphate pH6.8, 2% SDS) was added and the samples were mixed, centrifuged, and the supernatants removed. The amount of radioactivity bound to the hydroxyapatite was determined in a gamma counter.

Table 72 shows that the probes hybridize well to N. gonorrhoeae RNA and do not hybridize to the other species tested.

TABLE 72

| HYBRIDIZATION OF NEISSERIA GONORRHOEAE PROBES 1-3 TO NEISSERIA AND KINGELLA RNAS | | |
|---|---|---|
| Organisms | ATCC# | % Probe Bound |
| Kingella kingae | 23332 | 0.09 |
| Neisseria cinerea | 14685 | 0.04 |
| N. gonorrhoeae | 19424 | 48.4 |
| N. lactamica | 23970 | 0.07 |
| N. meningitidis serogroup A | 13077 | 0.04 |
| N. meningitidis serogroup B | 13090 | 0.04 |
| N. meningitidis serogroup C | 13102 | 0.04 |
| N. mucosa | 19696 | 0.07 |
| N. subflava | 14799 | 0.05 |

The following derivatives of Neisseria probes also have been made and used:

GAG GAT TCC GCA CAT GTC AAA ACC AGG

GAG GAT TCC GCA CAT GTC AAA ACC AGG TAA

CCC GCT ACC CGG TAC GTT C

CCG CTA CCC GGT ACG TTC.

Although the above examples of performance were determined using the standard assay format previously described, the specific probes may be used under a wide variety of experimental conditions. For example, additives may be included to the reaction solutions to provide optimal reaction conditions for accelerated hybridization. Such additives may include buffers, chelators, organic compounds and nucleic acid precipitating agents such as detergents, dihydroxybenzene, sodium dodecyl sulfate, sodium diisobutyl sulfosuccinate, sodium tetradecyl sulfate, sarkosyl and the alkali metal salts and ammonium salts of $(SO_4)^{2-}$, $(PO_4)^{3-}$, $Cl^-$ and $HCOO^-$. Such additives can be utilized by one skilled in the art to provide optimal conditions for the hybridization reaction to take place. These conditions for accelerated hybridization of single-stranded nucleic acid molecules into double-stranded molecules are the subject of EP-A-0167366 and EP-A-0229442.

The present invention can be carried out on nonviral organisms from purified samples or unpurified clinical samples such as sputum, feces, tissue, blood, spinal or synovial fluids serum, urine or other bodily fluids, or other samples such as environmental or food samples. Prior to cell breakage and hybridization, the cells can be suspended or placed in solution. In the case of the unpurified samples referred to above, the cells may remain intact and untreated in their own biological environment prior to the assay.

The probes of the present invention may be used in an assay either alone or in combination with different probes. Several individual probes also can be linked together during nucleic acid synthesis. This results in one probe molecule which contains multiple probe sequences, and therefore, multiple specificities. For example, a single nucleic acid molecule can be synthesized which contains both the Mycobacterium avium and the Mycobacterium intracellulare sequences described in Examples 1 and 2. When hybridized with either M.avium or M. intracellulare rRNA this probe will hybridize completely. If the two probe sequences were combined separately in an assay only one half of the mixed individual probes will hybridize with either M.avium or M. intracellulare rRNA. Other embodiments also may be practiced within the scope of the claims. For example, probes may be labelled using a variety of labels, as described within, and may be incorporated into diagnostic kits.

## Claims

1. A method for preparing a probe for use in a qualitative or quantitative hybridization assay which comprises constructing an oligonucleotide that is sufficiently complementary to hybridize under hybridization conditions to an rRNA sequence selected to be unique to a non-viral organism or group of non-viral organisms sought to be detected, said oligonucleotide being selected by:

    a) identifying one or more candidate variable regions by comparing one or more variable region rRNA sequences from a non-viral target organism or group of non-viral target organisms with one or more variable region rRNA sequences from the known nearest related organism to select a sequence unique to the rRNA of the target organism or organisms; and
    b) synthesizing an oligonucleotide complementary to the unique rRNA sequence,

    wherein the thermal stability of probe:target nucleic acid hybrids is greater than the thermal stability of probe:nontarget nucleic acid hybrids;
    wherein said target organism or organisms are selected from one or more species in a genus or one or more genera in a family.

2. The method of claim 1 wherein said region of rRNA is selected to have at least about a one base sequence difference from a corresponding rRNA sequence of the known nearest related organism to said non-viral organism or group of non-viral organisms sought to be detected.

3. The method of claim 1 wherein said region of rRNA is selected to have at least about a 10% or greater base sequence difference from the corresponding rRNA sequence of the known nearest related organism to said non-viral organism or group of non-viral organisms sought to be detected.

4. The method of claim 1 wherein said region of rRNA is chosen from the group consisting of 5S, 16S, and 23S rRNA.

5. The method of claim 1 wherein said region of rRNA is chosen from the group consisting of 5.0S, 5.8S, 18S and 28S rRNA.

6. The method of claim 1 wherein said oligonucleotide is at least about 10 nucleotides in length.

7. The method of claim 1 wherein said oligonucleotide is at least about 15 nucleotides in length.

8. The method of claim 1 wherein said oligonucleotide is at least about 20 nucleotides in length.

9. The method of claim 1 wherein said oligonucleotide is at least about 30 nucleotides in length.

10. The method of claim 1 wherein said oligonucleotide is about 20 nucleotides to about 50 nucleotides in length.

11. The method of claim 1 wherein said oligonucleotide is about 30 nucleotides to about 50 nucleotides in length.

**12.** The method of claim 2 wherein said oligonucleotide is at least about 10 nucleotides in length.

**13.** The method of claim 2 wherein said oligonucleotide is at least about 15 nucleotides in length.

**14.** The method of claim 2 wherein said oligonucleotide is at least about 20 nucleotides in length.

**15.** The method of claim 2 wherein said oligonucleotide is at least about 3 0 nucleotides in length.

**16.** The method of claim 2 wherein said oligonucleotide is about 20 nucleotides to about 50 nucleotides in length.

**17.** The method of claim 2 wherein said oligonucleotide is about 30 nucleotides to about 50 nucleotides in length.

**18.** The method of claim 2 wherein said oligonucleotide is at least about 10 nucleotides in length.

**19.** The method of claim 3 wherein said oligonucleotide is at least about 15 nucleotides in length.

**20.** The method of claim 3 wherein said oligonucleotide is at least about 20 nucleotides in length.

**21.** The method of claim 3 wherein said oligonucleotide is at least about 30 nucleotides in length.

**22.** The method of claim 3 wherein said oligonucleotide is about 20 nucleotides to about 50 nucleotides in length.

**23.** The method of claim 3 wherein said oligonucleotide is about 30 nucleotides to about 50 nucleotides in length.

**24.** The method of claim 1 wherein said probe is at least about 75% complementary to said region of rRNA.

**25.** The method of claim 2 wherein said oligonucleotide is at least about 75% complementary to said region of rRNA.

**26.** The method of claim 3 wherein said oligonucleotide is at least about 75% complementary to said region of rRNA.

**27.** The method of claim 1 wherein said probe is perfectly complementary to said region of rRNA.

**28.** The method of claim 2 wherein said probe is perfectly complementary to said region of rRNA.

**29.** The method of claim 3 wherein said probe is perfectly complementary to said region of rRNA.

**30.** A method for preparing a probe or combination of probes for use in a qualitative or quantitative hybridization assay which comprises constructing a nucleotide polymer that is sufficiently complementary to hybridize to a region of rDNA or rRNA selected to distinguish a target non-viral organism or first group of non-viral organisms sought to be detected from at least one nontarget organism or second group of nontarget organisms which may be present in a sample, wherein said nontarget organisms or group of organisms are close phylogenetic relatives of said target organisms or group of organisms, said region of rDNA or rRNA being selected by:

- comparing one or more rDNA or rRNA nucleotide base sequences of said non-viral organism or group of non-viral organisms sought to be detected with one or more rDNA or rRNA nucleotide base sequences of said nontarget organisms or group of nontarget organisms;
- aligning said rDNA or rRNA nucleotide base sequences of said non-viral organism or group of non-viral organisms with said rDNA or rRNA nucleotide base sequences of said nontarget organisms or group of organisms so as to identify regions of homology; and
- selecting said nucleotide polymer by maximizing the homology of said nucleotide polymer to the regions of said rDNA or rRNA of said non-viral organism or non-viral group of organisms sought to be detected while minimizing the homology of said nucleotide polymer to rDNA or rRNA sequences of said nontarget organism or group of organisms sought to be distinguished therefrom; wherein said organism or group of organisms sought to be detected is selected from one or more species in a genus or one or more genera in a family.

**31.** The method of claim 30 wherein said nucleotide polymer is at least about 90% homologous to the regions of said rDNA or rRNA of said non-viral organism or non-viral group of organisms sought to be detected.

**32.** The method of claim 30 wherein said probe oligonucleotide is less than about 90% homologous to rDNA or rRNA sequences of the close phylogenetic relatives sought to be distinguished therefrom.

**33.** The method of any of claims 30 to 32 comprising the further step of verifying said probe non-cross reactivity by hybridizing said probe oligonucleotide to non-viral organisms or groups of non-viral organisms sought to be distinguished by said probe.

**34.** A method for preparing a probe for use in a qualitative or quantitative hybridization assay which comprises constructing an oligonucleotide that is sufficiently complementary to hybridize to a region of rDNA or rRNA selected to be unique to a non-viral organism or group of non-viral organisms sought to be detected, said region of rDNA or rRNA being selected by:

- comparing one or more rDNA or rRNA nucleotide base sequences of said non-viral organism or group of non-viral organisms sought to be detected with one or more rDNA or rRNA nucleotide base sequences of its closest phylogenetic relatives;
- aligning said rDNA or rRNA nucleotide base sequences of said nonviral organism or group of non-viral organisms with said rDNA or rRNA sequences of said closest phylogenetic relatives, so as to reveal the interspecies hypervariable rDNA or rRNA regions;
- selecting said probe oligonucleotide in said interspecies hypervariable region by maximizing the homology of said probe oligonucleotide to the regions of said rDNA or rRNA of said non-viral organism or non-viral group of organisms sought to be detected while minimizing the homology of said probe oligonucleotide to rDNA or rRNA sequences of said closest phylogenetic relatives sought to be distinguished therefrom;

wherein said organism or group of organisms sought to be detected is selected from one or more species in a genus or one or more genera in a family.

**35.** The method of claim 34 wherein said probe oligonucleotide is at least about 90% homologous to the regions of said rDNA or rRNA of said non-viral organism or non-viral group of organisms sought to be detected.

**36.** The method of claim 34 wherein said probe oligonucleotide is less than about 90% homologous to rDNA or rRNA sequences of said closest phylogenetic relatives sought to be distinguished therefrom.

**37.** The method of any of claims 34 to 36 comprising the further step of verifying said probe non-cross reactivity by hybridizing said probe oligonucleotide to non-viral organisms or groups of non-viral organisms sought to be distinguished by said probe.

**38.** A nucleotide polymer hybridisation assay probe for a non-viral organism or a group of non-viral organisms in a substantially isolated form which comprises an oligonucleotide of from 10 to 100 nucleotides able to hybridise under stringent hybridisation conditions to at least 10 nucleotides in a variable region of rRNA or rDNA selected to be unique to said non-viral organism or group of non-viral organisms, wherein said probe hybridises under stringent hybridisation conditions to nucleic acid from one species of organism in a genus and fails to hybridise under said conditions to nucleic acid from other species in said genus, or wherein said probe hybridises under stringent hybridising conditions to nucleic acid from one or more members of said group of organisms and fails to hybridise to nucleic acid from organisms which are not members of said group, wherein said variable region corresponds to a region selected from the group consisting of:

bases 60-100, 120-150, 600-670, 820-860, 980-1050 and 1250-1290 of E. coli 16S rRNA; 270-390, 535-560, 1150-1200, 1440-1600, 1710-1750 and 2190-2330 of E. coli 23S rRNA;
bases 330-365 of E. coli 16S rRNA, where said nucleotide polymer hybridises under stringent conditions to species of the phylogenetic group bacteria;
bases 675-715 of E. coli 16S rRNA where said nucleotide polymer hybridises under stringent conditions to species of the phylogenetic group bacteria;
bases1080-1110 of E. coli 16S rRNA where said nucleotide polymer hybridises under stringent conditions to species of the phylogenetic group bacteria;
bases 460-490 of E. coli 23S rRNA, where said nucleotide polymer hybridises under stringent conditions to species of the phylogenetic group bacteria
bases1050-1080 of E. coli 23S rRNA where said nucleotide polymer hybridises under stringent conditions to species of the phylogenetic group bacteria

bases 1900-1960 of E. coli 23S rRNA where said nucleotide polymer hybridises under stringent conditions to species of the phylogenetic group bacteria;
bases 845-880 of S. cerevisiae 18 rRNA where said nucleotide polymer hybridises under stringent conditions to species of the phylogenetic group fungi;
bases 1960-2000 of S. cerevisiae 28S rRNA where said nucleotide polymer hybridises under stringent conditions to species of the phylogenetic group fungi
bases 1225-1270 of S. cerevisiae 28S rRNA where said nucleotide polymer hybridises under stringent conditions to species of the phylogenetic group fungi;
bases 185-225 of E. coli 16S rRNA, where said nucleotide polymer hybridises under stringent conditions to Mycobacterium avium;
bases 185-225 of E. coli 16S rRNA, where said nucleotide polymer hybridises under stringent conditions to Mycobacterium intracellulare;
bases 185-225 of E. coli 16S rRNA, where said nucleotide polymer hybridises under stringent conditions to species of the Mycobacterium tuberculosis complex bacteria;
bases 540-575 of E. coli 23S rRNA, where said nucleotide polymer hybridises under stringent conditions to species of the Mycobacterium tuberculosis complex bacteria;
bases 1155-1190 of E. coli 23S rRNA, where said nucleotide polymer hybridises under stringent conditions to species of the Mycobacterium tuberculosis complex bacteria;
bases 2195-2235 of E. coli 23S rRNA, where said nucleotide polymer hybridises under stringent conditions to species of the Mycobacterium tuberculosis complex bacteria;
bases 1025-1060 of E. coli 16S rRNA, where said nucleotide polymer hybridises under stringent conditions to species of the genus Mycobacterium;
bases 1440-1475 of E. coli 23S rRNA, where said nucleotide polymer hybridises under stringent conditions to species of the genus Mycobacterium;
bases 1515-1555 of E. coli 23S rRNA, where said nucleotide polymer hybridises under stringent conditions to species of the genus Mycobacterium;
bases 1570-1610 of E. coli 23S rRNA where said nucleotide polymer hybridises under stringent conditions to species of the genus Mycobacterium;
bases 190-230 of E. coli 16S rRNA, where said nucleotide polymer hybridises under stringent conditions to Mycoplasma pneumoniae:
bases 450-490 of E. coli 16S rRNA, where said nucleotide polymer hybridises under stringent conditions to Mycoplasma pneumoniae;
bases 820-860 of E. coli 16S rRNA where said nucleotide polymer hybridises under stringent conditions to Mycoplasma pneumoniae:
bases 1255-1290 of E. coli 16S rRNA where said nucleotide polymer hybridises under stringent conditions to Mycoplasma pneumoniae;
bases 65-120 of E. coli 5S rRNA where said nucleotide polymer hybridises under stringent conditions to Mycoplasma pneumoniae;
bases 630-675 of E. coli 16S rRNA where said nucleotide polymer hybridises under stringent conditions to species of the genus Legionella:
bases 975-1020 of E. coli 16S rRNA where said nucleotide polymer hybridises under stringent conditions to species of the genus Legionella;
bases 350-395 of E. coli 23S rRNA, where said nucleotide polymer hybridises under stringent conditions to species of the genus Legionella
bases 1585-1620 of E. coli 23S rRNA where said nucleotide polymer hybridises under stringent conditions to species of the genus Legionella;
bases 2280-2330 of E. coli 23S rRNA where said nucleotide polymer hybridises under stringent conditions to species of the genus Legionella;
bases 60-105, of E. coli 16S rRNA where said nucleotide polymer hybridises under stringent conditions to Chlamydia trachomatis;
bases 600-635 of E. coli 16S rRNA where said nucleotide polymer hybridises under stringent conditions to Chlamydia trachomatis;
bases 830-870 of E. coli 16S rRNA where said nucleotide polymer hybridises under stringent conditions to Chlamydia trachomatis;
bases 275-320 of E. coli 23S rRNA where said nucleotide polymer hybridises under stringent conditions to Chlamydia trachomatis;
330-365 of E. coli 23S rRNA where said nucleotide polymer hybridises under stringent conditions to Chlamydia trachomatis;

1160-1190 of E. coli 23S rRNA where said nucleotide polymer hybridises under stringent conditions to Chlamydia trachomatis;
1450-1490, of E. coli 23S rRNA where said nucleotide polymer hybridises under stringent conditions is Chlamydia trachomatis;
1510-1545 of E. coli 23S rRNA where said nucleotide polymer hybridises under stringent conditions to Chlamydia trachomatis;
1710-1750 of E. coli 23S rRNA where said nucleotide polymer hybridises under stringent conditions to Chlamydia trachomatis;
bases 980-1010 of E. coli 16S rRNA where said nucleotide polymer hybridises under stringent conditions to species of the genus Campylobacter;
bases 825-860 of E. coli 16S rRNA, where said nucleotide polymer hybridises under stringent conditions to species of the sub-generic group Streptococci known as Enterococci;
bases 365-405 of E. coli 23S rRNA, where said nucleotide polymer hybridises under stringent conditions to species of the sub-generic grouping known as group 1 Pseudomonas;
bases 305-340 of E. coli 16S rRNA, where said nucleotide polymer hybridises under stringent conditions to Enterobacter cloacae;
bases 270-305 of E. coli 23S rRNA, where said nucleotide polymer hybridises under stringent conditions to Proteus mirabilis;
bases 1125-1155 of E. coli 16S rRNA where the group of organisms belongs to the genus Salmonella;
bases 335-375 of E. coli 23S rRNA where said nucleotide polymer hybridises under stringent conditions to species of the genus Salmonella;
bases 995-1030 of E. coli 16S rRNA where said nucleotide polymer hybridises under stringent conditions to Escherichia coli;
bases 125-150 of E. coli 16S rRNA where the organism is Neisseria gonorrhoeae,
bases 455-485 of E. coli 16S rRNA where said nucleotide polymer hybridises under stringent conditions to Neisseria gonorrhoeae;
bases 980-1015 of E. coli 16S rRNA where said nucleotide polymer hybridises under stringent conditions to Neisseria gonorrhoeae;
or the complement thereof.

**39.** A nucleotide polymer hybridisation assay probe for a non-viral organism or a group of non-viral organisms in a substantially isolated form which comprises an oligonucleotide of from 10 to 100 nucleotides able to hybridise under stringent hybridisation conditions to at least 10 nucleotides in a variable region of rRNA or rDNA selected to be unique to said non-viral organism or group of non-viral organisms, wherein said probe hybridises under stringent hybridisation conditions to nucleic acid from one species of organism in a genus and fails to hybridise under said conditions to nucleic acid from other species in said genus, or wherein said probe hybridises under stringent hybridising conditions to nucleic acid from one or more members of said group of organisms and fails to hybridise to nucleic acid from organisms which are not members of said group, wherein said oligonucleotide comprises a sequence selected from the group consisting of:
where said group of organisms is the phylogenetic group bacteria, the sequences:

CCACTGCTGCCTCCCGTAGGAGTCTGGGCC;
CCAGATCTCTACGCATTTCACCGCTACACGTGG;
GCTCGTTGCGGGACTTAACCCAACAT;
GGGGTTCTTTTCGCCTTTCCCTCACGG;
GGCTGCTTCTAAGCCAACATCCTG;
GGACCGTTATAGTTACGGCCGCC;
GGTCGGAACTTACCCGACAAGGAATTTCGCTACC;
where said group of non-viral organisms is the phylogenetic group fungi, the sequences:
CCCGACCGTCCCTATTAATCATTACGATGG;
CCCGACCGTCCCTATTAATCATTACGATGGTCCTAGAAAC;
CGACTTGGCATGAAAACTATTCCTTCCTGTGG;
GCTCTTCATTCAATTGTCCACGTTCAATTAAGCAACAAGG;
GCTCTGCATTCAAAGGTCCGCGTTCAATAAAGAAACAGGG;
where said organism is Mycobacterium avium, the sequence:
ACCGCAAAAGCTTTCCACCAGAAGACATGCGTCTTGAG;
where said organism is Mycobacterium intracellulare, the sequence:
ACCGCAAAAGCTTTCCACCTAAAGACATGCGCCTAAAG;

where the group of non-viral organisms is the Mycobacterium tuberculosis complex bacteria, the sequences:
TAAAGCGCTTTCCACCACAAGACATGCATCCCGTG;
TGCCCTACCCACACCCACCACCAGGTGATGT;
CCATCACCACCCTCCTCCGGAGAGGAAAAGG;
CTGTCCCTAAACCCGATTCAGGGTTCGAGGTTAGATGC;
AGGCACTGTCCCTAAACCCGATTCAGGGTTC;
CCGCTAAAGCGCTTTCCACCACAAGACATGCATCCCG;
ACACCGCTAAAGCGCTTTCCACCACAAGACATGCATC;
where the group of non-viral organisms is the genus Mycobacterium, the sequences:
CCATGCACCACCTGCACACAGGCCACAAGG;
GGCTTGCCCCAGTATTACCACTGACTGGTACGG;
CACCGAATTCGCCTCAACCGGCTATGCGTCACCTC;
GGGGTACGGCCCGTGTGTGTGCTCGCTAGAGGC;
where said organism is Mycoplasma pneumoniae, the sequences:
GCTTGGTGCTTTCCTATTCTCACTGAAACAGCTACATTCGGC;
AATAACGAACCCTTGCAGGTCCTTTCAACTTTGAT;
CAGTCAAACTCTAGCCATTACCTGCTAAAGTCATT;
TACCGAGGGGATCGCCCCGACAGCTAGTAT;
CTTTACAGATTTGCTCACTTTTACAAGCTGGCGAC;
where said group of organisms is the genus Legionella, the sequences:
TACCCTCTCCCATACTCGAGTCAACCAGTATTATCTGACC;
GGATTTCACGTGTCCCGGCCTACTTGTTCGGGTGCGTAGTTC;
CATCTCTGCAAAATTCACTGTATGTCAAGGGTAGGTAAGG;
GCGGTACGGTTCTCTATAAGTTATGGCTAGC;
GTACCGAGGGTACCTTTGTGCT;
CACTCTTGGTACGATGTCCGAC;
where said organism is Chlamydia trachomatis, the sequences:
CCGACTCGGGGTTGAGCCCATCTTTGACAA;
TTACGTCCGACACGGATGGGGTTGAGACCATC;
CCGCCACTAAACAATCGTCGAAACAATTGCTCCGTTCGA;
CGTTACTCGGATGCCCAAATATCGCCACATTCG;
CATCCATCTTTCCAGATGTGTTCAACTAGGAGTCCTGATCC;
GAGGTCGGTCTTTCTCTCCTTTCGTCTACG;
CCGTTCTCATCGCTCTACGGACTCTTCCAATCG;
CGAAGATTCCCCTTGATCGCGACCTGATCT;
CCGGGGCTCCTATCGTTCCATAGTCACCCTAAAAG;
TACCGCGTGTCTTATCGACACACCCGCG;
where said group of organisms is the genus Campylobacter, the sequence:
GGTTCTTAGGATATCAAGCCCAGG;
Where said group of organisms is the sub-generic group Streptococci known as Enterococci, the sequence:
TGCAGCACTGAAGGGCGGAAACCCTCCAACACTTA;
where said group of organisms is the sub-generic group known as group 1 Pseudomonas, the sequence:
CAGACAAAGTTTCTCGTGCTCCGTCCTACTCGATT;
where said organism is Enterobacter cloacae, the sequence:
GTGTGTTTTCGTGTACGGGAGTTTCACCC;
where said organism is Proteus mirabilis, the sequence:
CCGTTCTCCTGACACTGCTATTGATTAAGACTC;
where said group of organisms is the genus Salmonella, the sequences:
CTCCTTTGAGTTCCCGACCTAATCGCTGGC;
CTCATCGAGCTCACAGCACATGCGCTTTTGTGTA;
where said organism is Escherichia coli, the sequence:
GCACATTCTCATCTCTGAAAACTTCCGTGG;
where said organism is Neisseria gonorrhoeae, the sequences:
CCGCCGCTACCCGGTAC;
TCATCGGCCGCCGATATTGGC;
GAGCATTCCGCACATGTCAAAACCAGGTA;
GAGATTCCGCACATGTCAAAACCAGG;
GAGGATTCCGCACATGTCAAAACCAGGTAA;

CCCGCTACCCGGTACGTTC;
CCGCTACCCGGTACGTTC.

**40.** A probe according to claim 39, wherein said group of non-viral organisms is the phylogenetic group bacteria and said oligonucleotide comprises the sequence CCACTGCTGCCTCCCGTAGGAGTCTGGGCC.

**41.** A probe according to claim 39, wherein said group of non-viral organisms is the phylogenetic group bacteria and said oligonucleotide comprises the sequence CCAGATCTCTACGCATTTCACCGCTACACGTGG.

**42.** A probe according to claim 39, wherein said group of non-viral organisms is the phylogenetic group bacteria and said oligonucleotide comprises the sequence GCTCGTTGCGGGACTTAACCCAACAT.

**43.** A probe according to claim 39, wherein said group of non-viral organisms is the phylogenetic group bacteria and said oligonucleotide comprises the sequence GGGGTTCTTTTCGCCTTTCCCTCACGG.

**44.** A probe according to claim 39, wherein said group of non-viral organisms is the phylogenetic group bacteria and said oligonucleotide comprises the sequence GGC TGCTTCTAAGCCAACATCCTG.

**45.** A probe according to claim 39, wherein said group of non-viral organisms is the phylogenetic group bacteria and said oligonucleotide comprises the sequence GGA CCGTTATAGTTACGGCCGCC.

**46.** A probe according to claim 39, wherein said group of non-viral organisms is the phylogenetic group bacteria and said oligonucleotide comprises the sequence GGTCGGAACTTACCCGACAAGGAATTTCGCTACC.

**47.** A probe according to claim 38, wherein said group of non-viral organisms is the phylogenetic group bacteria and said variable region corresponds to bases 330-365 of E. coli 16S rRNA, or the complement thereof.

**48.** A probe according to claim 38, wherein said group of non-viral organisms is the phylogenetic group bacteria and said variable region corresponds to bases 675-715 of E. coli 16S rRNA, or the complement thereof.

**49.** A probe according to claim 38, wherein said group of non-viral organisms is the phylogenetic group bacteria and said variable region corresponds to bases 1080-1110 of E. coli 16S rRNA, or the complement thereof.

**50.** A probe according to claim 38, wherein said group of non-viral organisms is the phylogenetic group bacteria and said variable region corresponds to bases 460-490 of E. coli 23S rRNA, or the complement thereof.

**51.** A probe according to claim 38, wherein said group of non-viral organisms is the phylogenetic group bacteria and said variable region corresponds to bases 1050-1080 of E. coli 23S rRNA, or the complement thereof.

**52.** A probe according to claim 38, wherein said group of non-viral organisms is the phylogenetic group bacteria and said variable region corresponds to bases 1900-1960 of E. coli 23S rRNA, or the complement thereof.

**53.** A probe according to claim 38, wherein said group of non-viral organisms is the phylogenetic group fungi and said variable region corresponds to bases 845-880 of S. cerevisiae 18S rRNA, or the complement thereof.

**54.** A probe according to claim 38, wherein said group of non-viral organisms is the phylogenetic group fungi and said variable region corresponds to bases 1960-2000 of S. cerevisiae 28S rRNA, or the complement thereof.

**55.** A probe according to claim 38, wherein said group of non-viral organisms is the phylogenetic group fungi and said variable region corresponds to bases 1225-1270 of S. cerevisiae 28S rRNA, or the complement thereof.

**56.** A probe according to claim 39, wherein said group of non-viral organisms is the phylogenetic group fungi and said oligonucleotide comprises the sequence
CCCGACCGTCCCTATTAATCATTACGATGG.

**57.** A probe according to claim 39, wherein said group of non-viral organisms is the phylogenetic group fungi and said oligonucleotide comprises the sequence
CCCGACCGTCCCTATTAATCATTACGATGGTCCTAGAAAC.

**58.** A probe according to claim 39, wherein said group of non-viral organisms is the phylogenetic group fungi and said oligonucleotide comprises the sequence CGACTTGGCATGAAAACTATTCCTTCCTGTGG.

**59.** A probe according to claim 39, wherein said group of non-viral organisms is the phylogenetic group fungi and said oligonucleotide comprises the sequence GCTCTTCATTCAATTGTCCACGTTCAATTAAGCAACAAGG.

**60.** A probe according to claim 39, wherein said group of non-viral organisms is the phylogenetic group fungi and said oligonucleotide comprises the sequence GCTCTGCATTCAAAGGTCCGCGTTCAATAAAGAAACAGGG.

**61.** A probe according to claim 39, wherein said organism is Mycobacterium avium and said oligonucleotide comprises the sequence ACCGCAAAAGCTTTCC ACCAGAAGACATGCGTCTTGAG.

**62.** A method according to any one of claims 1 to 37, or a probe according to claim 38, wherein said organism is Mycobacterium avium and wherein said variable region corresponds to bases 185-225 of E. coli 16S rRNA, or the complement thereof.

**63.** A probe according to claim 39, wherein said organism is Mycobacterium intracellulare and said oligonucleotide comprises the sequence ACCGCAAAAGCTTTCCACCTAAAGACATGCGCCTAAAG.

**64.** A method according to any one of claims 1 to 37, or a probe according to claim 38, wherein said organism is Mycobacterium intracellulare and said variable region corresponds to bases 185-225 of E. coli 16S rRNA, or the complement thereof.

**65.** A probe according to claim 39, wherein said group of non-viral organisms is the Mycobacterium tuberculosis-complex bacteria and said oligonucleotide comprises the sequence TAAAGCGCTTTCCACCACAAGACATGCATCCCGTG.

**66.** A probe according to claim 39, wherein said group of non-viral organisms is the Mycobacterium tuberculosis-complex bacteria and said oligonucleotide comprises the sequence TGCCCTACCCACACCCACCACCAGGTGATGT.

**67.** A probe according to claim 39, wherein said group of non-viral organisms is the Mycobacterium tuberculosis-complex bacteria and said oligonucleotide comprises the sequence CCATCACCACCCTCCTCCGGAGAGGAAAAGG.

**68.** A probe according to claim 39, wherein said group of non-viral organisms is the Mycobacterium tuberculosis-complex bacteria and said oligonucleotide comprises the sequence CTGTCCCTAAACCCGATTCAGGGTTCGAGGTTAGATGC.

**69.** A probe according to claim 39, wherein said group of non-viral organisms is the Mycobacterium tuberculosis-complex bacteria and said oligonucleotide comprises the sequence AGGCACTGTCCCTAAACCCGATTCAGGGTTC.

**70.** A probe according to claim 39, wherein said group of non-viral organisms is the Mycobacterium tuberculosis-complex bacteria and said oligonucleotide comprises the sequence CCGCTAAAGCGCTTTCCACCACAAGACATGCATCCCG.

**71.** A probe according to claim 39, wherein said group of non-viral organisms is the Mycobacterium tuberculosis-complex bacteria and said oligonucleotide comprises the sequence ACACCGCTAAAGCGCTTTCCACCACAAGACATGCATC.

**72.** A method according to any one of claims 1 to 37, or a probe according to claim 38, wherein said group of non-viral organisms is the Mycobacterium tuberculosis-complex bacteria and said variable region corresponds to bases 185-225 of E. coli 16S rRNA, or the complement thereof.

**73.** A method according to any one of claims 1 to 37, or a probe according to claim 38, wherein said group of non-viral organisms is the Mycobacterium tuberculosis-complex bacteria and said variable region corresponds to bases 540-575 of E. coli 23S rRNA, or the complement thereof.

**74.** A method according to any one of claims 1 to 37, or a probe according to claim 38, wherein said group of non-viral organisms is the Mycobacterium tuberculosis-complex bacteria and said variable region corresponds to bases 1155-1190 of E. coli 23S rRNA, or the complement thereof.

**75.** A method according to any one of claims 1 to 37, or a probe according to claim 38, wherein said group of non-viral organisms is the Mycobacterium tuberculosis-complex bacteria and said variable region corresponds to bases 2195-2235 of E. coli 23S rRNA, or the complement thereof.

**76.** A probe according to claim 39, wherein said group of organisms is the genus Mycobacterium and said oligonucleotide comprises the sequence
CCATGCACCACCTGCACACAGGCCACAAGG.

**77.** A probe according to claim 39, wherein said group of organisms is the genus Mycobacterium and said oligonucleotide comprises the sequence
GGCTTGCCCCAGTATTACCACTGACTGGTACGG.

**78.** A probe according to claim 39, wherein said group of organisms is the genus Mycobacterium and said oligonucleotide comprises the sequence
CACCGAATTCGCCTCAACCGGCTATGCGTCACCTC.

**79.** A probe according to claim 39, wherein said group of organisms is the genus Mycobacterium and said oligonucleotide comprises the sequence
GGGGTACGGCCCGTGTGTGTGCTCGCTAGAGGC.

**80.** A method according to any one of claims 1 to 37, or a probe according to claim 38, wherein said group of organisms is the genus Mycobacterium and said variable region corresponds to bases 1025-1060 of E. coli 16S rRNA, or the complement thereof.

**81.** A method according to any one of claims 1 to 37, or a probe according to claim 38, wherein said group of organisms is the genus Mycobacterium and said variable region corresponds to bases 1440-1475 of E. coli 23S rRNA, or the complement thereof.

**82.** A method according to any one of claims 1 to 37, or a probe according to claim 38, wherein said group of organisms is the genus Mycobacterium and said variable region corresponds to bases 1515-1555 of E. coli 23S rRNA, or the complement thereof.

**83.** A method according to any one of claims 1 to 37, or a probe according to claim 38, wherein said group of organisms is the genus Mycobacterium and said variable region corresponds to bases 1570-1610 of E. coli 23S rRNA, or the complement thereof.

**84.** A probe according to claim 39, wherein said organism is Mycoplasma pneumoniae and said oligonucleotide comprises the sequence
GCTTGGTGCTTTCCTATTCTCACTGAAACAGCTACATTCGGC.

**85.** A probe according to claim 39, wherein said organism is Mycoplasma pneumoniae and said oligonucleotide comprises the sequence
AATAACGAACCCTTGCAGGTCCTTTCAACTTTGAT.

**86.** A probe according to claim 39, wherein said organism is Mycoplasma pneumoniae and said oligonucleotide comprises the sequence
CAGTCAAACTCTAGCCATTACCTGCTAAAGTCATT.

**87.** A probe according to claim 39, wherein said organism is Mycoplasma pneumoniae and said oligonucleotide comprises the sequence
TACCGAGGGGATCGCCCCGACAGCTAGTAT.

**88.** A probe according to claim 39, wherein said organism is Mycoplasma pneumoniae and said oligonucleotide comprises the sequence
CTTTACAGATTTGCTCACTTTTACAAGCTGGCGAC.

**89.** A method according to any one of claims 1 to 37, or a probe according to claim 38, wherein said organism is Mycoplasma pneumoniae and said variable region corresponds to bases 190-230 of E. coli 16S rRNA, or the complement thereof.

**90.** A method according to any one of claims 1 to 37, or a probe according to claim 38, wherein said organism is Mycoplasma pneumoniae and said variable region corresponds to bases 450-490 of E. coli 16S rRNA. or the complement thereof.

**91.** A method according to any one of claims 1 to 37, or a probe according to claim 38, wherein said organism is Mycoplasma pneumoniae and said variable region corresponds to bases 820-860 of E. coli 16S rRNA, or the complement thereof.

**92.** A method according to any one of claims 1 to 37, or a probe according to claim 38 wherein said organism is Mycoplasma pneumoniae and said variable region corresponds to bases 1255-1290 of E. coli 16S rRNA, or the complement thereof.

**93.** A method according to any one of claims 1 to 37, or a probe according to claim 38, wherein said organism is Mycoplasma pneumoniae and said variable region corresponds to bases 65-120 of E. coli 5S rRNA, or the complement thereof.

**94.** A probe according to claim 39, wherein said group of organisms is the genus Legionella and said oligonucleotide comprises the sequence
TACCCTCTCCCATACTCGAGTCAACCAGTATTATCTGACC.

**95.** A probe according to claim 39, wherein said group of organisms is the genus Legionella and said oligonucleotide comprises the sequence
GGATTTCACGTGTCCCGGCCTACTTGTTCGGGTGCGTAGTTC.

**96.** A probe according to claim 39, wherein said group of organisms is the genus Legionella and said oligonucleotide comprises the sequence
CATCTCTGCAAAATTCACTGTATGTCAAGGGTAGGTAAGG.

**97.** A probe according to claim 39, wherein said group of organisms is the genus Legionella and said oligonucleotide comprises the sequence
GCGGTACGGTTCTCTATAAGTTATGGCTAGC.

**98.** A probe according to claim 39, wherein said group of organisms is the genus Legionella and said oligonucleotide comprises the sequence
GTACCGAGGGTACCTTTGTGCT.

**99.** A probe according to claim 39, wherein said group of organisms is the genus Legionella and said oligonucleotide comprises the sequence CACTCTTGGTACGATGTCCGAC.

**100.** A method according to anyone of claims 1 to 37, or a probe according to claim 38,
wherein said group of organisms is the genus Legionella and said variable region corresponds to bases 630-675 of E. coli 16S rRNA, or the complement thereof.

**101.** A method according to any one of claims 1 to 37, or a probe according to claim 38,
wherein said group of organisms is the genus Legionella and said variable region corresponds to bases 975-1020 of E. coli 16S rRNA, or the complement thereof.

**102.** A method according to any one of claims 1 to 37, or a probe according to claim 38, wherein said group of organisms is the genus Legionella and said variable region corresponds to bases 350-395 of E. coli 23S rRNA, or the complement thereof.

**103.** A method according to any one of claims 1 to 37, or a probe according to claim 38, wherein said group of organisms is the genus Legionella and said variable region corresponds to bases 1585-1620 of E. coli 23S rRNA, or the complement thereof.

**104.** A method according to any one of claims 1 to 37, or a probe according to claim 38, wherein said group of organisms is the genus Legionella and said variable region corresponds to bases 2280-2330 of E. coli 23S rRNA, or the complement thereof.

**105.** A probe according to claim 39, wherein said organism is Chlamydia trachomatis and said oligonucleotide comprises the sequence
CCGACTCGGGGTTGAGCCCATCTTTGACAA

**106.** A probe according to claim 39, wherein said organism is Chlamydia trachomatis and said oligonucleotide comprises the sequence
TTACGTCCGACACGGATGGGGTTGAGACCATC.

**107.** A probe according to claim 39, wherein said organism is Chlamydia trachomatis and said oligonucleotide comprises the sequence
CCGCCACTAAACAATCGTCGAAACAATTGCTCCGTTCGA.

**108.** A probe according to claim 39, wherein said organism is Chlamydia trachomatis and said oligonucleotide comprises the sequence
CGTTACTCGGATGCCCAAATATCGCCACATTCG.

**109.** A probe according to claim 39, wherein said organism is Chlamydia trachomatis and said oligonucleotide comprises the sequence
CATCCATCTTTCCAGATGTGTTCAACTAGGAGTCCTGATCC.

**110.** A probe according to claim 39, wherein said organism is Chlamydia trachomatis and said oligonucleotide comprises the sequence
GAGGTCGGTCTTTCTCTCCTTTCGTCTACG.

**111.** A probe according to claim 39, wherein said organism is Chlamydia trachomatis and said oligonucleotide comprises the sequence
CCGTTCTCATCGCTCTACGGACTCTTCCAATCG.

**112.** A probe according to claim 39, wherein said organism is Chlamydia trachomatis and said oligonucleotide comprises the sequence
CGAAGATTCCCCTTGATCGCGACCTGATCT.

**113.** A probe according to claim 39, wherein said organism is Chlamydia trachomatis and said oligonucleotide comprises the sequence
CCGGGGCTCCTATCGTTCCATAGTCACCCTAAAAG.

**114.** A probe according to claim 39, wherein said organism is Chlamydia trachomatis and said oligonucleotide comprises the sequence
TACCGCGTGTCTTATCGACACACCCGCG.

**115.** A method according to any one of claims 1 to 37, or a probe according to claim 38, wherein said organism is Chlamydia trachomatis and said variable region corresponds to bases 60-105 of E. coli 16S rRNA, or the complement thereof.

**116.** A method according to any one of claims 1 to 37, or a probe according to claim 38, wherein said organism is Chlamydia trachomatis and said variable region corresponds to bases 600-635 of E. coli 16S rRNA, or the complement thereof.

**117.** A method according to any one of claims 1 to 37, or a probe according to claim 38, wherein said organism is Chlamydia trachomatis and said variable region corresponds to bases 830-870 of E. coli 16S rRNA, or the complement thereof.

**118.** A method according to any one of claims 1 to 37, or a probe according to claim 38, wherein said organism is Chlamydia trachomatis and said variable region corresponds to bases 275-320 of E. coli 23S rRNA, or the complement thereof.

**119.** A method according to any one of claims 1 to 37, or a probe according to claim 38, wherein said organism is Chlamydia trachomatis and said variable region corresponds to 330-365 of E. coli 23S rRNA, or the complement thereof.

**120.** A method according to any one of claims 1 to 37, or a probe according to claim 38, wherein said organism is Chlamydia trachomatis and said variable region corresponds to bases 1160-1190 of E. coli 23S rRNA, or the complement thereof.

**121.** A method according to any one of claims 1 to 37, or a probe according to claim 38, wherein said organism is Chlamydia trachomatis and said variable region corresponds to bases 1450-1490 of E. coli 23S rRNA, or the complement thereof.

**122.** A method according to any one of claims 1 to 37, or a probe according to claim 38, wherein said organism is Chlamydia trachomatis and said variable region corresponds to bases 1510-1545 of E. coli 23S rRNA, or the complement thereof.

**123.** A method according to any one of claims 1 to 37, or a probe according to claim 38, wherein said organism is Chlamydia trachomatis and said variable region corresponds to bases 1710-1750 of E. coli 23S rRNA, or the complement thereof.

**124.** A probe according to claim 39, wherein said group of organisms is the genus Campylobacter and said oligonucleotide comprises the sequence
GGTTCTTAGGATATCAAGCCCAGG.

**125.** A method according to any one of claims 1 to 37, or a probe according to claim 38, wherein said group of organisms is the genus Campylobacter and said variable region corresponds to bases 980-1010 of E. coli 16S rRNA, or the complement thereof.

**126.** A probe according to claim 39, wherein said group of organisms is the sub-generic group of Streptococci known as Enterococci and said oligonucleotide comprises the sequence
TGCAGCACTGAAGGGCGGAAACCCTCCAACACTTA.

**127.** A method according to any one of claims 1 to 37, or a probe according to claim 38, wherein said group of organisms is the sub-generic group Streptococci known as Enterococci and said variable region corresponds to bases 825-860 of E. coli 16S rRNA, or the complement thereof.

**128.** A probe according to claim 39, wherein said group of organisms is the sub-generic grouping known as Group I Pseudomonas and said oligonucleotide comprises the sequence
CAGACAAAGTTTCTCGTGCTCCGTCCTACTCGATT.

**129.** A method according to any one of claims 1 to 37, or a probe according to claim 38, wherein said group of organisms is the sub-generic grouping known as Group I Pseudomonas and said variable region corresponds to bases 365-405 of E. coli 23S rRNA, or the complement thereof.

**130.** A probe according to claim 39, wherein said organism is Enterobacter cloacae and said oligonucleotide comprises the sequence
GTGTGTTTTCGTGTACGGGAGTTTCACCC.

**131.** A method according to any one of claims 1 to 37, or a probe according to claim 38, wherein said organism is Enterobacter cloacae and said variable region corresponds to bases 305-340 of E. coli 23S rRNA, or the complement thereof.

**132.** A probe according to claim 39, wherein said organism is Proteus mirabilis and said oligonucleotide comprises the sequence
CCGTTCTCCTGACACTGCTATTGATTAAGACTC.

**133.** A method according to any one of claims 1 to 37, or a probe according to claim 38, wherein said organism is Proteus mirabilis and said variable region corresponds to bases 270-305 of E. coli 23S rRNA, or the complement thereof.

**134.** A probe according to claim 39, wherein said group of organisms is the genus Salmonella and said oligonucleotide comprises the sequence
CTCCTTTGAGTTCCCGACCTAATCGCTGGC.

**135.** probe according to claim 39, wherein said group of organisms is the genus Salmonella and said oligonucleotide comprises the sequence
CTCATCGAGCTCACAGCACATGCGCTTTTGTGTA.

**136.** A method according to any one of claims 1 to 37, or a probe according to claim 38, wherein said group of organisms is the genus Salmonella and said variable region corresponds to bases 1125-1155 of E. coli 16S rRNA, or the complement thereof.

**137.** A method according to any one of claims 1 to 37, or a probe according to claim 38, wherein said group of organisms is the genus Salmonella and said variable region corresponds to bases 335-375 of E. coli 23S rRNA, or the complement thereof.

**138.** A probe according to claim 39, wherein said organism is Escherichia coli and said oligonucleotide comprises the sequence
GCACATTCTCATCTCTGAAAACTTCCGTGG.

**139.** A method according to any one of claims 1 to 37, or a probe according to claim 38, wherein said organism is Escherichia coli and said variable region corresponds to bases 995-1030 of E. coli 16S rRNA, or the complement thereof.

**140.** A probe according to claim 39, wherein said organism is Neisseria gonorrhoeae and said oligonucleotide comprises the sequence CCGCCGCTACCCGGTAC.

**141.** A probe according to claim 39, wherein said organism is Neisseria gonorrhoeae and said oligonucleotide comprises the sequence TCATCGGCCGCCGATATTGGC.

**142.** A probe according to claim 39, wherein said organism is Neisseria gonorrhoeae and said oligonucleotide comprises the sequence GAGCATTCCGCACATGTCAAAACCAGGTA.

**143.** A probe according to claim 39, wherein said organism is Neisseria gonorrhoeae and said oligonucleotide comprises the sequence GAGATTCCGCACATGTCAAAACCAGG.

**144.** A probe according to claim 39, wherein said organism is Neisseria gonorrhoeae and said oligonucleotide comprises the sequence GAGGATTCCGCACATGTCAAAACCAGGTAA.

**145.** A probe according to claim 39, wherein said organism is Neisseria gonorrhoeae and said oligonucleotide comprises the sequence CCCGCTACCCGGTACGTTC.

**146.** A probe according to claim 39, wherein said organism is Neisseria gonorrhoeae and said oligonucleotide comprises the sequence CCGCTACCCGGTACGTTC.

**147.** A method according to any one of claims 1 to 37, or a probe according to claim 38,

wherein said organism is Neisseria gonorrhoeae and said variable region corresponds to bases 125-150 of E. coli 16S rRNA, or the complement thereof.

**148.** A method according to any one of claims 1 to 37, or a probe according to claim 38, wherein said organism is Neisseria gonorrhoeae and said variable region corresponds to bases 455-485 of E. coli 16S rRNA, or the complement thereof.

**149.** A method according to any one of claims 1 to 37, or a probe according to claim 38, wherein said organism is Neisseria gonorrhoeae and said variable region corresponds to bases 980-1015 of E. coli 16S rRNA, or the complement thereof.

**150.** A nucleic acid hybrid formed between a nucleotide polymer or oligonucleotide according to any one of claims 47-56, 62, 64, 72-75, 80-83, 89-93, 100-104, 115, 123, 125, 127, 129, 131 133, 136, 137, 139 and 147-149 and a nucleic acid complementary thereto.

**151.** A nucleotide polymer which hybridises under stringent conditions to a probe according to any one of claims 40-46, 56-61, 63, 65-71, 76-79, 84-88, 94-99, 105-114, 124, 126, 128, 130, 132, 134, 135, 138 and 140-146, or to the complement thereof.

**152.** A nucleic acid hybrid according to claim 150 formed between an oligonucleotide and a nucleic acid complementary thereto.

**153.** The complement to the nucleotide polymer of claim 151.

**154.** A hybridisation assay comprising reacting together any rRNA or the complement thereof from a sample to be assayed for a non-viral organism or a group of non-viral organisms and a probe according to any one of claims 39 to 149, wherein said probe is at least about 75% complementary to a variable region of rRNA or rDNA selected to be unique to said non-viral organism or a group of non-viral organisms, under conditions such that hybridisation between the oligonucleotide probe or the nucleotide polymer and any sufficiently complementary sample rRNA or the complement thereof can occur, and observing and/or measuring said hybridisation.

**155.** The assay of claim 154 wherein said hybridisation between the oligonucleotide probe and any target sample rRNA is from at least about 10% to about 100%.

**156.** The assay of claim 154 wherein said oligonucleotide probe is cDNA.

**157.** The assay of claim 154 wherein said conditions include a temperature from about 25°C below Tm to about 1°C below Tm.

**158.** The assay of claim 154 which further comprises the parallel assay of a positive homologous control, or a positive heterologous control, or both.

**159.** The assay of claim 154 which further comprises the parallel assay of a negative control.

**160.** The assay of claim 154 wherein said conditions include agents for increased rates of hybridisation.

**161.** The assay of claim 154 wherein said conditions are such as to promote maximum hybridisation between the oligonucleotide probe and any complementary sample rRNA and minimum cross-reactivity between the oligonucleotide probe and any non-complementary sample rRNA.

**162.** The assay of claim 154 wherein said oligonucleotide probe is labelled.

**163.** The assay of claim 154 wherein said oligonucleotide probe is labelled with an isotopic, non-isotopic or chemiluminescent label.

**164.** The assay of claim 154 which further comprises the release of rRNA from the cells of said non-viral organism or group of non-viral organisms prior to the reacting together step.

**165.** The assay of claim 154 wherein said non-viral organism or group of non-viral organisms are Mycobacterium avium, Mycobacterium intracellulare, the Mycobacterium tuberculosis-complex bacteria, Mycobacterium genus, Mycoplasma pneumoniae, Legionella, Neisseria gonorrhoeae, Salmonella, Chlamydia trachomatis, Campylobacter, Proteus mirabilis Enterococcus, Enterobacter cloacae, Escherichia coli, Pseudomonas Group 1, bacteria or fungi.

**166.** The assay of claim 162, wherein said labelled oligonucleotide probe is about 20 nucleotides to about 50 nucleotides in length.

**167.** The assay of claim 162 wherein said labelled oligonucleotide probe is at least about 95% complementary to said variable region of rRNA.

**168.** The assay of claim 154 further comprising the use of one or more additional oligonucleotide probes of at least about 10 nucleotides in length and which are at least about 75% complementary to one or more additional variable regions of rRNA selected to be unique to said non-viral organisms.

**169.** The assay of claim 154 further comprising the use of one or more additional probes which identify one or more additional non-viral organisms, thereby expanding the group of non-viral organisms to be assayed.

## Patentansprüche

1. Verfahren zur Herstellung einer Sonde zur Verwendung in einem qualitativen oder quantitativen Hybridisierungsassay, das das Konstruieren eines Oligonukleotids, das ausreichend komplementär ist, um unter Hybridisierungsbedingungen an eine Region von rRNA zu hybridisieren, die so gewählt wird, daß sie für einen nicht-viralen Organismus oder eine Gruppe von nicht-viralen Organismen, welche(r) nachgewiesen werden soll, einzigartig ist, wobei das Oligonukleotid ausgewählt wird durch:

    a) Identifizieren einer oder mehrerer variabler Kandidatenregionen durch Vergleichen einer oder mehrerer variabler rRNA-Region-Sequenz(en) eines nicht-viralen Zielorganismus oder einer Gruppe von nicht-viralen Zielorganismen mit einer oder mehreren variablen rRNA-Region-Sequenzen des bekannten nächstverwandten Organismus, um eine Sequenz auszuwählen, die für die rRNA des Zielorganismus oder der -organismen einzigartig ist, und
    b) Synthetisieren eines Oligonukleotids, welches zu der einzigartigen rRNA-Sequenz komplementär ist, wobei die Wärmestabilität der Sonde:Zielnukleinsäure-Hybride größer ist als die Wärmestabilität der Sonde:Nicht-Zielnukleinsäure-Hybride,

    wobei der Zielorganismus oder die -organismen aus einer oder mehreren Arten in einer Gattung oder einer oder mehreren Gattungen in einer Familie ausgewählt sind.

2. Verfahren nach Anspruch 1, wobei die Region der rRNA so gewählt ist, daß sie mindestens etwa einen eine Base großen Sequenzunterschied von einer entsprechenden rRNA-Sequenz des bekannten nächstverwandten Organismus zu dem nicht-viralen Organismus oder der Gruppe nicht-viraler Organismen, welche(r) nachgewiesen werden soll, aufweist.

3. Verfahren nach Anspruch 1, wobei die rRNA-Region so gewählt ist, daß sie einen mindestens etwa 10%-igen oder größeren Basensequenzunterschied von der entsprechenden rRNA-Sequenz des bekannten nächstverwandten Organismus zu dem nicht-viralen Organismus oder der Gruppe nicht-viraler Organismen, welche(r) nachgewiesen werden soll, aufweist.

4. Verfahren nach Anspruch 1, wobei die rRNA-Region aus der 5S-, 16S- und 23S-rRNA umfassenden Gruppe gewählt ist.

5. Verfahren nach Anspruch 1, wobei die rRNA-Region aus der 5,OS-, 5,8S-, 18S- und 28S-rRNA umfassenden Gruppe gewählt ist.

6. Verfahren nach Anspruch 1, wobei das Oligonukleotid mindestens etwa 10 Nukleotide lang ist.

7. Verfahren nach Anspruch 1, wobei das Oligonukleotid mindestens etwa 15 Nukleotide lang ist.

**8.** Verfahren nach Anspruch 1, wobei das Oligonukleotid mindestens etwa 20 Nukleotide lang ist.

**9.** Verfahren nach Anspruch 1, wobei das Oligonukleotid mindestens etwa 30 Nukleotide lang ist.

**10.** Verfahren nach Anspruch 1, wobei das Oligonukleotid etwa 20 Nukleotide bis etwa 50 Nukleotide lang ist.

**11.** Verfahren nach Anspruch 1, wobei das Oligonukleotid etwa 30 Nukleotide bis etwa 50 Nukleotide lang ist.

**12.** Verfahren nach Anspruch 2, wobei das Oligonukleotid mindestens etwa 10 Nukleotide lang ist.

**13.** Verfahren nach Anspruch 2, wobei das Oligonukleotid mindestens etwa 15 Nukleotide lang ist.

**14.** Verfahren nach Anspruch 2, wobei das Oligonukleotid mindestens etwa 20 Nukleotide lang ist.

**15.** Verfahren nach Anspruch 2, wobei das Oligonukleotid mindestens etwa 30 Nukleotide lang ist.

**16.** Verfahren nach Anspruch 2, wobei das Oligonukleotid etwa 20 Nukleotide bis etwa 50 Nukleotide lang ist.

**17.** Verfahren nach Anspruch 2, wobei das Oligonukleotid etwa 30 Nukleotide bis etwa 50 Nukleotide lang ist.

**18.** Verfahren nach Anspruch 2, wobei das Oligonukleotid mindestens etwa 10 Nukleotide lang ist.

**19.** Verfahren nach Anspruch 3, wobei das Oligonukleotid mindestens etwa 15 Nukleotide lang ist.

**20.** Verfahren nach Anspruch 3, wobei das Oligonukleotid mindestens etwa 20 Nukleotide lang ist.

**21.** Verfahren nach Anspruch 3, wobei das Oligonukleotid mindestens etwa 30 Nukleotide lang ist.

**22.** Verfahren nach Anspruch 3, wobei das Oligonukleotid etwa 20 Nukleotide bis etwa 50 Nukleotide lang ist.

**23.** Verfahren nach Anspruch 3, wobei das Oligonukleotid etwa 30 Nukleotide bis etwa 50 Nukleotide lang ist.

**24.** Verfahren nach Anspruch 1, wobei die Sonde zu mindestens etwa 75% komplementär zu der rRNA-Region ist.

**25.** Verfahren nach Anspruch 2, wobei das Oligonukleotid zu mindestens etwa 75% komplementär zu der rRNA-Region ist.

**26.** Verfahren nach Anspruch 3, wobei das Oligonukleotid zu mindestens etwa 75% komplementär zu der rRNA-Region ist.

**27.** Verfahren nach Anspruch 1, wobei die Sonde vollständig komplementär zu der rRNA-Region ist.

**28.** Verfahren nach Anspruch 2, wobei die Sonde vollständig komplementär zu der rRNA-Region ist.

**29.** Verfahren nach Anspruch 3, wobei die Sonde vollständig komplementär zu der rRNA-Region ist.

**30.** Verfahren zur Herstellung einer Sonde oder einer Kombination von Sonden zur Verwendung in einem qualitativen oder quantitativen Hybridisierungsassay, welcher das Konstruieren eines Nukleotidpolymeren umfaßt, das ausreichend komplementär ist, um an eine Region von rDNA oder rRNA zu hybridisieren, die ausgewählt ist, um einen nicht-viralen Zielorganismus oder eine erste Gruppe nicht-viraler Organismen, welche(r) nachgewiesen werden soll, von mindestens einem Nicht-Zielorganismus oder einer zweiten Gruppe von Nicht-Zielorganismen, welche in einer Probe vorhanden sein können, zu unterscheiden, wobei die Nicht-Zielorganismen oder die Gruppe von

- organismen nahe phylogenetische Verwandte der Zielorganismen oder der Gruppe von -organismen sind und die rDNA- oder rRNA-Region ausgewählt wird durch:

  • Vergleichen einer oder mehrerer rDNA- oder rRNA-Nukleotidbasensequenzen des nicht-viralen Organismus oder der Gruppe von nicht-viralen Organismen, welche(r) nachgewiesen werden soll, mit einer oder mehreren rDNA- oder rRNA-Nukleotidbasensequenzen des Nicht-Zielorganismus oder der Gruppe von Nicht-Zielorganismen,

• Anordnen mit maximaler Übereinstimmung bzw. Aligning der rDNA- oder rRNA-Nukleotidbasensequenzen des nicht-viralen Organismus oder der Gruppe von nicht-viralen Organismen mit den rDNA- oder rRNA-Nukleotidbasensequenzen der Nicht-Zielorganismen oder -organismengruppe, so daß Homologieregionen identifiziert werden, und

• Auswählen des Nukleotidpolymeren durch Maximieren der Homologie des Nukleotidpolymeren zu den Regionen der rDNA oder rRNA des nicht-viralen Organismus oder der Gruppe von nicht-viralen Organismen, welche(r) nachgewiesen werden soll, während des Minimierens der Homologie des Nukleotidpolymeren zu rDNA- oder rRNA-Sequenzen des Nicht-Zielorganismus oder der -organismengruppe, welche(r) davon unterschieden werden soll, wobei der/die nachzuweisende Organismus oder Organismengruppe aus einer oder mehreren Arten in einer Gattung oder einer oder mehreren Gattungen in einer Familie ausgewählt sind.

**31.** Verfahren nach Anspruch 30, wobei das Nukleotidpolymer zu mindestens etwa 90% homolog zu den Regionen der rDNA oder rRNA des nachzuweisenden nicht-viralen Organismus oder der nachzuweisenden nicht-viralen Organismengruppe ist.

**32.** Verfahren nach Anspruch 30, wobei das Sondenoligonukleotid weniger als etwa 90% homolog zu rDNA- oder rRNA-Sequenzen der nahen phylogenetischen Verwandten, welche davon unterschieden werden sollen, ist.

**33.** Verfahren nach einem der Ansprüche 30 bis 32, umfassend den weiteren Schritt des Bestätigens der Nicht-Kreuzreaktivität der Sonde durch Hybridisieren des Sondenoligonukleotids mit nicht-viralen Organismen oder Gruppen von nicht-viralen Organismen, welche durch die Sonde unterschieden werden sollen.

**34.** Verfahren zur Herstellung einer Sonde zur Verwendung in einem qualitativen oder quantitativen Hybridisierungsassay, welches das Konstruieren eines Oligonukleotids umfaßt, das ausreichend komplementär ist, um an eine Region von rDNA oder rRNA zu hybridisieren, die so ausgewählt ist, daß sie für einen nicht-viralen Organismus oder eine Gruppe nicht-viraler Organismen, welche(r) nachgewiesen werden soll, einzigartig ist, wobei die Region von rDNA oder rRNA ausgewählt wird durch:

• Vergleichen einer oder mehrerer rDNA- oder rRNA-Nukleotidbasensequenzen des nicht-viralen Organismus oder der Gruppe von nicht-viralen Organismen, welche(r) nachgewiesen werden soll, mit einer oder mehreren rDNA- oder rRNA-Nukleotidbasensequenzen von dessen/deren nächsten phylogenetischen Verwandten,

• Anordnen mit maximaler Übereinstimmung der rDNA- oder rRNA-Nukleotidbasensequenzen des nicht-viralen Organismus oder der Gruppe nicht-viraler Organismen mit den rDNA- oder rRNA-Sequenzen der nächsten phylogenetischen Verwandten, so daß die zwischenartlichen hypervariablen rDNA- oder rRNA-Regionen enthüllt werden,

• Auswählen des Sondenoligonukleotids in der zwischenartlich hypervariablen Region durch Maximieren der Homologie des Sondenoligonukleotids zu den Regionen der rDNA oder rRNA des nicht-viralen Organismus oder der nicht-viralen Organismengruppe, welche(r) nachgewiesen werden soll, während man die Homologie des Sondenoligonukleotids zu rDNA- oder rRNA-Sequenzen der davon zu unterscheidenden nächsten phylogenetischen Verwandten minimiert, wobei der nachzuweisende Organismus oder die nachzuweisende Organismengruppe aus einer oder mehreren Arten in einer Gattung oder einer oder mehreren Gattungen in einer Familie ausgewählt wird.

**35.** Verfahren nach Anspruch 34, wobei das Sondenoligonukleotid zu mindestens etwa 90% homolog zu den Regionen der rDNA oder rRNA des nicht-viralen Organismus oder der nicht-viralen Organismengruppe, welche(r) nachgewiesen werden soll, ist.

**36.** Verfahren nach Anspruch 34, wobei das Sondenoligonukleotid weniger als etwa 90% homolog zu rDNA- oder rRNA-Sequenzen der nächsten phylogenetischen Verwandten, welche davon unterschieden werden sollen, ist.

**37.** Verfahren nach einem der Ansprüche 34 bis 36, umfassend den weiteren Schritt des Bestätigens der Nicht-Kreuzreaktivität der Sonde durch Hybridisieren des Sondenoligonukleotids mit nicht-viralen Organismen oder Gruppen von nicht-viralen Organismen, welche durch die Sonde unterschieden werden sollen.

**38.** Nukleotidpolymer-Hybridisierungsassaysonde für einen nicht-viralen Organismus oder eine Gruppe von nicht-viralen Organismen in einer im wesentlichen isolierten Form, welche ein Oligonukleotid von 10 bis 100 Nukleotiden umfaßt, das fähig ist, unter stringenten Hybridisierungsbedingungen an mindestens 10 Nukleotide in einer variablen Region von rRNA oder rDNA zu hybridisieren, welche so ausgewählt ist, daß sie für den nicht-viralen Organismus oder die Gruppe von nicht-viralen Organismen einzigartig ist, wobei die Sonde unter stringenten Hybridisierungsbedingungen an Nukleinsäure aus einer Organismenart in einer Gattung hybridisiert und unter den Bedingungen darin fehlschlägt, an Nukleinsäure aus anderen Arten in der Gattung zu hybridisieren, oder wobei die Sonde unter stringenten Hybridisierungsbedingungen an Nukleinsäure aus einem oder mehreren Vertreter(n) der Organismengruppe hybridisiert und darin fehlschlägt, an Nukleinsäure aus Organismen, welche nicht Vertreter der Gruppe sind, zu hybridisieren, wobei die variable Region einer Region entspricht, ausgewählt aus der Gruppe, bestehend aus:

den Basen 60-100, 120-150, 600-670, 820-860, 980-1050 und 1250-1290 von E. coli 16S-rRNA, 270-390, 535-560, 1150-1200, 1440-1600, 1710-1750 und 2190-2330 von E. coli 23S-rRNA,
den Basen 330-365 von E. coli 16S-rRNA, wobei das Nukleotidpolymer unter stringenten Bedingungen an Arten der phylogenetischen Gruppe der Bakterien hybridisiert,
den Basen 675-715 von E. coli 16S-rRNA, wobei das Nukleotidpolymer unter stringenten Bedingungen an Arten der phylogenetischen Gruppe der Bakterien hybridisiert,
den Basen 1080-1110 von E. coli 16S-rRNA, wobei das Nukleotidpolymer unter stringenten Bedingungen an Arten der phylogenetischen Gruppe der Bakterien hybridisiert,
den Basen 460-490 von E. coli 23S-rRNA, wobei das Nukleotidpolymer unter stringenten Bedingungen an Arten der phylogenetischen Gruppe der Bakterien hybridisiert,
den Basen 1050-1080 von E. coli 23S-rRNA, wobei das Nukleotidpolymer unter stringenten Bedingungen an Arten der phylogenetischen Gruppe der Bakterien hybridisiert,
den Basen 1900-1960 von E. coli 23S-rRNA, wobei das Nukleotidpolymer unter stringenten Bedingungen an Arten der phylogenetischen Gruppe der Bakterien hybridisiert,
den Basen 845-880 von S. cerevisiae 18-rRNA, wobei das Nukleotidpolymer unter stringenten Bedingungen an Arten der phylogenetischen Gruppe der Pilze hybridisiert,
den Basen 1960-2000 von S. cerevisiae 28S-rRNA, wobei das Nukleotidpolymer unter stringenten Bedingungen an Arten der phylogenetischen Gruppe der Pilze hybridisiert,
den Basen 1225-1270 von S. cerevisiae 28S-rRNA, wobei das Nukleotidpolymer unter stringenten Bedingungen an Arten der phylogenetischen Gruppe der Pilze hybridisiert,
den Basen 185-225 von E. coli 16S-rRNA, wobei das Nukleotidpolymer unter stringenten Bedingungen an Mycobacterium avium hybridisiert,
den Basen 185-225 von E. coli 16S-rRNA, wobei das Nukleotidpolymer unter stringenten Bedingungen an Mycobacterium intracellulare hybridisiert,
den Basen 185-225 von E. coli 16S-rRNA, wobei das Nukleotidpolymer unter stringenten Bedingungen an Arten von Bakterien des Mycobacterium tuberculosis-Komplexes hybridisiert,
den Basen 540-575 von E. coli 23S-rRNA, wobei das Nukleotidpolymer unter stringenten Bedingungen an Arten von Bakterien des Mycobacterium tuberculosis-Komplexes hybridisiert,
den Basen 1155-1190 von E. coli 23S-rRNA, wobei das Nukleotidpolymer unter stringenten Bedingungen an Arten von Bakterien des Mycobacterium tuberculosis-Komplexes hybridisiert,
den Basen 2195-2235 von E. coli 23S-rRNA, wobei das Nukleotidpolymer unter stringenten Bedingungen an Arten von Bakterien des Mycobacterium tuberculosis-Komplexes hybridisiert,
den Basen 1025-1060 von E. coli 16S-rRNA, wobei das Nukleotidpolymer unter stringenten Bedingungen an Arten der Gattung Mycobacterium hybridisiert,
den Basen 1440-1475 von E. coli 23S-rRNA, wobei das Nukleotidpolymer unter stringenten Bedingungen an Arten der Gattung Mycobacterium hybridisiert,
den Basen 1515-1555 von E. coli 23S-rRNA, wobei das Nukleotidpolymer unter stringenten Bedingungen an Arten der Gattung Mycobacterium hybridisiert,
den Basen 1570-1610 von E. coli 23S-rRNA, wobei das Nukleotidpolymer unter stringenten Bedingungen an Arten der Gattung Mycobacterium hybridisiert,
den Basen 190-230 von E. coli 16S-rRNA, wobei das Nukleotidpolymer unter stringenten Bedingungen an Mycoplasma pneumoniae hybridisiert,
den Basen 450-490 von E. coli 16S-rRNA, wobei das Nukleotidpolymer unter stringenten Bedingungen an Mycoplasma pneumoniae hybridisiert,
den Basen 820-860 von E. coli 16S-rRNA, wobei das Nukleotidpolymer unter stringenten Bedingungen an Mycoplasma pneumoniae hybridisiert,
den Basen 1255-1290 von E. coli 16S-rRNA, wobei das Nukleotidpolymer unter stringenten Bedingungen an Mycoplasma Pneumoniae hybridisiert,
den Basen 65-120 von E. coli 5S-rRNA, wobei das Nukleotidpolymer unter stringenten Bedingungen an Mycoplasma pneumoniae hybridisiert,
den Basen 630-675 von E. coli 16S-rRNA, wobei das Nukleotidpolymer unter stringenten Bedingungen an Arten der Gattung Legionella hybridisiert,
den Basen 975-1020 von E. coli 16S-rRNA, wobei das Nukleotidpolymer unter stringenten Bedingungen an Arten der Gattung Legionella hybridisiert,
den Basen 350-395 von E. coli 23S-rRNA, wobei das Nukleotidpolymer unter stringenten Bedingungen an Arten der Gattung Legionella hybridisiert,
den Basen 1585-1620 von E. coli 23S-rRNA, wobei das Nukleotidpolymer unter stringenten Bedingungen an Arten der Gattung Legionella hybridisiert,
den Basen 2280-2330 von E. coli 23S-rRNA, wobei das Nukleotidpolymer unter stringenten Bedingungen an Arten der Gattung Legionella hybridisiert,
den Basen 60-105 von E. coli 16S-rRNA, wobei das Nukleotidpolymer unter stringenten Bedingungen an Chlamydia trachomatis hybridisiert,
den Basen 600-635 von E. coli 16S-rRNA, wobei das Nukleotidpolymer unter stringenten Bedingungen an Chlamydia trachomatis hybridisiert,
den Basen 830-870 von E. coli 16S-rRNA, wobei das Nukleotidpolymer unter stringenten Bedingungen an Chlamydia trachomatis hybridisiert,
den Basen 275-320 von E. coli 23S-rRNA, wobei das Nukleotidpolymer unter stringenten Bedingungen an Chlamydia trachomatis hybridisiert,
den Basen 330-365 von E. coli 23S-rRNA, wobei das Nukleotidpolymer unter stringenten Bedingungen an Chlamydia trachomatis hybridisiert,
den Basen 1160-1190 von E. coli 23S-rRNA, wobei das Nukleotidpolymer unter stringenten Bedingungen an Chlamydia trachomatis hybridisiert,
den Basen 1450-1490 von E. coli 23S-rRNA, wobei das Nukleotidpolymer unter stringenten Bedingungen an Chlamydia trachomatis hybridisiert,
den Basen 1510-1545 von E. coli 23S-rRNA, wobei das Nukleotidpolymer unter stringenten Bedingungen an Chlamydia trachomatis hybridisiert,
den Basen 1710-1750 von E. coli 23S-rRNA, wobei das Nukleotidpolymer unter stringenten Bedingungen an Chlamydia trachomatis hybridisiert,
den Basen 980-1010 von E. coli 16S-rRNA, wobei das Nukleotidpolymer unter stringenten Bedingungen an Arten der Gattung Campylobacter hybridisiert,
den Basen 825-860 von E. coli 16S-rRNA, wobei das Nukleotidpolymer unter stringenten Bedingungen an Arten der als Enterococci bekannten Untergruppe der Gattung Streptococci hybridisiert,
den Basen 365-405 von E. coli 23S-rRNA, wobei das Nukleotidpolymer unter stringenten Bedingungen an Arten der als Pseudomonas-Gruppe 1 bekannten Gattungsuntergruppe hybridisiert,
den Basen 305-340 von E. coli 16S-rRNA, wobei das Nukleotidpolymer unter stringenten Bedingungen an Enterobacter cloacae hybridisiert,
den Basen 270-305 von E. coli 23S-rRNA, wobei das Nukleotidpolymer unter stringenten Bedingungen an Proteus mirabilis hybridisiert,
den Basen 1125-1155 von E. coli 16S-rRNA, wobei die Gruppe von Organismen der Gattung Salmonella angehört,
den Basen 335-375 von E. coli 23S-rRNA, wobei das Nukleotidpolymer unter stringenten Bedingungen an Arten der Gattung Salmonella hybridisiert,
den Basen 995-1030 von E. coli 16S-rRNA, wobei das Nukleotidpolymer unter stringenten Bedingungen an Escherichia coli hybridisiert,
den Basen 125-150 von E. coli 16S-rRNA, wobei der Organismus Neisseria gonorrhoeae ist,
den Basen 455-485 von E. coli 16S-rRNA, wobei das Nukleotidpolymer unter stringenten Bedingungen an Neisseria gonorrhoeae hybridisiert,
den Basen 980-1015 von E. coli 16S-rRNA, wobei das Nukleotidpolymer unter stringenten Bedingungen an Neisseria gonorrhoeae hybridisiert,
oder dem Komplement davon.

**39.** Nukleotidpolymer-Hybridisierungsassaysonde für einen nicht-viralen Organismus oder eine Gruppe von nicht-viralen Organismen in einer im wesentlichen isolierten Form, welche ein Oligonukleotid von 10 bis 100 Nukleotiden umfaßt, das fähig ist, unter stringenten Hybridisierungsbedingungen an mindestens 10 Nukleotide in einer variablen Region von rRNA oder rDNA zu hybridisieren, welche so ausgewählt ist, daß sie für den nicht-viralen Organismus oder eine Gruppe von nicht-viralen Organismen einzigartig ist, wobei die Sonde unter stringenten Hybridisierungsbedingungen an Nukleinsäure aus einer Organismenart in einer Gattung hybridisiert und unter den Bedingungen darin fehlschlägt, an Nukleinsäure aus anderen Arten in der Gattung zu hybridisieren, oder wobei die Sonde unter stringenten Hybridisierungsbedingungen an Nukleinsäure aus einem oder mehreren Vertreter(n) der Organismengruppe hybridisiert und darin fehlschlägt, an Nukleinsäure aus Organismen, welche nicht Vertreter der Gruppe sind, zu hybridisieren, wobei das Oligonukleotid eine Sequenz umfaßt, ausgewählt aus der Gruppe, bestehend aus: wenn es sich bei der Gruppe von Organismen um die phylogenetische Gruppe der Bakterien handelt, den Sequenzen:

CCACTGCTGCCTCCCGTAGGAGTCTGGGCC;
CCAGATCTCTACGCATTTCACCGCTACACGTGG;
GCTCGTTGCGGGACTTAACCCAACAT;
GGGGTTCTTTTCGCCTTTCCCTCACGG;
GGCTGCTTCTAAGCCAACATCCTG;
GGACCGTTATAGTTACGGCCGCC;
GGTCGGAACTTACCCGACAAGGAATTTCGCTACC;
wenn es sich bei der Gruppe von nicht-viralen Organismen um die phylogenetische Gruppe der Pilze handelt, den Sequenzen:
CCCGACCGTCCCTATTAATCATTACGATGG;
CCCGACCGTCCCTATTAATCATTACGATGGTCCTAGAAAC;
CGACTTGGCATGAAAACTATTCCTTCCTGTCC;
GCTCTTCATTCAATTGTCCACGTTCAATTAAGCAACAAGG;
GCTCTGCATTCAAAGGTCCGCGTTCAATAAAGAAACAGGG;
wenn es sich bei dem Organismus um Mycobacterium avium handelt, der Sequenz:
ACCGCAAAAGCTTTCCACCAGAAGACATGCGTCTTGAG;
wenn es sich bei dem Organismus um Mycobacterium intracellulare handelt, der Sequenz:
ACCGCAAAAGCTTTCCACCTAAAGACATGCGCCTAAAG;
wenn es sich bei der Gruppe nicht-viraler Organismen um die Bakterien des Mycobacterium tuberculosis-Komplexes handelt, den Sequenzen:
TAAAGCGCTTTCCACCACAAGACATGCATCCCGTG;
TGCCCTACCCACACCCACCACCAGGTGATGT;
CCATCACCACCCTCCTCCGGAGAGGAAAAGG;
CTGTCCCTAAACCCGATTCAGGGTTCGAGGTTAGATGC;
AGGCACTGTCCCTAAACCCGATTCAGGGTTC;
CCGCTAAAGCGCTTTCCACCACAAGACATGCATCCCG;
ACACCGCTAAAGCGCTTTCCACCACAAGACATGCATC;
wenn es sich bei der Gruppe nicht-viraler Organismen um die Gattung Mycobacterium handelt, den Sequenzen:
CCATGCACCACCTGCACACAGGCCACAAGG;
GGCTTGCCCCAGTATTACCACTGACTGGTACGG;
CACCGAATTCGCCTCAACCGGCTATGCGTCACCTC;
GGGGTACGGCCCGTGTGTGTGCTCGCTAGAGGC;
wenn es sich bei dem Organismus um Mycoplasma pneumoniae handelt, den Sequenzen:
GCTTGGTGCTTTCCTATTCTCACTGAAACAGCTACATTCGGC;
AATAACGAACCCTTGCAGGTCCTTTCAACTTTGAT;
CAGTCAAACTCTAGCCATTACCTGCTAAAGTCATT;
TACCGAGGGGATCGCCCCGACAGCTAGTAT;
CTTTACAGATTTGCTCACTTTTACAAGCTGGCGAC;
wenn es sich bei der Gruppe von Organismen um die Gattung Legionella handelt, den Sequenzen:
TACCCTCTCCCATACTCGAGTCAACCAGTATTATCTGACC;
GGATTTCACGTGTCCCGGCCTACTTGTTCGGGTGCGTAGTTC;
CATCTCTGCAAAATTCACTGTATGTCAAGGGTAGGTAAGG;
GCGGTACGGTTCTCTATAAGTTATGGCTAGC;
GTACCGAGGGTACCTTTGTGCT;
CACTCTTGGTACGATGTCCGAC;
wenn es sich bei dem Organismus um Chlamydia trachomatis handelt, den Sequenzen:
CCGACTCGGGGTTGAGCCCATCTTTGACAA;
TTACGTCCGACACGGATGGGGTTGAGACCATC;
CCGCCACTAAACAATCGTCGAAACAATTGCTCCGTTCGA;
CGTTACTCGGATGCCCAAATATCGCCACATTCG;
CATCCATCTTTCCAGATGTGTTCAACTAGGAGTCCTGATCC;
GAGGTCGGTCTTTCTCTCCTTTCGTCTACG;

CCGTTCTCATCGCTCTACGGACTCTTCCAATCG;
CGAAGATTCCCCTTGATCGCGACCTGATCT;
CCGGGGCTCCTATCGTTCCATAGTCACCCTAAAAG;
TACCGCGTGTCTTATCGACACACCCGCG;
wenn es sich bei der Gruppe von Organismen um die Gattung Campylobacter handelt, der Sequenz:
GGTTCTTAGGATATCAAGCCCAGG;
wenn es sich bei der Gruppe von Organismen um die als Enterococci bekannte Untergruppe der Gattung Streptococci handelt, der Sequenz:
TGCAGCACTGAAGGGCGGAAACCCTCCAACACTTA;
wenn es sich bei der Gruppe von Organismen um die als Pseudomonas-Gruppe 1 bekannte Gattungsuntergruppe handelt, der Sequenz:
CAGACAAAGTTTCTCGTGCTCCGTCCTACTCGATT;
wenn es sich bei dem Organismus um Enterobacter cloacae handelt, der Sequenz:
GTGTGTTTTCGTGTACGGGAGTTTCACCC;
wenn es sich bei dem Organismus um Proteus mirabilis handelt, der Sequenz:
CCGTTCTCCTGACACTGCTATTGATTAAGACTC;
wenn es sich bei der Gruppe von Organismen um die Gattung Salmonella handelt, den Sequenzen:
CTCCTTTGAGTTCCCGACCTAATCGCTGGC;
CTCATCGAGCTCACAGCACATGCGCTTTTGTGTA;
wenn es sich bei dem Organismus um Escherichia coli handelt, der Sequenz:
GCACATTCTCATCTCTGAAAACTTCCGTCC;
wenn es sich bei dem Organismus um Neisseria gonorrhoeae handelt, den Sequenzen:
CCGCCGCTACCCGGTAC;
TCATCGGCCGCCGATATTGGC;
GAGCATTCCGCACATGTCAAAACCAGGTA;
GAGATTCCGCACATGTCAAAACCAGG;
GAGGATTCCGCACATGTCAAAACCAGGTAA;
CCCGCTACCCGGTACGTTC;
CCGCTACCCGGTACGTTC.

**40.** Sonde nach Anspruch 39, wobei es sich bei der Gruppe von nicht-viralen Organismen um die phylogenetische Gruppe der Bakterien handelt und das Oligonukleotid die folgende Sequenz umfaßt:
CCACTGCTGCCTCCCGTAGGAGTCTGGGCC

**41.** Sonde nach Anspruch 39, wobei es sich bei der Gruppe von nicht-viralen Organismen um die phylogenetische Gruppe der Bakterien handelt und das Oligonukleotid die folgende Sequenz umfaßt:
CCAGATCTCTACGCATTTCACCGCTACACGTGG.

**42.** Sonde nach Anspruch 39, wobei es sich bei der Gruppe von nicht-viralen Organismen um die phylogenetische Gruppe der Bakterien handelt und das Oligonukleotid die folgende Sequenz umfaßt:
GCTCGTTGCGGGACTTAACCCAACAT

**43.** Sonde nach Anspruch 39, wobei es sich bei der Gruppe von nicht-viralen Organismen um die phylogenetische Gruppe der Bakterien handelt und das Oligonukleotid die folgende Sequenz umfaßt:
CCGGTTCTTTTCGCCTTTCCCTCACGG

**44.** Sonde nach Anspruch 39, wobei es sich bei der Gruppe von nicht-viralen Organismen um die phylogenetische Gruppe der Bakterien handelt und das Oligonukleotid die folgende Sequenz umfaßt:
GGC TGCTTCTAAGCCAACATCCTG

**45.** Sonde nach Anspruch 39, wobei es sich bei der Gruppe von nicht-viralen Organismen um die phylogenetische Gruppe der Bakterien handelt und das Oligonukleotid die folgende Sequenz umfaßt:
GGACCGTTATAGTTACGGCCGCC

**46.** Sonde nach Anspruch 39, wobei es sich bei der Gruppe von nicht-viralen Organismen um die phylogenetische Gruppe der Bakterien handelt und das Oligonukleotid die folgende Sequenz umfaßt:
GGTCGGAACTTACCCGACAAGGAATTTCGCTACC

**47.** Sonde nach Anspruch 38, wobei es sich bei der Gruppe von nicht-viralen Organismen um die phylogenetische Gruppe der Bakterien handelt und die variable Region den Basen 330-365 von E. coli 16S-rRNA oder dem Komplement davon entspricht.

**48.** Sonde nach Anspruch 38, wobei es sich bei der Gruppe von nicht-viralen Organismen um die phylogenetische Gruppe der Bakterien handelt und die variable Region den Basen 675-715 von E. coli 16S-rRNA oder dem Komplement davon entspricht.

**49.** Sonde nach Anspruch 38, wobei es sich bei der Gruppe von nicht-viralen Organismen um die phylogenetische Gruppe der Bakterien handelt und die variable Region den Basen 1080-1110 von E. coli 16S-rRNA oder dem Komplement davon entspricht.

**50.** Sonde nach Anspruch 38, wobei es sich bei der Gruppe von nicht-viralen Organismen um die phylogenetische Gruppe der Bakterien handelt und die variable Region den Basen 460-490 von E. coli 23S-rRNA oder dem Komplement davon entspricht.

**51.** Sonde nach Anspruch 38, wobei es sich bei der Gruppe von nicht-viralen Organismen um die phylogenetische Gruppe der Bakterien handelt und die variable Region den Basen 1050-1080 von E. coli 23S-rRNA oder dem Komplement davon entspricht.

**52.** Sonde nach Anspruch 38, wobei es sich bei der Gruppe von nicht-viralen Organismen um die phylogenetische Gruppe der Bakterien handelt und die variable Region den Basen 1900-1960 von E. coli 23S-rRNA oder dem Komplement davon entspricht.

**53.** Sonde nach Anspruch 38, wobei es sich bei der Gruppe von nicht-viralen Organismen um die phylogenetische Gruppe der Pilze handelt und die variable Region den Basen 845-880 von S. cerevisiae 18S-rRNA oder dem Komplement davon entspricht.

**54.** Sonde nach Anspruch 38, wobei es sich bei der Gruppe von nicht-viralen Organismen um die phylogenetische Gruppe der Pilze handelt und die variable Region den Basen 1960-2000 von S. cerevisiae 28S-rRNA oder dem Komplement davon entspricht.

**55.** Sonde nach Anspruch 38, wobei es sich bei der Gruppe von nicht-viralen Organismen um die phylogenetische Gruppe der Pilze handelt und die variable Region den Basen 1225-1270 von S. cerevisiae 28S-rRNA oder dem Komplement davon entspricht.

**56.** Sonde nach Anspruch 39, wobei es sich bei der Gruppe von nicht-viralen Organismen um die phylogenetische Gruppe der Pilze handelt und das Oligonukleotid die folgende Sequenz umfaßt:
CCCGACCGTCCCTATTAATCATTACGATGG

**57.** Sonde nach Anspruch 39, wobei es sich bei der Gruppe von nicht-viralen Organismen um die phylogenetische Gruppe der Pilze handelt und das Oligonukleotid die folgende Sequenz umfaßt:
CCCGACCGTCCCTATTAATCATTACGATGGTCCTAGAAAC.

**58.** Sonde nach Anspruch 39, wobei es sich bei der Gruppe von nicht-viralen Organismen um die phylogenetische Gruppe der Pilze handelt und das Oligonukleotid die folgende Sequenz umfaßt:
CGACTTGGCATGAAAACTATTCCTTCCTGTGG

**59.** Sonde nach Anspruch 39, wobei es sich bei der Gruppe von nicht-viralen Organismen um die phylogenetische Gruppe der Pilze handelt und das Oligonukleotid die folgende Sequenz umfaßt:
GCTCTTCATTCAATTGTCCACGTTCAATTAAGCAACAAGG

**60.** Sonde nach Anspruch 39, wobei es sich bei der Gruppe von nicht-viralen Organismen um die phylogenetische Gruppe der Pilze handelt und das Oligonukleotid die folgende Sequenz umfaßt:
GCTCTGCATTCAAAGGTCCGCGTTCAATAAAGAAACAGGG

**61.** Sonde nach Anspruch 39, wobei es sich bei dem Organismus um Mycobacterium avium handelt und das Oligonukleotid die folgende Sequenz umfaßt:

ACCGCAAAAGCTTTCC ACCAGAAGACATGCGTCTTGAG

**62.** Verfahren nach einem der Ansprüche 1 bis 37 oder Sonde nach Anspruch 38, wobei es sich bei dem Organismus um Mycobacterium avium handelt und die variable Region den Basen 185-225 von E. coli 16S-rRNA oder dem Komplement davon entspricht.

**63.** Sonde nach Anspruch 39, wobei es sich bei dem Organismus um Mycobacterium intracellulare handelt und das Oligonukleotid die folgende Sequenz umfaßt:
ACCGCAAAAGCTTTCCACCTAAAGACATCCCCCTAAAG

**64.** Verfahren nach einem der Ansprüche 1 bis 37 oder Sonde nach Anspruch 38, wobei der Organismus Mycobacterium intracellulare ist und die variable Region den Basen 185-225 von E. coli 16S-rRNA oder dem Komplement davon entspricht.

**65.** Sonde nach Anspruch 39, wobei es sich bei der Gruppe von nicht-viralen Organismen um die Bakterien des Mycobacterium tuberculosis-Komplexes handelt und das Oligonukleotid die folgende Sequenz umfaßt:
TAAAGCGCTTTCCACCACAAGACATGCATCCCGTG

**66.** Sonde nach Anspruch 39, wobei es sich bei der Gruppe von nicht-viralen Organismen um die Bakterien des Mycobacterium tuberculosis-Komplexes handelt und das Oligonukleotid die folgende Sequenz umfaßt:
TGCCCTACCCACACCCACCACCAGGTGATGT

**67.** Sonde nach Anspruch 39, wobei es sich bei der Gruppe von nicht-viralen Organismen um die Bakterien des Mycobacterium tuberculosis-Komplexes handelt und das Oligonukleotid die folgende Sequenz umfaßt:
CCATCACCACCCTCCTCCGGAGAGGAAAAGG,

**68.** Sonde nach Anspruch 39, wobei es sich bei der Gruppe von nicht-viralen Organismen um die Bakterien des Mycobacterium tuberculosis-Komplexes handelt und das Oligonukleotid die folgende Sequenz umfaßt:
CTGTCCCTAAACCCGATTCAGGGTTCGAGGTTAGATGC

**69.** Sonde nach Anspruch 39, wobei es sich bei der Gruppe von nicht-viralen Organismen um die Bakterien des Mycobacterium tuberculosis-Komplexes handelt und das Oligonukleotid die folgende Sequenz umfaßt:
AGGCACTGTCCCTAAACCCGATTCAGGGTTC.

**70.** Sonde nach Anspruch 39, wobei es sich bei der Gruppe von nicht-viralen Organismen um die Bakterien des Mycobacterium tuberculosis-Komplexes handelt und das Oligonukleotid die folgende Sequenz umfaßt:
CCGCTAAAGCGCTTTCCACCACAAGACATGCATCCCG.

**71.** Sonde nach Anspruch 39, wobei es sich bei der Gruppe von nicht-viralen Organismen um die Bakterien des Mycobacterium tuberculosis-Komplexes handelt und das Oligonukleotid die folgende Sequenz umfaßt:
ACACCGCTAAAGCGCTTTCCACCACAAGACATGCATC

**72.** Verfahren nach einem der Ansprüche 1 bis 37 oder Sonde nach Anspruch 38, wobei es sich bei der Gruppe von nicht-viralen Organismen um die Bakterien des Mycobacterium tuberculosis-Komplexes handelt und die variable Region den Basen 185-225 von E. coli 16S-rRNA oder dem Komplement davon entspricht.

**73.** Verfahren nach einem der Ansprüche 1 bis 37 oder Sonde nach Anspruch 38, wobei es sich bei der Gruppe von nicht-viralen Organismen um die Bakterien des Mycobacterium tuberculosis-Komplexes handelt und die variable Region den Basen 540-575 von E. coli 23S-rRNA oder dem Komplement davon entspricht.

**74.** Verfahren nach einem der Ansprüche 1 bis 37 oder Sonde nach Anspruch 38, wobei es sich bei der Gruppe von nicht-viralen Organismen um die Bakterien des Mycobacterium tuberculosis-Komplexes handelt und die variable Region den Basen 1155-1190 von E. coli 23S-rRNA oder dem Komplement davon entspricht.

**75.** Verfahren nach einem der Ansprüche 1 bis 37 oder Sonde nach Anspruch 38, wobei es sich bei der Gruppe von nicht-viralen Organismen um die Bakterien des Mycobacterium tuberculosis-Komplexes handelt und die variable Region den Basen 2195-2235 von E. coli 23S-rRNA oder dem Komplement davon entspricht.

**76.** Sonde nach Anspruch 39, wobei es sich bei der Gruppe von Organismen um die Gattung Mycobacterium handelt und das Oligonukleotid die folgende Sequenz umfaßt:
CCATGCACCACCTGCACACAGGCCACAAGG.

**77.** Sonde nach Anspruch 39, wobei es sich bei der Gruppe von Organismen um die Gattung Mycobacterium handelt und das Oligonukleotid die folgende Sequenz umfaßt:
GGCTTGCCCCAGTATTACCACTGACTGGTACGG

**78.** Sonde nach Anspruch 39, wobei es sich bei der Gruppe von Organismen um die Gattung Mycobacterium handelt und das Oligonukleotid die folgende Sequenz umfaßt:
CACCGAATTCGCCTCAACCGGCTATGCGTCACCTC.

**79.** Sonde nach Anspruch 39, wobei es sich bei der Gruppe von Organismen um die Gattung Mycobacterium handelt und das Oligonukleotid die folgende Sequenz umfaßt:
GGGGTACGGCCCGTGTGTGTGCTCGCTAGAGGC

**80.** Verfahren nach einem der Ansprüche 1 bis 37 oder Sonde nach Anspruch 38, wobei es sich bei der Gruppe von Organismen um die Gattung Mycobacterium handelt und die variable Region den Basen 1025-1060 von E. coli 16S-rRNA oder dem Komplement davon entspricht.

**81.** Verfahren nach einem der Ansprüche 1 bis 37 oder Sonde nach Anspruch 38, wobei es sich bei der Gruppe von Organismen um die Gattung Mycobacterium handelt und die variable Region den Basen 1440-1475 von E. coli 23S-rRNA oder dem Komplement davon entspricht.

**82.** Verfahren nach einem der Ansprüche 1 bis 37 oder Sonde nach Anspruch 38, wobei es sich bei der Gruppe von Organismen um die Gattung Mycobacterium handelt und die variable Region den Basen 1515-1555 von E. coli 23S-rRNA oder dem Komplement davon entspricht.

**83.** Verfahren nach einem der Ansprüche 1 bis 37 oder Sonde nach Anspruch 38, wobei es sich bei der Gruppe von Organismen um die Gattung Mycobacterium handelt und die variable Region den Basen 1570-1610 von E. coli 23S-rRNA oder dem Komplement davon entspricht.

**84.** Sonde nach Anspruch 39, wobei es sich bei dem Organismus um Mycoplasma pneumoniae handelt und das Oligonukleotid die folgende Sequenz umfaßt:
GCTTGGTGCTTTCCTATTCTCACTGAAACAGCTACATTCGGC

**85.** Sonde nach Anspruch 39, wobei es sich bei dem Organismus um Mycoplasma pneumoniae handelt und das Oligonukleotid die folgende Sequenz umfaßt:
AATAACGAACCCTTGCAGGTCCTTTCAACTTTGAT

**86.** Sonde nach Anspruch 39, wobei es sich bei dem Organismus um Mycoplasma pneumoniae handelt und das Oligonukleotid die folgende Sequenz umfaßt:
CAGTCAAACTCTAGCCATTACCTGCTAAAGTCATT

**87.** Sonde nach Anspruch 39, wobei es sich bei dem Organismus um Mycoplasma pneumoniae handelt und das Oligonukleotid die folgende Sequenz umfaßt:
TACCGAGGGGATCGCCCCGACAGCTAGTAT

**88.** Sonde nach Anspruch 39, wobei es sich bei dem Organismus um Mycoplasma pneumoniae handelt und das Oligonukleotid die folgende Sequenz umfaßt:
CTTTACAGATTTGCTCACTTTTACAAGCTCGCGAC

**89.** Verfahren nach einem der Ansprüche 1 bis 37 oder Sonde nach Anspruch 38, wobei es sich bei dem Organismus um Mycoplasma pneumoniae handelt und die variable Region den Basen 190-230 von E. coli 16S-rRNA oder dem Komplement davon entspricht.

**90.** Verfahren nach einem der Ansprüche 1 bis 37 oder Sonde nach Anspruch 38, wobei es sich bei dem Organismus um Mycoplasma pneumoniae handelt und die variable Region den Basen 450-490 von E. coli 16S-rRNA oder dem Komplement davon entspricht.

**91.** Verfahren nach einem der Ansprüche 1 bis 37 oder Sonde nach Anspruch 38, wobei es sich bei dem Organismus um Mycoplasma pneumoniae handelt und die variable Region den Basen 820-860 von E. coli 16S-rRNA oder dem Komplement davon entspricht.

**92.** Verfahren nach einem der Ansprüche 1 bis 37 oder Sonde nach Anspruch 38, wobei es sich bei dem Organismus um Mycoplasma pneumoniae handelt und die variable Region den Basen 1255-1290 von E. coli 16S-rRNA oder dem Komplement davon entspricht.

**93.** Verfahren nach einem der Ansprüche 1 bis 37 oder Sonde nach Anspruch 38, wobei es sich bei dem Organismus um Mycoplasma pneumoniae handelt und die variable Region den Basen 65-120 von E. coli 5S-rRNA oder dem Komplement davon entspricht.

**94.** Sonde nach Anspruch 39, wobei es sich bei der Gruppe von Organismen um die Gattung Legionella handelt und das Oligonukleotid die folgende Sequenz umfaßt:
TACCCTCTCCCATACTCGAGTCAACCAGTATTATCTGACC

**95.** Sonde nach Anspruch 39, wobei es sich bei der Gruppe von Organismen um die Gattung Legionella handelt und das Oligonukleotid die folgende Sequenz umfaßt:
GGATTTCACGTGTCCCGGCCTACTTGTTCGGGTGCGTAGTTC.

**96.** Sonde nach Anspruch 39, wobei es sich bei der Gruppe von Organismen um die Gattung Legionella handelt und das Oligonukleotid die folgende Sequenz umfaßt:
CATCTCTGCAAAATTCACTGTATCTCAAGGCTAGGTAAGG

**97.** Sonde nach Anspruch 39, wobei es sich bei der Gruppe von Organismen um die Gattung Legionella handelt und das Oligonukleotid die folgende Sequenz umfaßt:
GCGGTACOCTTCTCTATAACTTATGGCTACC

**98.** Sonde nach Anspruch 39, wobei es sich bei der Gruppe von Organismen um die Gattung Legionella handelt und das Oligonukleotid die folgende Sequenz umfaßt:
GTA CCGAGGGTACCTTTGTGCT

**99.** Sonde nach Anspruch 39, wobei es sich bei der Gruppe von Organismen um die Gattung Legionella handelt und das Oligonukleotid die folgende Sequenz umfaßt:
CACTCTTGGTACGATGTCCGAC

**100.** Verfahren nach einem der Ansprüche 1 bis 37 oder Sonde nach Anspruch 38, wobei es sich bei der Gruppe von Organismen um die Gattung Legionella handelt und die variable Region den Basen 630-675 von E. coli 16S-rRNA oder dem Komplement davon entspricht.

**101.** Verfahren nach einem der Ansprüche 1 bis 37 oder Sonde nach Anspruch 38, wobei es sich bei der Gruppe von Organismen um die Gattung Legionella handelt und die variable Region den Basen 975-1020 von E. coli 16S-rRNA oder dem Komplement davon entspricht.

**102.** Verfahren nach einem der Ansprüche 1 bis 37 oder Sonde nach Anspruch 38, wobei es sich bei der Gruppe von Organismen um die Gattung Legionella handelt und die variable Region den Basen 350-395 von E. coli 23S-rRNA oder dem Komplement davon entspricht.

**103.** Verfahren nach einem der Ansprüche 1 bis 37 oder Sonde nach Anspruch 38, wobei es sich bei der Gruppe von Organismen um die Gattung Legionella handelt und die variable Region den Basen 1585-1620 von E. coli 23S-rRNA oder dem Komplement davon entspricht.

**104.** Verfahren nach einem der Ansprüche 1 bis 37 oder Sonde nach Anspruch 38, wobei es sich bei der Gruppe von Organismen um die Gattung Legionella handelt und die variable Region den Basen 2280-2330 von E. coli 23S-rRNA oder dem Komplement davon entspricht.

**105.** Sonde nach Anspruch 39, wobei es sich bei dem Organismus um Chlamydia trachomatis handelt und das Oligonukleotid die folgende Sequenz umfaßt:
CCGACTCGGGGTTGAGCCCATCTTTGACAA

**106.** Sonde nach Anspruch 39, wobei es sich bei dem Organismus um Chlamydia trachomatis handelt und das Oligonukleotid die folgende Sequenz umfaßt:
TTACGTCCGACACGGATGGGGTTGAGACCATC

**107.** Sonde nach Anspruch 39, wobei es sich bei dem Organismus um Chlamydia trachomatis handelt und das Oligonukleotid die folgende Sequenz umfaßt:
CCGCCACTAAACAATCGTCGAAACAATTGCTCCGTTCGA

**108.** Sonde nach Anspruch 39, wobei es sich bei dem Organismus um Chlamydia trachomatis handelt und das Oligonukleotid die folgende Sequenz umfaßt:
CGTTACTCGGATGCCCAAATATCGCCACATTCG

**109.** Sonde nach Anspruch 39, wobei es sich bei dem Organismus um Chlamydia trachomatis handelt und das Oligonukleotid die folgende Sequenz umfaßt:
CATCCATCTTTCCAGATGTGTTCAACTAGGAGTCCTGATCC

**110.** Sonde nach Anspruch 39, wobei es sich bei dem Organismus um Chlamydia trachomatis handelt und das Oligonukleotid die folgende Sequenz umfaßt:
GAGGTCGGTCTTTCTCTCCTTTCGTCTACG

**111.** Sonde nach Anspruch 39, wobei es sich bei dem Organismus um Chlamydia trachomatis handelt und das Oligonukleotid die folgende Sequenz umfaßt:
CCGTTCTCATCGCTCTACGGACTCTTCCAATCG

**112.** Sonde nach Anspruch 39, wobei es sich bei dem Organismus um Chlamydia trachomatis handelt und das Oligonukleotid die folgende Sequenz umfaßt:
CGAAGATTCCCCTTGATCGCGACCTGATCT

**113.** Sonde nach Anspruch 39, wobei es sich bei dem Organismus um Chlamydia trachomatis handelt und das Oligonukleotid die folgende Sequenz umfaßt:
CCGGGGCTCCTATCGTTCCATAGTCACCCTAAAAG

**114.** Sonde nach Anspruch 39, wobei es sich bei dem Organismus um Chlamydia trachomatis handelt und das Oligonukleotid die folgende Sequenz umfaßt:
TACCGCGTGTCTTATCGACACACCCGCG

**115.** Verfahren nach einem der Ansprüche 1 bis 37 oder Sonde nach Anspruch 38, wobei es sich bei dem Organismus um Chlamydia trachomatis handelt und die variable Region den Basen 60-105 von E. coli 16S-rRNA oder dem Komplement davon entspricht.

**116.** Verfahren nach einem der Ansprüche 1 bis 37 oder Sonde nach Anspruch 38, wobei es sich bei dem Organismus um Chlamydia trachomatis handelt und die variable Region den Basen 600-635 von E. coli 16S-rRNA oder dem Komplement davon entspricht.

**117.** Verfahren nach einem der Ansprüche 1 bis 37 oder Sonde nach Anspruch 38, wobei es sich bei dem Organismus um Chlamydia trachomatis handelt und die variable Region den Basen 830-870 von E. coli 16S-rRNA oder dem Komplement davon entspricht.

**118.** Verfahren nach einem der Ansprüche 1 bis 37 oder Sonde nach Anspruch 38, wobei es sich bei dem Organismus um Chlamydia trachomatis handelt und die variable Region den Basen 275-320 von E. coli 23S-rRNA oder dem Komplement davon entspricht.

**119.** Verfahren nach einem der Ansprüche 1 bis 37 oder Sonde nach Anspruch 38, wobei es sich bei dem Organismus um Chlamydia trachomatis handelt und die variable Region den Basen 330-365 von E. coli 23S-rRNA oder dem Komplement davon entspricht.

**120.** Verfahren nach einem der Ansprüche 1 bis 37 oder Sonde nach Anspruch 38, wobei es sich bei dem Organismus um Chlamydia trachomatis handelt und die variable Region den Basen 1160-1190 von E. coli 23S-rRNA oder dem Komplement davon entspricht.

**121.** Verfahren nach einem der Ansprüche 1 bis 37 oder Sonde nach Anspruch 38, wobei es sich bei dem Organismus um Chlamydia trachomatis handelt und die variable Region den Basen 1450-1490 von E. coli 23S-rRNA oder dem Komplement davon entspricht.

**122.** Verfahren nach einem der Ansprüche 1 bis 37 oder Sonde nach Anspruch 38, wobei es sich bei dem Organismus um Chlamydia trachomatis handelt und die variable Region den Basen 1510-1545 von E. coli 23S-rRNA oder dem Komplement davon entspricht.

**123.** Verfahren nach einem der Ansprüche 1 bis 37 oder Sonde nach Anspruch 38, wobei es sich bei dem Organismus um Chlamydia trachomatis handelt und die variable Region den Basen 1710-1750 von E. coli 23S-rRNA oder dem Komplement davon entspricht.

**124.** Sonde nach Anspruch 39, wobei es sich bei der Gruppe von Organismen um die Gattung Campylobacter handelt und das Oligonukleotid die folgende Sequenz umfaßt:
GGTTCTTAGGATATCAAGCCCAGG

**125.** Verfahren nach einem der Ansprüche 1 bis 37 oder Sonde nach Anspruch 38, wobei es sich bei der Gruppe von Organismen um die Gattung Campylobacter handelt und die variable Region den Basen 980-1010 von E. coli 16S-rRNA oder dem Komplement davon entspricht.

**126.** Sonde nach Anspruch 39, wobei es sich bei der Gruppe von Organismen um die als Enterococci bekannte Untergruppe der Gattung Streptococci handelt und das Oligonukleotid die folgende Sequenz umfaßt:
TGCAGCACTGAAGGGCGGAAACCCTCCAACACTTA

**127.** Verfahren nach einem der Ansprüche 1 bis 37 oder Sonde nach Anspruch 38, wobei es sich bei der Gruppe von Organismen um die als Enterococci bekannte Untergruppe der Gattung Streptococci handelt und die variable Region den Basen 825-860 von E. coli 16S-rRNA oder dem Komplement davon entspricht.

**128.** Sonde nach Anspruch 39, wobei es sich bei der Gruppe von Organismen um die als Pseudomonas-Gruppe 1 bekannte Gattungsuntergruppe handelt und das Oligonukleotid die folgende Sequenz umfaßt:
CAGACAAAGTTTCTCGTGCTCCGTCCT’ACTCGATT

**129.** Verfahren nach einem der Ansprüche 1 bis 37 oder Sonde nach Anspruch 38, wobei es sich bei der Gruppe von Organismen um die als Pseudomonas-Gruppe 1 bekannte Gattungsuntergruppe handelt und die variable Region den Basen 365-405 von E. coli 23S-rRNA oder dem Komplement davon entspricht.

**130.** Sonde nach Anspruch 39, wobei es sich bei dem Organismus um Enterobacter cloacae handelt und das Oligonukleotid die folgende Sequenz umfaßt:
GTGTGTTTTCGTGTACGGGAGTTTCACCC

**131.** Verfahren nach einem der Ansprüche 1 bis 37 oder Sonde nach Anspruch 38, wobei es sich bei dem Organismus um Enterobacter cloacae handelt und die variable Region den Basen 305-340 von E. coli 23S-rRNA oder dem Komplement davon entspricht.

**132.** Sonde nach Anspruch 39, wobei es sich bei dem Organismus um Proteus mirabilis handelt und das Oligonukleotid die folgende Sequenz umfaßt:
CCGTTCTCCTGACACTGCTATTGATTAAGACTC

**133.** Verfahren nach einem der Ansprüche 1 bis 37 oder Sonde nach Anspruch 38, wobei es sich bei dem Organismus um Proteus mirabilis handelt und die variable Region den Basen 270-305 von E. coli 23S-rRNA oder dem Komplement davon entspricht.

**134.** Sonde nach Anspruch 39, wobei es sich bei der Gruppe von Organismen um die Gattung Salmonella handelt und das Oligonukleotid die folgende Sequenz umfaßt: CTCCTTTGAGTTCCCGACCTAATCGCTGGC.

**135.** Sonde nach Anspruch 39, wobei es sich bei der Gruppe von Organismen um die Gattung Salmonella handelt und das Oligonukleotid die folgende Sequenz umfaßt: CTCATCGAGCTCACAGCACATGCGCTTTTGTGTA

**136.** Verfahren nach einem der Ansprüche 1 bis 37 oder Sonde nach Anspruch 38, wobei es sich bei der Gruppe von Organismen um die Gattung Salmonella handelt und die variable Region den Basen 1125-1155 von E. coli 16S-rRNA oder dem Komplement davon entspricht.

**137.** Verfahren nach einem der Ansprüche 1 bis 37 oder Sonde nach Anspruch 38, wobei es sich bei der Gruppe von Organismen um die Gattung Salmonella handelt und die variable Region den Basen 335-375 von E. coli 23S-rRNA oder dem Komplement davon entspricht.

**138.** Sonde nach Anspruch 39, wobei es sich bei dem Organismus um Escherichia coli handelt und das Oligonukleotid die folgende Sequenz umfaßt: GCACATTCTCATCTCTGAAAACTTCCGTGG

**139.** Verfahren nach einem der Ansprüche 1 bis 37 oder Sonde nach Anspruch 38, wobei es sich bei dem Organismus um Escherichia coli handelt und die variable Region den Basen 995-1030 von E. coli 16S-rRNA oder dem Komplement davon entspricht.

**140.** Sonde nach Anspruch 39, wobei es sich bei dem Organismus um Neisseria gonorrhoeae handelt und das Oligonukleotid die folgende Sequenz umfaßt: CCGCCGCTACCCGGTAC

**141.** Sonde nach Anspruch 39, wobei es sich bei dem Organismus um Neisseria gonorrhoeae handelt und das Oligonukleotid die folgende Sequenz umfaßt: TCATCGGCCGCCGATATTGGC.

**142.** Sonde nach Anspruch 39, wobei es sich bei dem Organismus um Neisseria gonorrhoeae handelt und das Oligonukleotid die folgende Sequenz umfaßt: GAGCATTCCGCACATGTCAAAACCAGGTA

**143.** Sonde nach Anspruch 39, wobei es sich bei dem Organismus um Neisseria gonorrhoeae handelt und das Oligonukleotid die folgende Sequenz umfaßt: GAGATTCCGCACATGTCAAAACCAGG

**144.** Sonde nach Anspruch 39, wobei es sich bei dem Organismus um Neisseria gonorrhoeae handelt und das Oligonukleotid die folgende Sequenz umfaßt: GAGGATTCCGCACATGTCAAAACCACGTAA

**145.** Sonde nach Anspruch 39, wobei es sich bei dem Organismus um Neisseria gonorrhoeae handelt und das Oligonukleotid die folgende Sequenz umfaßt: CCCGCTACCCGGTACGTTC.

**146.** Sonde nach Anspruch 39, wobei es sich bei dem Organismus um Neisseria gonorrhoeae handelt und das Oligonukleotid die folgende Sequenz umfaßt: CCGCTACCCGGTACGTTC

**147.** Verfahren nach einem der Ansprüche 1 bis 37 oder Sonde nach Anspruch 38, wobei es sich bei dem Organismus um Neisseria gonorrhoeae handelt und die variable Region den Basen 125-150 von E. coli 16S-rRNA oder dem Komplement davon entspricht.

**148.** Verfahren nach einem der Ansprüche 1 bis 37 oder Sonde nach Anspruch 38, wobei es sich bei dem Organismus um Neisseria gonorrhoeae handelt und die variable Region den Basen 455-485 von E. coli 16S-rRNA oder dem Komplement davon entspricht.

**149.** Verfahren nach einem der Ansprüche 1 bis 37 oder Sonde nach Anspruch 38, wobei es sich bei dem Organismus um Neisseria gonorrhoeae handelt und die variable Region den Basen 980-1015 von E. coli 16S-rRNA oder dem Komplement davon entspricht.

**150.** Nukleinsäurehybrid, gebildet zwischen einem Nukleotidpolymer oder Oligonukleotid nach einem der Ansprüche 47-56, 62, 64, 72-75, 80-83, 89-93, 100-104, 115-123, 125, 127, 129, 131, 133, 136, 137, 139 und 147-149 und einer dazu komplementären Nukleinsäure.

**151.** Nukleotidpolymer, welches unter stringenten Bedingungen an eine Sonde nach einem der Ansprüche 40-46, 56-61, 63, 65-71, 76-79, 84-88, 94-99, 105-114, 124, 126, 128, 130, 132, 134, 135, 138 und 140-146 oder das Komplement davon hybridisiert.

**152.** Nukleinsäurehybrid nach Anspruch 150, gebildet zwischen einem Oligonukleotid und einer dazu komplementären Nukleinsäure.

**153.** Komplement zu dem Nukleotidpolymer nach Anspruch 151.

**154.** Hybridisierungsassay, welcher das Miteinander-Umsetzen jeglicher rRNA oder des Komplements davon aus einer hinsichtlich eines nicht-viralen Organismus oder einer Gruppe von nicht-viralen Organismen zu testenden Probe mit einer Sonde nach einem der Ansprüche 39 bis 149,
wobei die Sonde zu mindestens etwa 75% komplementär zu einer variablen Region von rRNA oder rDNA ist, welche so ausgewählt ist, daß sie für den nicht-viralen Organismus oder eine Gruppe von nicht-viralen Organismen einzigartig ist, unter solchen Bedingungen, daß die Hybridisierung zwischen der Oligonukleotidsonde oder dem Nukleotidpolymer und irgendeiner ausreichend komplementären Proben-rRNA oder dem Komplement davon stattfinden kann, und das Beobachten und/oder Messen der Hybridisierung umfaßt.

**155.** Assay nach Anspruch 154, wobei die Hybridisierung zwischen der Oligonukleotidsonde und jeglicher Ziel-Proben-rRNA sich auf mindestens etwa 10% bis etwa 100% beläuft.

**156.** Assay nach Anspruch 154, wobei die Oligonukleotidsonde cDNA ist.

**157.** Assay nach Anspruch 154, wobei die Bedingungen eine Temperatur von etwa 25°C unterhalb der Tm bis etwa 1 °C unterhalb der Tm umfassen.

**158.** Assay nach Anspruch 154, der ferner den Parallelassay einer homologen Positivkontrolle oder einer heterologen Positivkontrolle oder beides umfaßt.

**159.** Assay nach Anspruch 154, der ferner den Parallelassay einer Negativkontrolle umfaßt.

**160.** Assay nach Anspruch 154, wobei die Bedingungen Mittel für gesteigerte Hybridisierungsraten umfassen.

**161.** Assay nach Anspruch 154, wobei die Bedingungen so sind, daß die maximale Hybridisierung zwischen der Oligonukleotidsonde und irgendeiner komplementären Proben-rRNA und eine minimale Kreuzreaktivität zwischen der Oligonukleotidsonde und irgendeiner nicht-komplementären Proben-rRNA gefördert wird.

**162.** Assay nach Anspruch 154, wobei die Oligonukleotidsonde markiert ist.

**163.** Assay nach Anspruch 154, wobei die Oligonukleotidsonde mit einer Isotop-, Nicht-Isotop- oder Chemolumineszenz-Markierung markiert ist.

**164.** Assay nach Anspruch 154, der ferner die Freisetzung von rRNA aus den Zellen des nicht-viralen Organismus oder der Gruppe nicht-viraler Organismen vor dem Schritt des Miteinander-Umsetzens umfaßt.

**165.** Assay nach Anspruch 154, wobei es sich bei dem nicht-viralen Organismus oder der Gruppe nicht-viraler Organismen um Mycobacterium avium, Mycobacterium intracellulare, die Bakterien des Mycobacterium tuberculosis-Komplexes, die Gattung Mycobacterium, Mycoplasma pneumoniae, Legionella, Neisseria gonorrhoeae, Salmonella, Chlamydia trachomatis, Campylobacter, Proteus mirabilis, Enterococcus, Enterobacter cloacae, Escherichia coli, Pseudomonas-Gruppe 1, Bakterien oder Pilze handelt.

**166.** Assay nach Anspruch 162, wobei die markierte Oligonukleotidsonde etwa 20 Nukleotide bis etwa 50 Nukleotide lang ist.

**167.** Assay nach Anspruch 162, wobei die markierte Oligonukleotidsonde zu mindestens etwa 95% komplementär zu der variablen rRNA-Region ist.

**168.** Assay nach Anspruch 154, der ferner die Verwendung einer oder mehrerer zusätzlicher Oligonukleotidsonden von mindestens etwa 10 Nukleotiden Länge umfaßt, welche zu mindestens etwa 75% komplementär zu einer oder mehreren zusätzlichen variablen Region(en) von rRNA sind, die ausgewählt sind, um für die nicht-viralen Organismen einzigartig zu sein.

**169.** Assay nach Anspruch 154, der ferner die Verwendung einer oder mehrerer zusätzlicher Sonden umfaßt, die einen oder mehrere zusätzliche nicht-virale Organismen identifizieren, wodurch die Gruppe der zu testenden nicht-viralen Organismen erweitert wird.

## Revendications

**1.** Méthode de préparation d'une sonde destinée à être utilisée dans un test d'hybridation qualitatif ou quantitatif, qui comprend la construction d'un oligonucléotide qui est suffisamment complémentaire pour s'hybrider, dans des conditions d'hybridation, à une séquence d'ARNr choisie pour être caractéristique d'un organisme non viral ou d'un groupe d'organismes non viraux que l'on cherche à détecter, ledit oligonucléotide étant choisi :

a) en identifiant une ou plusieurs régions variables candidates en comparant une ou plusieurs séquences d'ARNr de régions variables provenant d'un organisme cible non viral ou d'un groupe d'organismes cibles non viraux avec une ou plusieurs séquences d'ARNr de régions variables provenant de l'organisme connu le plus étroitement apparenté pour sélectionner une séquence unique à l'ARNr du ou des organismes cibles ; et
b) en synthétisant un oligonucléotide complémentaire de la séquence d'ARNr unique, la stabilité thermique des hybrides sonde : acide nucléique cible étant supérieure à la stabilité thermique des hybrides sonde : acide nucléique non cible ;

dans laquelle ledit ou lesdits organismes cibles sont choisis parmi une ou plusieurs espèces dans un genre ou un ou plusieurs genres dans une famille.

**2.** Méthode suivant la revendication 1, dans laquelle ladite région d'ARNr est choisie pour avoir au moins environ une différence de séquence d'une base par rapport à une séquence d'ARNr correspondante de l'organisme connu le plus étroitement apparenté audit organisme non viral ou groupe d'organismes non viraux que l'on cherche à détecter.

**3.** Méthode suivant la revendication 1, dans laquelle ladite région d'ARNr est choisie pour avoir une différence de séquence de bases approximativement égale ou supérieure à 10 % par rapport à la séquence d'ARNr correspondante de l'organisme connu le plus étroitement apparenté audit organisme non viral ou groupe d'organismes non viraux que l'on cherche à détecter.

**4.** Méthode suivant la revendication 1, dans laquelle ladite région d'ARNr est choisie dans le groupe constitué des ARNr 5S, 16S et 23S.

**5.** Méthode suivant la revendication 1, dans laquelle ladite région d'ARNr est choisie dans le groupe constitué des ARNr 5, 0S, 5, 8S, 18S et 28S.

**6.** Méthode suivant la revendication 1, dans laquelle ledit oligonucléotide a une longueur d'au moins environ 10 nucléotides.

**7.** Méthode suivant la revendication 1, dans laquelle ledit oligonucléotide a une longueur d'au moins environ 15 nucléotides.

**8.** Méthode suivant la revendication 1, dans laquelle ledit oligonucléotide a une longueur d'au moins environ 20 nucléotides.

**9.** Méthode suivant la revendication 1, dans laquelle ledit oligonucléotide a une longueur d'au moins environ 30 nucléotides.

**10.** Méthode suivant la revendication 1, dans laquelle ledit oligonucléotide a une longueur d'environ 20 nucléotides à environ 50 nucléotides.

**11.** Méthode suivant la revendication 1, dans laquelle ledit oligonucléotide a une longueur d'environ 30 nucléotides à environ 50 nucléotides.

**12.** Méthode suivant la revendication 2, dans laquelle ledit oligonucléotide a une longueur d'au moins environ 10 nucléotides.

**13.** Méthode suivant la revendication 2, dans laquelle ledit oligonucléotide a une longueur d'au moins environ 15 nucléotides.

**14.** Méthode suivant la revendication 2, dans laquelle ledit oligonucléotide a une longueur d'au moins environ 20 nucléotides.

**15.** Méthode suivant la revendication 2, dans laquelle ledit oligonucléotide a une longueur d'au moins environ 30 nucléotides.

**16.** Méthode suivant la revendication 2, dans laquelle ledit oligonucléotide a une longueur d'environ 20 nucléotides à environ 50 nucléotides.

**17.** Méthode suivant la revendication 2, dans laquelle ledit oligonucléotide a une longueur d'environ 30 nucléotides à environ 50 nucléotides.

**18.** Méthode suivant la revendication 2, dans laquelle ledit oligonucléotide a une longueur d'au moins environ 10 nucléotides.

**19.** Méthode suivant la revendication 3, dans laquelle ledit oligonucléotide a une longueur d'au moins environ 15 nucléotides.

**20.** Méthode suivant la revendication 3, dans laquelle ledit oligonucléotide a une longueur d'au moins environ 20 nucléotides.

**21.** Méthode suivant la revendication 3, dans laquelle ledit oligonucléotide a une longueur d'au moins environ 30 nucléotides.

**22.** Méthode suivant la revendication 3, dans laquelle ledit oligonucléotide a une longueur d'environ 20 nucléotides à environ 50 nucléotides.

**23.** Méthode suivant la revendication 3, dans laquelle ledit oligonucléotide a une longueur d'environ 30 nucléotides à environ 50 nucléotides.

**24.** Méthode suivant la revendication 1, dans laquelle ladite sonde est au moins environ à 75 % complémentaire de ladite région d'ARNr.

**25.** Méthode suivant la revendication 2, dans laquelle ledit oligonucléotide est au moins environ à 75 % complémentaire de ladite région d'ARNr.

**26.** Méthode suivant la revendication 3, dans laquelle ledit oligonucléotide est au moins environ à 75 % complémentaire de ladite région d'ARNr.

**27.** Méthode suivant la revendication 1, dans laquelle ladite sonde est parfaitement complémentaire de ladite région d'ARNr.

**28.** Méthode suivant la revendication 2, dans laquelle ladite sonde est parfaitement complémentaire de ladite région d'ARNr.

**29.** Méthode suivant la revendication 3, dans laquelle ladite sonde est parfaitement complémentaire de ladite région d'ARNr.

**30.** Méthode de préparation d'une sonde ou d'une combinaison de sondes, destinée à être utilisée dans un test d'hybridation qualitatif ou quantitatif, qui comprend la construction d'un polymère nucléotidique qui est suffisamment complémentaire pour s'hybrider à une région d'ADNr ou d'ARNr choisie pour distinguer un organisme non viral cible ou un premier groupe d'organismes non viraux que l'on cherche à détecter d'au moins un organisme non cible ou d'un second groupe d'organismes non cibles qui peuvent être présents dans un échantillon, lesdits organismes non cibles ou ledit groupe d'organismes non cibles étant des relations phylogénétiques proches desdits organismes cibles ou dudit groupe d'organismes cibles, ladite région d'ADNr ou d'ARNr étant choisie par les étapes consistant:

- à comparer une ou plusieurs séquences de bases nucléotidiques d'ADNr ou d'ARNr dudit organisme non viral

ou groupe d'organismes non viraux que l'on cherche à détecter avec une ou plusieurs séquences de bases nucléotidiques d'ADNr ou d'ARNr desdits organismes non cibles ou groupe d'organismes non cibles ;

- à aligner lesdites séquences de bases nucléotidiques d'ADNr ou d'ARNr dudit organisme non viral ou groupe d'organismes non viraux avec lesdites séquences de bases nucléotidiques d'ADNr ou d'ARNr desdits organismes non cibles

ou groupe d'organismes non cibles de façon à identifier les régions d'homologie ; et

- à sélectionner ledit polymère nucléotidique en rendant maximale l'homologie dudit polymère nucléotidique avec les régions dudit ADNr ou ARNr dudit organisme non viral ou groupe d'organismes non viraux que l'on cherche à détecter, tout en rendant minimale l'homologie dudit polymère nucléotidique avec les séquences d'ADNr ou d'ARNr dudit organisme non cible ou groupe d'organismes non cibles que l'on cherche à distinguer des premiers ; ledit organisme ou groupe d'organismes que l'on cherche à détecter étant choisi parmi une ou plusieurs espèces dans un genre ou un ou plusieurs genres dans une famille.

**31.** Méthode suivant la revendication 30, dans laquelle ledit polymère nucléotidique est homologue à au moins environ 90 % avec les régions dudit ADNr ou ARNr dudit organisme non viral ou groupe d'organismes non viraux que l'on cherche à détecter.

**32.** Méthode suivant la revendication 30, dans laquelle ledit oligonucléotide sonde est homologue à moins d'environ 90 % avec les séquences d'ADNr ou d'ARNr des relations phylogénétiques proches que l'on cherche à distinguer des premières.

**33.** Méthode suivant l'une quelconque des revendications 30 à 32, comprenant l'étape supplémentaire consistant à vérifier l'absence de réactivité croisée de ladite sonde en hybridant ledit oligonucléotide sonde aux organismes non viraux ou groupes d'organismes non viraux que l'on cherche à distinguer par ladite sonde.

**34.** Méthode de préparation d'une sonde destinée à être utilisée dans un test d'hybridation qualitatif ou quantitatif, qui comprend la construction d'un oligonucléotide qui est suffisamment complémentaire pour s'hybrider à une région d'ADNr ou d'ARNr choisie pour être caractéristique d'un organisme non viral ou d'un groupe d'organismes non viraux que l'on cherche à détecter, ladite région d'ADNr ou d'ARNr étant choisie par les étapes consistant :

- à comparer une ou plusieurs séquences de bases nucléotidiques d'ADNr ou d'ARNr dudit organisme non viral

ou groupe d'organismes non viraux que l'on cherche à détecter avec une ou plusieurs séquences de bases nucléotidiques d'ADNr ou d'ARNr de ses relations phylogénétiques les plus proches ;

- à aligner lesdites séquences de bases nucléotidiques d'ADNr ou d'ARNr dudit organisme non viral ou groupe d'organismes non viraux avec lesdites séquences d'ADNr ou d'ARNr desdites relations phylogénétiques les plus proches, de façon à révéler les régions d'ADNr ou d'ARNr hypervariables interespèces ;
• à sélectionner ledit oligonucléotide sonde dans ladite région hypervariable interespèce en rendant maximale l'homologie dudit oligonucléotide sonde avec les régions dudit ADNr ou ARNr dudit organisme non viral ou groupe d'organismes non viraux que l'on cherche à détecter, tout en rendant minimale l'homologie dudit oligonucléotide sonde avec les séquences d'ADNr ou d'ARNr desdites relations phylogénétiques les plus proches l'on cherche à distinguer des premiers ; dans laquelle ledit organisme ou groupe d'organismes que l'on cherche à détecter est choisi parmi une ou plusieurs espèces dans un genre ou un ou plusieurs genres dans une famille.

**35.** Méthode de la revendication 34, dans laquelle ledit oligonucléotide sonde est au moins à environ 90 % homologue avec les régions dudit ADNr ou ARNr dudit organisme non viral ou groupe d'organismes non viraux que l'on cherche à détecter.

**36.** Méthode de la revendication 34, dans laquelle ledit oligonucléotide sonde est homologue à moins d'environ 90 % avec les séquences d'ADNr ou d'ARNr desdites relations phylogénétiques proches que l'on cherche à distinguer des premiers.

**37.** Méthode de l'une quelconque des revendications 34 à 36, comprenant l'étape supplémentaire consistant à vérifier l'absence de réactivité croisée de ladite sonde en hybridant ledit oligonucléotide sonde aux organismes non viraux ou groupes d'organismes non viraux que l'on cherche à distinguer par ladite sonde.

**38.** Sonde polymère nucléotidique de test d'hybridation pour un organisme non viral ou un groupe d'organismes non viraux sous une forme sensiblement isolée, qui comprend un oligonucléotide de 10 à 100 nucléotides capable de s'hybrider dans des conditions d'hybridation stringentes à au moins 10 nucléotides dans une région variable d'ADNr ou d'ARNr choisie pour être caractéristique dudit organisme non viral
ou groupe d'organismes non viraux, dans laquelle ladite sonde s'hybride dans des conditions d'hybridation stringentes à un acide nucléique provenant d'une espèce d'organisme dans un genre et ne parvient pas à s'hybrider dans lesdites conditions à un acide nucléique provenant d'une autre espèce dans ledit genre, ou dans laquelle ladite sonde s'hybride dans des conditions d'hybridation stringentes à un acide nucléique provenant d'un ou plusieurs membres dudit groupe d'organismes et ne parvient pas à s'hybrider à un acide nucléique provenant d'organismes qui ne sont pas membres dudit groupe, ladite région variable correspondant à une région choisie dans le groupe consistant en :

les bases 60-100, 120-150, 600-670, 820-860, -980-1050 et 1250-1290 de l'ARNr 16S de E. coli ; 270-390, 535-560, 1150-1200, 1440-1600, 1710-1750 et 2190-2330 de l'ARNr 23S de E. coli ;
les bases 330-365 de l'ARNr 16S de E. coli, ledit polymère de nucléotides s'hybridant dans des conditions stringentes à des espèces du groupe phylogénétique des bactéries ;
les bases 675-715 de l'ARNr 16S de E. coli, ledit polymère de nucléotides s'hybridant dans des conditions stringentes à des espèces du groupe phylogénétique des bactéries ;
les bases 1080-1110 de l'ARNr 16S de E. coli, ledit polymère de nucléotides s'hybridant dans des conditions stringentes à des espèces du groupe phylogénétique des bactéries ;
les bases 460-490 de l'ARNr 23S de E. coli, ledit polymère de nucléotides s'hybridant dans des conditions stringentes à des espèces du groupe phylogénétique des bactéries ;
les bases 1050-1080 de l'ARNr 23S de E. coli, ledit polymère de nucléotides s'hybridant dans des conditions stringentes à des espèces du groupe phylogénétique des bactéries ;
les bases 1900-1960 de l'ARNr 23S de E. coli, ledit polymère de nucléotides s'hybridant dans des conditions stringentes à des espèces du groupe phylogénétique des bactéries ;
les bases 845-880 de l'ARNr 18 de S. cerevisiae, ledit polymère de nucléotides s'hybridant dans des conditions stringentes à des espèces du groupe phylogénétique des champignons ;
les bases 1960-2000 de l'ARNr 28S de S. cerevisiae, ledit polymère de nucléotides s'hybridant dans des conditions stringentes à des espèces du groupe phylogénétique des champignons ;
les bases 1225-1270 de l'ARNr 28S de S. cerevisiae, ledit polymère de nucléotides s'hybridant dans des conditions stringentes à des espèces du groupe phylogénétique des champignons ;
les bases 185-225 de l'ARNr 16S de E. coli, ledit polymère de nucléotides s'hybridant dans des conditions stringentes à Mycobacterium avium ;
les bases 185-225 de l'ARNr 16S de E. coli, ledit polymère de nucléotides s'hybridant dans des conditions stringentes à Mycobacterium intracellulare ;
les bases 185-225 de l'ARNr 16S de E. coli, ledit polymère de nucléotides s'hybridant dans des conditions stringentes à des espèces de bactéries du complexe à Mycobacterium tuberculosis ;

les bases 540-575 de l'ARNr 23S de E. coli, ledit polymère de nucléotides s'hybridant dans des conditions stringentes à des espèces de bactéries du complexe à Mycobacterium tuberculosis ;

les bases 1155-1190 de l'ARNr 23S de E. coli, ledit polymère de nucléotides s'hybridant dans des conditions stringentes à des espèces de bactéries du complexe à Mycobacterium tuberculosis;

les bases 2195-2235 de l'ARNr 23S de E. coli, ledit polymère de nucléotides s'hybridant dans des conditions stringentes à des espèces de bactéries du complexe à Mycobacterium tuberculosis ;

les bases 1025-1060 de l'ARNr 16S de E. coli, ledit polymère de nucléotides s'hybridant dans des conditions stringentes à des espèces du genre Mycobacterium ;

les bases 1440-1475 de l'ARNr 23S de E. coli, ledit polymère de nucléotides s'hybridant dans des conditions stringentes à des espèces du genre Mycobacterium ;

les bases 1515-1555 de l'ARNr 23S de E. coli, ledit polymère de nucléotides s'hybridant dans des conditions stringentes à des espèces du genre Mycobacterium ;

les bases 1570-1610 de l'ARNr 23S de E. coli, ledit polymère de nucléotides s'hybridant dans des conditions stringentes à des espèces du genre Mycobacterium ;

les bases 190-2-30 de l'ARNr 16S de E. coli, ledit polymère de nucléotides s'hybridant dans des conditions stringentes à Mycoplasma pneumoniae ;

les bases 450-490 de l'ARNr 16S de E. coli, ledit polymère de nucléotides s'hybridant dans des conditions stringentes à Mycoplasma pneumoniae ;

les bases 820-860 de l'ARNr 16S de E. coli, ledit polymère de nucléotides s'hybridant dans des conditions stringentes à Mycoplasma pneumoniae ;

les bases 1255-1290 de l'ARNr 16S de E. coli, ledit polymère de nucléotides s'hybridant dans des conditions stringentes à Mycoplasma pneumoniae ;

les bases 65-120 de l'ARNr 5S de E. coli, ledit polymère de nucléotides s'hybridant dans des conditions stringentes à Mycoplasma pneumoniae ;

les bases 630-675 de l'ARNr 16S de E. coli, ledit polymère de nucléotides s'hybridant dans des conditions stringentes à des espèces du genre Legionella ;

les bases 975-1020 de l'ARNr 16S de E. coli, ledit polymère de nucléotides s'hybridant dans des conditions stringentes à des espèces du genre Legionella ;

les bases 350-395 de l'ARNr 23S de E. coli, ledit polymère de nucléotides s'hybridant dans des conditions stringentes à des espèces du genre Legionella ;

les bases 1585-1620 de l'ARNr 23S de E. coli, ledit polymère de nucléotides s'hybridant dans des conditions stringentes à des espèces du genre Legionella ;

les bases 2280-2330 de l'ARNr 23S de E. coli, ledit polymère de nucléotides s'hybridant dans des conditions stringentes à des espèces du genre Legionella ;

les bases 60-105 de l'ARNr 16S de E. coli, ledit polymère de nucléotides s'hybridant dans des conditions stringentes à Chlamydia trachomatis ;

les bases 600-635 de l'ARNr 16S de E. coli, ledit polymère de nucléotides s'hybridant dans des conditions stringentes à Chlamydia trachomatis ;

les bases 830-870 de l'ARNr 16S de E. coli, ledit polymère de nucléotides s'hybridant dans des conditions stringentes à Chlamydia trachomatis ;

les bases 275-320 de l'ARNr 23S de E. coli, ledit polymère de nucléotides s'hybridant dans des conditions stringentes à Chlamydia trachomatis ;

les bases 330-365 de l'ARNr 23S de E. coli, ledit polymère de nucléotides s'hybridant dans des conditions stringentes à Chlamydia trachomatis ;

les bases 1160-1190 de l'ARNr 23S de E. coli, ledit polymère de nucléotides s'hybridant dans des conditions stringentes à Chlamydia trachomatis ;

les bases 1450-1490 de l'ARNr 23S de E. coli, ledit polymère de nucléotides s'hybridant dans des conditions stringentes à Chlamydia trachomatis ;

les bases 1510-1545 de l'ARNr 23S de E. coli, ledit polymère de nucléotides s'hybridant dans des conditions stringentes à Chlamydia trachomatis ;

les bases 1710-1750 de l'ARNr 23S de E. coli, ledit polymère de nucléotides s'hybridant dans des conditions stringentes à Chlamydia trachomatis ;

les bases 980-1010 de l'ARNr 16S de E. coli, ledit polymère de nucléotides s'hybridant dans des conditions stringentes à des espèces du genre Campylobacter ;

les bases 825-860 de l'ARNr 16S de E. coli, ledit polymère de nucléotides s'hybridant dans des conditions stringentes à des espèces du groupe sous-générique de Streptococci connu sous le nom d'Enterococci ;

les bases 365-405 de l'ARNr 23S de E. coli, ledit polymère de nucléotides s'hybridant dans des conditions stringentes à des espèces du groupement sous-générique connu sous le nom de Pseudomonas du groupe 1 ;

les bases 305-340 de l'ARNr 16S de E. coli, ledit polymère de nucléotides s'hybridant dans des conditions stringentes à Enterobacter cloacae ;
les bases 270-305 der l'ARNr 23S de E. coli, ledit polymère de nucléotides s'hybridant dans des conditions stringentes à Proteus mirabilis ;
les bases 1125-1155 de l'ARNr 16S de E. coli, ledit groupe d'organismes appartenant au genre Salmonella ;
les bases 335-375 de l'ARNr 23S de E. coli, ledit polymère de nucléotides s'hybridant dans des conditions stringentes à des espèces du genre Salmonella ;
les bases 995-1030 de l'ARNr 16S de E. coli, ledit polymère de nucléotides s'hybridant dans des conditions stringentes à Escherichia coli ;
les bases 125-150 de l'ARNr 16S de E. coli, l'organisme étant Neisseria gonorrhoeae ;
les bases 445-485 de l'ARNr 16S de E. coli, ledit polymère de nucléotides s'hybridant dans des conditions stringentes à Neisseria gonorrhoeae ;
les bases 980-1015 de l'ARNr 16S de E. coli, ledit polymère de nucléotides s'hybridant dans des conditions stringentes à Neisseria gonorrhoeae ;
ou son complément.

**39.** Sonde polymère nucléotidique de test d'hybridation pour un organisme non viral ou un groupe d'organismes non viraux sous une forme sensiblement isolée, qui comprend un oligonucléotide de 10 à 100 nucléotides capable de s'hybrider dans des conditions d'hybridation stringentes à au moins 10 nucléotides dans une région variable d'ARNr ou d'ADNr choisie pour être caractéristique dudit organisme non viral ou groupe d'organismes non viraux, dans laquelle ladite sonde s'hybride dans des conditions d'hybridation stringentes à un acide nucléique provenant d'une espèce d'organismes dans un genre et ne parvient à s'hybrider dans lesdites conditions à un acide nucléique provenant d'une autre espèce dans ledit genre, ou dans laquelle ladite sonde s'hybride dans des conditions d'hybridation stringentes à un acide nucléique provenant d'un ou plusieurs membres dudit groupe d'organismes et ne parvient pas à s'hybrider à un acide nucléique provenant d'organismes qui ne sont pas des membres dudit groupe, ledit oligonucléotide comprenant une séquence choisie dans le groupe consistant en :
lorsque ledit groupe d'organismes est le groupe phylogénétique des bactéries, les séquences :

CCACTGCTGCCTCCCGTAGGAGTCTGGGCC;
CCAGATCTCTACGCATTTCACCGCTACACGTGG;
GCTCGTTGCGGGACTTAACCCAACAT;
GGGGTTCTTTTCGCCTTTCCCTCACGG;
GGCTGCTTCTAAGCCAACATCCTG;
GGACCGTTATAGTTACGGCCGCC;
GGTCGGAACTTACCCGACAAGGAATTTCGCTACC;
lorsque ledit groupe d'organismes non viraux est le groupe phylogénétique des champignons, les séquences :
CCCGACCGTCCCTATTAATCATTACGATGG;
CCCGACCGTCCCTATTAATCATTACGATGGTCCTAGAAAC;
CCACTTGGCATCAAAACTATTCCTTCCTGTGG;
GCTCTTCATTCAATTGTCCACGTTCAATTAAGCAACAAGG;
GCTCTGCATTCAAAGGTCCGCGTTCAATAAAGAAACAGGG;
lorsque ledit organisme est Mycobacterium avium, la séquence :
ACCGCAAAAGCTTTCCACCAGAAGACATGCGTCTTGAG;
lorsque ledit organisme est Mycobacterium intracellulare, la séquence :
ACCGCAAAAGCTTTCCACCTAAAGACATGCGCCTAAAG;
lorsque le groupe d'organismes non viraux est constitué des bactéries du complexe Mycobacterium tuberculosis, les séquences :
TAAAGCGCTTTCCACCACAAGACATGCATCCCGTG;
TGCCCTACCCACACCCACCACCAGGTGATGT;
CCATCACCACCCTCCTCCGGAGAGGAAAAGG;
CTGTCCCTAAACCCGATTCAGGGTTCGAGGTTAGATGC;
AGGCACTGTCCCTAAACCCGATTCAGGGTTC;
CCGCTAAAGCGCTTTCCACCACAAGAGATGCATCCCG;
ACACCGCTAAAGCGCTTTCCACCACAAGACATGCATC;
lorsque le groupe d'organismes non viraux est le genre Mycobacterium, les séquences :
CCATGCACCACCTGCACACAGGCCACAAGG;
GGCTTGCCCCAGTATTACCACTGACTGGTACGG;
CACCGAATTCGCCTCAACCGGCTATCCGTCACCTC;

GGGGTACGGCCCGTGTGTGTGCTCGCTAGAGGC;
lorsque ledit organisme est Mycoplasma pneumoniae, les séquences :
GCTTGGTGCTTTCCTATTCTCACTGAAACAGCTACATTCGGC;
AATAACGAACCCTTGCACGTCCTTTCAACTTTGAT;
CAGTCAAACTCTAGCCATTACCTGCTAAAGTCATT;
TACCGAGGGGATCGCCCCGACAGCTAGTAT;
CTTTACAGATTTGCTCACTTTTACAAGCTGGCGAC;
lorsque ledit groupe d'organismes est le genre Legionella, les séquences :
TACCCTCTCCCATACTCGAGTCAACCAGTATTATCTGACC;
GGATTTCACGTGTCCCGGCCTACTTGTTCGGGTGCGTAGTTC;
CATCTCTGCAAAATTCACTGTATGTCAAGGGTAGGTAAGG;
GCGGTACGGTTCTCTATAAGTTATGGCTAGC;
GTACCGAGGGTACCTTTGTGCT;
CACTCTTGGTACGATGTCCGAC;
lorsque ledit organisme est Chlamydia trachomatis, les séquences :
CCGACTCGGGGTTGAGCCCATCTTTGACAA;
TTACGTCCGACACGGATGGGGTTGAGACCATC;
CCGCCACTAAACAATCGTCGAAACAATTGCTCCGTTCGA;
CGTTACTCGGATGCCCAAATATCGCCACATTCG;
CATCCATCTTTCCAGATGTGTTCAACTAGGAGTCCTGATCC;
GAGGTCGGTCTTTCTCTCCTTTCGTCTACG;
CGGTTCTCATCGCTCTAGGGACTCTTCCAATCG;
CGAAGATTCCCCTTGATCGCGACCTGATCT;
CCGGGGCTCCTATCGTTCCATAGTCACCCTAAAAG;
TACCGCGTGTCTTATCGACACACCCGCG;
lorsque ledit groupe d'organismes est Campylobacter, la séquence :
GGTTCTTAGGATATCAAGCCCAGG;
lorsque ledit groupe d'organismes est le groupe sub-générique Streptococci connu sous le nom d'Enterococci, la séquence :
TGCAGCACTGAAGGGCGGAAACCCTCCAACACTTA,
lorsque ledit groupe d'organismes est le groupe sub-générique connu sous le nom de Pseudomonas du groupe 1, la séquence :
CAGACAAAGTTTCTCGTGCTCCCTCCTACTCGATT;
lorsque ledit organisme est Enterobacter cloacae, la séquence :
GTGTGTTTTCGTGTACGGGAGTTTCACCC;
lorsque ledit organisme est Proteus mirabilis, la séquence :
CCGTTCTCCTGACACTGCTATTGATTAAGACTC;
lorsque ledit groupe d'organismes est le genre Salmonella, les séquences :
CTCCTTTGAGTTCCCGACCTAATCGCTGGC;
CTCATGGAGCTGACAGCACATGCGCTTTTGTGTA;
lorsque ledit organisme est Escherichia coli, la séquence :
GCACATTCTCATCTCTGAAAACTTCCGTGG;
lorsque ledit organisme est Neisseria gonorrhoeae, les séquences :
CCGCCGCTACCCGGTAC;
TCATCGGCCGCCGATATTGGC;
GAGCATTCCGCACATGTCAAAACCAGGTA;
GAGATTCCGCACATGTCAAAACCAGG;
GAGGATTCCGCACATGTCAAAACCAGGTAA;
CCCGCTACCCGGTACGTTC;
CCGCTACCCGGTACGTTC.

**40.** Sonde suivant la revendication 39, dans laquelle ledit groupe d'organismes non viraux est le groupe phylogénétique des bactéries et ledit oligonucléotide comprend la séquence
CCACTGCTGCCTCCCGTAGGAGTCTGGGCC.

**41.** Sonde suivant la revendication 39, dans laquelle ledit groupe d'organismes non viraux est le groupe phylogénétique des bactéries et ledit oligonucléotide comprend la séquence
CCAGATCTCTACGCATTTCACCGCTACACGTGG.

**42.** Sonde suivant la revendication 39, dans laquelle ledit groupe d'organismes non viraux est le groupe phylogénétique des bactéries et ledit oligonucléotide comprend la séquence GCTCGTTGCGGGACTTAACCCAACAT,

**43.** Sonde suivant la revendication 39, dans laquelle ledit groupe d'organismes non viraux est le groupe phylogénétique des bactéries et ledit oligonucléotide comprend la séquence GCTCGTTGCGGGACTTAACCCAACAT.

**44.** Sonde suivant la revendication 39, dans laquelle ledit groupe d'organismes non viraux est le groupe phylogénétique des bactéries et ledit oligonucléotide comprend la séquence GGC TGCTTCTAAGCCAACATCCTG.

**45.** Sonde suivant la revendication 39, dans laquelle ledit groupe d'organismes non viraux est le groupe phylogénétique des bactéries et ledit oligonucléotide comprend la séquence GGA CCGTTATAGTTACGGCCGCC.

**46.** Sonde suivant la revendication 39, dans laquelle ledit groupe d'organismes non viraux est le groupe phylogénétique des bactéries et ledit oligonucléotide comprend la séquence OGTCGGAACTTACCCGACAAGGAATTTCGCTACC.

**47.** Sonde suivant la revendication 38, dans laquelle ledit groupe d'organismes non viraux est le groupe phylogénétique des bactéries et ladite région variable correspond aux bases 330-365 de l'ARNr 16S de E. coli, ou son complément.

**48.** Sonde suivant la revendication 38, dans laquelle ledit groupe d'organismes non viraux est le groupe phylogénétique des bactéries et ladite région variable correspond aux bases 675-715 de l'ARNr 16S de E. coli, ou son complément.

**49.** Sonde suivant la revendication 38, dans laquelle ledit groupe d'organismes non viraux est le groupe phylogénétique des bactéries et ladite région variable correspond aux bases 1080-1110 de l'ARNr 16S de E. coli, ou son complément.

**50.** Sonde suivant la revendication 38, dans laquelle ledit groupe d'organismes non viraux est le groupe phylogénétique des bactéries et ladite région variable correspond aux bases 460-490 de l'ARNr 23S de E. coli, ou son complément.

**51.** Sonde suivant la revendication 38, dans laquelle ledit groupe d'organismes non viraux est le groupe phylogénétique des bactéries et ladite région variable correspond aux bases 1050-1080 de l'ARNr 23S de E. coli, ou son complément.

**52.** Sonde suivant la revendication 38, dans laquelle ledit groupe d'organismes non viraux est le groupe phylogénétique des bactéries et ladite région variable correspond aux bases 1900-1960 de l'ARNr 23S de E. coli, ou son complément.

**53.** Sonde suivant la revendication 38, dans laquelle ledit groupe d'organismes non viraux est le groupe phylogénétique des champignons et ladite région variable correspond aux bases 845-880 de l'ARNr 18S de S. cerevisiae, ou son complément.

**54.** Sonde suivant la revendication 38, dans laquelle ledit groupe d'organismes non viraux est le groupe phylogénétique des champignons et ladite région variable correspond aux bases 1960-2000 de l'ARNr 28S de S. cerevisiae, ou son complément.

**55.** Sonde suivant la revendication 38, dans laquelle ledit groupe d'organismes non viraux est le groupe phylogénétique des champignons et ladite région variable correspond aux bases 1225-1270 de l'ARNr 28S de S. cerevisiae, ou son complément.

**56.** Sonde suivant la revendication 39, dans laquelle ledit groupe d'organismes non viraux est le groupe phylogénétique des champignons et ledit oligonucléotide comprend la séquence CCCGACCGTCCCTATTAATCATTACGATGG.

**57.** Sonde suivant la revendication 39, dans laquelle ledit groupe d'organismes non viraux est le groupe phylogénétique des champignons et ledit oligonucléotide comprend la séquence CCCGACCGTCCCTATTAATCATTACGATGGTCCTAGAAAC.

**58.** Sonde suivant la revendication 39, dans laquelle ledit groupe d'organismes non viraux est le groupe phylogénétique des champignons et ledit oligonucléotide comprend la séquence CGACTTGGCATGAAAACTATTCCTTCCTGTGG.

**59.** Sonde suivant la revendication 39, dans laquelle ledit groupe d'organismes non viraux est le groupe phylogénétique des champignons et ledit oligonucléotide comprend la séquence GCTCTTCATTCAATTGTCCACGTTCAATTAAGCAACAAGG.

**60.** Sonde suivant la revendication 39, dans laquelle ledit groupe d'organismes non viraux est le groupe phylogénétique des champignons et ledit oligonucléotide comprend la séquence GCTCTGCATTCAAAGGTCCGCGTTCAATAAAGAAACAGGG.

**61.** Sonde suivant la revendication 39, dans laquelle ledit organisme est Mycobacterium avium et ledit oligonucléotide comprend la séquence ACCGCAAAAGCTTTCC ACCAGAAGACATGCGTCTTGAG.

**62.** Méthode suivant l'une quelconque des revendications 1 à 37, ou sonde suivant la revendication 38, dans laquelle ledit organisme est Mycobacterium avium et dans laquelle ladite région variable correspond aux bases 185-225 de l'ARNr 16S de E. coli, ou son complément.

**63.** Sonde suivant la revendication 39, dans laquelle ledit organisme est Mycobacterium intracellulare et ledit oligonucléotide comprend la séquence ACCGCAAAAGCTTTCCACCTAAAGACATGCGCCTAAAG.

**64.** Méthode suivant l'une quelconque des revendications 1 à 37, ou sonde suivant la revendication 38, dans laquelle ledit organisme est Mycobacterium intracellulare et ladite région variable correspond aux bases 185-225 de l'ARNr 16S de E. coli, ou son complément.

**65.** Sonde suivant la revendication 39, dans laquelle ledit groupe d'organismes non viraux est constitué des bactéries du complexe à Mycobacterium tuberculosis et ledit oligonucléotide comprend la séquence TAAAGCGCTTTCCACCACAAGACATGCATCCCGTG.

**66.** Sonde suivant la revendication 39, dans laquelle ledit groupe d'organismes non viraux est constitué des bactéries du complexe à Mycobacterium tuberculosis et ledit oligonucléotide comprend la séquence TGCCCTACCCACACCCACCACCAGGTGATGT.

**67.** Sonde suivant la revendication 39, dans laquelle ledit groupe d'organismes non viraux est constitué des bactéries du complexe à Mycobacterium tuberculosis et ledit oligonucléotide comprend la séquence CCATCACCACCCTCCTCCGGAGAGGAAAAGG.

**68.** Sonde suivant la revendication 39, dans laquelle ledit groupe d'organismes non viraux est constitué des bactéries du complexe à Mycobacterium tuberculosis et ledit oligonucléotide comprend la séquence CTGTCCCTAAACCCGATTCAGGGTTCGAGGTTAGATGC.

**69.** Sonde suivant la revendication 39, dans laquelle ledit groupe d'organismes non viraux est constitué des bactéries du complexe à Mycobacterium tuberculosis et ledit oligonucléotide comprend la séquence AGGCACTGTCCCTAAACCCGATTCAGGGTTC.

**70.** Sonde suivant la revendication 39, dans laquelle ledit groupe d'organismes non viraux est constitué des bactéries du complexe à Mycobacterium tuberculosis et ledit oligonucléotide comprend la séquence CCGCTAAAGCGCTTTCCACCACAAGACATGCATCCCG.

**71.** Sonde suivant la revendication 39, dans laquelle ledit groupe d'organismes non viraux est constitué des bactéries du complexe à Mycobacterium tuberculosis et ledit oligonucléotide comprend la séquence ACACCGCTAAAGCGCMCCACCACAAGACATGCATC.

**72.** Méthode suivant l'une quelconque des revendications 1 à 37, ou sonde suivant la revendication 38, dans laquelle ledit groupe d'organismes non viraux est constitué des bactéries du complexe à Mycobacterium tuberculosis et ladite région variable correspond aux bases 185-225 de l'ARNr 16S de E. coli, ou son complément.

**73.** Méthode suivant l'une quelconque des revendications 1 à 37, ou sonde suivant la revendication 38, dans laquelle ledit groupe d'organismes non viraux est constitué des bactéries du complexe à Mycobacterium tuberculosis et ladite région variable correspond aux bases 540-575 de l'ARNr 23S de E. coli, ou son complément.

**74.** Méthode suivant l'une quelconque des revendications 1 à 37, ou sonde suivant la revendication 38, dans laquelle ledit groupe d'organismes non viraux est constitué des bactéries du complexe à Mycobacterium tuberculosis et ladite région variable correspond aux bases 1155-1190 de l'ARNr 23S de E. coli, ou son complément.

**75.** Méthode suivant l'une quelconque des revendications 1 à 37, ou sonde suivant la revendication 38, dans laquelle ledit groupe d'organismes non viraux est constitué des bactéries du complexe à Mycobacterium tuberculosis et ladite région variable correspond aux bases 2195-2235 de l'ARNr 23S de E. coli, ou son complément.

**76.** Sonde suivant la revendication 39, dans laquelle ledit groupe d'organismes est le genre Mycobacterium et ledit oligonucléotide comprend la séquence
CCATGCACCACCTGCACACAGGCCACAAGG.

**77.** Sonde suivant la revendication 39, dans laquelle ledit groupe d'organismes est le genre Mycobacterium et ledit oligonucléotide comprend la séquence
GGCTTGCCCCAGTATTACCACTGACTGGTACGG.

**78.** Sonde suivant la revendication 39, dans laquelle ledit groupe d'organismes est le genre Mycobacterium et ledit oligonucléotide comprend la séquence
CACCGAATTCGCCTCAACCGGCTATGCGTCACCTC.

**79.** Sonde suivant la revendication 39, dans laquelle ledit groupe d'organismes est le genre Mycobacterium et ledit oligonucléotide comprend la séquence
GGGGTACGGCCCGTGTGTGTGCTCGCTAGAGGC.

**80.** Méthode suivant l'une quelconque des revendications 1 à 37, ou sonde suivant la revendication 38, dans laquelle ledit groupe d'organismes est le genre Mycobacterium et ladite région variable correspond aux bases 1025-1060 de l'ARNr 16S de E. coli, ou son complément.

**81.** Méthode suivant l'une quelconque des revendications 1 à 37, ou sonde suivant la revendication 38, dans laquelle ledit groupe d'organismes est le genre Mycobacterium et ladite région variable correspond aux bases 1440-1475 de l'ARNr 23S de E. coli, ou son complément.

**82.** Méthode suivant l'une quelconque des revendications 1 à 37, ou sonde suivant la revendication 38, dans laquelle ledit groupe d'organismes est le genre Mycobacterium et ladite région variable correspond aux bases 1515-1555 de l'ARNr 23S de E. coli, ou son complément.

**83.** Méthode suivant l'une quelconque des revendications 1 à 37, ou sonde suivant la revendication 38, dans laquelle ledit groupe d'organismes est le genre Mycobacterium et ladite région variable correspond aux bases 1570-1610 de l'ARNr 23S de E. coli, ou son complément.

**84.** Sonde suivant la revendication 39, dans laquelle ledit organisme est Mycoplasma pneumoniae et ledit oligonucléotide comprend la séquence
GCTTGGTGCTTTCCTATTCTCACTGAAACAGCTACATTCGGC.

**85.** Sonde suivant la revendication 39, dans laquelle ledit organisme est Mycoplasma pneumoniae et ledit oligonucléotide comprend la séquence
AATAACGAAGCCTTGCAGGTCCTTTCAACTTTGAT.

**86.** Sonde suivant la revendication 39, dans laquelle ledit organisme est Mycoplasma pneumoniae et ledit oligonucléotide comprend la séquence
CAGTCAAACTCTAGCCATTACCTGCTAAAGTCATT.

**87.** Sonde suivant la revendication 39, dans laquelle ledit organisme est Mycoplasma pneumoniae et ledit oligonucléotide comprend la séquence
TACCGAGGGGATCGCCCCGACAGCTAGTAT.

**88.** Sonde suivant la revendication 39, dans laquelle ledit organisme est Mycoplasma pneumoniae et ledit oligonucléotide comprend la séquence
CTTTACAGATTTGCTCACTTTTACAAGCTGGCGAC.

**89.** Méthode suivant l'une quelconque des revendications 1 à 37, ou sonde suivant la revendication 38, dans laquelle ledit organisme est Mycoplasma pneumoniae et ladite région variable correspond aux bases 190-230 de l'ARNr 16S de E. coli, ou son complément.

**90.** Méthode suivant l'une quelconque des revendications 1 à 37, ou sonde suivant la revendication 38, dans laquelle ledit organisme est Mycoplasma pneumoniae et ladite région variable correspond aux bases 450-490 de l'ARNr 16S de E. coli, ou son complément.

**91.** Méthode suivant l'une quelconque des revendications 1 à 37, ou sonde suivant la revendication 38, dans laquelle ledit organisme est Mycoplasma pneumoniae et ladite région variable correspond aux bases 820-860 de l'ARNr 16S de E. coli, ou son complément.

**92.** Méthode suivant l'une quelconque des revendications 1 à 37, ou sonde suivant la revendication 38, dans laquelle ledit organisme est Mycoplasma pneumoniae et ladite région variable correspond aux bases 1255-1290 de l'ARNr 16S de E. coli, ou son complément.

**93.** Méthode suivant l'une quelconque des revendications 1 à 37, ou sonde suivant la revendication 38, dans laquelle ledit organisme est Mycoplasma pneumoniae et ladite région variable correspond aux bases 65-120 de l'ARNr 5S de E. coli, ou son complément.

**94.** Sonde suivant la revendication 39, dans laquelle ledit groupe d'organismes est le genre Legionella et ledit oligonucléotide comprend la séquence
TACCCTCTCCCATAGTCGAGTCAACCAGTATTATCTGACC.

**95.** Sonde suivant la revendication 39, dans laquelle ledit groupe d'organismes est le genre Legionella et ledit oligonucléotide comprend la séquence
GGATTTCACGTGTCCCCGGCCTACTTGTTCGGGTGCGTAGTTC.

**96.** Sonde suivant la revendication 39, dans laquelle ledit groupe d'organismes est le genre Legionella et ledit oligonucléotide comprend la séquence
CATCTCTGCAAAATTCACTGTATGTCAAGGGTAGGTAAGG.

**97.** Sonde suivant la revendication 39, dans laquelle ledit groupe d'organismes est le genre Legionella et ledit oligonucléotide comprend la séquence
GCGGTACGGTTCTCTATAAGTTATGGCTAGC.

**98.** Sonde suivant la revendication 39, dans laquelle ledit groupe d'organismes est le genre Legionella et ledit oligonucléotide comprend la séquence
GTACCGAGGGTACCTTTGTGCT.

**99.** Sonde suivant la revendication 39, dans laquelle ledit groupe d'organismes est le genre Legionella et ledit oligonucléotide comprend la séquence CACTCTTGGTACGATGTCCGAC.

**100.** Méthode suivant l'une quelconque des revendications 1 à 37, ou sonde suivant la revendication 38, dans laquelle ledit groupe d'organismes est le genre Legionella et ladite région variable correspond aux bases 630-675 de l'ARNr 16S de E. coli, ou son complément.

**101.** Méthode suivant l'une quelconque des revendications 1 à 37, ou sonde suivant la revendication 38, dans laquelle ledit groupe d'organismes est le genre Legionella et ladite région variable- corre-spond aux bases 975-1020 de l'ARNr 16S de E. coli, ou son complément.

**102.** Méthode suivant l'une quelconque des revendications 1 à 37, ou sonde suivant la revendication 38, dans laquelle ledit groupe d'organismes est le genre Legionella et ladite région variable correspond aux bases 350-395 de l'ARNr 23S de E. coli, ou son complément.

**103.** Méthode suivant l'une quelconque des revendications 1 à 37, ou sonde suivant la revendication 38, dans laquelle ledit groupe d'organismes est le genre Legionella et ladite région variable correspond aux bases 1585-1620 de l'ARNr 23S de E. coli, ou son complément.

**104.** Méthode suivant l'une quelconque des revendications 1 à 37, ou sonde suivant la revendication 38, dans laquelle ledit groupe d'organismes est le genre Legionella et ladite région variable correspond aux bases 2280-2330 de l'ARNr 23S de E. coli, ou son complément.

**105.** Sonde suivant la revendication 39, dans laquelle ledit organisme est Chlamydia trachomatis et ledit oligonucléotide comprend la séquence
CCGACTCGCGGTTGAGCCCATCTTTGACAA

**106.** Sonde suivant la revendication 39, dans laquelle ledit organisme est Chlamydia trachomatis et ledit oligonucléotide comprend la séquence
TTACGTCCGACACGGATGGGGTTGAGACCATC.

**107.** Sonde suivant la revendication 39, dans laquelle ledit organisme est Chlamydia trachomatis et ledit oligonucléotide comprend la séquence
CCGCCACTAAACAATCGTCGAAACAATTGCTCCGTTCGA.

**108.** Sonde suivant la revendication 39, dans laquelle ledit organisme est Chlamydia trachomatis et ledit oligonucléotide comprend la séquence
CGTTACTCGGATGCCCAAATATCGCCACATTCG.

**109.** Sonde suivant la revendication 39, dans laquelle ledit organisme est Chlamydia trachomatis et ledit oligonucléotide comprend la séquence
CATCCATCTTTCCAGATGTGTTCAACTAGGAGTCCTGATCC.

**110.** Sonde suivant la revendication 39, dans laquelle ledit organisme est Chlamydia trachomatis et ledit oligonucléotide comprend la séquence
GAGGTCGGTCTTTCTCTCCTTTCGTCTACG.

**111.** Sonde suivant la revendication 39, dans laquelle ledit organisme est Chlamydia trachomatis et ledit oligonucléotide comprend la séquence
CCGTTCTCATCGCTCTACGGACTCTTCCAATCG.

**112.** Sonde suivant la revendication 39, dans laquelle ledit organisme est Chlamydia trachomatis et ledit oligonucléotide comprend la séquence
CGAAGATTCCCCTTGATCGCGACCTGATCT.

**113.** Sonde suivant la revendication 39, dans laquelle ledit organisme est Chlamydia trachomatis et ledit oligonucléotide comprend la séquence
CCGGGGCTCCTATCGTTCCATAGTCACCCTAAAAG.

**114.** Sonde suivant la revendication 39, dans laquelle ledit organisme est Chlamydia trachomatis et ledit oligonucléotide comprend la séquence
TACCGCGTGTCTTATCGACACACCCGCG.

**115.** Méthode suivant l'une quelconque des revendications 1 à 37, ou sonde suivant la revendication 38, dans laquelle ledit organisme est Chlamydia trachomatis et ladite région variable correspond aux bases 60-105 de l'ARNr 16S de E. coli, ou son complément.

**116.** Méthode suivant l'une quelconque des revendications 1 à 37, ou sonde suivant la revendication 38, dans laquelle ledit organisme est Chlamydia trachomatis et ladite région variable correspond aux bases 600-635 de l'ARNr 16S de E. coli, ou son complément.

**117.** Méthode suivant l'une quelconque des revendications 1 à 37, ou sonde suivant la revendication 38, dans laquelle ledit organisme est Chlamydia trachomatis et ladite région variable correspond aux bases 8-30-870 de l'ARNr 16S de E. coli, ou son complément.

**118.** Méthode suivant l'une quelconque des revendications 1 à 37, ou sonde suivant la revendication 38, dans laquelle ledit organisme est Chlamydia trachomatis et ladite région variable correspond aux bases 275-320 de l'ARNr 23S de E. coli, ou son complément.

**119.** Méthode suivant l'une quelconque des revendications 1 à 37, ou sonde suivant la revendication 38, dans laquelle ledit organisme est Chlamydia trachomatis et ladite région variable correspond aux bases 330-365 de l'ARNr 23S de E. coli, ou son complément.

**120.** Méthode suivant l'une quelconque des revendications 1 à 37, ou sonde suivant la revendication 38, dans laquelle ledit organisme est Chlamydia trachomatis et ladite région variable correspond aux bases 1160-1190 de l'ARNr 23S de E. coli, ou son complément.

**121.** Méthode suivant l'une quelconque des revendications 1 à 37, ou sonde suivant la revendication 38, dans laquelle ledit organisme est Chlamydia trachomatis et ladite région variable correspond aux bases 1450-1490 de l'ARNr 23S de E. coli, ou son complément.

**122.** Méthode suivant l'une quelconque des revendications 1 à 37, ou sonde suivant la revendication 38, dans laquelle ledit organisme est Chlamydia trachomatis et ladite région variable correspond aux bases 1510-1545 de l'ARNr 23S de E. coli, ou son complément.

**123.** Méthode suivant l'une quelconque des revendications 1 à 37, ou sonde suivant la revendication 38, dans laquelle ledit organisme est Chlamydia trachomatis et ladite région variable correspond aux bases 1710-1750 de l'ARNr 23S de E. coli, ou son complément.

**124.** Sonde suivant la revendication 39, dans laquelle ledit groupe d'organismes est le genre Campylobacter et ledit oligonucléotide comprend la séquence
GGTTCTTAGGATATCAAGCCCAGG.

**125.** Méthode suivant l'une quelconque des revendications 1 à 37, ou sonde suivant la revendication 38, dans laquelle ledit groupe d'organismes -est le genre Campylobacter et ladite région variable correspond aux bases 980-1010 de l'ARNr 16S de E. coli, ou son complément.

**126.** Sonde suivant la revendication 39, dans laquelle ledit groupe d'organismes est le groupe sous-générique de Streptococci connu sous le nom d'Enterococci et ledit oligonucléotide comprend la séquence
TGCAGCACTGAAGGGCGGAAACCCTCCAACACTTA.

**127.** Méthode suivant l'une quelconque des revendications 1 à 37, ou sonde suivant la revendication 38, dans laquelle ledit groupe d'organismes est le groupe sous-générique de Streptococci connu sous le nom d'Enterococci et ladite région variable correspond aux bases 825-860 de l'ARNr 16S de E. coli, ou son complément.

**128.** Sonde suivant la revendication 39, dans laquelle ledit groupe d'organismes est le groupe sous-générique connu sous le nom de Pseudomonas du Groupe 1 et ledit oligonucléotide comprend la séquence
CAGACAAAGTTTCTCGTGCTCCGTCCTACTCGATT.

**129.** Méthode suivant l'une quelconque des revendications 1 à 37, ou sonde suivant la revendication 38, dans laquelle ledit groupe d'organismes est le groupement sous-générique connu sous le nom de Pseudomonas du Groupe 1 et ladite région variable correspond aux bases 365-405 de l'ARNr 23S de E. coli, ou son complément.

**130.** Sonde suivant la revendication 39, dans laquelle ledit organisme est Enterobacter cloacae et ledit oligonucléotide comprend la séquence
GTGTGTTTTCGTGTACGGGAGTTTCACCC.

**131.** Méthode suivant l'une quelconque des revendications 1 à 37, ou sonde suivant la revendication 38, dans laquelle ledit organisme est Enterobacter cloacae et ladite région variable correspond aux bases 305-340 de l'ARNr 23S de E. coli, ou son complément.

**132.** Sonde suivant la revendication 39, dans laquelle ledit organisme est Proteus mirabilis et ledit oligonucléotide comprend la séquence
CCGTTCTCCTGACACTGCTATTGATTAAGACTC.

**133.** Méthode suivant l'une quelconque des revendications 1 à 37, ou sonde suivant la revendication 38, dans laquelle ledit organisme est Proteus mirabilis et ladite région variable correspond aux bases 270-305 de l'ARNr 23S de E. coli, ou son complément.

**134.** Sonde suivant la revendication 39, dans laquelle ledit groupe d'organismes est le genre Salmonella et ledit oligonucléotide comprend la séquence
CTCCTTTGAGTTCCCGACCTAATCGCTGGC.

**135.** Sonde suivant la revendication 39, dans laquelle ledit groupe d'organismes est le genre Salmonella et ledit oligonucléotide comprend la séquence
CTCATCGAGCTGACAGCACATGCGCTTTTGTGTA.

**136.** Méthode suivant l'une quelconque des revendications 1 à 37, ou sonde suivant la revendication 38, dans laquelle ledit groupe d'organismes est le genre Salmonella et ladite région variable correspond aux bases 1125-1155 de l'ARNr 16S de E. coli, ou son complément.

**137.** Méthode suivant l'une quelconque des revendications 1 à 37, ou sonde suivant la revendication 38, dans laquelle ledit groupe d'organismes est le genre Salmonella et ladite région variable correspond aux bases 335-375 de l'ARNr 23S de E. coli, ou son complément.

**138.** Sonde suivant la revendication 39, dans laquelle ledit organisme est Escherichia coli et ledit oligonucléotide comprend la séquence
GCACATTCTCATCTCTGAAAACTTCCGTGG.

**139.** Méthode suivant l'une quelconque des revendications 1 à 37, ou sonde suivant la revendication 38, dans laquelle ledit organisme est Escherichia coli et ladite région variable correspond aux bases 995-1030 de l'ARNr 16S de E. coli, ou son complément.

**140.** Sonde suivant la revendication 39, dans laquelle ledit organisme est Neisseria gonorrhoeae et ledit oligonucléotide comprend la séquence CCGCCGCTACCCGGTAC.

**141.** Sonde suivant la revendication 39, dans laquelle ledit organisme est Neisseria gonorrhoeae et ledit oligonucléotide comprend la séquence TCATCGGCCGCCGATATTGGC.

**142.** Sonde suivant la revendication 39, dans laquelle ledit organisme est Neisseria gonorrhoeae et ledit oligonucléotide comprend la séquence GAGCATTCCGCACATGTCAAAACCAGGTA.

**143.** Sonde suivant la revendication 39, dans laquelle ledit organisme est Neisseria gonorrhoeae et ledit oligonucléotide comprend la séquence GAGATTCCGCACATGTCAAAACCAGG.

**144.** Sonde suivant la revendication 39, dans laquelle ledit organisme est Neisseria gonorrhoeae et ledit oligonucléotide comprend la séquence GAGGATTCCGCACATGTCAAAACCAGGTAA.

**145.** Sonde suivant la revendication 39, dans laquelle ledit organisme est Neisseria gonorrhoeae et ledit oligonucléotide comprend la séquence CCCGCTACCCGGTACGTTC.

**146.** Sonde suivant la revendication 39, dans laquelle ledit organisme est Neisseria gonorrhoeae et ledit oligonucléotide comprend la séquence CCGCTACCCGGTACGTTC.

**147.** Méthode suivant l'une quelconque des revendications 1 à 37, ou sonde suivant la revendication 38, dans laquelle ledit organisme est Neisseria gonorrhoeae et ladite région variable correspond aux bases 125-150 de l'ARNr 16S de E. coli, ou son complément.

**148.** Méthode suivant l'une quelconque des revendications 1 à 37, ou sonde suivant la revendication 38, dans laquelle ledit organisme est Neisseria gonorrhoeae et ladite région variable correspond aux bases 455-485 de l'ARNr 16S de E. coli, ou son complément.

**149.** Méthode suivant l'une quelconque des revendications 1 à 37, ou sonde suivant la revendication 38, dans laquelle ledit organisme est Neisseria gonorrhoeae et ladite région variable correspond aux bases 980-1015 de l'ARNr 16S de E. coli, ou son complément.

**150.** Hybride d'acides nucléiques formé entre un polymère de nucléotides ou un oligonucléotide suivant l'une quelconques des revendications 47-56, 62, 64, 72-75, 80-83, 89-93, 100-104, 115-123, 125, 127, 129, 131, 133, 136, 137, 139 et 147-149 et un acide nucléique complémentaire de celui-ci.

**151.** Polymère de nucléotides qui s'hybride dans des conditions stringentes à une sonde suivant l'une quelconques des revendications 40-46, 56-61, 63, 65-71, 76-79, 84-88, 94-99, 105-114, 124, 126, 128, 130, 132, 134, 135, 138 et 140-146 ou à son complément.

**152.** Hybride d'acides nucléiques suivant la revendication 150, formé entre un oligonucléotide et un acide nucléique complémentaire de celui-ci.

**153.** Complément du polymère de nucléotides de la revendication 151.

**154.** Test d'hybridation comprenant les étapes consistant à faire réagir ensemble tout ARNr ou son complément provenant d'un échantillon à tester pour la présence d'un organisme non viral ou d'un groupe d'organismes non viraux et une sonde suivant l'une quelconque des revendications 39 à 149, dans lequel ladite sonde est complémentaire au moins à environ 75 % avec une région variable d'ARNr ou d'ADNr choisie de manière à être caractéristique dudit organisme non viral ou d'un groupe d'organismes non viraux, dans des conditions telles que l'hybridation entre la sonde oligonucléotidique ou le polymère de nucléotides et n'importe quel ARNr d'échantillon suffisamment complémentaire ou son complément puisse se produire, et observer et/ou mesurer ladite hybridation.

**155.** Test de la revendication 154, dans lequel ladite hybridation entre la sonde oligonucléotidique et n'importe quel ARNr d'échantillon cible est au moins d'environ 10 % à environ 100 %.

**156.** Test de la revendication 154, dans lequel ladite sonde oligonucléotidique est un ADNc.

**157.** Test de la revendication 154, dans lequel lesdites conditions comprennent une température d'environ 25°C en dessous de Tm à environ 1°C en dessous de Tm.

**158.** Test de la revendication 154, qui comprend en outre le test parallèle d'un témoin homologue positif, ou d'un témoin hétérologue positif, ou les deux.

**159.** Test de la revendication 154, qui comprend en outre le test parallèle d'un témoin négatif.

**160.** Test de la revendication 154, dans lequel lesdites conditions comprennent des agents permettant d'augmenter les taux d'hybridation.

**161.** Test de la revendication 154, dans lequel lesdites conditions sont celles qui favorisent l'hybridation maximale entre la sonde oligonucléotidique et un quelconque ARNr d'échantillon complémentaire et réduisent au minimum la réactivité croisée entre la sonde oligonucléotidique et un quelconque ARNr d'échantillon non complémentaire.

**162.** Test de la revendication 154, dans lequel ladite sonde oligonucléotidique est marquée.

**163.** Test de la revendication 154, dans lequel ladite sonde oligonucléotidique- est marquée avec un marqueur isotopique, non isotopique ou chimioluminescent.

**164.** Test de la revendication 154, qui comprend en outre la libération d'ARNr des cellules dudit organisme non viral ou groupe d'organismes non viraux préalablement à l'étape consistant à les faire réagir ensemble.

**165.** Test de la revendication 154, dans lequel ledit organisme non viral ou groupe d'organismes non viraux est Mycobacterium avium, Mycobacterium intracellulare, les bactéries du complexe à Mycobacterium tuberculosis, le genre Mycobacterium, Mycoplasma pneumoniae, Legionella, Neisseria gonorrhoeae, Salmonella, Chlamydia trachomatis, Campylobacter, Proteus mirabilis, Enterococcus, Enterobacter cloacae, E. coli, Pseudomonas du Groupe 1, des bactéries ou des champignons.

**166.** Test de la revendication 162, dans lequel ladite sonde oligonucléotidique marquée a une longueur d'environ 20 à environ 50 nucléotides.

**167.** Test de la revendication 162, dans lequel ladite sonde oligonucléotidique marquée est au moins environ à 95 % complémentaire de ladite région variable d'ARNr.

**168.** Test de la revendication 154, comprenant en outre l'utilisation d'une ou plusieurs sources oligonucléotidiques supplémentaires d'une longueur d'au moins environ 10 nucléotides et qui sont au moins environ à 75 % complémentaires d'une
ou plusieurs régions variables supplémentaires d'ARNr choisies pour être caractéristiques desdits organismes non viraux.

**169.** Test de la revendication 154, comprenant en outre l'utilisation d'une ou plusieurs sondes supplémentaires qui identifient un ou plusieurs organismes non viraux supplémentaires, étendant ainsi le groupe des organismes non viraux à tester.

| | | |
|---|---|---|
| *Escherichia coli* | AAAUUGAAGAGUUUGAUCAUGGCUCAGAUUGAACGCUGGCGGCAGGCCUAACACAUGCAAGUCGAACGGUAACAGGAAGAAGCUUGCUUCUUUGCUGACG | 100 |
| *Escherichia coli* | AGUGGCGGACGGGUGAGUAAUGUCUGGGAAACUGCCUGAUGGAGGGGGAUAACUACUGGAAACGGUAGCUAAUACCGCAUAACGUCGCAAGACCAAAGAG | 200 |
| *Escherichia coli* | GGGGACCUUCGGGCCUCUUGCCAUCGGAUGUGCCCAGAUGGGAUUAGCUAGUAGGUGGGGUAACGGCUCACCUAGGCGACGAUCCCUAGCUGGUCUGAGA | 300 |
| *Escherichia coli* | GGAUGACCAGCCACACUGGAACUGAGACACGGUCCAGACUCCUACGGGAGGCAGCAGUGGGGAAUAUUGCACAAUGGGCGCAAGCCUGAUGCAGCCAUGC | 400 |
| *Escherichia coli* | CGCGUGUAUGAAGAAGGCCUUCGGGUUGUAAAGUACUUUCAGCGGGGAGGAAGGGAGUAAAGUUAAUACCUUUGCUCAUUGACGUUACCCGCAGAAGAAG | 500 |
| *Escherichia coli* | CACCGGCUAACUCCGUGCCAGCAGCCGCGGUAAUACGGAGGGUGCAAGCGUUAAUCGGAAUUACUGGGCGUAAAGCGCACGCAGGCGGUUUGUUAAGUCA | 600 |
| *Escherichia coli* | GAUGUGAAAUCCCCGGGCUCAACCUGGGAACUGCAUCUGAUACUGGCAAGCUUGAGUCUCGUAGAGGGGGGUAGAAUUCCAGGUGUAGCGGUGAAAUGCG | 700 |
| *Escherichia coli* | UAGAGAUCUGGAGGAAUACCGGUGGCGAAGGCGGCCCCCUGGACGAAGACUGACGCUCAGGUGCGAAAGCGUGGGGAGCAAACAGGAUUAGAUACCCUGG | 800 |
| *Escherichia coli* | UAGUCCACGCCGUAAACGAUGUCGACUUGGAGGUUGUGCCCUUGAGGCGUGGCUUCCGGAGCUAACGCGUUAAGUCGACCGCCUGGGGAGUACGGCCGCA | 900 |
| *Escherichia coli* | AGGUUAAAACUCAAAUGAAUUGACGGGGGCCCGCACAAGCGGUGGAGCAUGUGGUUUAAUUCGAUGCAACGCGAAGAACCUUACCUGGUCUUGACAUCCA | 1000 |
| *Escherichia coli* | CGGAAGUUUUCAGAGAUGAGAAUGUGCCUUCGGGAACCGUGAGACAGGUGCUGCAUGGCUGUCGUCAGCUCGUGUUGUGAAAUGUUGGGUUAAGUCCCGC | 1100 |
| *Escherichia coli* | AACGAGCGCAACCCUUAUCCUUUGUUGCCAGCGGUCCGGCCGGGAACUCAAAGGAGACUGCCAGUGAUAAACUGGAGGAAGGUGGGGAUGACGUCAAGUC | 1200 |
| *Escherichia coli* | AUCAUGGCCCUUACGACCAGGGCUACACACGUGCUACAAUGGCGCAUACAAAGAGAAGCGACCUCGCGAGAGCAAGCGGACCUCAUAAAGUGCGUCGUAG | 1300 |
| *Escherichia coli* | UCCGGAUUGGAGUCUGCAACUCGACUCCAUGAAGUCGGAAUCGCUAGUAAUCGUGGAUCAGAAUGCCACGGUGAAUACGUUCCCGGGCCUUGUACACACC | 1400 |
| *Escherichia coli* | GCCCGUCACACCAUGGGAGUGGGUUGCAAAAGAAGUAGGUAGCUUAACCUUCGGGAGGGCGCUUACCACUUUGUGAUUCAUGACUGGGGUGAAGUCGUAA | 1500 |
| *Escherichia coli* | CAAGGUAACCGUAGGGGAACCUGCGGUUGGAUCACCUCCUUA | 1542 |

FIG. 1

| | | |
|---|---|---|
| *Escherichia coli* | GGUUAAGCGACUAAGCGUACACGGUGGAUGCCCUGGCAGUCAGAGGCGAUGAAGGACGUGCUAAUCUGCGAUAAGCGUCGGUAAGGUGAUAUGAACCGUU | 100 |
| *Escherichia coli* | AUAACCGGCGAUUUCCGAAUGGGGAAACCCAGUGUGUUUCGACACACUAUCAUUAACUGAAUCCAUAGGUUAAUGAGGCGAACCGGGGGAACUGAAACAU | 200 |
| *Escherichia coli* | CUAAGUACCCCGAGGAAAAGAAAUCAACCGAGAUUCCCCCAGUAGCGGCGAGCGAACGGGGAGCAGCCCAGAGCCUGAAUCAGUGUGUGUGUUAGUGGAA | 300 |
| *Escherichia coli* | GCGUCUGGAAAGGCGCGCGAUACAGGGUGACAGCCCCGUACACAAAAAUGCACAUGCUGUGAGCUCGAUGAGUAGGGCGGGACACGUGGUAUCCUGUCUG | 400 |
| *Escherichia coli* | AAUAUGGGGGGACCAUCCUCCAAGGCUAAAUACUCCUGACUGACCGAUAGUGAACCAGUACCGUGAGGGAAAGGCGAAAAGAACCCCGGCGAGGGGAGUG | 500 |
| *Escherichia coli* | AAAAAGAACCUGAAACCGUGUACGUACAAGCAGUGGGAGCACGCUUAGGCGUGUGACUGCGUACCUUUUGUAUAAUGGGUCAGCGACUUAUAUUCUGUAG | 600 |
| *Escherichia coli* | CAAGGUUAACCGAAUAGGGGAGCCGAAGGGAAACCGAGUCUUAACUGGGCGUUAAGUUGCAGGGUAUAGACCCGAAACCCGGUGAUCUAGCCAUGGGCAG | 700 |
| *Escherichia coli* | GUUGAAGGUUGGGUAACACUAACUGGAGGACCGAACCGACUAAUGUUGAAAAAUUAGCGGAUGACUUGUGGCUGGGGGUGAAAGGCCAAUCAAACCGGGA | 800 |
| *Escherichia coli* | GAUAGCUGGUUCUCCCCGAAAGCUAUUUAGGUAGCGCCUCGUGAAUUCAUCUCCGGGGGUAGAGCACUGUUUCGGCAAGGGGGUCAUCCCGACUUACCAA | 900 |
| *Escherichia coli* | CCCGAUGCAAACUGCGAAUACCGGAGAAUGUUAUCACGGGAGACACACGGCGGGUGCUAACGUCCGUCGUGAAGAGGGAAACAACCCAGACCGCCAGCUA | 1000 |
| *Escherichia coli* | AGGUCCCAAAGUCAUGGUUAAGUGGGAAACGAUGUGGGAAGGCCCAGACAGCCAGGAUGUUGGCUUAGAAGCAGCCAUCAUUUAAAGAAAGCGUAAUAGC | 1100 |
| *Escherichia coli* | UCACUGGUCGAGUCGGCCUGCGCGGAAGAUGUAACGGGGCUAAACCAUGCACCGAAGCUGCGGCAGCGACGCUUAUGCGUUGUUGGGUAGGGGAGCGUUC | 1200 |
| *Escherichia coli* | UGUAAGCCUGCGAAGGUGUGCUGUGAGGCAUGCUGGAGGUAUCAGAAGUGCGAAUGCUGACAUAAGUAACGAUAAAGCGGGUGAAAAGCCCGCUCGCCGG | 1300 |
| *Escherichia coli* | AAGACCAAGGGUUCCUGUCCAACGUUAAUCGGGGCAGGGUGAGUCGACCCCUAAGGCGAGGCCGAAAGGCGUAGUCGAUGGGAAACAGGUUAAUAUUCCU | 1400 |
| *Escherichia coli* | GUACUUGGUGUUACUGCGAAGGGGGGACGGAGAAGGCUAUGUUGGCCGGGCGACGGUUGUCCCGGUUUAAGCGUGUAGGCUGGUUUUCCAGGCAAAUCCG | 1500 |
| *Escherichia coli* | GAAAAUCAAGGCUGAGGCGUGAUGACGAGGCACUACGGUGCUGAAGCAACAAAUGCCCUGCUUCCAGGAAAAGCCUCUAAGCAUCAGGUAACAUCAAAUC | 1600 |
| *Escherichia coli* | GUACCCCAAACCGACACAGGUGGUCAGGUAGAGAAUACCAAGGCGCUUGAGAGAACUCGGGUGAAGGAACUAGGCAAAAUGGUGCCGUAACUUCGGGAGA | 1700 |
| *Escherichia coli* | AGGCACGCUGAUAUGUAGGUGAGGUCCCUCGCGGAUGGAGCUGAAAUCAGUCGAAGAUACCAGCUGGCUGCAACUGUUUAUUAAAAACACAGCACUGUGC | 1800 |
| *Escherichia coli* | AAACACGAAAGUGGACGUAUACGGUGUGACGCCUGCCCGGUGCCGGAAGGUUAAUUGAUGGGGUUAGCGCAAGCGAAGCUCUUGAUCGAAGCCCCGGUAA | 1900 |
| *Escherichia coli* | ACGGCGGCCGUAACUAUAACGGUCCUAAGGUAGCGAAAUUCCUUGUCGGGUAAGUUCCGACCUGCACGAAUGGCGUAAUGAUGGCCAGGCUGUCUCCACC | 2000 |
| *Escherichia coli* | CGAGACUCAGUGAAAUUGAACUCGCUGUGAAGAUGCAGUGUACCCGCGGCAAGACGGAAAGACCCCGUGAACCUUUACUAUAGCUUGACACUGAACAUUG | 2100 |
| *Escherichia coli* | AGCCUUGAUGUGUAGGAUAGGUGGGAGGCUUUGAAGUGUGGACGCCAGUCUGCAUGGAGCCGACCUUGAAAUACCACCCUUUAAUGUUUGAUGUUCUAAC | 2200 |
| *Escherichia coli* | GUUGACCCGUAAUCCGGGUUGCGGACAGUGUCUGGUGGGUAGUUUGACUGGGGCGGUCUCCUCCUAAAGAGUAACGGAGGAGCACGAAGGUUGGCUAAUC | 2300 |
| *Escherichia coli* | CUGGUCGGACAUCAGGAGGUUAGUGCAAUGGCAUAAGCCAGCUUGACUGCGAGCGUGACGGCGCGAGCAGGUGCGAAAGCAGGUCAUAGUGAUCCGGUGG | 2400 |
| *Escherichia coli* | UUCUGAAUGGAAGGGCCAUCGCUCAACGGAUAAAAGGUACUCCGGGGAUAACAGGCUGAUACCGCCCAAGAGUUCAUAUCGACGGCGGUGUUUGGCACCU | 2500 |
| *Escherichia coli* | CGAUGUCGGCUCAUCACAUCCUGGGGCUGAAGUAGGUCCCAAGGGUAUGGCUGUUCGCCAUUUAAAGUGGUACGCGAGCUGGGUUUAGAACGUCGUGAGA | 2600 |
| *Escherichia coli* | CAGUUCGGUCCCUAUCUGCCGUGGGCGCUGGAGAACUGAGGGGGGCUGCUCCUAGUACGAGAGGACCGGAGUGGACGCAUCACUGGUGUUCGGGUUGUCA | 2700 |
| *Escherichia coli* | UGCCAAUGGCACUGCCCGGUAGCUAAAUGCGGAAGAGAUAAGUGCUGAAAGCAUCUAAGCACGAAACUUGCCCCGAGAUGAGUUCUCCCUGACCCUUUAA | 2800 |
| *Escherichia coli* | GGGUCCUGAAGGAACGUUGAAGACGACGACGUUGAUAGGCCGGGUGUGUAAGCGCAGCGAUGCGUUGAGCUAACCGGUACUAAUGAACCGUGAGGCUUAA | 2900 |
| *Escherichia coli* | CCUU | 2904 |

FIG. 2

| | |
|---|---|
| *Escherichia coli* | UGCCUGGCGGCCGUAGCGCGGUGGUCCCACCUGACCCCAUGCCGAACUCAGAAGUGAAACGCCGUAGCGCCGAUGGUAGUGUGGGGUCUCCCCAUGCGAG |
| *Escherichia coli* | AGUAGGGAACUGCCAGGCAU |

FIG. 3

| | | |
|---|---|---|
| S.cerevisiae.sc | UAUCUGGUUGAUCCUGCCAGUAGUCAUAUGCUUGUCUCAAAGAUUAAGCCAUGCAUGUCUAAGUAUAAGCAAUUUAUACAGUGAAACUGCGAAUGGCUCA | 100 |
| S.cerevisiae.sc | UUAAAUCAGUUAUCGUUUAUUUGAUAGUUCCUUUACUACAUGGUAUAACCGUGGUAAUUCUAGAGCUAAUACAUGCUUAAAAUCUCGACCCUUUGGAAGA | 200 |
| S.cerevisiae.sc | GAUGUAUUUAUUAGAUAAAAAAUCAAUGUCUUCGCACUCUUUGAUGAUUCAUAAUAACUUUUCGAAUCGCAUGGCCUUGUGCUGGCGAUGGUUCAUUCAA | 300 |
| S.cerevisiae.sc | AUUUCUGCCCUAUCAACUUUCGAUGGUAGGAUAGUGGCCUACCAUGGUUUCAACGGGUAACGGGGAAUAAGGGUUCGAUUCCGGAGAGGGAGCCUGAGAA | 400 |
| S.cerevisiae.sc | ACGGCUACCACAUCCAAGGAAGGCAGCAGGCGCGCAAAUUACCCAAUCCUAAUUCAGGGAGGUAGUGACAAUAAAUAACGAUACAGGGCCCAUUCGGGUC | 500 |
| S.cerevisiae.sc | UUGUAAUUGGAAUGAGUACAAUGUAAAUACCUUAACGAGGAACAAUUGGAGGGCAAGUCUGGUGCCAGCAGCCGCGGUAAUUCCAGCUCCAAUAGCGUAU | 600 |
| S.cerevisiae.sc | AUUAAAGUUGUUGCAGUUAAAAAGCUCGUAGUUGAACUUUGGGCCCGGUUGGCCGGUCCGAUUUUUUCGUGUACUGGAUUUCCAACGGGGCCUUUCCUUC | 700 |
| S.cerevisiae.sc | UGGCUAACCUUGAGUCCUUGUGGCUCUUGGCGAACCAGGACUUUUACUUUGAAAAAAUUAGAGUGUUCAAAGCAGGCGUAUUGCUCGAAUAUAUUAGCAU | 800 |
| S.cerevisiae.sc | GGAAUAAUAGAAUAGGACGUUUGGUUCUAUUUUGUUGGUUUCUAGGACCAUCGUAAUGAUUAAUAGGGACGGUCGGGGGCAUCGGUAUUCAAUUGUCGAG | 900 |
| S.cerevisiae.sc | GUGAAAUUCUUGGAUUUAUUGAAGACUAACUACUGCGAAAGCGUUUGCCAAGGACGUUUUCGUUAAUCAAGAACGAAAGUUGAGGGAUCGAAGACGAUCU | 1000 |
| S.cerevisiae.sc | GAUACCGUCGUAGUCUUAACCAUAAACUAUGCCGACUAGGGAUCGGGUGGUGUUUUUUUAAUGACCCACUCGGUACCUUACGAGAAAUCAAAGUCUUUGG | 1100 |
| S.cerevisiae.sc | GUUCUGGGGGGAGUAUGGUCGCAAAGGCUGAAACUUAAAGGAAUUGACGGAAGGGCACCACCAGGAGUGGAGCCUGCGGCUUAAUUUGACUCAACACGGG | 1200 |
| S.cerevisiae.sc | GAAACUCACCAGGUCCAGACACAAUAAGGAUUGACAGAUUGAGAGCUCUUUCUUGAUUUUGUGGGUGGUGGUGCAUGGCCGUUUCUCAGUUGGUGGAGUG | 1300 |
| S.cerevisiae.sc | AUUUGUCUGCUUAAUUGCGAUAACGAACGAGACCUUAACCUACUAAAUAGUGGUGCUAGCAUUUGCUGGUUAUCCACUUCUUAGAGGGACUAUCGGUUUC | 1400 |
| S.cerevisiae.sc | AAGCCGAUGGAAGUUUGAGGCAAUAACAGGUCUGUGAUGCCCUUAGAACGUUCUGGGCCGCACGCGCGCUACACUGACGGAGCCAGCGAGUCUAACCUUG | 1500 |
| S.cerevisiae.sc | GCCGAGAGGUCUUGGUAAUCUUGUGAAACUCCGUCGUGCUGGGGAUAGAGCAUUGUAAUUAUUGCUCUUCAACGAGGAAUUCCUAGUAAGCGCAAGUCAU | 1600 |
| S.cerevisiae.sc | CAGCUUGCGUUGAUUACGUCCCUGCCCUUUGUACACACCGCCCGUCGCUAGUACCGAUUGAAUGGCUUAGUGAGGCCUCAGGAUCUGCUUAGAGAAGGGG | 1700 |
| S.cerevisiae.sc | GCAACUCCAUCUCAGAGCGGAGAAUUUGGACAAACUUGGUCAUUUGGAGGAACUAAAAGUCGUAACAAGGUUUCCGUAGGUGAACCUGCGGAAGGAUCAU | 1800 |
| S.cerevisiae.sc | UA | 1802 |

FIG. 4

| | | |
|---|---|---|
| *S.cerevisiae* | AAACUUUCAACAACGGAUCUCUUGGUUCUCGCAUCGAUGAAGAACGCAGCGAAAUGCGAUACGUAAUGUGAAUUGCAGAAUUCCGUGAAUCAUCGAAUCUUUGAACGCACAUUGCGCCCC | 120 |
| *S.cerevisiae* | UUGGUAUUCCAGGGGGCAUGCCUGUUUGAGCGUCAUUUGUUUGACCUCAAAUCAGGUAGGAGUACCCGCUGAACUUAAGCAUAUCAAUAAGCGGAGGAAAAGAAACCAACCGGAUUGCCU | 240 |
| *S.cerevisiae* | UAGUAACGGCGAGUGAAGCGGCAAAAGCUCAAAUUUGAAAUCUGGUACCUUCGGUGCCCGAGUUGUAAUUUGGAGAGGGCAACUUUGGGGCCGUUCCUUGUCUAUGUUCCUUGGAACAGG | 360 |
| *S.cerevisiae* | ACGUCAUAGAGGGUGAGCAUCCCGUGUGGCGAGGAGUGCGGUUCUUUGUAAAGUGCCUUCGAAGAGUCGAGUUGUUUGGGAAUGCAGCUCUAAGUGGGUGGUAAAUUCCAUCUAAAGCUA | 480 |
| *S.cerevisiae* | AAUAUUGGCGAGAGACCGAUAGCGAACAAGUACAGUGAUGGAAAGAUGAAAAGAACUUUGAAAAGAGAGUGAAAAAGUACGUGAAAUUGUUGAAAGGGAAGGGCAUUUGAUCAGACAUGG | 600 |
| *S.cerevisiae* | UGUUUUGUGCCCUCUGCUCCUUGUGGGUAGGGGAAUCUCGCAUUUCACUGGGCCAGCAUCAGUUUUGGUGGCAGGAUAAAUCCAUAGGAAUGUAGCUUGCCUCGGUAAGUAUUAUAGCCU | 720 |
| *S.cerevisiae* | GUGGGAAUACUGCCAGCUGGGACUGAGGACUGCGACGUAAGUCAAGGAUGCUGGCAUAAUGGUUAUAUGCCGCCCGUCUUGAAACACGGACCAAGGAGUCUAACGUCUAUGCGAGUGUUU | 840 |
| *S.cerevisiae* | GGGUGUAAAACCCAUACGCGUAAUGAAAGUGAACGUAGGUUGGGGCCUCGCAAGAGGUGCACAAUCGACCGAUCCUGAUGUCUUCGGAUGGAUUUGAGUAAGAGCAUAGCUGUUGGGACC | 960 |
| *S.cerevisiae* | CGAAAGAUGGUGAACUAUGCCUGAAUAGGGUGAAGCCAGAGGAAACUCUGGUGGAGGCUCGUAGCGGUUCUGACGUGCAAAUCGAUCGUCGAAUUUGGGUAUAGGGGCGAAAGACUAAUC | 1080 |
| *S.cerevisiae* | GAACCAUCUAGUAGCUGGUUCCUGCCGAAGUUUCCCUCAGGAUAGCAGAAGCUCGUAUCAGUUUUAUGAGGUAAAGCGAAUGAUUAGAGGUUCCGGGGUCGAAAUGACCUUGACCUAUUC | 1200 |
| *S.cerevisiae* | UCAAACUUUAAAUAUGUAAGAAGUCCUUGUUACUUAAUUGAACGUGGACAUUUGAAUGAAGAGCUUUUAGUGGGCCAUUUUUGGUAAGCAGAACUGGCGAUGCGGGAUGAACCGAACGUA | 1320 |
| *S.cerevisiae* | GAGUUAAGGUGCCGGAAUACACGCUCAUCAGACACCACAAAAGGUGUUAGUUCAUCUAGACAGCCGGACGGUGGCCAUGGAAGUCGGAAUCCGCUAAGGAGUGUGUAACAACUCACCGGC | 1440 |
| *S.cerevisiae* | CGAAUGAACUAGCCCUGAAAAUGGAUGGCGCUCAAGCGUGUUACCUAUACUCUACCGUCAGGGUUGAUAUGAUGCCCUGACGAGUAGGCAGGCGUGGAGGUCAGUGACGAAGCCUAGACC | 1560 |
| *S.cerevisiae* | GUAAGGUCGGGUCGAACGGCCUCUAGUGCAGAUCUUGGUGGUAGUAGCAAAUAUUCAAAUGAGAACUUUGAAGACUGAAGUGGGGAAAGGUUCCACGUCAACAGCAGUUGGACGUGGGUU | 1680 |
| *S.cerevisiae* | AGUCGAUCCUAAGAGAUGGGGAAGCUCCGUUUCAAAGGCCUGAUUUUAUGCAGGCCACCAUCGAAAGGGAAUCCGGUAAGAUUCCGGAACUUGGAUAUGGAUUCUUCACGGUAACGUAAC | 1800 |
| *S.cerevisiae* | UGAAUGUGGAGACGUCGGCGCGAGCCCUGGGAGGAGUUAUCUUUUCUUCUUAACAGCUUAUCACCCCGGAAUUGGUUUAUCCGGAGAUGGGGUCUUAUGGCUGGAAGAGGCCAGCACCUU | 1920 |
| *S.cerevisiae* | UGCUGGCUCCGGUGCGCUUGUGACGGCCCGUGAAAAUCCACAGGAAGGAAUAGUUUUCAUGCUAGGUCGUACUGAUAACCGCAGCAGGUCUCCAAGGUGAACAGCCUCUAGUUGAUAGAA | 2040 |
| *S.cerevisiae* | UAAUGUAGAUAAGGGAAGUCGGCAAAAUAGAUCCGUAACUUCGGGAUAAGGAUUGGCUCUAAGGGUCGGGUAGUGAGGGCCUUGGUCAGACGCAGCGGGCGUGCUUGUGGACUGCUUGGU | 2160 |
| *S.cerevisiae* | GGGGCUUGCUCUGCUAGGCGGACUACUUGCGUGCCUUGUUGUAGACGGCCUUGGUAGGUCUCUUGUAGACCGUCGCUUGCUACAAUUAACAGAUCAACUUAGAACUGGUACGGACAAGGG | 2280 |
| *S.cerevisiae* | GAAUCUGACUGUCUAAUUAAAACAUAGCAUUGCGAUGGUCAGAAAGUGAUGUUGACGCAAUGUGAUUUCUGCCCAGUGCUCUGAAUGUCAAAGUGAAGAAAUUCAACCAAGCGCGAGUAA | 2400 |
| *S.cerevisiae* | ACGGCGGGAGUAACUAUGACUCUCUUAAGGUAGCCAAAUGCCUCGUCAUCUAAUUAGUGACGCGCAUGAAUGGAUUAACGAGAUUCCCACUGUCCCUAUCUACUAUCUAGCGAAACCACA | 2520 |
| *S.cerevisiae* | GCCAAGGGAACGGGCUUGGCAGAAUCAGCGGGGAAAGAAGACCCUGUUGAGCUUGACUCUAGUUUGACAUUGUGAAGAGACAUAGAGGGUGUAGAAUAAGUGGGAGCUUCGGCGCCAGUG | 2640 |
| *S.cerevisiae* | AAAUACCACUACCUUUAUAGUUUCUUUACUUAUUCAAUGAAGCGGAGCUGGAAUUCAUUUUCCACGUUCUAGCAUUCAAGGUCCCAUUCGGGGCUGAUCCGGGUUGAAGACAUUGUCAGG | 2760 |
| *S.cerevisiae* | UGGGGAGUUUGGCUGGGGCGGCACAUCUGUUAAACGAUAACGCAGAUGUCCUAAGGGGGGCUCAUGGAGAACAGAAAUCUCCAGUAGAACAAAAGGGUAAAGCCCCUUAGUUUGAUUUCA | 2880 |
| *S.cerevisiae* | GUGUGAAUACAAACCAUUGAAAGUGUGGCCUAUCGAUCCUUUAGUCCCUCGGAAUUUGAGGCUAGAGGUGCCAGAAAAGUUACCACAGGGAUAACUGGCUUGUGGCAGUCAAGCGUUCAU | 3000 |
| *S.cerevisiae* | AGCGACAUUGCUUUUUGAUUCUUCGAUGUCGGCUCUUCCUAUCAUACCGAAGCAGAAUUCGGUAAGCGUUGGAUUGUUCACCCACUAAUAGGGAACAUGAGCUGGGUUUAGACCGUCGUG | 3120 |
| *S.cerevisiae* | AGACAGGUUAGUUUUACCCUACUGAUGAAUGUUACCAGCAAUAGUAAUUGAACUUAGUACGAGAGGAACAGUUCAUUCGGAUAAUUGGUUUUUGCGGCUGUCUGAUCAGGCAUUGCCGCG | 3240 |
| *S.cerevisiae* | AAGCACCAUCCGCUGGAUUAUGGCUGAACGCCUCUAAGUCAGAAUCCAUGCUAGAACGCGGUGAUUUCUUUGCUCCACACAAUAUAGAUGGAUACGAAUAAGGCGUCCUUGUGGCGUCGC | 3360 |
| *S.cerevisiae* | UGAACCAUAGCAGGCUAGCAACGGUGCACUUGGCGGAAAGGCCUUGGGUGCUUGCUGGCGAAUUGCAAUGUCAUUUUGCGUGGGGAUAAAUCAUUUGUAUACGACUUAGAUGUACAACGG | 3480 |
| *S.cerevisiae* | GGUAUUGUAAGCGGUAGAGUAGCCUUGUUGUUACGAUCUGCUGAGAUUAAGCCUUUGUUGUCUGAUUUGU | 3550 |
| *S.cerevisiae* | | 3550 |
| *S.cerevisiae* | | 3550 |

FIG. 5

FIG. 6

## Summary of 16S rRNA Analysis

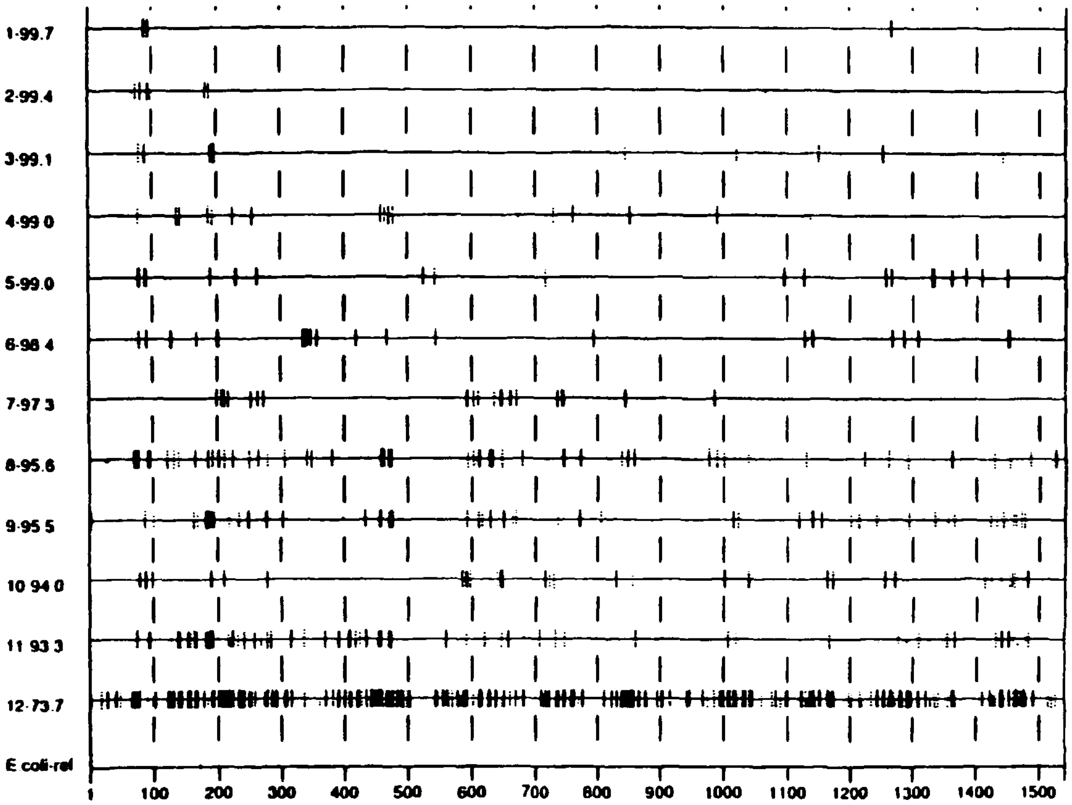

LEGEND: SUMMARY of 16S rRNA Analysis (Listing of Bacteria and percent similarity included in analysis)

1. 99.7% Clostridium botulinum G-Clostridium subterminale;
2. 99.4% Streptococcus cremoris-Streptococcus lactis;
3. 99.1% Lactobacillus lactis-Lactobacillus delbrueckii;
4. 99.0% Neisseria gonorrhoeae-Neisseria meningitidis;
5. 99.0% Mycobacterium intracellulare Mycobacterium avium;
6. 98.4% Mycobacterium avium-Mycobacterium tuberculosis;
7. 97.3% Pseudomonas alcaligenes-Pseudomonas stutzeri;
8. 95.6% Chlamydia psittaci-Chlamydia trachomatis;
9. 95.5% Spiroplasma citri-Spiroplasma mirum;
10. 94.0% Clostridium lituseburense-Clostridium sodellii;
11. 93.3% Listeria monocytogenes-Brochothrix thermosphacta;
12. 73.7% Escherichia coli-Bacteroides fragilis.

FIG. 7

## Summary of 23S rRNA Analysis

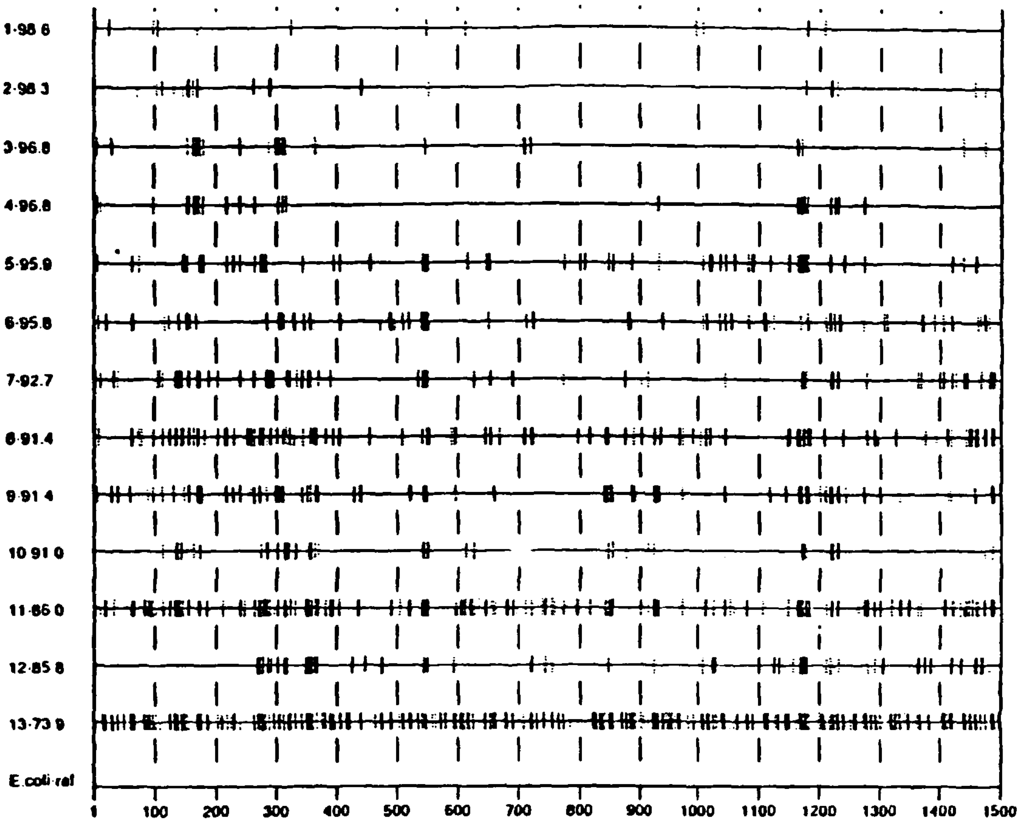


LEGEND: SUMMARY of 23S rRNA Analysis (Listing of Bacteria and percent similarity included in analysis)

1. 98.6% Neisseria gonorrhoeae-Neisseria meningitidis;
2. 98.3% Proteus mirabilis-Proteus vulgaris;
3. 96.8% Mycobacterium intracellulare-Mycobacterium avium;
4. 96.8% Mycobacterium avium-Mycobacterium asiaticum;
5. 95.9% Mycobacterium tuberculosis-Mycobacterium kansasii;
6. 95.8% Nicotiana tabacum (tobacco)-Zea mays (maize);
7. 92.7% Proteus vulgaris-Klebsiella rhinoscleromatis;
8. 91.4% Bacillus stearothermophilus-Bacillus subtilis;
9. 91.4% Mycobacterium intracellulare-Mycobacterium fortuitum;
10. 91.0% Escherichia coli-Klebsiella rhinoscleromatis;
11. 86.0% Escherichia coli-Pseudomonas aeruginosa;
12. 85.8% Chlamydia trachomatis-Chlamydia psittaci;
13. 73.9% Escherichia coli-Anacystis nidulans.

FIG. 8

## Summary of 23S rRNA Analysis

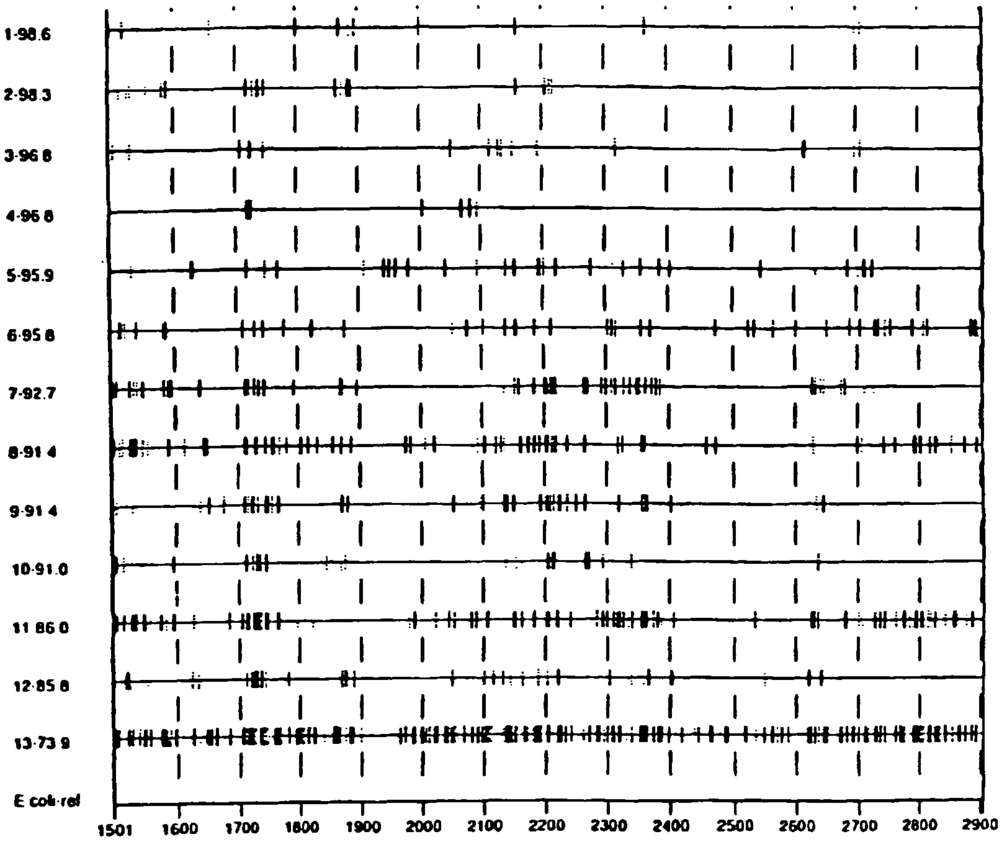


LEGEND: SUMMARY of 23S rRNA Analysis (Listing of Bacteria and percent similarity included in analysis)

1. 98.6% Neisseria gonorrhoeae-Neisseria meningitidis;

2. 98.3% Proteus mirabilis-Proteus vulgaris;

3. 96.8% Mycobacterium intracellulare-Mycobacterium avium;

4. 96.8% Mycobacterium avium-Mycobacterium asiaticum;

5. 95.9% Mycobacterium tuberculosis-Mycobacterium kansasii;

6. 95.8% Nicotiana tabacum (tobacco)-Zea mays (maize);

7. 92.7% Proteus vulgaris-Klebsiella rhinoscleromatis;

8. 91.4% Bacillus stearothermophilus-Bacillus subtilis;

9. 91.4% Mycobacterium intracellulare-Mycobacterium tortuitum;

10. 91.0% Escherichia coli-Klebsiella rhinoscleromatis;

11. 86.0% Escherichia coli-Pseudomonas aeruginosa;

12. 85.8% Chlamydia trachomatis-Chlamydia psittaci;

13. 73.9% Escherichia coli-Anacystis nidulans.

Summary of 16S rRNA PROBE locations

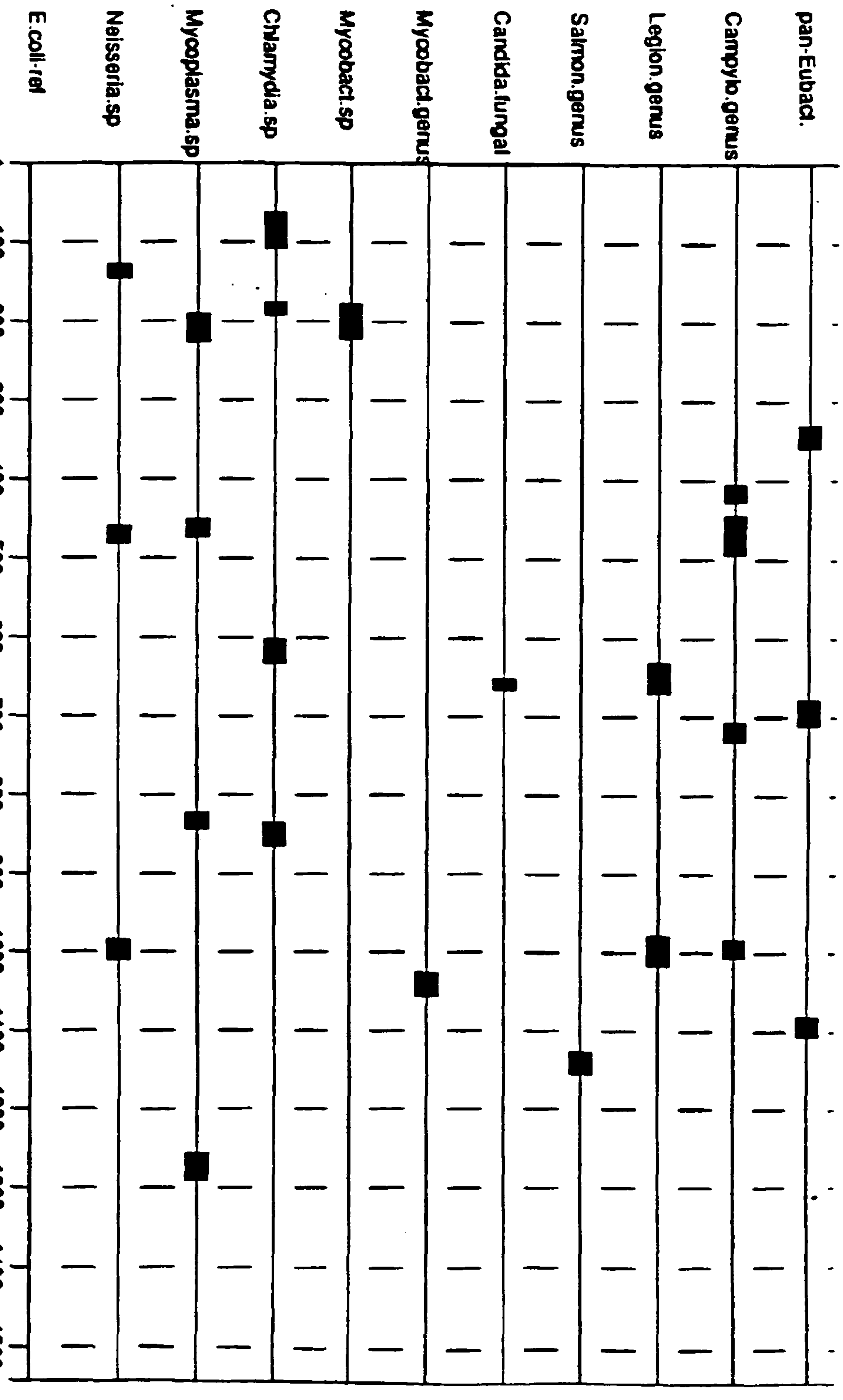


FIG. 9

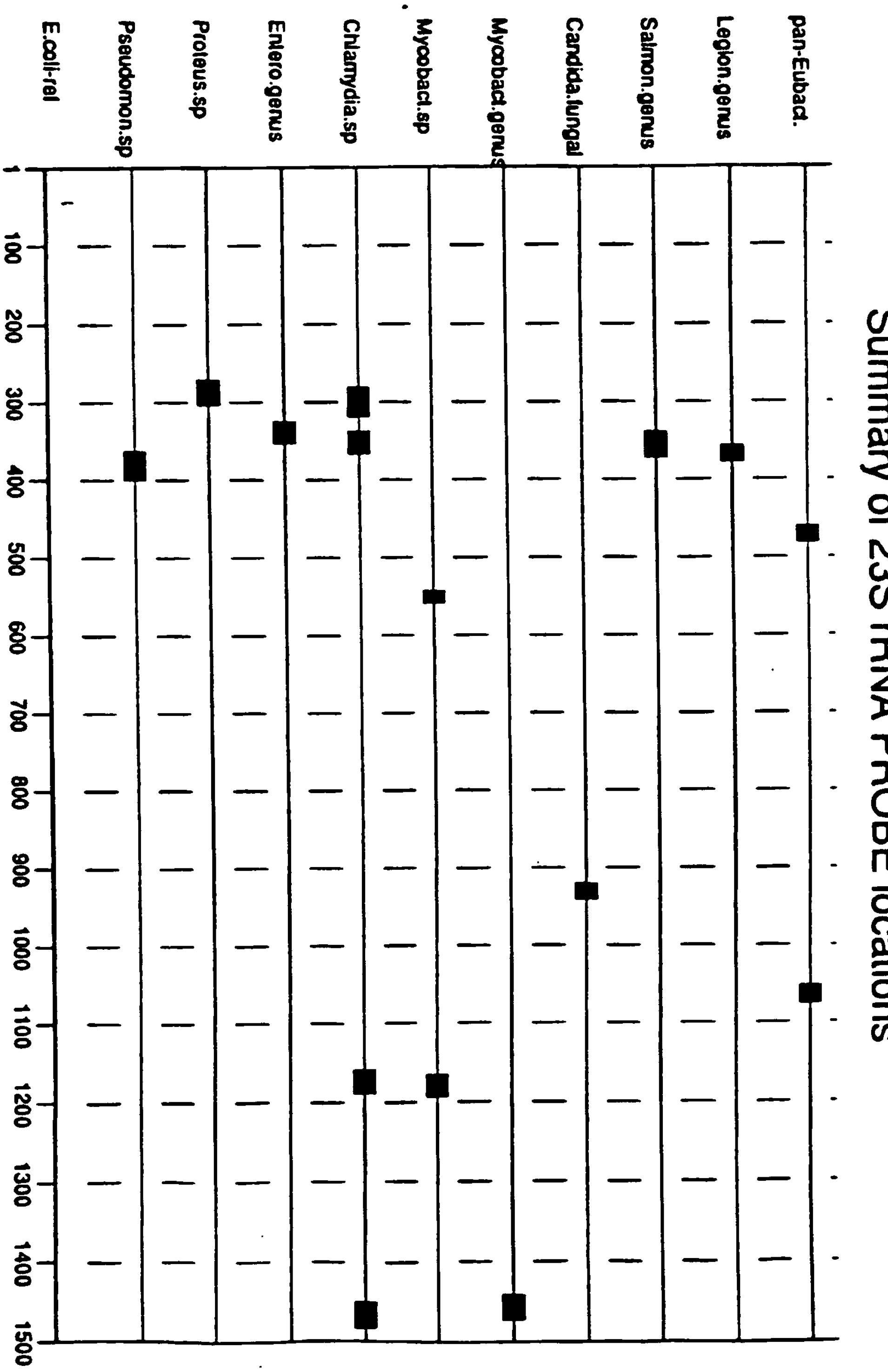
Summary of 23S rRNA PROBE locations
pan-Eubact.
Legion.genus
Salmon.genus
Candida.fungal
Mycobact.genus
Mycobact.sp
Chlamydia.sp
Entero.genus
Proteus.sp
Pseudomon.sp
E.coli-rel
1
100
200
300
400
500
600
700
800
900
1000
1100
1200
1300
1400
1500

FIG. 10

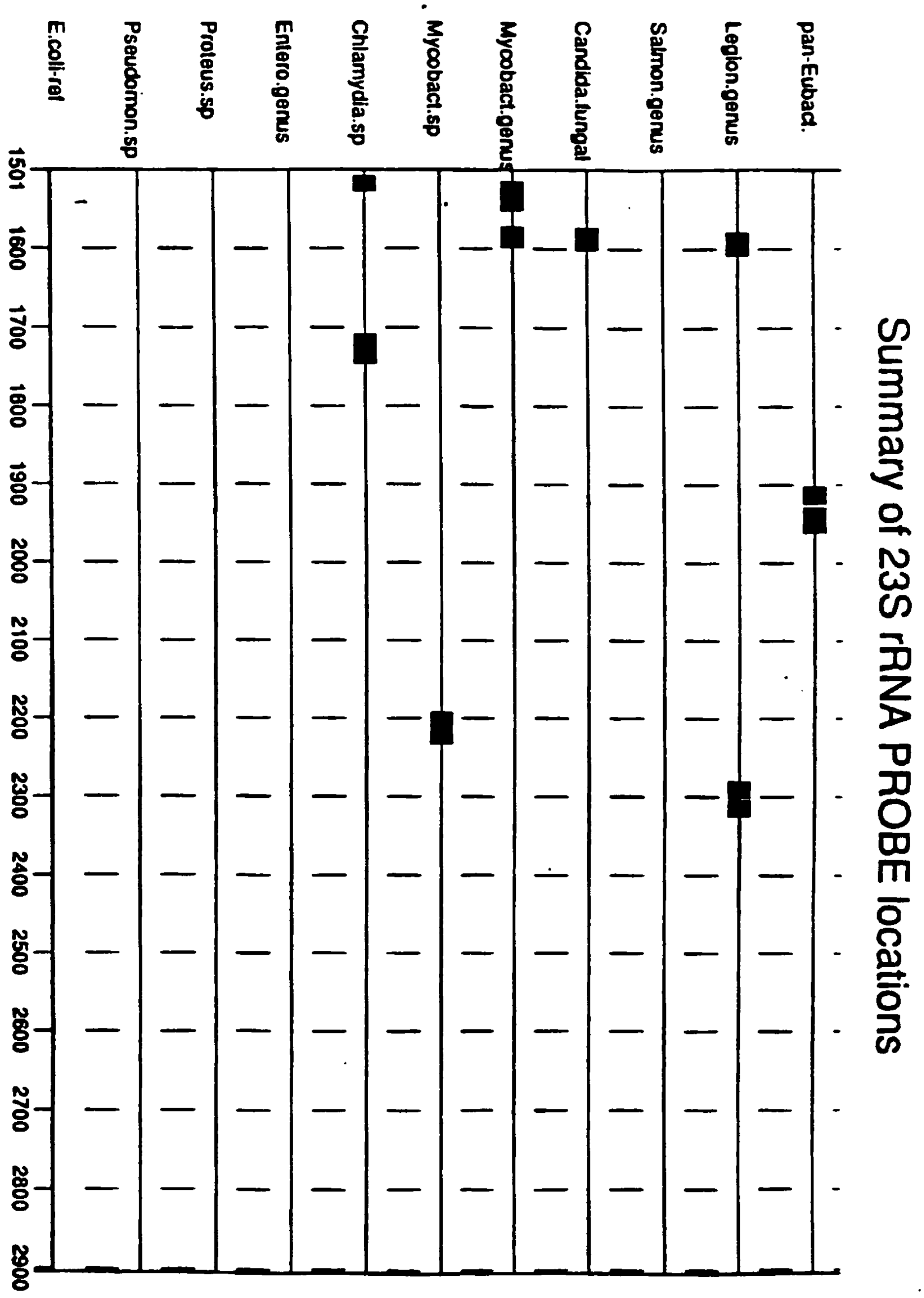
Summary of 23S rRNA PROBE locations
pan-Eubact.
Legion.genus
Salmon.genus
Candida.fungal
Mycobact.genus
Mycobact.sp
Chlamydia.sp
Entero.genus
Proteus.sp
Pseudomon.sp
E.coli-ref
1501
1600
1700
1800
1900
2000
2100
2200
2300
2400
2500
2600
2700
2800
2900

FIG. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0131052 A
- WO 8402721 A
- EP 0133671 A
- EP 0232085 A
- EP 0245129 A
- EP 0250662 A
- EP 0277237 A
- EP 0229442 A
- EP 0167366 A


### Non-patent literature cited in the description


- **LANE, D.J. et al.** *Proc. Nat'l Acad. Sci. USA,* 1985, vol. 82, 6955-6959
- **BARONE, A.D. et al.** *Nucleic Acids Research,* 1984, vol. 12, 4051-4060
- **MAXAM, A.M. ; GILBERT, W.** *Proc. Nat'l. Acad. Sci. USA,* 1980, vol. 74, 560-564
- **GOOD, R.C. et al.** *J. Infect. Dis.,* 1982, vol. 146, 829-833
- **GILL, V.J. et al.** *J. Clin. Microbio,* 1985, vol. 22, 543-546
- **LANE, D.J. et al.** *Proc. Nat. Acad. Sci. USA,* 1985, vol. 82, 6955
- **ROGERS, M.J. et al.** *Proc. Natl. Acad. Sci. USA,* 1985, vol. 82, 1160-1164
- **HORI, H. et al.** *Nucl. Acids Res.,* 1981, vol. 9, 5407-5410
- **WALKER, R.T. et al.** *Nucl. Acids Res.,* 1982, vol. 10, 6363-6367
- **FOX, G.E. ; WOESE, C.R.** *Nature,* 1975, vol. 256, 505-507
- **KINGSBURY, D.T. ; E. WEISS.** *J. Bacteriol.,* 1968, vol. 96, 1421-23
- **MOULDER, J.W.** *ASM News,* 1984, vol. 50 (8
- **WEISBERG, W.G.** *J. Bacteriol,* 1986, vol. 167, 570-574
- **ROMANIUK, P.J. et al.** *J. Bacteriol,* 1987, vol. 169, 2137-2141
- **BROSIUS et al.** *Proc. Natl. Acad. Sci. U.S.A.,* 1978, vol. 75, 4801-4805
- **CARBON et al.** *Nuc. Acids Res.,* 1981, vol. 9, 2325-2333
- **YANG et al.** *Proc. Natl. Acad. Sci. U.S.A.,* 1985, vol. 82, 4443
- **TOMIOKA ; SUGIURA.** *Mol. Gen. Genet.,* 1983, vol. 191, 46
- **DOUGLAS ; DOOLITTLE.** *Nuc. Acids Res.,* 1984, vol. 12, 3373
- **GREEN et al.** *Gene,* 1985, vol. 37, 261
- **KOP et al.** *DNA,* 1984, vol. 3, 347
- **WEISBURG et al.** *J. Bacteriol.,* 1985, vol. 164, 230
- **WEISBURG et al.** *J. Bacteriol,* 1986, vol. 167, 570
- **OYAIZU ; WOESE.** *System. Appl. Microbiol.,* 1985, vol. 6, 257
- **BROSIUS et al.** *Proc. Natl. Acad. Sci. U.S.A.,* 1980, vol. 77, 201
- **WOESE et al.** *Science,* 1985, vol. 229, 762
- **FRYDENBERG ; CHRISTIANSEN.** *DNA,* 1985, vol. 4, 127
- **CARBON et al.** *Nuc. Acids Res.,* 1981, vol. 9, 2325
- **WEISBURG et al.** *Science,* 1985, vol. 230, 556
- **RUBSTOV et al.** *Nuc. Acids Res.,* 1980, vol. 8, 5779
- **GEORGIEV et al.** *Nuc. Acids Res.,* 1981, vol. 9, 6953
- **TORCZYNSKI et al.** *DNA,* 1985, vol. 4, 283
- **GONZALEZ et al.** *Proc. Natl. Acad. Sci. U.S.A.,* 1985, vol. 82, 7666
- **KINGSBURY, D.T.** *J. Bacteriol.,* 1967, vol. 94, 870-874
- Bacterial Systematics. *Int'l J. System. Bacteriol.,* 1987, vol. 37, 463-464